# EUROPEAN PATENT SPECIFICATION

(11) **EP 3 194 382 B1**
(45) Date of publication and mention of the grant of the patent: **08.09.2021**
(21) Application number: 15771410.6
(22) Date of filing: 16.09.2015
(51) Int. Cl.: C07D 401/14, C07D 413/14, C07D 403/04, C07D 403/14, C07D 417/14, C07D 487/04, C07D 491/10, C07D 495/04, A61K 31/505, A61K 31/519, A61K 31/53, A61P 9/00, A61P 25/00, A61P 15/00, A61P 3/00

(54) **PYRAZOLE DERIVATIVES AS SGC STIMULATORS**
PYRAZOLDERIVATE ALS SGC-STIMULATOREN
DÉRIVÉS DE PYRAZOLE UTILISÉS COMME STIMULATEURS DE SGC

(30) Priority: 17.09.2014 US 201462051557 P; 13.08.2015 US 201562204710 P
(43) Date of publication of application: 26.07.2017
(73) Proprietor: Cyclerion Therapeutics, Inc., Cambridge, MA 02142 (US)
(72) Inventor: BARDEN, Timothy Claude, Cambridge, Massachusetts 02142 (US); SHEPPECK, James Edward, Newtown, Pennsylvania 19940 (US); RENNIE, Glen Robert, Somerville, Massachusetts 02144 (US); RENHOWE, Paul Allan, Sudbury, Massachusetts 01776 (US); PERL, Nicholas, Somerville, Massachusetts 02143 (US); NAKAI, Takashi, Newton, Massachusetts 02458 (US); MERMERIAN, Ara, Waltham, Massachusetts 02453 (US); LEE, Thomas Wai-Ho, Lexington, Massachusetts 02420 (US); JUNG, Joon, Newton, Massachusetts 02459 (US); JIA, James, Belmont, Massachusetts 02478 (US); IYER, Karthik, Cambridge, Massachusetts 02142 (US); IYENGAR, Rajesh R., West Newton, Massachusetts 02465 (US); IM, G-Yoon Jamie, Cambridge, Massachusetts 02139 (US)
(74) Representative: Elkington and Fife LLP
(86) International application number: PCT/US2015/050468
(87) International publication number: WO 2016/044447

(56) References cited:
- WO-A1-2008/031513
- WO-A1-2012/003405
- WO-A1-2013/101830
- WO-A1-2014/047111
- WO-A1-2014/047325
- WO-A1-2015/089182
- WO-A1-2015/106268
- WO-A2-2014/144100
- None

## Description

### FIELD OF THE INVENTION

The present disclosure relates to stimulators of soluble guanylate cyclase (sGC), pharmaceutical formulations comprising them and their uses thereof, alone or in combination with one or more additional agents, for treating and/or preventing various diseases, wherein an increase in the concentration of nitric oxide (NO) or an increase in the concentration of cyclic Guanosine Monophosphate (cGMP) might be desirable.

### BACKGROUND OF THE INVENTION

Soluble guanylate cyclase (sGC) is the primary receptor for nitric oxide (NO) *in vivo.* sGC can be activated *via* both NO-dependent and NO-independent mechanisms. In response to this activation, sGC converts GTP into the secondary messenger cyclic GMP (cGMP). The increased level of cGMP, in turn, modulates the activity of downstream effectors including protein kinases, phosphodiesterases (PDEs) and ion channels.

In the body, NO is synthesized from arginine and oxygen by various nitric oxide synthase (NOS) enzymes and by sequential reduction of inorganic nitrate. Three distinct isoforms of NOS have been identified: inducible NOS (iNOS or NOS II) found in activated macrophage cells; constitutive neuronal NOS (nNOS or NOS I), involved in neurotransmission and long term potentiation; and constitutive endothelial NOS (eNOS or NOS III) which regulates smooth muscle relaxation and blood pressure.

Experimental and clinical evidence indicates that reduced bioavailability and/or responsiveness to endogenously produced NO contributes to the development of cardiovascular, endothelial, renal and hepatic disease, as well as erectile dysfunction and other sexual disorders (e.g. female sexual disorder or vaginal atrophy). In particular, the NO signaling pathway is altered in cardiovascular diseases, including, for instance, systemic and pulmonary hypertension, heart failure, angina, stroke, thrombosis and other thromboembolic diseases, peripheral arterial disease, fibrosis of the liver, lung or kidney and atherosclerosis.

sGC stimulators are also useful in the treatment of lipid related disorders such as e.g., dyslipidemia, hypercholesterolemia, hypertriglyceridemia, sitosterolemia, fatty liver disease, and hepatitis.

Pulmonary hypertension (PH) is a disease characterized by sustained elevation of blood pressure in the pulmonary vasculature (pulmonary artery, pulmonary vein and pulmonary capillaries), which results in right heart hypertrophy, eventually leading to right heart failure and death. In PH, the bioactivity of NO and other vasodilators such as prostacyclin is reduced, whereas the production of endogenous vasoconstrictors such as endothelin is increased, resulting in excessive pulmonary vasoconstriction. sGC stimulators have been used to treat PH because they promote smooth muscle relaxation, which leads to vasodilation.

Treatment with NO-independent sGC stimulators also promoted smooth muscle relaxation in the corpus cavernosum of healthy rabbits, rats and humans, causing penile erection, indicating that sGC stimulators are useful for treating erectile dysfunction.

NO-independent, heme-dependent, sGC stimulators, such as those disclosed herein, have several important differentiating characteristics, including crucial dependency on the presence of the reduced prosthetic heme moiety for their activity, strong synergistic enzyme activation when combined with NO and stimulation of the synthesis of cGMP by direct stimulation of sGC, independent of NO. The benzylindazole compound YC-1 was the first sGC stimulator to be identified. Additional sGC stimulators with improved potency and specificity for sGC have since been developed. These compounds have been shown to produce anti-aggregatory, anti-proliferative and vasodilatory effects.

Since compounds that stimulate sGC in an NO-independent manner offer considerable advantages over other current alternative therapies, there is a need to develop novel stimulators of sGC. They are potentially useful in the prevention, management and treatment of disorders such as pulmonary hypertension, arterial hypertension, heart failure, atherosclerosis, inflammation, thrombosis, renal fibrosis and failure, liver cirrhosis, lung fibrosis, erectile dysfunction, female sexual arousal disorder and vaginal atrophy and other cardiovascular disorders; they are also potentially useful for the prevention, management and treatment of lipid related disorders.

WO 2012/003405 discloses compounds that are useful as stimulators of sGC, particularly NO-independent, heme-dependent stimulators. WO 2013/101830 discloses 2-Benzyl, 3-(pyrimidin-2-yl) substituted pyrazoles, and their use as stimulators of sGC, particularly NO - independent, heme - dependent stimulators. WO 2014/047111 discloses compounds that are useful as stimulators of sGC, particularly NO-independent, heme-dependent stimulators. WO 2014/047325 discloses compounds that are useful as sGC stimulators. WO 2015/089182 discloses compounds that are useful as soluble sGC stimulators. WO 2015/106268 discloses methods, uses, pharmaceutical compositions and kits comprising a sGC stimulator or a pharmaceutically acceptable salt thereof, alone or in combination with one or more additional therapeutic agents, for the treatment of a neuromuscular disorder associated with loss or alteration of function of nitric oxide synthase (NOS).

### SUMMARY OF THE INVENTION

The present invention is directed to a compound in accordance with claim 1.

The invention is also directed to a pharmaceutical composition in accordance with claim 2.

The invention also provides a compound or pharmaceutical composition for use in accordance with claim 3.

### DETAILED DESCRIPTION OF THE INVENTION

Reference will now be made in detail to certain embodiments of the invention, examples of which are illustrated in the accompanying structures and formulae. While the invention will be described in conjunction with the enumerated embodiments, it will be understood that they are not intended to limit the invention to those embodiments. Rather, the invention is intended to cover all alternatives, modifications and equivalents included within the scope of the present invention as defined by the claims. The present invention is not limited to the methods and materials described herein but include any methods and materials similar or equivalent to those described herein that could be used in the practice of the present invention. In the event that one or more of the incorporated literature references, patents or similar materials differ from or contradict this application, including but not limited to defined terms, term usage, described techniques or the like, this application controls.

### Definitions and general terminology

For purposes of this disclosure, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry", 5th Ed., Smith, M. B. and March, J., eds. John Wiley & Sons, New York: 2001.

A compound, such as a compound of Table IA or Table IB or other compounds herein disclosed, may be present in its free form (e.g. an amorphous form, or a crystalline form or a polymorph). Under certain conditions, compounds may also form co-forms. As used herein, the term co-form is synonymous with the term multi-component crystalline form. When one of the components in the co-form has clearly transferred a proton to the other component, the resulting co-form is referred to as a "salt". The formation of a salt is determined by how large the difference is in the pKas between the partners that form the mixture. For purposes of this disclosure, compounds include pharmaceutically acceptable salts, even if the term "pharmaceutically acceptable salts" is not explicitly noted.

Unless only one of the isomers is drawn or named specifically, structures depicted herein are also meant to include all stereoisomeric (e.g., enantiomeric, diastereomeric, atropoisomeric and cis-trans isomeric) forms of the structure; for example, the R and S configurations for each asymmetric center, *Ra* and *Sa* configurations for each asymmetric axis, *(Z)* and *(E)* double bond configurations, and *cis* and *trans* conformational isomers. Therefore, single stereochemical isomers as well as racemates, and mixtures of enantiomers, diastereomers, and *cis-trans* isomers (double bond or conformational) of the present compounds are within the scope of the present disclosure. Unless otherwise stated, all tautomeric forms of the compounds of the present disclosure are also within the scope of the invention. As an example, a substituent drawn as below: 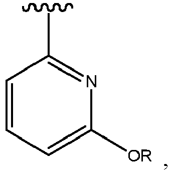 wherein R may be hydrogen, would include both compounds shown below:

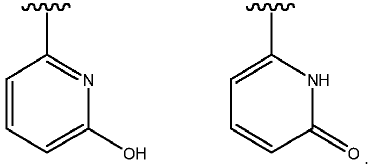

The present disclosure also embraces isotopically-labeled compounds which are identical to those recited herein, but for the fact that one or more atoms are replaced by an atom having an atomic mass or mass number different from the atomic mass or mass number usually found in nature. All isotopes of any particular atom or element as specified are contemplated within the scope of the compounds of the invention, and their uses. Exemplary isotopes that can be incorporated into compounds of the invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I, and ¹²⁵I, respectively. Certain isotopically-labeled compounds of the present invention (e.g., those labeled with ³H and ¹⁴C) are useful in compound and/or substrate tissue distribution assays. Tritiated (i.e., ³H) and carbon-14 (i.e., ¹⁴C) isotopes are useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C, and ¹⁸F are useful for positron emission tomography (PET) studies to examine substrate receptor occupancy. Isotopically labeled compounds of the present invention can generally be prepared by following procedures analogous to those disclosed in the Schemes and/or in the Examples herein below, by substituting an isotopically labeled reagent for a non-isotopically labeled reagent.

The compounds of the invention are defined herein by their chemical structures and/or chemical names. Where a compound is referred to by both a chemical structure and a chemical name, and the chemical structure and chemical name conflict, the chemical structure is determinative of the compound's identity.

### Compounds

The compounds of the invention are selected from those depicted in Table IA or Table IB

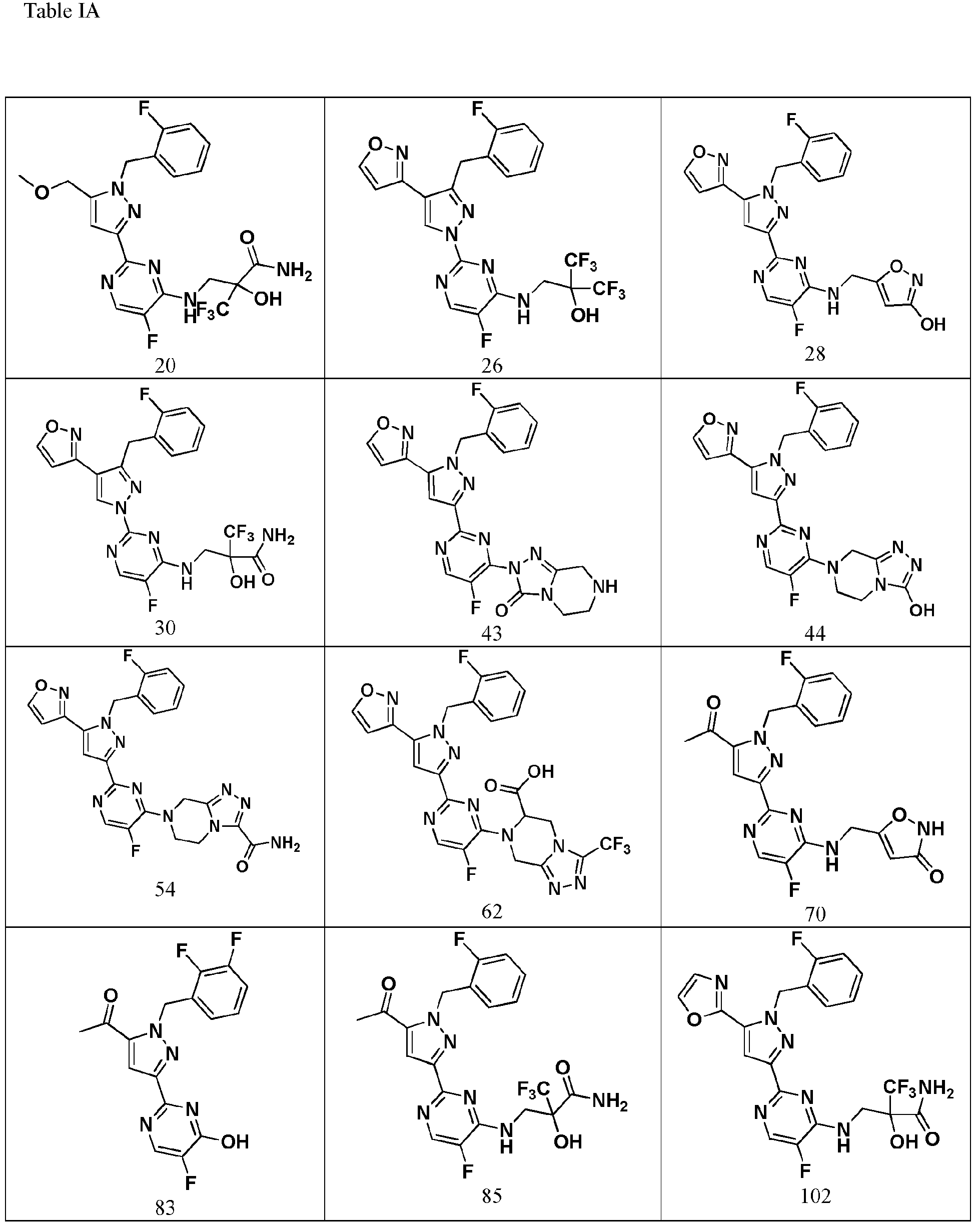

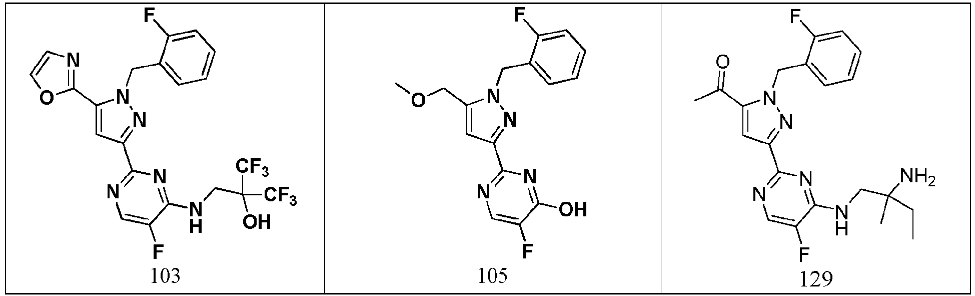

### Methods of preparing the compounds

The compounds of the invention may be prepared according to the schemes and examples depicted and described below. Unless otherwise specified, the starting materials and various intermediates may be obtained from commercial sources, prepared from commercially available compounds or prepared using well-known synthetic methods.

General synthetic procedures for the compounds of this invention are described below. The synthetic schemes are presented as examples and do not limit the scope of the invention in any way.

### General Procedure A

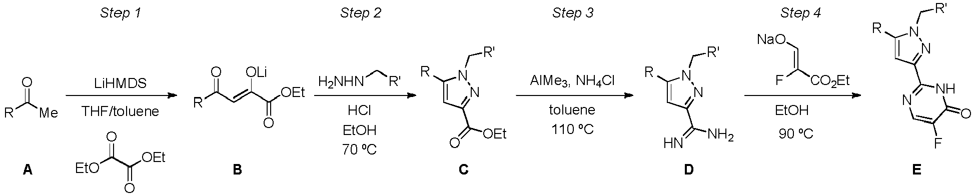

### Step 1:

*Dione enolate formation:* To a solution of ketone **A** in THF cooled to -78 °C, LiHMDS (e.g., 0.9 equiv, 1.0 M in toluene) was added dropwise via syringe. The reaction was allowed to warm to 0 °C, then charged with diethyl oxalate (1.2 equiv). At this time, the reaction was warmed to room temperature and stirred at that temperature until judged complete (e.g., using either TLC or LC/MS analysis). Once the reaction was complete (reaction time was typically 45 minutes), the product dione enolate **B** was used "as-is" in Step 2, i.e., the cyclization step, without any further purification.

### Step 2:

*Pyrazole formation:* Dione enolate **B** was diluted with ethanol and consecutively charged with HCl (e.g., 3 equiv, 1.25 M solution in ethanol) and arylhydrazine hydrate (e.g., 1.15 equiv). The reaction mixture was heated to 70 °C and stirred at this temperature until cyclization was deemed complete (e.g., by LC/MS analysis, typically 30 minutes). Once complete, the reaction mixture was treated carefully with solid sodium bicarbonate (e.g., 4 equiv) and diluted with dichloromethane and water. Layers were separated, and aqueous layer was futher diluted with water before extraction with dichloromethane (3x). The combined organics were washed with brine, dried over MgSO₄, filtered, and concentrated in vacuo. The resulting pyrazole C was then purified by SiO2 chromatography using an appropriate gradient of EtOAc in hexanes.

### Step 3:

*Amidine formation:* To a suspension of NH4Cl (e.g., 5 equiv) in toluene cooled to 0 °C was added AlMe₃ (e.g., 5 equiv, 2.0M solution in toluene) dropwise via syringe. The reaction was allowed to warm to room temperature, and stirred at this temperature until no more bubbling was observed. Pyrazole C was added in 1 portion to the reaction mixture, heated to 110 °C, and stirred at this temperature until judged complete (e.g., using either TLC or LC/MS analysis). Once complete, the reaction was cooled, treated with excess methanol, and stirred vigorously for 1 hour at room temperature. The thick slurry was filtered, and the resulting solid cake was washed with methanol. The filtrate was concentrated in vacuo, and the resulting solids were re-suspended in an ethyl acetate : isopropyl alcohol = 5:1 solvent mixture. The reaction was further treated with saturated sodium carbonate solution, and stirred for 10 minutes before the layers are separated. The aqueous layer was extracted with the ethyl acetate : isopropyl alcohol = 5:1 solvent mixture (3x), and the combined organics were washed with brine. The organics were further dried over MgSO4, filtered, and the solvent removed in vacuo. The product amidine **D** was used as-is in subsequent steps without further purification.

### Step 4:

*Pyrimidone formation:* Amidine **D** was suspended in ethanol, and stirred vigorously at 23 °C to encourage full solvation. The reaction was further treated with sodium 3-ethoxy-2-fluoro-3-oxoprop-1-en-1-olate (e.g., 3 equiv.), and the flask was equipped with a reflux condenser. The reaction was placed into a pre-heated oil bath maintained at 90 °C and stirred until full consumption of starting material was observed on the LC/MS (reaction times were typically 1 h). The contents were cooled to 23 °C, and the reaction mixture acidified with HCl (e.g., 3 equiv., 1.25M solution in EtOH). The mixture was stirred for 30 minutes, and the majority of the solvent was removed in vacuo. Contents were re-suspended in ether and water (1:1 mixture), and the resulting slurry was stirred for 20 min. The suspension was vacuum filtered, and the solid cake was rinsed with additional water and ether and dried on high vacuum overnight. The resulting pyrimidone E was used as-is in subsequent steps without further purification.

### General procedure B

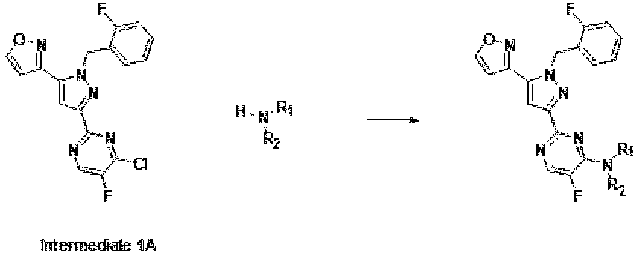

A solution of amino nucleophile (3 equiv.), triethylamine (10 equiv.), and **Intermediate-1A** (1 equiv.) was stirred in dioxane and water (2:1 ratio) at 90 °C until complete consumption of starting material was observed by LC/MS. The solution was diluted with aqueous IN hydrochloric acid and dichloromethane. The layers were then separated and the aqueous layer was extracted with dichloromethane. The organics were combined, dried over magnesium sulfate, filtered, and the solvent was removed in vacuo. Purification yielded the desired product.

### General procedure C

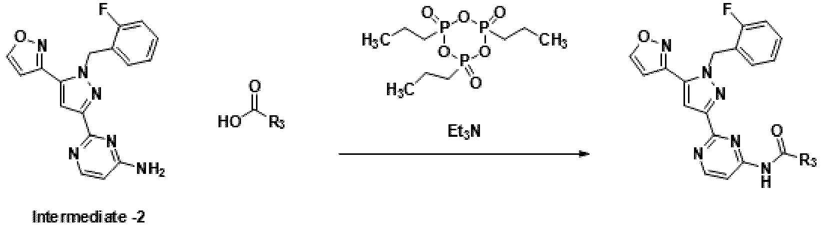

A mixture of **Intermediate-2** (this intermediate was described in previously published patent application WO2012/3405 A1; 1 equivalent) and carboxylic acid (1.1 equivalent) in *N*,*N*-dimethylformamide was treated with triethylamine (4 equivalent) followed by a 50% in ethyl acetate solution of propylphosphonic anhydride (T3P, 1.4 equivalent). The reaction was heated to 80 °C for 24 h, after which the reaction was diluted with water and IN hydrochloric acid solution. Contents were extracted with dichloromethane, then ethyl acetate. The combined organic layers were dried over sodium sulfate, filtered, and concentrated in vacuo. Purification yielded the desired product.

### Pharmaceutically acceptable salts of the invention.

In all instances described herein, the term "compound" also includes a pharmaceutically acceptable salt of the compound, whether or not the phrase "pharmaceutically acceptable salt" is actually used. The phrase "pharmaceutically acceptable salt," as used herein, refers to pharmaceutically acceptable organic or inorganic salts of a compound of Table IA or Table IB. The pharmaceutically acceptable salts of a compound of Table IA or Table IB are used in medicine. Salts that are not pharmaceutically acceptable may, however, be useful in the preparation of a compound of Table IA or Table IB or of their pharmaceutically acceptable salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

Pharmaceutically acceptable salts of the compounds described herein include those derived from the compounds with inorganic acids, organic acids or bases. In some embodiments, the salts can be prepared in situ during the final isolation and purification of the compounds. In other embodiments the salts can be prepared from the free form of the compound in a separate synthetic step.

When a compound of Table IA or Table IB is acidic or contains a sufficiently acidic bioisostere, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particular embodiments include ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N, N.sup.1-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

When a compound of Table IBA or Table IB is basic or contains a sufficiently basic bioisostere, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particular embodiments include citric, hydrobromic, hydrochloric, maleic, phosphoric, sulfuric and tartaric acids. Other exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethane sulfonate, benzenesulfonate, p-toluenesulfonate, and pamoate (i.e., 1,1'-methylene-bis-(2-hydroxy-3-naphthoate)) salts.

The preparation of the pharmaceutically acceptable salts described above and other typical pharmaceutically acceptable salts is more fully described by Berg et al., "Pharmaceutical Salts," J. Pharm. Sci., 1977:66:1-19.

In addition to the compounds described herein, their pharmaceutically acceptable salts may also be employed in compositions to treat or prevent the herein identified disorders.

In all instances described herein, the term "compound" also includes a pharmaceutically acceptable salt of the compound, whether or not the phrase "pharmaceutically acceptable salt" is actually used

### Pharmaceutical compositions and methods of administration.

The compounds herein disclosed, and their pharmaceutically acceptable salts thereof may be formulated as pharmaceutical compositions or "formulations".

A typical formulation is prepared by mixing a compound of Table IA or Table IB, or a pharmaceutically acceptable salt thereof, and a carrier, diluent or excipient. Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which a compound of Table IA or Table IB is being formulated. Solvents are generally selected based on solvents recognized by persons skilled in the art as safe (GRAS-Generally Regarded as Safe) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The formulations may also include other types of excipients such as one or more buffers, stabilizing agents, antiadherents, surfactants, wetting agents, lubricating agents, emulsifiers, binders, suspending agents, disintegrants, fillers, sorbents, coatings (e.g. enteric or slow release) preservatives, antioxidants, opaquing agents, glidants, processing aids, colorants, sweeteners, perfuming agents, flavoring agents and other known additives to provide an elegant presentation of the drug (i.e., a compound of Table IA or Table IB or pharmaceutical composition thereof) or aid in the manufacturing of the pharmaceutical product (i.e., medicament).

The formulations may be prepared using conventional dissolution and mixing procedures. For example, the bulk drug substance (i.e., a compound of Table IA or Table IB, a pharmaceutically acceptable salt thereof, or a stabilized form of the compound, such as a complex with a cyclodextrin derivative or other known complexation agent) is dissolved in a suitable solvent in the presence of one or more of the excipients described above. A compound having the desired degree of purity is optionally mixed with pharmaceutically acceptable diluents, carriers, excipients or stabilizers, in the form of a lyophilized formulation, milled powder, or an aqueous solution. Formulation may be conducted by mixing at ambient temperature at the appropriate pH, and at the desired degree of purity, with physiologically acceptable carriers. The pH of the formulation depends mainly on the particular use and the concentration of compound, but may range from about 3 to about 8. When the agent described herein is a solid amorphous dispersion formed by a solvent process, additives may be added directly to the spray-drying solution when forming the mixture such as the additive is dissolved or suspended in the solution as a slurry which can then be spray dried. Alternatively, the additives may be added following spray-drying process to aid in the forming of the final formulated product.

The compound of Table IA or Table IB or a pharmaceutically acceptable salt thereof is typically formulated into pharmaceutical dosage forms to provide an easily controllable dosage of the drug and to enable patient compliance with the prescribed regimen. Pharmaceutical formulations of a compound of Table IA or Table IB, or a pharmaceutically acceptable salt thereof, may be prepared for various routes and types of administration. Various dosage forms may exist for the same compound, since different medical conditions may warrant different routes of administration.

The amount of active ingredient that may be combined with the carrier material to produce a single dosage form will vary depending upon the subject treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight: weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur. As a general proposition, the initial pharmaceutically effective amount of the inhibitor administered will be in the range of about 0.01-100 mg/kg per dose, namely about 0.1 to 20 mg/kg of patient body weight per day, with the typical initial range of compound used being 0.3 to 15 mg/kg/day.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The therapeutically or pharmaceutically effective amount of the compound to be administered will be governed by such considerations, and is the minimum amount necessary to ameliorate, cure or treat the disease or disorder or one or more of its symptoms.

The pharmaceutical compositions of compounds of Table IA or Table IB will be formulated, dosed, and administered in a fashion, i.e., amounts, concentrations, schedules, course, vehicles, and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners, such as the age, weight, and response of the individual patient.

The term "prophylactically effective amount" refers to an amount effective in preventing or substantially lessening the chances of acquiring a disease or disorder or in reducing the severity of the disease or disorder before it is acquired or reducing the severity of one or more of its symptoms before the symptoms develop. Roughly, prophylactic measures are divided between *primary* prophylaxis (to prevent the development of a disease) and *secondary* prophylaxis (whereby the disease has already developed and the patient is protected against worsening of this process).

Acceptable diluents, carriers, excipients, and stabilizers are those that are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, tretralose or sorbitol; salt-forming counter-ions such as sodium; metal complexes (e.g. Zn-protein complexes); and/or non-ionic surfactants such as TWEEN™, PLURONICS™ or polyethylene glycol (PEG). The active pharmaceutical ingredients may also be entrapped in microcapsules prepared, for example, by coacervation techniques or by interfacial polymerization, e.g., hydroxymethylcellulose or gelatin-microcapsules and poly-(methylmethacylate) microcapsules, respectively; in colloidal drug delivery systems (for example, liposomes, albumin microspheres, microemulsions, nano-particles and nanocapsules) or in macroemulsions. Such techniques are disclosed in Remington's: The Science and Practice of Pharmacy, 21st Edition, University of the Sciences in Philadelphia, Eds., 2005 (hereafter "Remington's").

"Controlled drug delivery systems" supply the drug to the body in a manner precisely controlled to suit the drug and the conditions being treated. The primary aim is to achieve a therapeutic drug concentration at the site of action for the desired duration of time. The term "controlled release" is often used to refer to a variety of methods that modify release of drug from a dosage form. This term includes preparations labeled as "extended release", "delayed release", "modified release" or "sustained release". In general, one can provide for controlled release of the agents described herein through the use of a wide variety of polymeric carriers and controlled release systems including erodible and non-erodible matrices, osmotic control devices, various reservoir devices, enteric coatings and multiparticulate control devices.

"Sustained-release preparations" are the most common applications of controlled release. Suitable examples of sustained-release preparations include semipermeable matrices of solid hydrophobic polymers containing the compound, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and gamma-ethyl-L-glutamate, non-degradable ethylene-vinyl acetate, degradable lactic acid-glycolic acid copolymers, and poly-D-(-)-3-hydroxybutyric acid.

"Immediate-release preparations" may also be prepared. The objective of these formulations is to get the drug into the bloodstream and to the site of action as rapidly as possible. For instance, for rapid dissolution, most tablets are designed to undergo rapid disintegration to granules and subsequent deaggregation to fine particles. This provides a larger surface area exposed to the dissolution medium, resulting in a faster dissolution rate.

Agents described herein can be incorporated into an erodible or non-erodible polymeric matrix controlled release device. By an erodible matrix is meant aqueous-erodible or water-swellable or aqueous-soluble in the sense of being either erodible or swellable or dissolvable in pure water or requiring the presence of an acid or base to ionize the polymeric matrix sufficiently to cause erosion or dissolution. When contacted with the aqueous environment of use, the erodible polymeric matrix imbibes water and forms an aqueous-swollen gel or matrix that entraps the agent described herein. The aqueous-swollen matrix gradually erodes, swells, disintegrates or dissolves in the environment of use, thereby controlling the release of a compound described herein to the environment of use. One ingredient of this water-swollen matrix is the water-swellable, erodible, or soluble polymer, which may generally be described as an osmopolymer, hydrogel or water-swellable polymer. Such polymers may be linear, branched, or cross linked. The polymers may be homopolymers or copolymers. In certain embodiments, they may be synthetic polymers derived from vinyl, acrylate, methacrylate, urethane, ester and oxide monomers. In other embodiments, they can be derivatives of naturally occurring polymers such as polysaccharides (e.g. chitin, chitosan, dextran and pullulan; gum agar, gum arabic, gum karaya, locust bean gum, gum tragacanth, carrageenans, gum ghatti, guar gum, xanthan gum and scleroglucan), starches (e.g. dextrin and maltodextrin), hydrophilic colloids (e.g. pectin), phosphatides (e.g. lecithin), alginates (e.g. ammonium alginate, sodium, potassium or calcium alginate, propylene glycol alginate), gelatin, collagen, and cellulosics. Cellulosics are cellulose polymer that has been modified by reaction of at least a portion of the hydroxyl groups on the saccharide repeat units with a compound to form an ester-linked or an ether-linked substituent. For example, the cellulosic ethyl cellulose has an ether linked ethyl substituent attached to the saccharide repeat unit, while the cellulosic cellulose acetate has an ester linked acetate substituent. In certain embodiments, the cellulosics for the erodible matrix comprises aqueous-soluble and aqueous-erodible cellulosics can include, for example, ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC). In certain embodiments, the cellulosics comprises various grades of low viscosity (MW less than or equal to 50,000 daltons, for example, the Dow Methocel™ series E5, E15LV, E50LV and K100LY) and high viscosity (MW greater than 50,000 daltons, for example, E4MCR, E10MCR, K4M, K15M and K100M and the Methocel™ K series) HPMC. Other commercially available types of HPMC include the Shin Etsu Metolose 90SH series.

Other materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGIT®, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl) methacrylate, and (trimethylaminoethyl) methacrylate chloride.

Alternatively, the agents of the present invention may be administered by or incorporated into a non-erodible matrix device. In such devices, an agent described herein is distributed in an inert matrix. The agent is released by diffusion through the inert matrix. Examples of materials suitable for the inert matrix include insoluble plastics (e.g methyl acrylate-methyl methacrylate copolymers, polyvinyl chloride, polyethylene), hydrophilic polymers (e.g. ethyl cellulose, cellulose acetate, cross linked polyvinylpyrrolidone (also known as crospovidone)), and fatty compounds (e.g. carnauba wax, microcrystalline wax, and triglycerides). Such devices are described further in Remington: The Science and Practice of Pharmacy, 20th edition (2000).

As noted above, the agents described herein may also be incorporated into an osmotic control device. Such devices generally include a core containing one or more agents as described herein and a water permeable, non-dissolving and non-eroding coating surrounding the core which controls the influx of water into the core from an aqueous environment of use so as to cause drug release by extrusion of some or all of the core to the environment of use. In certain embodiments, the coating is polymeric, aqueous-permeable, and has at least one delivery port. The core of the osmotic device optionally includes an osmotic agent which acts to imbibe water from the surrounding environment via such a semi-permeable membrane. The osmotic agent contained in the core of this device may be an aqueous-swellable hydrophilic polymer or it may be an osmogen, also known as an osmagent. Pressure is generated within the device which forces the agent(s) out of the device via an orifice (of a size designed to minimize solute diffusion while preventing the build-up of a hydrostatic pressure head). Non limiting examples of osmotic control devices are disclosed in U. S. Patent Application Serial No. 09/495,061.

The amount of water-swellable hydrophilic polymers present in the core may range from about 5 to about 80 wt% (including for example, 10 to 50 wt%). Non limiting examples of core materials include hydrophilic vinyl and acrylic polymers, polysaccharides such as calcium alginate, polyethylene oxide (PEO), polyethylene glycol (PEG), polypropylene glycol (PPG), poly (2-hydroxyethyl methacrylate), poly (acrylic) acid, poly (methacrylic) acid, polyvinylpyrrolidone (PVP) and cross linked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl cellulose (CEC), sodium alginate, polycarbophil, gelatin, xanthan gum, and sodium starch glycolat. Other materials include hydrogels comprising interpenetrating networks of polymers that may be formed by addition or by condensation polymerization, the components of which may comprise hydrophilic and hydrophobic monomers such as those just mentioned. Water-swellable hydrophilic polymers include but are not limited to PEO, PEG, PVP, sodium croscarmellose, HPMC, sodium starch glycolate, polyacrylic acid and cross linked versions or mixtures thereof.

The core may also include an osmogen (or osmagent). The amount of osmogen present in the core may range from about 2 to about 70 wt% (including, for example, from 10 to 50 wt%). Typical classes of suitable osmogens are water-soluble organic acids, salts and sugars that are capable of imbibing water to thereby effect an osmotic pressure gradient across the barrier of the surrounding coating. Typical useful osmogens include but are not limited to magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. In certain embodiments, the osmogen is glucose, lactose, sucrose, mannitol, xylitol, sodium chloride, including combinations thereof.

The rate of drug delivery is controlled by such factors as the permeability and thickness of the coating, the osmotic pressure of the drug-containing layer, the degree of hydrophilicity of the hydrogel layer, and the surface area of the device. Those skilled in the art will appreciate that increasing the thickness of the coating will reduce the release rate, while any of the following will increase the release rate: increasing the permeability of the coating; increasing the hydrophilicity of the hydrogel layer; increasing the osmotic pressure of the drug-containing layer; or increasing the device's surface area.

In certain embodiments, entrainment of particles of agents described herein in the extruding fluid during operation of such osmotic device is desirable. For the particles to be well entrained, the agent drug form is dispersed in the fluid before the particles have an opportunity to settle in the tablet core. One means of accomplishing this is by adding a disintegrant that serves to break up the compressed core into its particulate components. Non limiting examples of standard disintegrants include materials such as sodium starch glycolate (e. g., Explotab™ CLV), microcrystalline cellulose (e. g., Avicel™), microcrystalline silicified cellulose (e. g., ProSoIv™) and croscarmellose sodium (e. g., Ac-Di-Sol™), and other disintegrants known to those skilled in the art. Depending upon the particular formulation, some disintegrants work better than others. Several disintegrants tend to form gels as they swell with water, thus hindering drug delivery from the device. Non-gelling, non-swelling disintegrants provide a more rapid dispersion of the drug particles within the core as water enters the core. In certain embodiments, non-gelling, non-swelling disintegrants are resins, for example, ion-exchange resins. In one embodiment, the resin is Amberlite™ IRP 88 (available from Rohm and Haas, Philadelphia, PA). When used, the disintegrant is present in amounts ranging from about 1-25% of the core agent.

Another example of an osmotic device is an osmotic capsule. The capsule shell or portion of the capsule shell can be semipermeable. The capsule can be filled either by a powder or liquid consisting of an agent described herein, excipients that imbibe water to provide osmotic potential, and/or a water-swellable polymer, or optionally solubilizing excipients. The capsule core can also be made such that it has a bilayer or multilayer agent analogous to the bilayer, trilayer or concentric geometries described above.

Another class of osmotic device useful in this invention comprises coated swellable tablets, for example, as described in EP378404. Coated swellable tablets comprise a tablet core comprising an agent described herein and a swelling material, preferably a hydrophilic polymer, coated with a membrane, which contains holes, or pores through which, in the aqueous use environment, the hydrophilic polymer can extrude and carry out the agent. Alternatively, the membrane may contain polymeric or low molecular weight water-soluble porosigens. Porosigens dissolve in the aqueous use environment, providing pores through which the hydrophilic polymer and agent may extrude. Examples of porosigens are water-soluble polymers such as HPMC, PEG, and low molecular weight compounds such as glycerol, sucrose, glucose, and sodium chloride. In addition, pores may be formed in the coating by drilling holes in the coating using a laser or other mechanical means. In this class of osmotic devices, the membrane material may comprise any film-forming polymer, including polymers which are water permeable or impermeable, providing that the membrane deposited on the tablet core is porous or contains water-soluble porosigens or possesses a macroscopic hole for water ingress and drug release. Embodiments of this class of sustained release devices may also be multilayered, as described, for example, in EP378404.

When an agent described herein is a liquid or oil, such as a lipid vehicle formulation, for example as described in WO05/011634, the osmotic controlled-release device may comprise a soft-gel or gelatin capsule formed with a composite wall and comprising the liquid formulation where the wall comprises a barrier layer formed over the external surface of the capsule, an expandable layer formed over the barrier layer, and a semipermeable layer formed over the expandable layer. A delivery port connects the liquid formulation with the aqueous use environment. Such devices are described, for example, in US6419952, US6342249, US5324280, US4672850, US4627850, US4203440, and US3995631.

As further noted above, the agents described herein may be provided in the form of microparticulates, generally ranging in size from about 10µm to about 2mm (including, for example, from about 100µm to 1mm in diameter). Such multiparticulates may be packaged, for example, in a capsule such as a gelatin capsule or a capsule formed from an aqueous-soluble polymer such as HPMCAS, HPMC or starch; dosed as a suspension or slurry in a liquid ; or they may be formed into a tablet, caplet, or pill by compression or other processes known in the art. Such multiparticulates may be made by any known process, such as wet- and dry-granulation processes, extrusion/spheronization, roller-compaction, melt-congealing, or by spray-coating seed cores. For example, in wet-and dry-granulation processes, the agent described herein and optional excipients may be granulated to form multiparticulates of the desired size.

The agents can be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology, New York: Marcel Dekker, 1992, volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifier") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

The compounds described herein can be incorporated into pharmaceutically-acceptable nanoparticle, nanosphere, and nanocapsule formulations (Delie and Blanco-Prieto, 2005, Molecule 10:65-80). Nanocapsules can generally entrap compounds in a stable and reproducible way. To avoid side effects due to intracellular polymeric overloading, ultrafine particles (sized around 0.1 µm) can be designed using polymers able to be degraded in vivo (e.g. biodegradable polyalkyl-cyanoacrylate nanoparticles). Such particles are described in the prior art.

Implantable devices coated with a compound of this invention are another embodiment of the present invention. The compounds may also be coated on implantable medical devices, such as beads, or co-formulated with a polymer or other molecule, to provide a "drug depot", thus permitting the drug to be released over a longer time period than administration of an aqueous solution of the drug. Suitable coatings and the general preparation of coated implantable devices are described in U.S. Pat. Nos. 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition.

The formulations include those suitable for the administration routes detailed herein. The formulations may conveniently be presented in unit dosage form and may be prepared by any of the methods well known in the art of pharmacy. Techniques and formulations generally are found in Remington's. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

The terms "administer", "administering" or "administration" in reference to a compound, composition or formulation of the invention means introducing the compound into the system of the animal in need of treatment. When a compound of the invention is provided in combination with one or more other active agents, "administration" and its variants are each understood to include concurrent and/or sequential introduction of the compound and the other active agents.

The compositions described herein may be administered systemically or locally, e.g.: orally (e.g. using capsules, powders, solutions, suspensions, tablets, sublingual tablets and the like), by inhalation (e.g. with an aerosol, gas, inhaler, nebulizer or the like), to the ear (e.g. using ear drops), topically (e.g. using creams, gels, liniments, lotions, ointments, pastes, transdermal patches, etc.), ophthalmically (e.g. with eye drops, ophthalmic gels, ophthalmic ointments), rectally (e.g. using enemas or suppositories), nasally, buccally, vaginally (e.g. using douches, intrauterine devices, vaginal suppositories, vaginal rings or tablets, etc), via an implanted reservoir or the like, or parenterally depending on the severity and type of the disease being treated. The term "parenteral" as used herein includes, but is not limited to, subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

The pharmaceutical compositions described herein may be orally administered in any orally acceptable dosage form including, but not limited to, capsules, tablets, aqueous suspensions or solutions. Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. Tablets may be uncoated or may be coated by known techniques including microencapsulation to mask an unpleasant taste or to delay disintegration and adsorption in the gastrointestinal tract and thereby provide a sustained action over a longer period. For example, a time delay material such as glyceryl monostearate or glyceryl distearate alone or with a wax may be employed. A water soluble taste masking material such as hydroxypropyl-methylcellulose or hydroxypropyl-cellulose may be employed.

Formulations of a compound of Table IA or Table IB that are suitable for oral administration may be prepared as discrete units such as tablets, pills, troches, lozenges, aqueous or oil suspensions, dispersible powders or granules, emulsions, hard or soft capsules, e.g. gelatin capsules, syrups or elixirs. Formulations of a compound intended for oral use may be prepared according to any method known to the art for the manufacture of pharmaceutical compositions.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

The active compounds can also be in microencapsulated form with one or more excipients as noted above.

When aqueous suspensions are required for oral use, the active ingredient is combined with emulsifying and suspending agents. If desired, certain sweetening and/or flavoring agents may be added. Syrups and elixirs may be formulated with sweetening agents, for example glycerol, propylene glycol, sorbitol or sucrose. Such formulations may also contain a demulcent, a preservative, flavoring and coloring agents and antioxidant.

Sterile injectable forms of the compositions described herein (e.g. for parenteral administration) may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of injectable formulations.

Oily suspensions may be formulated by suspending a compound of Table IA or Table IB in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Aqueous suspensions of a compound of Table IA or Table IB contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound described herein, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsulated matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

The injectable solutions or microemulsions may be introduced into a patient's bloodstream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

Compositions for rectal or vaginal administration are preferably suppositories which can be prepared by mixing the compounds described herein with suitable non-irritating excipients or carriers such as cocoa butter, beeswax, polyethylene glycol or a suppository wax which are solid at ambient temperature but liquid at body temperature and therefore melt in the rectum or vaginal cavity and release the active compound. Other formulations suitable for vaginal administration may be presented as pessaries, tampons, creams, gels, pastes, foams or sprays.

The pharmaceutical compositions described herein may also be administered topically, especially when the target of treatment includes areas or organs readily accessible by topical application, including diseases of the eye, the ear, the skin, or the lower intestinal tract. Suitable topical formulations are readily prepared for each of these areas or organs.

Dosage forms for topical or transdermal administration of a compound described herein include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel. Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum. For treatment of the eye or other external tissues, e.g., mouth and skin, the formulations may be applied as a topical ointment or cream containing the active ingredient(s) in an amount of, for example, 0.075 to 20% w/w. When formulated in an ointment, the active ingredients may be employed with either an oil-based, paraffinic or a water-miscible ointment base.

Alternatively, the active ingredients may be formulated in a cream with an oil-in-water cream base. If desired, the aqueous phase of the cream base may include a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulfoxide and related analogs.

The oily phase of emulsions prepared using a compound of Table IA or Table IB may be constituted from known ingredients in a known manner. While the phase may comprise merely an emulsifier (otherwise known as an emulgent), it desirably comprises a mixture of at least one emulsifier with a fat or an oil or with both a fat and an oil. A hydrophilic emulsifier may be included together with a lipophilic emulsifier which acts as a stabilizer. In some embodiments, the emulsifier includes both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations. Emulgents and emulsion stabilizers suitable for use in the formulation of a compound of Table IA or Table IB include Tween™-60, Span™-80, cetostearyl alcohol, benzyl alcohol, myristyl alcohol, glyceryl mono-stearate and sodium lauryl sulfate.

The pharmaceutical compositions may also be administered by nasal aerosol or by inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. Formulations suitable for intrapulmonary or nasal administration have a particle size for example in the range of 0.1 to 500 micros (including particles in a range between 0.1 and 500 microns in increments microns such as 0.5, 1, 30, 35 microns, etc) which is administered by rapid inhalation through the nasal passage or by inhalation through the mouth so as to reach the alveolar sacs.

The pharmaceutical composition (or formulation) for use may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

In another aspect, a compound of Table IA or Table IB or a pharmaceutically acceptable salt thereof may be formulated in a veterinary composition comprising a veterinary carrier. Veterinary carriers are materials useful for the purpose of administering the composition and may be solid, liquid or gaseous materials which are otherwise inert or accepable in the veterinary art and are compatible with the active ingredient. These veterinary compositions may be administered parenterally, orally or by any other desired route.

### Therapeutic methods

In another aspect, the invention relates to the treatment of certain disorders by using sGC stimulators, either alone or in combination, or their pharmaceutically acceptable salts or pharmaceutical compositions comprising them, in a patient in need thereof.

The present disclosure relates to stimulators of soluble guanylate cyclase (sGC), pharmaceutical formulations thereof and their use, alone or in combination with one or more additional agents, for treating and/or preventing various diseases, wherein an increase in the concentration of NO or an increase in the concentration of cGMP might be desirable.

Increased production of NO or increased concentration of cGMP in a tissue leads to vasodilation, inhibition of platelet aggregation and adhesion, anti-hypertensive effects, anti-remodeling effects, anti-fibrotic, anti-apoptotic effects, anti-inflammatory effects and neuronal signal transmission effects, among other effects.

In other embodiments, the compounds here disclosed are sGC stimulators that may be useful in the prevention and/or treatment of diseases and disorders characterized by undesirable reduced bioavailability of and/or sensitivity to NO in a biological system (e.g., in the human body), such as those associated with conditions of oxidative stress or nitrosative stress.

The term "cardiovascular disease" (or "cardiovascular disorder") as used herein, refers to a disease based on the abnormal symptoms of circulatory organs such as the heart, blood vessels (arteries, capillaries, and veins) or both. The term also includes any disease that affects the cardiovascular system in general, including cardiac disease, vascular diseases of the brain, vascular diseases of the kidney, liver and associated organs, or lung, and peripheral arterial disease, among others.

A "sGC-related cardiovascular disease" is one for which the NO/sGC/cGMP system is known or suspected to be involved and is a cardiovascular disease that can be treated or prevented by sGC activation/stimulation, by activation of a NO synthase, or by addition of NO or an NO-donor or an NO precursor such as L-Arginine or L-citruline, or by inhibition of a PDE (phosphodiesterase) enzyme responsible for the breakdown of cGMP, or a combination of the any of the above methods.

The term "vasodilation" as used herein, refers to the widening of blood vessels. It results from relaxation of smooth muscle cells within the vessel walls, in particular in the large veins, large arteries, and smaller arterioles. In essence, the process is the opposite of "vasoconstriction", which is the narrowing of blood vessels. When blood vessels dilate, the flow of blood is increased due to a decrease in vascular resistance. Therefore, dilation of arterial blood vessels (mainly the arterioles) decreases blood pressure. The response may be intrinsic (due to local processes in the surrounding tissue) or extrinsic (due to hormones or the nervous system). In addition, the response may be localized to a specific organ (depending on the metabolic needs of a particular tissue, as during strenuous exercise), or it may be systemic (seen throughout the entire systemic circulation).

The term "vasoconstriction" as used herein refers to the narrowing of a blood vessel due to muscle contraction. Vasoconstriction is one mechanism by which the body regulates and maintains mean arterial pressure (MAP). Generalized vasoconstriction usually results in an increase in systemic blood pressure, but it may also occur in specific tissues, causing a localized reduction in blood flow.

As used herein, the term "bronchoconstriction" is used to define the constriction of the airways in the lungs due to the tightening of surrounding smooth muscle, with consequent coughing, wheezing, and shortness of breath. The condition has a number of causes, the most common being asthma. Exercise and allergies can bring on the symptoms in an otherwise asymptomatic individual. Other conditions such as chronic obstructive pulmonary disease (COPD) can also present with bronchoconstriction.

Throughout this disclosure, the terms "hypertension", "arterial hypertension" or "high blood pressure (HBP)" are used interchangeably and refer to an extremely common and highly preventable chronic condition in which blood pressure (BP) in the arteries is higher than normal or desired. If not properly controlled, it represents a significant risk factor for several serious cardiovascular and renal conditions. Hypertension may be a primary disease, called "essential hypertension" or "idiopathic hypertension", or it may be caused by or related to other diseases, in which case it is classified as "secondary hypertension". Essential hypertension accounts for 90-95% of all cases.

As used herein, the term "resistant hypertension" refers to hypertension that remains above goal blood pressure (usually less than 140/90 mmHg, although a lower goal of less than 130/80 mmHg is recommended for patients with comorbid diabetes or kidney disease), in spite of concurrent use of three antihypertensive agents belonging to different antihypertensive drug classes. People who require four or more drugs to control their blood pressure are also considered to have resistant hypertension. Hypertension is an extremely common comorbid condition in diabetes, affecting ∼20-60% of patients with diabetes, depending on obesity, ethnicity, and age. This type of hypertension is herein refered to as "diabetic hypertension". In type 2 diabetes, hypertension is often present as part of the metabolic syndrome of insulin resistance also including central obesity and dyslipidemia. In type 1 diabetes, hypertension may reflect the onset of diabetic nephropathy.

"Pulmonary hypertension (PH)", as used herein, is a disease characterized by sustained elevations of blood pressure in the pulmonary vasculature (pulmonary artery, pulmonary vein and pulmonary capillaries), which results in right heart hypertrophy, eventually leading to right heart failure and death. Common symptoms of PH include shortness of breath, dizziness and fainting, all of which are exacerbated by exertion. Without treatment, median life expectancy following diagnosis is 2.8 years. PH exists in many different forms, which are categorized according to their etiology. Categories include pulmonary arterial hypertension (PAH), PH with left heart disease, PH associated with lung diseases and /or hypoxaemia, PH due to chronic thrombotic and/or embolic disease and miscellaneous PH. PAH is rare in the general population, but the prevalence increases in association with certain common conditions such as HIV infection, scleroderma and sickle cell disease. Other forms of PH are generally more common than PAH, and, for instance, the association of PH with chronic obstructive pulmonary disease (COPD) is of particular concern. Current treatment for pulmonary hypertension depends on the stage and the mechanism of the disease.

The term "coronary artery disease" refers to a condition in which the blood supply to the heart muscle is partially or completely blocked (ischemia of the heart muscle or myocardium). This reduced blood supply to the myocardium may result in a number of "acute myocardial syndromes": chest pain ("angina", also called "angina pectoris", stable or unstable) and different types of heart attacks ("myocardial infarction" or MI). One common cause of coronary artery disease is "atherosclerosis" which refers to hardening of the arteries, due to fatty deposits in the artery walls which then may progress through formation of atherosclerotic plaques, to narrowing and eventually blockage of blood flow to the in the artery. This process of atherosclerosis may affect other arteries as well, not just those of the heart. A blood clot is the most common cause of the blockage of the artery, as usually the artery is already partially blocked due to atherosclerotic plaque (atheroma), the atheroma may rupture or tear, leading to the formation of a clot. Occasionally, coronary artery disease is caused by spasm of a coronary artery, which can occur spontaneously or as a result of the use of certain drugs (e.g., cocaine, nicotine). Rarely, the cause of coronary artery disease is a birth defect, a viral infection (e.g., Kawasaki disease), systemic lupus erythematosus (lupus), inflammation of the arteries (arteritis), a blood clot that travelled from a heart chamber into one of the coronary arteries or physical damage (e.g., from injury or radiation therapy).

"Unstable angina", as used herein, refers to a change in the pattern of angina symptoms including prolonged or worsening angina and new onset of severe symptoms.

MI can be classified into two types: "Non-ST-segment elevation" MI and "ST-segment elevation" MI. The complications of acute coronary syndromes depend on how much, how long, and where the coronary artery is blocked. If the blockage affects a large amount of heart muscle, the heart will not pump effectively. If the blockage shuts off blood flow to the electrical system of the heart, the heart rhythm may be affected. When a heart attack occurs, part of the myocardium dies. Dead tissue and the scar tissue that replaces it, does not contract. The scar tissue sometimes even expands or bulges when the rest of the heart tries to contract. Consequently there is less muscle to pump blood. If enough muscle dies, the heart's pumping ability may be so reduced that the heart cannot meet the body's demands for oxygen and blood. Heart failure, low blood pressure or both then develop. If more than half of the myocardium is damaged or dies, the heart generally cannot function and severe disability or death is likely.

As used herein "Heart Failure" (HF) is a progressive disorder of left ventricular (LV) myocardial remodeling that culminates in a complex clinical syndrome in which impaired cardiac function and circulatory congestion are the defining features, and results in insufficient delivery of blood and nutrients to body tissues. The condition occurs when the heart is damaged or overworked and unable to pump out all the blood that returns to it from the systemic circulation. As less blood is pumped out, blood returning to the heart backs up and fluid builds up in other parts of the body. Heart failure also impairs the kidneys' ability to dispose of sodium and water, complicating fluid retention further. Heart failure is characterized by autonomic dysfunction, neuro-hormonal activation and overproduction of cytokines, which contribute to progressive circulatory failure. Symptoms of heart failure include: dyspnea (shortness of breath) while exercising or resting and waking at night due to sudden breathlessness, both indicative of pulmonary edema; general fatigue or weakness; edema of the feet, ankles and legs; rapid weight gain; or chronic cough, including that producing mucus or blood. Depending on its clinical presentation, heart failure is classified as *de novo*, transient, acute, post-acute or chronic. Acute heart failure, i.e., the rapid or gradual onset of symptoms requiring urgent therapy, may develop *de novo* or as a result of chronic heart failure becoming decompensated. The term "Heart failure" is often used to mean "chronic heart failure". The terms "congestive heart failure (CHF)" or "congestive cardiac failure (CCF)" are often used interchangeably with chronic heart failure. Common causes of heart failure include coronary artery disease including a previous myocardial infarction (heart attack), high blood pressure, atrial fibrillation, valvular heart disease, and cardiomyopathy. These cause heart failure by changing either the structure or the functioning of the heart.

There are two main types of heart failure: "heart failure due to reduced ejection fraction (HFREF)", also known as "heart failure due to left ventricular systolic dysfunction" or "systolic heart failure", and "heart failure with preserved ejection fraction (HFPEF)", also known as "diastolic heart failure" or "heart failure with normal ejection fraction (HFNEF)". Ejection fraction is the proportion of blood in the heart pumped out of the heart during a single contraction. It is a percentage with normal being between 50 and 75%.

The term "acute" (as in "acute HF") is used to mean rapid onset, and "chronic" refers to long duration. Chronic heart failure is a long term situation, usually with stable treated symptomatology. "Acute decompensated" heart failure is worsening or decompensated heart failure, referring to episodes in which a person can be characterized as having a change in heart failure signs and symptoms resulting in a need for urgent therapy or hospitalization. Heart failure may also occur in situations of high output (then it is termed "high output cardiac failure") where the ventricular systolic function is normal but the heart cannot deal with an important augmentation of blood volume.

In cardiovascular physiology, the term "Ejection Fraction (EF)" is defined as the fraction of blood in the left and right ventricles that is pumped out with each heartbeat or cardiac cycle. In finite mathematics allowed by medical imaging, EF is applied to both the right ventricle, which ejects blood via the pulmonary valve into the pulmonary circulation, or the left ventricle, which ejects blood via the aortic valve into the cerebral and systemic circulation.

The term "heart failure with preserved ejection fraction (HFPEF)" is commonly understood to refer to a manifestation of signs and symptoms of heart failure with an ejection fraction greater than 55%. It is characterized by a decrease in left ventricular compliance, leading to increased pressure in the left ventricle. Increased left atrial size is often seen with HFPEF as a result of the poor left ventricular function. There is an increased risk for congestive heart failure, atrial fibrillation, and pulmonary hypertension. Risk factors are hypertension, hyperlipidemia, diabetes, smoking, and obstructive sleep apnea. In this type of heart failure, the heart muscle contracts well but the ventricle does not fill with blood well in the relaxation phase.

The term "heart failure with reduced ejection fraction (HFREF)" refers to heart failure in which the ejection fraction is less than 40%.

Diabetes is a common comorbidity in patients with heart failure and is associated with poorer outcomes as well as potentially compromising the efficacy of treatments. Other important comorbidities include systemic hypertension, chronic airflow obstruction, sleep apnea, cognitive dysfunction, anemia, chronic kidney disease and arthritis. Chronic left heart failure is frequently associated with the development of pulmonary hypertension. The frequency of certain comorbidities varies by gender: among women, hypertension and thyroid disease are more common, while men more commonly suffer from chronic obstructive pulmonary disease (COPD), peripheral vascular disease, coronary artery disease and renal insufficiency. Depression is a frequent comorbidity of heart failure and the two conditions can and often do complicate one another. Cachexia has long been recognized as a serious and frequent complication of heart failure, affecting up to 15% of all heart failure patients and being associated with poor prognosis. Cardiac cachexia is defined as the nonedematous, non-voluntary loss of at least 6% of body weight over a period of six months.

The term "arrhythmias", as used herein, refers to abnormal heart rhythms that occur in more than 90 % of people who have had a heart attack. Sometimes the problem is with the part of the heart that triggers the heartbeat and the heart rate may be too slow, other times the problems may cause the heart to beat too rapidly or irregularly. Sometimes the signal to beat is not conducted from one part of the heart to the other and the heartbeat may slow or stop. In addition areas of the myocardium that have not died but have poor blood flow may be irritable. This causes heart rhythm problems such as ventricular tachycardia or ventricular fibrillation. This may lead to cardiac arrest if the heart stops pumping entirely.

The "pericardium" is the sack or membrane that surrounds the heart. "Pericarditis" or inflammation of this membrane may develop as a result of a heart attack and may result in fever, pericardial effusion, inflammation of the membranes covering the lungs (pleura), pleural effusion, and joint pain. Other complications after a heart attack may include malfunction of the mitral valve, rupture of the heart muscle, a bulge in the wall of the ventricle (ventricular aneurysm), blood clots, and low blood pressure.

The term "cardiomyopathy" refers to the progressive impairment of the structure and function of the muscular walls of the heart chambers. The main types of cardiomyopathies are dilated, hypertrophic and restrictive. Cardiomyophaties often cause symptoms of heart failure, and they may also cause chest pain, fainting and sudden death.

The terms "mitral valve regurgitation", "mitral regurgitation", "mitral insufficiency" or "mitral incompetence" refer to a situation in which the mitral valve of the heart doesn't close tightly, allowing blood to flow backward in the heart. As a result, blood can't move through the heart or to the rest of the body as efficiently, resulting in fatigue or shortness of breath.

The term "sleep apnea" refers to the most common of the sleep-disordered breathing disorders. It is a condition characterized by intermittent, cyclical reductions or total cessations of airflow, which may or may not involve obstruction of the upper airway. There are three types of sleep apnea: obstructive sleep apnea, the most common form, central sleep apnea and mixed sleep apnea.

"Central sleep apnea (CSA)", is caused by a malfunction in the brain's normal signal to breathe, rather than physical blockage of the airway. The lack of respiratory effort leads to an increase in carbon dioxide in the blood, which may rouse the patient. CSA is rare in the general population, but is a relatively common occurrence in patients with systolic heart failure.

As used herein, the term "metabolic syndrome", "insulin resistance syndrome" or "syndrome X", refers to a group or clustering of metabolic conditions (abdominal obesity, elevated fasting glucose, "dyslipidemia" (i.e,. elevated lipid levels) and elevated blood pressure (HBP)) which occur together more often than by chance alone and that together promote the development of type 2 diabetes and cardiovascular disease. Metabolic syndrome is characterized by a specific lipid profile of increased triglycerides, decreased high-density lipoprotein cholesterol (HDL-cholesterol) and in some cases moderately elevated low-density lipoprotein cholesterol (LDL-cholesterol) levels, as well as accelerated progression of "atherosclerotic disease" due to the pressure of the component risk factors. There are several types of dyslipidemias: "hypercholesterolemia" refers to elevated levels of cholesterol. Familial hypercholesterolemia is a specific form of hypercholesterolemia due to a defect on chromosome 19 (19p13.1-13.3). "Hyperglyceridemia" refers to elevated levels of glycerides (e.g., "hypertrigliceridemia" involves elevated levels of triglycerides). "Hyperlipoproteinemia" refers to elevated levels of lipoproteins (usually LDL unless otherwise specified).

The term "steatosis" refers to the abnormal retention of lipids within a cell. It usually reflects an impairment of the normal processes of synthesis and elimination of triglycerides. Excess fat accumulates in vesicles that displace the cytoplasm of the cell. In severe cases the cell may burst. Usually steatosis is observed in the liver as it is the organ mostly associated with fat metabolism. It can also be observed in the heart, kidneys and muscle tissue.

As used herein, the term "peripheral vascular disease (PVD)", also commonly referred to as "peripheral arterial disease (PAD)" or "peripheral artery occlusive disease (PAOD)", refers to the obstruction of large arteries *not* within the coronary, aortic arch vasculature, or the brain. PVD can result from atherosclerosis, inflammatory processes leading to stenosis, an embolism, thrombus formation or other types of occlusions. It causes either acute or chronic "ischemia (lack of blood supply)". Often PVD is a term used to refer to atherosclerotic blockages found in the lower extremity. PVD also includes a subset of diseases classified as microvascular diseases resulting from episodic narrowing of the arteries (e.g., "Raynaud's phenomenon"), or widening thereof (erythromelalgia), i.e., vascular spasms. Peripheral arterial diseases include occlusive thrombotic vasculitis, peripheral arterial occlusive disease, Raynaud's disease, and Raynaud's syndrome. Common symptoms are cold leg or feet, intermittent claudication, lower limb pain and critical limb ischemia (lower limb ulcers and necrosis). Diagnosis and treatment guidelines for peripheral arterial disease can be found in Eur. J. Vasco Endovasc. Surg, 2007, 33(1), S1.

The term "stenosis" as used herein refers to an abnormal narrowing in a blood vessel or other tubular organ or structure. It is also sometimes called a "stricture" (as in urethral stricture). The term "coarctation" is a synonym, but is commonly used only in the context of aortic coarctation. The term "restenosis" refers to the recurrence of stenosis after a procedure.

The term "thrombosis" refers to the formation of a blood clot ("thrombus") inside a blood vessel, obstructing the flow of blood through the circulatory system. When a blood vessel is injured, the body uses platelets (thrombocytes) and fibrin to form a blood clot to prevent blood loss. Alternatively, even when a blood vessel is not injured, blood clots may form in the body if the proper conditions present themselves. If the clotting is too severe and the clot breaks free, the traveling clot is now known as an "embolus". The term "thromboembolism" refers to the combination of thrombosis and its main complication, "embolism". When a thrombus occupies more than 75% of surface area of the lumen of an artery, blood flow to the tissue supplied is reduced enough to cause symptoms because of decreased oxygen (hypoxia) and accumulation of metabolic products like lactic acid ("gout"). More than 90% obstruction can result in anoxia, the complete deprivation of oxygen and "infarction", a mode of cell death.

An "embolism" (plural embolisms) is the event of lodging of an embolus (a detached intravascular mass capable of clogging arterial capillary beds at a site far from its origin) into a narrow capillary vessel of an arterial bed which causes a blockage (vascular occlusion) in a distant part of the body. This is not to be confused with a thrombus which blocks at the site of origin. The material that forms the embolism can have a number of different origins: if the material is blood the "embolus" is termed a "thrombus"; the solid material could also comprise fat, bacterial remains, infected tissue, etc.

"Ischemia" is a restriction in blood supply to tissues, causing a shortage of oxygen and glucose needed for cellular metabolism (to keep tissue alive). Ischemia is generally caused by problems with blood vessels, with resultant damage to or dysfunction of tissue. It also means local anemia in a given part of a body sometimes resulting from congestion (such as vasoconstriction, thrombosis or embolism). If the "ischemia" takes place in the heart muscle (or "myocardium") the ischemia is termed myocardial ischemia. Other types of ischemia are for instance cerebral ischemia, critical limb ischemia and the like.

"Reperfusion" occurs when blood supply returns to the tissue after a period of ischemia. Upon restoration of circulation to the tissue, inflammatory and oxidative stress processes may develop. One example of this chain of events is ischemia-reperfusion associated with organ transplants.

"Reperfusion injury" is the tissue damage caused when blood supply returns to the tissue after a period of ischemia and inflammation and oxidative damage ensue rather than restoration of normal function. Reperfusion of ischemic issues is often associated with microvascular injury, particularly due to the increased permeability of capillaries and arterioles that lead to an increase in diffusion and fluid filtration across the tissues. The activated endothelial cells produce more reactive oxygen species but less NO following reperfusion, and the imbalance results in an inflammatory response. White blood cells, carried to the area by the newly returned blood flow, release a host of inflammatory factors and free radicals in response to tissue damage. The restored blood flow brings with it oxygen that damages cellular proteins, DNA and plasma membranes. This process of ischemia-reperfusion is also thought to be responsible for formation and failure to heal of chronic wounds, (e.g., pressure sores or diabetic ulcers).

The term "angiopathy" as used herein is the generic term for a disease of the blood vessels (arteries, veins, and capillaries). The most common and most prevalent angiopathy is "diabetic angiopathy", a common complication of chronic diabetes. Another common type of angiopathy is "cerebral amyloid angiopathy" (CAA), also known as congophilic angiopathy, wherein amyloid deposits form in the walls of the blood vessels of the central nervous system. The term *congophilic* is used because the presence of the abnormal aggregations of amyloid can be demonstrated by microscopic examination of brain tissue after application of a special stain called Congo red. The amyloid material is only found in the brain and as such the disease is not related to other forms of amyloidosis.

A "stroke", or cerebrovascular accident (CVA), is the rapid loss of brain function(s) due to disturbance in the blood supply to the brain. This can be due to "ischemia" (lack of blood flow with resultant insufficient oxygen and glucose supply to the tissue) caused by blockage (thrombosis, arterial embolism, fat accumulation or a spasm), or a hemorrhage (leakage of blood). As a result, the affected area of the brain cannot function, which might result in an inability to move one or more limbs on one side of the body, inability to understand or formulate speech, or an inability to see one side of the visual field. Risk factors for stroke include old age, hypertension, previous stroke or transient ischemic attack (TIA), diabetes, high cholesterol, cigarette smoking and atrial fibrillation. High blood pressure is the most important modifiable risk factor of stroke. An "ischemic stroke" is occasionally treated in a hospital with thrombolysis (also known as a "clot buster"), and some hemorrhagic strokes benefit from neurosurgery. Prevention of recurrence may involve the administration of antiplatelet drugs such as aspirin and dipyridamole, control and reduction of hypertension, and the use of statins. Selected patients may benefit from carotid endarterectomy and the use of anticoagulants.

"Vascular dementia" is the 2nd most common cause of dementia among the elderly. It is more common among men and usually begins after age 70. It occurs more often in people who have vascular risk factors (e.g, hypertension, diabetes mellitus, hyperlipidemia, smoking) and in those who have had several strokes. Many people have both vascular dementia and Alzheimer disease. Vascular dementia typically occurs when multiple small cerebral infarcts (or sometimes hemorrhages) cause enough neuronal or axonal loss to impair brain function. Vascular dementias include the following types: multiple lacunar infarction (wherein small blood vessels are affected and infarcts occur deep within hemispheric white and gray matter); multi-infarct dementia (wherein medium-sized blood vessels are affected); strategic single-infarct dementia (wherein a single infarct occurs in a crucial area of the brain such as the angular gyrus or the thalamus; Binswanger dementia or subcortical arteriosclerotic encephalopathy (wherein small-vessel dementia is associated with severe, poorly controlled hypertension and systemic vascular disease and which causes diffuse and irregular loss of axons and myelin with widespread gliosis, tissue death due to an infarction, or loss of blood supply to the white matter of the brain).

The term "glioma" refers to a type of tumor that starts in the brain or spine. It is called a glioma because it arises from glial cells. The most common site of gliomas is the brain. Gliomas make up about 30% of all brain and central nervous system tumors and 80% of all malignant brain tumors.

According to the American Psychiatric Association's Diagnostic and Statistical Manual of Mental Disorders, Fourth Edition (DSM-IV), the term "sexual dysfunction" encompasses a series of conditions "characterized by disturbances in sexual desire and in the psychophysiological changes associated with the sexual response cycle"; while problems of this type are common, sexual dysfunction is only considered to exist when the problems cause distress for the patient. Sexual dysfunction can be either physical or psychological in origin. It can exist as a primary condition, generally hormonal in nature, although most often it is secondary to other medical conditions or to drug therapy for said conditions. All types of sexual dysfunction can be further classified as life-long, acquired, situational or generalized (or combinations thereof).

The DSM-IV-TR specifies five major categories of "female sexual dysfunction": sexual desire/interest disorders; "sexual arousal disorders (including genital, subjective and combined)"; orgasmic disorder; dyspareunia and vaginismus; and persistent sexual arousal disorder.

"Female sexual arousal disorder (FSAD)" is defined as a persistent or recurring inability to attain or maintain sufficient levels of sexual excitement, causing personal distress. FSAD encompasses both the lack of subjective feelings of excitement (i.e., subjective sexual arousal disorder) and the lack of somatic responses such as lubrication and swelling (i.e., genital/physical sexual arousal disorder). FSAD may be strictly psychological in origin, although it generally is caused or complicated by medical or physiological factors. Hypoestrogenism is the most common physiologic condition associated with FSAD, which leads to urogenital atrophy and a decrease in vaginal lubrication.

As used herein, "erectile dysfunction (ED)" is a male sexual dysfunction characterized by the inability to develop or maintain an erection of the penis during sexual performance. A penile erection is the hydraulic effect of blood entering and being retained in sponge-like bodies within the penis. The process is often initiated as a result of sexual arousal, when signals are transmitted from the brain to nerves in the penis. Erectile dysfunction is indicated when an erection is difficult to produce. The most important organic causes are cardiovascular disease and diabetes, neurological problems (for example, trauma from prostatectomy surgery), hormonal insufficiencies (hypogonadism) and drug side effects.

In one embodiment, compounds of **Table IA or Table IB** that are stimulators of sGC, and their pharmaceutically acceptable salts thereof, are therefore useful in the prevention and/or treatment of the following types of cardiac, pulmonary, peripheral, hepatic, kidney, or cerebral vascular/endothelial disorders, conditions and diseases related to circulation:
- disorders related to high blood pressure and decreased coronary blood flow; increased acute and chronic coronary blood pressure; arterial hypertension; vascular disorder resulting from cardiac and renal complications; vascular disorders resulting from heart disease, stroke, cerebral ischemia or renal failure; resistant hypertension; diabetic hypertension; essential hypertension; secondary hypertension; gestational hypertension; pre-eclampsia; portal hypertension; myocardial infarction;
- heart failure, HFPEF, HFREF; acute and chronic HF; more specific forms of HF: acute decompensated HF, right ventricular failure, left ventricular failure, total HF, ischemic cardiomyopathy, dilatated cardiomyopathy, congenital heart defects, HF with valvular defects, mitral valve stenosis, mitral valve insufficiency, aortic valve stenosis, aortic valve insufficiency, tricuspid stenosis, tricuspic insufficiency, pulmonary valve stenosis, pulmonary valve insufficiency, combined valvular defects; diabetic heart failure; alcoholic cardiomyopathy or storage cardiomyopathies; diastolic HF, systolic HF; acute phases of an existing chronic HF (worsening HF); diastolic or systolic dysfunction; coronary insufficiency; arrhythmias; reduction of ventricular preload; cardiac hypertrophy; heart failure/cardiorenal syndrome; portal hypertension; endothelial dysfunction or injury; disturbances of atrial and ventricular rhythm and conduction disturbances: atrioventricular blocks of degree I-III (AVB I-III), supraventricular tachyarrhythmia, atrial fibrillation, atrial flutter, ventricular fibrillation, ventricular flutter, ventricular tachyarrhythmia, torsade-de-pointes tachycardia, atrial and ventricular extrasystoles, AV-junction extrasystoles, sick-sinus syndrome, syncopes, AV-node reentry tachycardia; Wolff-Parkinson-White syndrome or acute coronary syndrome; Boxer cardiomyopathy; premature ventricular contraction; cardiomyopathy; cancer-induced cardiomyopathy;
- thromboembolic disorders and ischemias; myocardial ischemia; infarction; myocardial infarction; heart attack; myocardial insufficiency; endothelial dysfunction; stroke; transient ischemic attacks (TIAs); obstructive thromboanginitis; stable or unstable angina pectoris; coronary spasms or spasms of the peripheral arteries; variant angina; Prinzmetal's angina; cardiac hypertrophy; preeclampsia; thrombogenic disorders; ischemia-reperfusion damage; ischemia-reperfusion associated with organ transplant; ischemia-reperfusion associated with lung transplant, pulmonary transplant, cardiac transplant, venus graft failure; conserving blood substituents in trauma patients;
- peripheral vascular disease; peripheral arterial disease; peripheral occlusive arterial disease; hypertonia; Raynaud's syndrome or phenomenon (primary and secondary); Raynaud's disease; critical limb ischemia; peripheral embolism; intermittent claudication; vaso-occlusive crisis; muscular dystrophy, Duchenne muscular dystrophy, Becker muscular dystrophy; microcirculation abnormalities; control of vascular leakage or permeability; lumbar spinal canal stenosis; occlusive thrombotic vasculitis; thrombotic vasculitis; peripheral perfusion disturbances; arterial and venous thrombosis; microalbuminuria; peripheral and autonomic neuropathies; diabetic microangiopathies;
- edema; renal edema due to heart failure;
- Alzheimer's disease; Parkinson's disease; vascular dementias; vascular cognitive impairment; cerebral vasospasm; congenital myasthenic syndrome; subarachnoid hemorrhage; traumatic brain injury; improving perception, capacity for concentration, capacity for learning or memory performance after cognitive disturbances such as those ocurring in mild cognitive impairment, age-related learning and memory disturbances, age-related memory loss, vascular dementia, head injury, stroke, post-stroke dementia, post-traumatic head injury, general disturbances of concentration and disturbances of concentration in children with learning and memory problems; Lewy body dementia; dementia with frontal lobe degeneration including Pick's syndrome; progressive nuclear palsy; dementia with corticobasal degeneration; Amyotropic Lateral Sclerosis (ALS); Huntington's disease; demyelination; Multiple Sclerosis; thalamic degeneration; Creutzfeldt-Jakob dementia; HIV-dementia; schizophrenia with dementia or Korsakoff psychosis; Multiple System Atrophy and other forms of Parkinsonism Plus; movement disorders; neuroprotection; anxiety, tension and depression or post-traumatic stress disorder (PTSD); bipolar disorder; schizophrenia; CNS-related sexual dysfunction and sleep disturbances; pathological eating disorders and use of luxury foods and addictive drugs; controlling cerebral perfusion; migraines; prophylaxis and control of consequences of cerebral infarction (apoplexia cerebri); prophylaxis and control of consequences of stroke, cerebral ischemias and head injury;
- shock; cardiogenic shock; sepsis; septic shock; anaphylactic shock; aneurysm; control of leukocyte activation; inhibition or modulation of platelet aggregation; multiple organ dysfunction syndrome (MODS); multiple organ failure (MOF);
- pulmonary/respiratory conditions: pulmonary hypertension (PH); pulmonary arterial hypertension (PAH), and associated pulmonary vascular remodeling; vascular remodeling in the form of localized thrombosis and right heart hypertrophy; pulmonary hypertonia; primary pulmonary hypertension; secondary pulmonary hypertension; familial pulmonary hypertension; sporadic pulmonary hypertension; pre-capillary pulmonary hypertension; idiopathic pulmonary hypertension; other forms of PH; PH associated with left ventricular disease, HIV, SCD, thromboembolism (CTEPH), sarcoidosis, COPD, pulmonary fibrosis, acute respiratory distress syndrome (ARDS), acute lung injury, alpha-1-antitrypsin deficiency (AATD), pulmonary emphysema, smoking-induced emphysema and cystic fibrosis (CF); thrombotic pulmonary arteriopathy; plexogenic pulmonary arteriopathy; cystic fibrosis; bronchoconstriction or pulmonary bronchoconstriction; acute respiratory distress syndrome; lung fibrosis, lung transplant; asthmatic diseases;
- pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary veno-occlusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, other coagulation disorders, chronic thromboembolism, pulmonary embolism; pulmonary embolism due to tumor, parasites or foreign material; connective tissue disease, lupus, lupus nephritis, schistosomiasis, sarcoidosis, chronic obstructive pulmonary disease, asthma, emphysema, chronic bronchitis, pulmonary capillary hemangiomatosis, histiocytosis X, lymphangiomatosis, compressed pulmonary vessels; compressed pulmonary vessels due to adenopathy, tumor or fibrosing mediastinitis;
- arterosclerotic diseases or conditions: atherosclerosis; atherosclerosis associated with endothelial injury, platelet and monocyte adhesion and aggregation, smooth muscle proliferation or migration; restenosis; restenosis developed after thrombolysis therapies, percutaneous transluminal angioplasties (PTAs), transluminal coronary angioplasties (PTCAs), heart transplant, bypass operations or inflammatory processes;
- micro and macrovascular damage (vasculitis); increased levels of fibrinogen and low density DLD; increased concentration of plasminogen activator inhibitor 1 (PA-1);
- metabolic syndrome; metabolic diseases or diseases associated with metabolic syndrome: obesity; excessive subcutaneous fat; excessive adiposity; diabetes; high blood pressure; lipid related disorders, hyperlipidemias, dyslipidemia, hypercholesterolemias, decreased high-density lipoprotein cholesterol (HDL-cholesterol), moderately elevated low-density lipoprotein cholesterol (LDL-cholesterol) levels, hypertriglyceridemias, hyperglyceridemia, hypolipoproteinanemias, sitosterolemia, fatty liver disease, hepatitis; preeclampsia; polycystic kidney disease progression; liver steatosis or abnormal lipid accumulation in the liver; steatosis of the heart, kidneys or muscle; alphabetalipoproteinemia; sitosterolemia; xanthomatosis; Tangier disease; hyperammonemia and related dieases; hepatic encephalopaties; other toxic encephalopaties; Reye syndrome;
- sexual, gynecological and urological disorders of conditions: erectile dysfunction; impotence; premature ejaculation; female sexual dysfunction; female sexual arousal dysfunction; hypoactive sexual arousal disorder; vaginal atrophy; dyspaneuria; atrophic vaginitis; benign prostatic hyperplasia (BPH), prostatic hypertrophy, prostatic enlargement; bladder outlet obstruction; bladder pain syndrome (BPS); interstitial cystitis (IC); overactive bladder; neurogenic bladder and incontinence; diabetic nephropathy; primary and secondary dysmenhorrea; lower urinary tract syndromes (LUTS); endometriosis; pelvic pains; benign and malignant diseases of the organs of the male and female urogenital system;
- chronic kidney disease; acute and chronic renal insufficiency; acute and chronic renal failure; lupus nephritis; underlying or related kidney diseases: hypoperfusion, intradialytic hypotension, obstructive uropathy, glomerulopathies, glomerulonephritis, acute glomerulonephritis, glomerulosclerosis, tubulointerstitial diseases, nephropathic diseases, primary and congenital kidney diseases, nephritis; diseases characterized by abnormally reduced creatinine and or water excretion; diseases characterized by abnormally increased blood concentrations of urea, nitrogen, potassium and/or creatinine; diseases characterized by altered activity of renal enzymes, diseases characterized by alterened activity of glutamyl synthetase; diseases characterized by altered urine osmolarity or urine volume; diseases characterized by increased microalbuminuria, diseases characterized by macroalbuminuria; diseases characterized by lesions of glomeruli and arterioles, tubular dilatation, hyperphosphatemia and/or need for dialysis; sequelae of renal insufficiency; renal-insufficiency related pulmonary enema; renal-insufficiency related to HF; renal insufficiency related to uremia or anemia; elecrolyte disturbances (herkalemia, hyponatremia); disturbances of bone and carbohydrate metabolism;
- ocular diseases or disorders such as glaucoma, retinopathy and diabetic retinopathy.

The term "Inflammation" refers to the complex biological response of vascular tissues to harmful stimuli, such as pathogens, damaged cells, or irritants. The classical signs of acute inflammation are pain, heat, redness, swelling, and loss of function. Inflammation is a protective attempt by the organism to remove the injurious stimuli and to initiate the healing process. Inflammation is not a synonym for infection, even though the two are often correlated (the former often being a result of the latter). Inflammation can also occur in the absence of infection, although such types of inflammation are usually maladaptive (such as in atherosclerosis). Inflammation is a stereotyped response, and therefore it is considered as a mechanism of innate immunity, as compared to adaptive immunity, which is specific for each pathogen. Progressive destruction of tissue in the absence of inflammation would compromise the survival of the organism. On the other hand, chronic inflammation might lead to a host of diseases, such as hay fever, periodontitis, atherosclerosis, rheumatoid arthritis, and even cancer (e.g., gallbladder carcinoma). It is for that reason that inflammation is normally closely regulated by the body. Inflammation can be classified as either *acute* or *chronic.* "Acute inflammation" is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as "chronic inflammation", leads to a progressive shift in the type of cells present at the site of inflammation and is characterized by simultaneous destruction and healing of the tissue from the inflammatory process.

In another embodiment, compounds of **Table IA or Table IB** that are stimulators of sGC, and their pharmaceutically acceptable salts thereof, are therefore useful in the prevention and/or treatment of the following types of cardiac, pulmonary, peripheral, hepatic, kidney, digestive or Central Nervous System disorders, conditions and diseases which may involve inflammation or an inflammatory process:
- heart muscle inflammation (myocarditis); chronic myocarditis; acute myocarditis; viral myocarditis;
- vasculitis; pancreatitis; peritonitis; rheumatoid diseases;
- inflammatory disease of the kidney; immunological kidney diseases: kidney transplant rejection, immune complex-induced kidney disease, nephropathy induced by toxins, constrast medium-induced nephropathy; diabetic and non-diabetic nephropathy, pyelonephritis, renal cysts, nephrosclerosis, hypertensive nephrosclerosis and nephrotic syndrome;
- chronic interstitial inflammations. inflammatory bowel diseases (IBD), Crohn's, Ulcerative Colitis (UC);
- inflammatory skin diseases;
- inflammatory diseases of the eye, blepharitis, dry eye syndrome, and Sjögren's Syndrome; eye fibrosis.

The term "wound healing" refers to the intricate process where the skin (or another organ or tissue) repairs itself after injury. For instance, in normal skin, the epidermis (outermost layer) and dermis (inner or deeper layer) exist in a steady-state equilibrium, forming a protective barrier against the external environment. Once the protective barrier is broken, the normal (physiologic) process of wound healing is immediately set in motion. The classic model of wound healing is divided into three or four sequential, yet overlapping, phases: (1) hemostasis (not considered a phase by some authors), (2) inflammation, (3) proliferation and (4) remodeling. Upon injury to the skin, a set of complex biochemical events takes place in a closely orchestrated cascade to repair the damage. Within the first few minutes after the injury, platelets adhere to the site of injury, become activated, and aggregate (join together), followed by activation of the coagulation cascade which forms a clot of aggregated platelets in a mesh of cross-linked fibrin protein. This clot stops active bleeding ("hemostasis"). During the inflammation phase, bacteria and cell debris are phagocytosed and removed from the wound by white blood cells. Platelet-derived growth factors (stored in the alpha granules of the platelets) are released into the wound that cause the migration and division of cells during the proliferative phase. The proliferation phase is characterized by angiogenesis, collagen deposition, granulation tissue formation, epithelialization, and wound contraction. In "angiogenesis", vascular endothelial cells form new blood vessels. In "fibroplasia" and granulation tissue formation, fibroblasts grow and form a new, provisional extracellular matrix (ECM) by excreting collagen and fibronectin. Concurrently, "re-epithelialization" of the epidermis occurs, in which epithelial cells proliferate and 'crawl' atop the wound bed, providing cover for the new tissue. During wound contraction, myofibroblasts decrease the size of the wound by gripping the wound edges and contracting using a mechanism that resembles that in smooth muscle cells. When the cells' roles are close to complete, unneeded cells undergo apoptosis. During maturation and remodeling, collagen is remodeled and realigned along tension lines, and cells that are no longer needed are removed by apoptosis. However, this process is not only complex but fragile, and is susceptible to interruption or failure leading to the formation of non-healing chronic wounds (one example includes diabetic wounds or ulcers, and, in particular, diabetic foot ulcers). Factors that contribute to non-healing chronic wounds are diabetes, venous or arterial disease, infection, and metabolic deficiencies of old age.

The terms "bone healing", or "fracture healing" refers to a proliferative physiological process in which the body facilitates the repair of a bone fracture. In the process of fracture healing, several phases of recovery facilitate the proliferation and protection of the areas surrounding fractures and dislocations. The length of the process depends on the extent of the injury, and usual margins of two to three weeks are given for the reparation of most upper bodily fractures; anywhere above four weeks given for lower bodily injury. The healing process is mainly determined by the "periosteum" (the connective tissue membrane covering the bone). The periosteum is one source of precursor cells which develop into "chondroblasts" and osteoblasts that are essential to the healing of bone. The bone marrow (when present), endosteum, small blood vessels, and fibroblasts are other sources of precursor cells.

In another embodiment, compounds of Table IA or Table IB, that are stimulators of sGC and their pharmaceutically acceptable salts thereof, are therefore useful in the treatment of the following types of diseases, disorders or conditions in which stimulation of the processes of wound or bone healing would be desirable:
- wound or ulcer healing in diabetics; microvascular perfusion improvement; microvascular perfusion improvement following injury or to counteract the inflammatory response in perioperative care; anal fissures; diabetic ulcers; diabetic foot ulcers); bone healing; osteoclastic bone resorption and remodeling; and new bone formation.

The term "connective tissue" (CT) refers to a kind of animal tissue that supports, connects, or separates different types of tissues and organs of the body. It is one of the four general classes of animal tissues, the others being epithelial, muscle, and nervous tissues. Connective tissue is found everywhere, including in the central nervous system. It is located in between other tissues. All CT has three main components--ground substances, fibers and cells--and all these components are immersed in the body fluids.

The term "connective tissue disorder or condition" refers to any condition that involves abnormalities in connective tissue in one or more parts of the body. Certain disorders are characterized by over-activity of the immune system with resulting inflammation and systemic damage to the tissues, usually with replacement of normal tissue (e.g., normal tissue of a certain organ) with connective tissue. Other disorders involve biochemical abnormalities or structural defects of the connective tissue itself. Some of these disorders are inherited, and some are of unknown etiology.

When connective tissue diseases are of autoimmune origin they are classified as "rheumatic disorders", "autoimmune rheumatic disorders" or "autoimmune collagen-vascular disorders".

In an "autoimmune disorder", antibodies or other cells produced by the body attack the body's own tissues. Many autoimmune disorders affect connective tissue in a variety of organs. In autoimmune disorders, inflammation and the immune response may result in connective tissue damage, around the joints and also in other tissues, including vital organs, such as the kidneys or organs of the gastrointestinal tract. The sac that surrounds the heart (pericardium), the membrane that covers the lungs (pleura), the mediastinum (an undelineated group of structures in the thorax, surrounded by loose connective tissue, containing the heart, the great vessels of the heart, the esophagus, the trachea, the phrenic nerve, the cardiac nerve, the thoracic duct, the thymus, and the lymph nodes of the central chest) and even the brain may be affected.

The term "fibrosis" as used herein refers to the accumulation of connective tissue or fibrous tissue (scar tissue, collagen) in a certain organ or part of the body. If fibrosis arises from a single cell line it is called a "fibroma". Fibrosis occurs as the body attempts to repair and replace damaged cells, and thus can be a reactive, benign or a pathological state. Physiological fibrosis is similar to the process of scarring. A pathological state develops when the tissue in question is repeatedly and continuously damaged. A single episode of injury, even if severe, does not usually cause fibrosis. If injury is repeated or continuous (for instance as it occurs in chronic hepatitis) the body attempts to repair the damage, but the attempts result instead in excessive accumulation of scar tissue. Scar tissue starts to replace regular tissue of the organ which performs certain functions that the scar tissue is not able to perform; it can also interfere with blood flow and limit blood supply to other cells. As a result, these other functional cells start to die and more scar tissue is formed. When this occurs in the liver, blood pressure in the vein that carries blood from the intestine to the liver (portal vein) increases, giving rise to the condition known as "portal hypertension".

The term "sclerosis" refers to the hardening or stiffening of tissue or a structure or organ that would normally be flexible, usually by replacement of normal organ specific tissue with connective tissue.

There are many types of fibroses or fibrotic diseases including but not limited to pulmonary fibrosis (idiopathic pulmonary fibrosis, cystic fibrosis), fibrosis of the liver (or "cirrhosis"), endomyocardial fibrosis, old myocardial infarction, atrial fibrosis, mediastinal fibrosis, myelofibrosis (affecting the bone marrow), retroperitoneal fibrosis, progressive massive fibrosis (affects the lungs), nephrogenic fibrosis (affecting the skin), Crohn's disease, arthrofibrosis, Peyronie's disease (affecting the penis), Dupuytren's contracture (affecting the hands and fingers), some forms of adhesive capsulitis (affecting the shoulders).

There are many types of scleroses or "sclerotic diseases" including but not limited to Amyotropic Lateral Sclerosis (ALS); atherosclerosis; focal segmental glomerulosclerosis and nephrotic syndrome; hippocampal sclerosis (affecting the brain); lichen sclerosus (a disease that hardens connective tissue of the vagina and penis); liver sclerosis (chirrhosis); multiple sclerosis or focal sclerosis (diseases that affects coordination); osteosclerosis (a disease in which bone densitiy is significantly reduced); otosclerosis (disease affecting the ears); tuberous sclerosis (rare genetic disease affecting multiple systems); primary sclerosing cholanginitis (hardening of the bile duct); primary lateral sclerosis (progressive muscle weakness in the voluntary muscles); and keloids.

The term "scleroderma" or "systemic sclerosis" or "progressive systemic scleroderma" refers to a condition which involves scarring of the joints, skin and internal organs as well as blood vessel abnormalities. Systemic sclerosis can sometimes occur in limited forms, for examples sometimes affecting just the skin or mainly only certain parts of the skin or as CREST syndrome (wherein peripheral areas of the skin but not the trunk are involved). The usual initial symptom of systemic sclerosis is swelling, then thickening and tightening of the skin at the end of the fingers. "Raynaud's phenomenon", in which fingers suddenly and temporarily become very pale and tingle or become numb, painful or both, is common.

The term "polymyositis" refers to muscle inflammation. The term "dermatomyositis", refers to muscle inflammation that is accompanied by skin inflammation. The term "polychondritis" refers to cartilage inflammation.

The term "oesinophilic fasciitis" refers to a rare disorder in which oesinophilic immune cells are released and results in inflammation and hardening of the "fasciae" which is the layer of tough fibrous tissue beneath the skin, on top and between the muscles. The fasciae becomes painfully inflamed and swollen and gradually hardens in the arms and legs. As the skin of the arms and legs progressively hardens, they become difficult to move. Eventually the become stuck in unusual positions. Sometimes, if the arms are involved the person may develop carpal tunnel syndrome.

In another embodiment, specific diseases of disorders which may be treated and/or prevented by administering an sGC stimulator of **Table IA or Table IB** that are stimulators of sGC, and their pharmaceutically acceptable salts thereof, include but are not limited to the following type of diseases involving inflammation, autoimmunity or fibrosis (i.e., fibrotic diseases):
- urogenital system disorders: diabetic nephropathy; renal fibrosis and renal failure resulting from chronic kidney diseases or insufficiency; renal fibrosis and renal failure due to accumulation/deposition and tissue injury; renal sclerosis; progressive sclerosis; glomerulonephritis; focal segmental glomerulosclerosis; nephrotic syndrome; prostate hypertrophy; kidney fibrosis; interstitial renal fibrosis;
- pulmonary system disorders: pulmonary fibrosis; idiopathic pulmonary fibrosis; cystic fibrosis; progressive massive fibrosis; progressive massive fibrosis thataffects the lungs);
- disorders affecting the heart: endomyocardial fibrosis; old myocardial infarction; atrial fibrosis; cardiac interstitial fibrosis; cardiac remodeling and fibrosis; cardiac hypertrophy;
- disorders of the liver and related organs: liver sclerosis or cirrhosis; liver cirrhosis associated with chronic liver disease; hepatic fibrosis; hepatic stellate cell activation; hepatic fibrous collagen and total collagen accumulation; liver disease of necro-inflammatory and/or of immunological origin; primary biliary cirrhosis; primary sclerosing cholanginitis; other cholestatic liver diseases: those associated with granulomatous liver diseases, liver malignancies, intrahepatic cholestasis of pregnancy, hepatitis, sepsis, drugs or toxins, graft-versus-host disease, post-liver transplantation, choledocholithiasis, bile duct tumors, pancreatic carcinoma, Mirizzi's syndrome, AIDS cholangiopathy or parasites; schistosomiasis;
- digestive diseases or disorders: Crohn's disease; Ulcerative Colitis; sclerosis of the gastro-intestinal tract;
- diseases of the skin or the eyes: nephrogenic fibrosis; keloids; fibrotic topical or skin disorders or conditions; dermal fibrosis; scleroderma, skin fibrosis; morphea; hypertrophic scars; naevi; proliferative vitroretinopathy; sarcoids; granulomas; eye fibrosis;
- diseases affecting the nervous system: Amyotropic Lateral Sclerosis (ALS); hippocampal sclerosis, multiple sclerosis (MS); focal sclerosis; primary lateral sclerosis;
- diseases of the bones; osteosclerosis;
- otosclerosis; other hearing diseases or disorders; hearing impairment, partial or total hearing loss; partial or total deafness; tinnitus; noise-induced hearing loss;
- other diseases involving autoimmunity, inflammation or fibrosis: scleroderma; localized scleroderma or circumscribed scleroderma; mediastinal fibrosis; fibrosis mediastinitis; myelofibrosis; retroperitoneal fibrosis; arthrofibrosis; Peyronie's disease; Dupuytren's contracture; lichen sclerosus; some forms of adhesive capsulitis; atherosclerosis; tuberous sclerosis; systemic sclerosis; polymyositis; dermatomyositis; polychondritis; oesinophilic fasciitis; Systemic Lupus Erythematosus or lupus; bone marrow fibrosis, myelofibrosis or osteomyelofibrosis; sarcoidosis; uterine fibroids; endometriosis.

In another embodiment, specific diseases of disorders which may be treated and/or prevented by administering an sGC stimulator of **Table IA or Table IB** that are stimulators of sGC, and their pharmaceutically acceptable salts thereof, include but are not limited to: certain types of cancers; Sickle Cell Disease; Sickle Cell Anemia; cancer metastasis; osteoporosis; gastroparesis; functional dyspepsia; diabetic complications; alopecia or hair loss; diseases associated with endothelial dysfunction; neurologic disorders associated with decreased nitric oxide production; arginosuccinic aciduria; neuromuscular diseases: Duchenne muscular dystrophy (DMD), Becker muscular dystrophy (BMD), limb girdle muscular dystrophies, distal myopathies, type I and type II myotonic dystrophies, facio-scapulo-peroneal muscular dystrophy, autosomal and X-linked Emery-Dreifuss muscular dystrophy, oculopharyngeal muscular dystrophy, amyotrophic lateral sclerosis and spinal muscle atrophy (SMA).

In another embodiment, compounds of the invention can be delivered in the form of implanted devices, such as stents. A stent is a mesh 'tube' inserted into a natural passage/conduit in the body to prevent or counteract a disease-induced, localized flow constriction. The term may also refer to a tube used to temporarily hold such a natural conduit open to allow access for surgery.

A drug-eluting stent (DES) is a peripheral or coronary stent (a scaffold) placed into narrowed, diseased peripheral or coronary arteries that slowly releases a drug to block cell proliferation, usually smooth muscle cell proliferation. This prevents fibrosis that, together with clots (thrombus), could otherwise block the stented artery, a process called restenosis. The stent is usually placed within the peripheral or coronary artery by an Interventional Cardiologist or Interventional Radiologist during an angioplasty procedure. Drugs commonly used in DES in order to block cell proliferation include paclitaxel or rapamycin analogues.

In some embodiments of the invention, a sGC stimulator of the invention can be delivered by means of a drug-eluting stent coated with said sGC stimulator. A drug-eluting stent coated with a sGC stimulator of the invention may be useful in the prevention of stent restenosis and thrombosis during percutaneous coronary interventions. A drug-eluting stent coated with a sGC stimulator of the invention may be able to prevent smooth cell proliferation as well as to assist re-vascularization and re-generation of the endothelial tissue of the artery in which the stent is inserted.

An alternative to percutaneous coronary intervention for the treatment of intractable angina due to coronary artery occlusive disease is the procedure named Coronary Artery Bypass Grafting (CABG). CABG provides only palliation of an ongoing process that is further complicated by the rapid development of graft atherosclerosis. The saphenous vein graft is the most commonly used conduit in CABG surgery. The long-term clinical success of venous CABG is hampered for three main reasons: accelerated graft atherosclerosis, incomplete endothelialization and thrombosis.

In some embodiments, a sGC stimulator of the invention can be used for the prevention of saphenous graft failure during CABG. Compounds of the invention may assist the process of endothelialization and help prevent thrombosis. In this indication, the sGC stimulator is delivered locally in the form of a gel.

The terms, "disease", "disorder" and "condition" may be used interchangeably here to refer to an sGC, cGMP and/or NO mediated medical or pathological condition.

As used herein, the terms "subject" and "patient" are used interchangeably. The terms "subject" and "patient" refer to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In some embodiments, the subject is a non-human animal such as a farm animal (e.g., a horse, cow, pig or sheep), or a pet (e.g., a dog, cat, guinea pig or rabbit). In some embodiments, the subject is a human.

The invention also provides a method for treating one of the above diseases, conditions and disorders in a subject, comprising administering a therapeutically effective amount of a compound of **Table IA or Table IB,** or a pharmaceutically acceptable salt thereof, to the subject in need of the treatment. Alternatively, the invention provides the use of a compound of **Table IA or Table IB,** or a pharmaceutically acceptable salt thereof, in the treatment of one of these diseases, conditions and disorders in a subject in need of the treatment. The invention further provides a method of making or manufacturing a medicament useful for treating one of these diseases, conditions and disorders comprising using a compound of **Table IA or Table IB,** or a pharmaceutically acceptable salt thereof.

The term "biological sample", as used herein, refers to an *in vitro* or *ex vivo* sample, and includes, without limitation, cell cultures or extracts thereof; biopsied material obtained from a mammal or extracts thereof; blood, saliva, urine, faeces, semen, tears, lymphatic fluid, ocular fluid, vitreous humour, or other body fluids or extracts thereof.

"Treat", "treating" or "treatment" with regard to a disorder or disease refers to alleviating or abrogating the cause and/or the effects of the disorder or disease. As used herein, the terms "treat", "treatment" and "treating" refer to the reduction or amelioration of the progression, severity and/or duration of an sGC, cGMP and/or NO mediated condition, or the amelioration of one or more symptoms (preferably, one or more discernable symptoms) of said condition (i.e., "managing" without "curing" the condition), resulting from the administration of one or more therapies (e.g., one or more therapeutic agents such as a compound or composition of the invention). In specific embodiments, the terms "treat"; "treatment" and "treating" refer to the amelioration of at least one measurable physical parameter of an sGC, cGMP and/or NO mediated condition. In other embodiments the terms "treat", "treatment" and "treating" refer to the inhibition of the progression of an sGC, cGMP and/or NO mediated condition, either physically by, e.g., stabilization of a discernable symptom or physiologically by, e.g., stabilization of a physical parameter, or both.

The term "preventing" as used herein refers to administering a medicament beforehand to avert or forestall the appearance of one or more symptoms of a disease or disorder. The person of ordinary skill in the medical art recognizes that the term "prevent" is not an absolute term. In the medical art it is understood to refer to the prophylactic administration of a drug to substantially diminish the likelihood or seriousness of a condition, or symptom of the condition and this is the sense intended in this disclosure. The Physician's Desk Reference, a standard text in the field, uses the term "prevent" hundreds of times. As used therein, the terms "prevent", "preventing" and "prevention" with regard to a disorder or disease, refer to averting the cause, effects, symptoms or progression of a disease or disorder prior to the disease or disorder fully manifesting itself.

In one embodiment, the methods of the invention are a preventative or "pre-emptive" measure to a patient, specifically a human, having a predisposition (e.g., a genetic predisposition) to developing an sGC, cGMP and/or NO related disease, disorder or symptom.

In other embodiments, the methods of the invention are a preventative or "pre-emptive" measure to a patient, specifically a human, suffering from a disease, disorder or condition that makes him at risk of developing an sGC, cGMP or NO related disease, disorder or symptom.

The compounds and pharmaceutical compositions described herein can be used alone or in combination therapy for the treatment or prevention of a disease or disorder mediated, regulated or influenced by sGC, cGMP and/or NO.

Compounds and compositions here disclosed are also useful for veterinary treatment of companion animals, exotic animals and farm animals, including, without limitation, dogs, cats, mice, rats, hamsters, gerbils, guinea pigs, rabbits, horses, pigs and cattle.

In other embodiments, the invention provides a method of stimulating sGC activity in a biological sample, comprising contacting said biological sample with a compound or composition of the invention. Use of a sGC stimulator in a biological sample is useful for a variety of purposes known to one of skill in the art. Examples of such purposes include, without limitation, biological assays and biological specimen storage.

### Combination Therapies

The compounds and pharmaceutical compositions described herein can be used in combination therapy with one or more additional therapeutic agents. For combination treatment with more than one active agent, where the active agents are in separate dosage formulations, the active agents may be administered separately or in conjunction. In addition, the administration of one element may be prior to, concurrent to, or subsequent to the administration of the other agent.

When co-administered with other agents, e.g., when co-administered with another pain medication, an "effective amount" of the second agent will depend on the type of drug used. Suitable dosages are known for approved agents and can be adjusted by the skilled artisan according to the condition of the subject, the type of condition(s) being treated and the amount of a compound described herein being used. In cases where no amount is expressly noted, an effective amount should be assumed. For example, compounds described herein can be administered to a subject in a dosage range from between about 0.01 to about 10,000 mg/kg body weight/day, about 0.01 to about 5000 mg/kg body weight/day, about 0.01 to about 3000 mg/kg body weight/day, about 0.01 to about 1000 mg/kg body weight/day, about 0.01 to about 500 mg/kg body weight/day, about 0.01 to about 300 mg/kg body weight/day, about 0.01 to about 100 mg/kg body weight/day.

When "combination therapy" is employed, an effective amount can be achieved using a first amount of a compound of Table IA or Table IB or a pharmaceutically acceptable salt thereof and a second amount of an additional suitable therapeutic agent.

In one embodiment of this invention, a compound of Table IA or Table IB and the additional therapeutic agent are each administered in an effective amount (i.e., each in an amount which would be therapeutically effective if administered alone). In another embodiment, the compound of Table IA or Table IB and the additional therapeutic agent are each administered in an amount which alone does not provide a therapeutic effect (a sub-therapeutic dose). In yet another embodiment, the compound of Table IA or Table IB can be administered in an effective amount, while the additional therapeutic agent is administered in a sub-therapeutic dose. In still another embodiment, the compound of Table IA or Table IB can be administered in a sub-therapeutic dose, while the additional therapeutic agent, for example, a suitable cancer-therapeutic agent is administered in an effective amount.

As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject.

Co-administration encompasses administration of the first and second amounts of the compounds in an essentially simultaneous manner, such as in a single pharmaceutical composition, for example, capsule or tablet having a fixed ratio of first and second amounts, or in multiple, separate capsules or tablets for each. In addition, such co administration also encompasses use of each compound in a sequential manner in either order. When co-administration involves the separate administration of the first amount of a compound of Table IA or Table IB and a second amount of an additional therapeutic agent, the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, a compound of Table IA or Table IB and the second therapeutic agent can be administered in any order within about 24 hours of each other, within about 16 hours of each other, within about 8 hours of each other, within about 4 hours of each other, within about 1 hour of each other or within about 30 minutes of each other.

More, specifically, a first therapy (e.g., a prophylactic or therapeutic agent such as a compound described herein) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., a prophylactic or therapeutic agent such as an anti-cancer agent) to a subject.

Examples of other therapeutic agents that may be combined with a compound of this disclosure, either administered separately or in the same pharmaceutical composition include, but are not limited to:
(1) Endothelium-derived releasing factor (EDRF);
(2) NO donors such as a nitrosothiol, a nitrite, a sydnonimine, a NONOate, a N-nitrosoamine, a N-hydroxyl nitrosamine, a nitrosimine, nitrotyrosine, a diazetine dioxide, an oxatriazole 5-imine, an oxime, a hydroxylamine, a N-hydroxyguanidine, a hydroxyurea or a furoxan. Some examples of these types of compounds include: glyceryl trinitrate (also known as GTN, nitroglycerin, nitroglycerine, and trinitrogylcerin), the nitrate ester of glycerol; sodium nitroprusside (SNP), wherein a molecule of nitric oxide is coordinated to iron metal forming a square bipyramidal complex; 3-morpholinosydnonimine (SIN-1), a zwitterionic compound formed by combination of a morpholine and a sydnonimine; S-nitroso-N-acetylpenicillamine (SNAP), an N-acetylated amino acid derivative with a nitrosothiol functional group; diethylenetriamine/NO (DETA/NO), a compound of nitric oxide covalently linked to diethylenetriamine; and NCX4016, an m-nitroxymethyl phenyl ester of acetyl salicylic acid. More specific examples of some of these classes of NO donors include: the classic nitrovasodilators, such as organic nitrate and nitrite esters, including nitroglycerin, amyl nitrite, isosorbide dinitrate, isosorbide 5-mononitrate, and nicorandil; Isosorbide (Dilatrate®-SR, Imdur®, Ismo®, Isordil®, Isordil®, Titradose®, Monoket®), FK 409 (NOR-3); FR 144420 (NOR-4); 3-morpholinosydnonimine; Linsidomine chlorohydrate ("SIN-1"); S-nitroso-N-acetylpenicillamine ("SNAP"); AZD3582 (CINOD lead compound), NCX 4016, NCX 701, NCX 1022, HCT 1026, NCX 1015, NCX 950, NCX 1000, NCX 1020, AZD 4717, NCX 1510/NCX 1512, NCX 2216, and NCX 4040 (all available from NicOx S.A.), S-nitrosoglutathione (GSNO), Sodium Nitroprusside, S-nitrosoglutathione mono-ethyl-ester (GSNO-ester), 6-(2-hydroxy-1-methyl-nitrosohydrazino)-*N*-methyl-1-hexanamine (NOC-9) or diethylamine NONOate. Nitric oxide donors are also as disclosed in U.S. Pat. Nos. 5,155,137, 5,366,997, 5,405,919, 5,650,442, 5,700,830, 5,632,981, 6,290,981, 5,691,423 5,721,365, 5,714,511, 6,511,911, and 5,814,666, Chrysselis et al. (2002) J Med Chem. 45:5406-9 (such as NO donors 14 and 17), and Nitric Oxide Donors for Pharmaceutical and Biological Research, Eds: Peng George Wang, Tingwei Bill Cai, Naoyuki Taniguchi, Wiley, 2005;
(3) Other substances that enhance cGMP concentrations such as protoporphyrin IX, arachidonic acid and phenyl hydrazine derivatives;
(4) Nitric Oxide Synthase substrates: for example, n-hydroxyguanidine based analogs, such as N[G]-hydroxy-L-arginine (NOHA), 1-(3, 4-dimethoxy-2-chlorobenzylideneamino)-3-hydroxyguanidine, and PR5 (1-(3, 4-dimethoxy-2-chlorobenzylideneamino)-3-hydroxyguanidine); L-arginine derivatives (such as homo-Arg, homo-NOHA, N-tert-butyloxy- and N-(3-methyl-2-butenyl)oxy-L-arginine, canavanine, epsilon guanidine-carpoic acid, agmatine, hydroxyl-agmatine, and L-tyrosyl-L-arginine); N-alkyl-N'-hydroxyguanidines (such as N-cyclopropyl-N'-hydroxyguanidine and N-butyl-N'-hydroxyguanidine), N-aryl-N'-hydroxyguanidines (such as N-phenyl-N'-hydroxyguanidine and its para-substituted derivatives which bear -F, -Cl, -methyl, -OH substituents, respectively); guanidine derivatives such as 3-(trifluormethyl) propylguanidine; and others reviewed in Cali et al. (2005, Current Topics in Medicinal Chemistry 5:721-736);
(5) Compounds which enhance eNOS transcription: for example those described in WO 02/064146, WO 02/064545, WO 02/064546 and WO 02/064565, and corresponding patent documents such as US2003/0008915, US2003/0022935, US2003/0022939 and US2003/0055093. Other eNOS transcriptional enhancers including those described in US20050101599 (e.g. 2,2-difluorobenzo[1,3]dioxol-5-carboxylic acid indan-2-ylamide, and 4-fluoro-N-(indan-2-yl)-benzamide), and Sanofi-Aventis compounds AVE3085 and AVE9488 (CA Registry NO. 916514-70-0; Schafer et al., Journal of Thrombosis and Homeostasis 2005; Volume 3, Supplement 1: abstract number P1487);
(6) NO independent heme-independent sGC activators, including, but not limited to: BAY 58-2667 (see patent publication DE19943635) 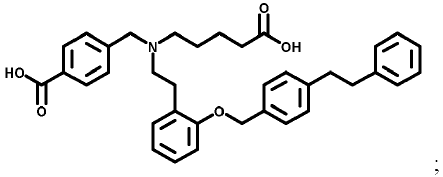 HMR-1766 (ataciguat sodium, see patent publication WO2000002851) 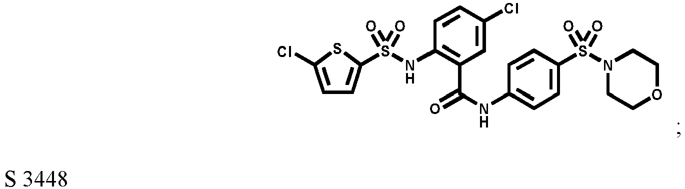 (2-(4-chloro-phenylsulfonylamino)-4,5-dimethoxy-N-(4-(thiomorpholine-4-sulfonyl)-phenyl)-benzam ide (see patent publications DE19830430 and WO2000002851) 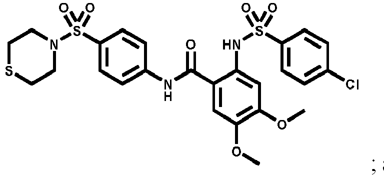 and HMR-1069 (Sanofi-Aventis).
(7) Heme-dependent sGC stimulators including, but not limited to:
YC-1 (see patent publications EP667345 and DE19744026) 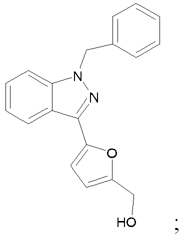
Riociguat (BAY 63-2521, Adempas, commercial product, described in DE19834044) 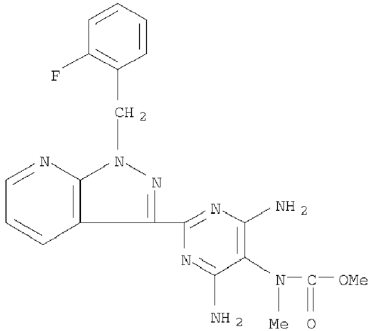
Neliciguat (BAY 60-4552, described in WO 2003095451) 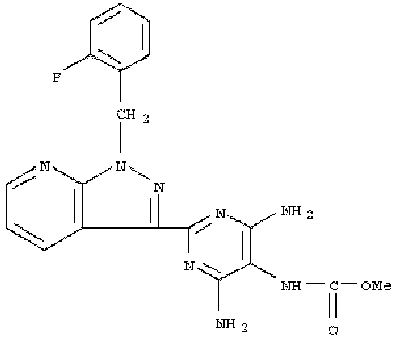 Vericiguat (BAY 1021189, clinical backup to Riociguat),
BAY 41-2272 (described in DE19834047 and DE19942809) 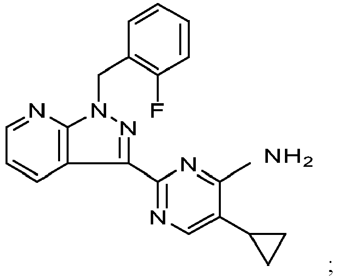
BAY 41-8543 (described in DE19834044) 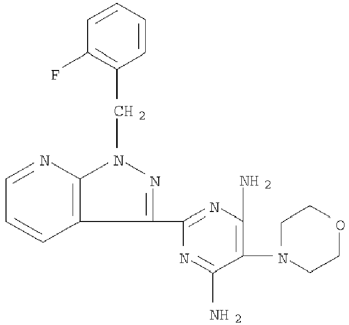
Etriciguat (described in WO 2003086407) 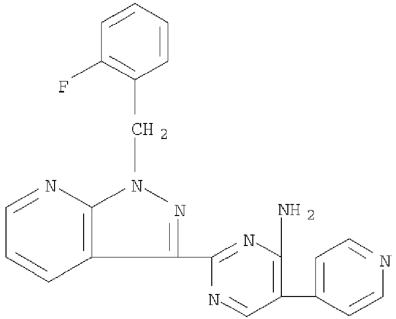
CFM-1571 (see patent publication WO2000027394) 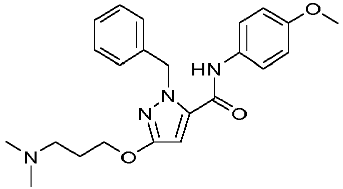
A-344905, its acrylamide analogue A-350619 and the aminopyrimidine analogue A-778935. 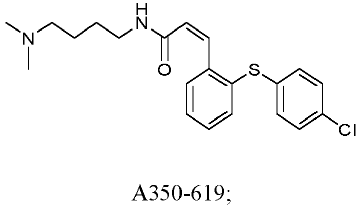 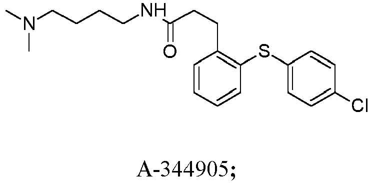 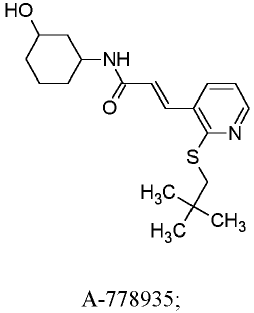
Compounds disclosed in one of publications: US20090209556, US8455638, US20110118282 (WO2009032249), US20100292192, US20110201621, US7947664, US8053455 (WO2009094242), US20100216764, US8507512, (WO2010099054) US20110218202 (WO2010065275), US20130012511 (WO2011119518), US20130072492 (WO2011149921), US20130210798 (WO2012058132) and other compounds disclosed in Tetrahedron Letters (2003), 44(48): 8661-8663.
(8) Compounds that inhibit the degradation of cGMP, such as:
PDE5 inhibitors, such as, for example, Sildenafil (Viagra®) and other related agents such as Avanafil, Lodenafil, Mirodenafil, Sildenafil citrate (Revatio®), Tadalafil (Cialis® or Adcirca®), Vardenafil (Levitra®) and Udenafil; Alprostadil; and Dipyridamole; PF-00489791
PDE9 inhibitors, such as, for example, PF-04447943;

(9) Calcium channel blockers such as:
Dihydropyridine calcium channel blockers: Amlodipine (Norvasc), Aranidipine (Sapresta), Azelnidipine (Calblock), Barnidipine (HypoCa), Benidipine (Coniel), Cilnidipine (Atelec, Cinalong, Siscard), Clevidipine (Cleviprex), Diltiazem, Efonidipine (Landel), Felodipine (Plendil), Lacidipine (Motens, Lacipil), Lercanidipine (Zanidip), Manidipine (Calslot, Madipine), Nicardipine (Cardene, Carden SR), Nifedipine (Procardia, Adalat), Nilvadipine (Nivadil), Nimodipine (Nimotop), Nisoldipine (Baymycard, Sular, Syscor), Nitrendipine (Cardif, Nitrepin, Baylotensin), Pranidipine (Acalas), Isradipine (Lomir);
Phenylalkylamine calcium channel blockers: Verapamil (Calan, Isoptin) 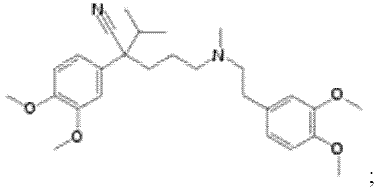 Gallopamil (Procorum, D600);
Benzothiazepines: Diltiazem (Cardizem); 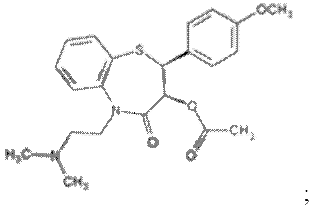
Nonselective calcium channel inhibitors such as: mibefradil, bepridil and fluspirilene, fendiline;

(10) Endothelin receptor antagonists (ERAs): for instance the dual (ET_{A} and ET_{B}) endothelin receptor antagonist Bosentan (marketed as Tracleer®); Sitaxentan, marketed under the name Thelin®; Ambrisentan is marketed as Letairis® in U.S; dual/nonselective endothelin antagonist Actelion-1, that entered clinical trials in 2008;
(11) Prostacyclin derivatives or analogues: for instance prostacyclin (prostaglandin I₂), Epoprostenol (synthetic prostacyclin, marketed as Flolan®); Treprostinil (Remodulin®), Iloprost (Ilomedin®), Iloprost (marketed as Ventavis®); oral and inhaled forms of Remodulin® that are under development; Beraprost, an oral prostanoid available in Japan and South Korea;
(12) Antihyperlipidemics such as: bile acid sequestrants (e.g., Cholestyramine, Colestipol, Colestilan and Colesevelam); statins such as Atorvastatin, Simvastatin, Lovastatin, Fluvastatin, Pitavastatin, Rosuvastatin and Pravastatin; ; cholesterol absorption inhibitors such as Ezetimibe; other lipid lowering agents such as Icosapent ethyl ester, Omega-3-acid ethyl esters, Reducol;; fibric acid derivatives such as Clofibrate, Bezafibrate, Clinofibrate, Gemfibrozil, Ronifibrate, Binifibrate, Fenofirate, Ciprofibrate, Choline fenofibrate; nicotinic acid derivatives such as Acipimox and Niacin; also combinations of statins, niacin, intestinal cholesterol absorption-inhibiting supplements (ezetimibe and others) and fibrates; antiplatelet therapies such as Clopidogrel bisulfate;
(13) Anticoagulants, such as the following types:
- Coumarines (Vitamin K antagonists): Warfarin® (Coumadin) mostly used in the US and UK; Acenocoumarol® and Phenprocoumon®, mainly used in other countries; Phenindione®;
- Heparin and derivative substances such as: Heparin; low molecular weight heparin, Fondaparinux and Idraparinux;
- Direct thrombin inhibitors such as: Argatroban, Lepirudin, Bivalirudin and Dabigatran; Ximelagatran (Exanta®), not approved in the US;
- Tissue plasminogen activators, used to dissolve clots and unblock arteries, such as Alteplase;

(14) Antiplatelet drugs: for instance thienopyridines such as Lopidogrel and Ticlopidine; Dipyridamole; Aspirin;
(15) ACE inhibitors, for example the following types:
- Sulfhydryl-containing agents such as Captopril (trade name Capoten®), the first ACE inhibitor and Zofenopril;
- Dicarboxylate-containing agents such as Enalapril (Vasotec/Renitec®); Ramipril (Altace/Tritace/Ramace/Ramiwin®); Quinapril (Accupril®), Perindopril (Coversyl/Aceon®); Lisinopril (Lisodur/Lopril/Novatec/Prinivil/Zestril®) and Benazepril (Lotensin®);
- Phosphonate-containing agents such as: Fosinopril;
- Naturally occurring ACE inhibitors such as: Casokinins and lactokinins, which are breakdown products of casein and whey that occur naturally after ingestion of milk products, especially cultured milk; The Lactotripeptides Val-Pro-Pro and Ile-Pro-Pro produced by the probiotic *Lactobacillus helveticus* or derived from casein also have ACE-inhibiting and antihypertensive functions;
- Other ACE inhibitors such as Alacepril, Delapril, Cilazapril, Imidapril, Trandolapril, Temocapril, Moexipril, Spirapril,

(16) Supplemental oxygen therapy;
(17) Beta blockers, such as the following types:
- Non-selective agents: Alprenolol®, Bucindolol®, Carteolol®, Carvedilol® (has additional α-blocking activity), Labetalol® (has additional α-blocking activity), Nadolol®, Penbutolol® (has intrinsic sympathomimetic activity), Pindolol® (has intrinsic sympathomimetic activity), Oxprenonol, Acebutolol, Sotalol, Mepindolol, Celiprolol, Arotinolol, Tertatolol, Amosulalol, Nipradilol, Propranolol® and Timolol®;
- β₁-Selective agents: Acebutolol® (has intrinsic sympathomimetic activity), Atenolol®, Betaxolol®, Bisoprolol®, Celiprolol®, Dobutamine hydrochloride, Irsogladine maleate, Carvedilol, Talinolol, Esmolol®, Metoprolol® and Nebivolol®;
- β₂-Selective agents: Butaxamine® (weak α-adrenergic agonist activity);

(18) Antiarrhythmic agents such as the following types:
- Type I (sodium channel blockers): Quinidine, Lidocaine, Phenytoin, Propafenone
- Type III (potassium channel blockers): Amiodarone, Dofetilide, Sotalol
- Type V: Adenosine, Digoxin

(19) Diuretics such as: Thiazide diuretics, e.g., Chlorothiazide, Chlorthalidone, and Hydrochlorothiazide, Bendroflumethiazide, Cyclopenthiazide, Methyclothiazide, Polythiazide , Quinethazone, Xipamide, Metolazone, Indapamide, Cicletanine; Loop diuretics, such as Furosemide and Toresamide; potassium-sparing diuretics such as Amiloride, Spironolactone, Canrenoate potassium, Eplerenone and Triamterene; combinations of these agents; other diuretics such as Acetazolamid and Carperitide
(20a) Direct-acting vasodilators such as Hydralazine hydrochloride, Diazoxide, Sodium nitroprusside, Cadralazine; other vasodilators such as Isosorbide dinitrate and Isosorbide 5-mononitrate;
(20b) Exogenous vasodilators such as:
- Adenocard®, an adenosine agonist, primarily used as an anti-arrhythmic;
- Alpha blockers (which block the vasoconstricting effect of adrenaline): Alpha-1-adrenoceptor antagonists such as Prazosin, Indoramin, Urapidil, Bunazosin, Terazosin, Doxazosin
- Atrial natriuretic peptide (ANP);
- Ethanol;
- Histamine-inducers, which complement proteins C3a, C4a and C5a work by triggering histamine release from mast cells and basophil granulocytes;
- Tetrahydrocannabinol (THC), major active chemical in marijuana which has minor vasodilatory effects;
- Papaverine, an alkaloid found in the opium poppy papaver somniferum;b

(21) Bronchodilators: there are two major types of bronchodilator, β₂ agonists and anticholinergics, exemplified below:
- β₂ agonists: Salbutamol® or albuterol (common brand name: Ventolin) and Terbutaline® are short acting β₂ agonists for rapid relief of COPD symptoms. Long acting β₂ agonists (LABAs) such as Salmeterol® and Formoterol®;
- anticholinergics: Ipratropium® is the most widely prescribed short acting anticholinergic drug. Tiotropium® is the most commonly prescribed long-acting anticholinergic drug in COPD;
- Theophylline®, a bronchodilator and phosphodiesterase inhibitor;

(22) Corticosteroids: such as beclomethasone, methylprednisolone, betamethasone, prednisone, prenisolone, triamcinolone, dexamethasone, fluticasone, flunisolide and hydrocortisone, and corticosteroid analogs such as budesonide
(23) Dietary supplements such as, for example: omega-3 oils; folid acid, niacin, zinc, copper, Korean red ginseng root, ginkgo, pine bark, *Tribulus terrestris,* arginine, *Avena sativa,* horny goat weed, maca root, muira puama, saw palmetto, and Swedish flower pollen; Vitamin C, Vitamin E, Vitamin K2; Testosterone supplements, Testosterone transdermal patch; Zoraxel, Naltrexone, Bremelanotide (formerly PT-141), Melanotan II, hMaxi-K; Prelox: a Proprietary mix/combination of naturally occurring ingredients, L-arginine aspartate and Pycnogenol;
(24) PGD2 receptor antagonists including, but not limited to, compounds described as having PGD2 antagonizing activity in United States Published Applications US20020022218, US20010051624, and US20030055077, PCT Published Applications WO9700853, WO9825919, WO03066046, WO03066047, WO03101961, WO03101981, WO04007451, WO0178697, WO04032848, WO03097042, WO03097598, WO03022814, WO03022813, and WO04058164, European Patent Applications EP945450 and EP944614, and those listed in: Torisu et al. 2004 Bioorg Med Chem Lett 14:4557, Torisu et al. 2004 Bioorg Med Chem Lett 2004 14:4891, and Torisu et al. 2004 Bioorg & Med Chem 2004 12:4685;
(25) Immunosuppressants such as cyclosporine (cyclosporine A, Sandimmune® Neoral®), tacrolimus (FK-506, Prograf®), rapamycin (sirolimus, Rapamune®) and other FK-506 type immunosuppressants, and mycophenolate, e.g., mycophenolate mofetil (CellCept®);
(26) Non-steroidal anti-asthmatics such as β2-agonists (e.g., terbutaline, metaproterenol, fenoterol, isoetharine, albuterol, salmeterol, bitolterol and pirbuterol) and β2-agonist-corticosteroid combinations (e.g., salmeterol-fluticasone (Advair®), formoterol-budesonid (Symbicort®)), theophylline, cromolyn, cromolyn sodium, nedocromil, atropine, ipratropium, ipratropium bromide, leukotriene biosynthesis inhibitors (zileuton, BAY 1005);
(27) Non-steroidal anti-inflammatory agents (NSAIDs) such as propionic acid derivatives (e.g., alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen), acetic acid derivatives (e.g., indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin and zomepirac), fenamic acid derivatives (e.g., flufenamic acid, meclofenamic acid, mefenamic acid, niflumic acid and tolfenamic acid), biphenylcarboxylic acid derivatives (e.g., diflunisal and flufenisal), oxicams (e.g., isoxicam, piroxicam, sudoxicam and tenoxican), salicylates (e.g., acetyl salicylic acid and sulfasalazine) and the pyrazolones (e.g., apazone, bezpiperylon, feprazone, mofebutazone, oxyphenbutazone and phenylbutazone);
(28) Cyclooxygenase-2 (COX-2) inhibitors such as celecoxib (Celebrex®), rofecoxib (Vioxx®), valdecoxib, etoricoxib, parecoxib and lumiracoxib;
(opioid analgesics such as codeine, fentanyl, hydromorphone, levorphanol, meperidine, methadone, morphine, oxycodone, oxymorphone, propoxyphene, buprenorphine, butorphanol, dezocine, nalbuphine and pentazocine; and
(29) Anti-diabetic agents such as insulin and insulin mimetics, sulfonylureas (e.g., Glyburide, Glybenclamide, Glipizide, Gliclazide, Gliquidone, Glimepiride, Meglinatide, Tolbutamide, Chlorpropamide, Acetohexamide, Tolazamide), biguanides, e.g., metformin (Glucophage®), α-glucosidase inhibitors (such as Acarbose, Epalrestat, Voglibose, Miglitol), thiazolidinone compounds, e.g., rosiglitazone (Avandia®), troglitazone (Rezulin®), ciglitazone, pioglitazone (Actos®) and englitazone; insulin sensitizers such as Pioglitazone and Rosiglitazone; Insulin secretagogues such as Repaglinide, Nateglinide and Mitiglinide; Incretin mimetics such as Exanatide and Liraglutide; Amylin analogues such as Pramlintide; glucose lowering agents such as Chromiumm picolinate (optinally combined with biotin); dipeptidyl peptidase IV inhibitors such as Sitagliptin, Vildagliptin, Saxagliptin, Alogliptin and Linagliptin; vaccines currently being developed for the treatment of diabetes; AVE-0277, Alum-GAD, BHT-3021, IBC-VS01; cytokine targeted therapies in development for the treatment of diabetes such as Anakinra, Canakinumab, Diacerein,Gevokizumab, LY-2189102, MABP-1, GIT-027; drugs in development for the treatment of diabetes:

| **Drugs in development for the treatment of diabetes** | | | |
|---|---|---|---|
| Dapagliflozin | AstraZeneca/ Bristol-Myers Squibb | SGLT-2 Inhibitors | Recommended Approval |
| Alogliptin benzoate/metformin hydrochloride | Takeda | Insulin Sensitizers/ Dipeptidyl Peptidase IV (CD26; DPP-IV; DP-IV) Inhibitors | Pre-Reg istered |
| Anagliptin | Kowa/ Sanwa | Dipeptidyl Peptidase IV (CD26; DPP-IV; DP-IV) Inhibitors | Pre-Registered |
| Insulin degludec | Novo Nordisk | | Pre-Registered |
| Insulin degludec/insulin aspart | Novo Nordisk | | Pre-Reg istered |
| Insulin human (rDNA origin) inhalation powder | MannKind | | Pre-Reg istered |
| Lixisenatide | Sanofi | Insulin Secretagogues/ GLP-1 Receptor Agonists | Pre-Reg istered |
| Recombinant human insulin | Biodel | | Pre-Registered |
| Teneligliptin | Mitsubishi Tanabe Pharma | Dipeptidyl Peptidase IV (CD26; DPP-IV; DP-IV) Inhibitors | Pre-Reg istered |
| AVE-0277 | Andromeda Biotech/ Teva | | Phase III |
| Albiglutide | GlaxoSmithKline | GLP-1 Receptor Agonists | Phase III |
| Aleglitazar | Roche | PPARalpha Agonists/ PPARgamma Agonists | Phase III |
| Atorvastatin calcium/glimepiride | GlaxoSmithKline | K(ATP) Channel Blockers/ Dipeptidyl Peptidase IV (CD26; DPP-IV; DP-IV) Inhibitors/ HMG-CoA Reductase Inhibitors/ TNFSF6 Expression Inhibitors | Phase III |
| BYK-324677 | Nycomed | | Phase III |
| Balaqlitazone | Dr. Reddy's Laboratories | Insulin Sensitizers/ PPARgamma Partial Agonists | Phase III |
| CSG-452 | Chugai Pharmaceutical | SGLT-2 Inhibitors | Phase III |
| Canagliflozin | Johnson & Johnson/ Mitsubishi Tanabe Pharma | SGLT-2 Inhibitors | Phase III |

| Canagliflozin/metformin hydrochloride | Johnson & Johnson | SGLT-2 Inhibitors/ Insulin Sensitizers | Phase III |
|---|---|---|---|
| Dapagliflozin/Metformin hydrochloride | AstraZeneca/ Bristol-Myers Squibb | SGLT-2 Inhibitors/ Insulin Sensitizers | Phase III |
| Dulaglutide | Lilly | Insulin Secretagogues/ GLP-1 Receptor Agonists | Phase III |
| Empagliflozin | Boehringer Ingelheim/ Lilly | SGLT-2 Inhibitors | Phase III |
| Empagliflozin/linagliptin | Boehringer Ingelheim/ Lilly | SGLT-2 Inhibitors/ Dipeptidyl Peptidase IV (CD26; DPP-IV; DP-IV) Inhibitors | Phase III |
| Gemigliptin | LG Life Sciences | Dipeptidyl Peptidase IV (CD26; DPP-IV; DP-IV) Inhibitors | Phase III |
| Hepatic-directed vesicle insulin | Diasome Pharmaceuticals | | Phase III |
| Human isophane insulin | Wockhardt | | Phase III |
| IN-105 | Biocon | | Phase III |
| Insulin degludec/liraglutide | Novo Nordisk | Insulin Secretagogues/ GLP-1 Receptor Agonists | Phase III |
| Insulin glargine | Sanofi | | Phase III |
| Ipragliflozin L-proline | Astellas Pharma/ Kotobuki | SGLT-2 Inhibitors | Phase III |
| LY-2605541 | Lilly | | Phase III |
| LY-2963016 | Lilly | | Phase III |
| Lixisenatide/Insulin glargine | Sanofi | Insulin Secretagogues/ GLP-1 Receptor Agonists | Phase III |
| Lobeglitazone sulfate | Chong Kun Dang Pharm (CKD Pharm) | PPARalpha Agonists/ PPARgamma Agonists/ Insulin Sensitizers | Phase III |
| Luseogliflozin | Taisho | SGLT-2 Inhibitors | Phase III |
| Otelixizumab | Tolerx | Anti-CD3 | Phase III |
| Ranolazine | Gilead | Sodium Channel Blockers | Phase III |
| Recombinant human insulin | National Institute of Health Sciences | | Phase III |
| Sitagliptin phosphate monohydrate/pioglitazone hydrochloride | Merck & Co. | PPARgamma Agonists/ Insulin Sensitizers/ Dipeptidyl Peptidase IV (CD26; DPP-IV; DP-IV) Inhibitors | Phase III |
| Sitagliptin/atorvastatin calcium | Merck & Co. | Dipeptidyl Peptidase IV (CD26; DPP-IV; DP-IV) Inhibitors/ HMG-CoA Reductase Inhibitors/ TNFSF6 Expression Inhibitors | Phase III |
| TAK-875 | Takeda | Free Fatty Acid Receptor 1 (FFAR1; GPR40) Agonists/ Insulin Secretagogues | Phase III |
| Π-401 | 7TM Pharma | Cannabinoid CB1 Antagonists | Phase I |

| Π-401 | Transition Therapeutics | | Phase I |
|---|---|---|---|
| ZYH-2 | Cadila Healthcare (d/b/a Zydus Cadila) | PPARalpha Ligands/ PPARgamma Ligands | Phase I |
| ZYO-1 | Cadila Healthcare (d/b/a Zydus Cadila) | Cannabinoid CB1 Antagonists | Phase I |
| 701645 | Cellonis Biotechnologies | | Phase I |
| 701499 | Cellonis Biotechnologies | | Phase I |
| 743300 | University of California, San Francisco | | Phase I |
| 448661 | University of Pittsburgh | | Phase I |
| AD-1 | National Institute Pharma Res Dev | | Clinical |
| Colesevelam hydrochloride | Daiichi Sankyo | Bile Acid Sequestrants | Clinical |
| DBPR-108 | National Health Research Institutes/ ScinoPharm | | IND Filed |
| Nodlin | Biolaxy | | IND Filed |
| PSN-491 | Prosidion | Glucose-Dependent Insulinotropic Receptor (GDIR, GPR119) Agonists/ Dipeptidyl Peptidase IV (CD26; DPP-IV; DP-IV) Inhibitors | IND Filed |
| Tolimidone | Melior Discovery | Lyn Kinase Activators | IND Filed |
| ZYD-1 | Cadila Healthcare (d/b/a Zydus Cadila) | GLP-1 Receptor Agonists | IND Filed |
| ZYOG-1 | Cadila Healthcare (d/b/a Zydus Cadila) | GLP-1 Receptor Agonists | IND Filed |

(30) HDL cholesterol-increasing agents such as Anacetrapib, MK-524A, CER-001, DRL-17822, Dalcetrapib, JTT-302, RVX-000222, TA-8995;
(31) Antiobesity drugs such as Methamphetamine hydrochloride, Amfepramone hydrochloride (Tenuate ®), Phentermine (Ionamin ®), Benzfetamine hydrochloride (Didrex ®), Phendimetrazine tartrate (Bontril®, Prelu-2 ®, Plegine ®), Mazindol (Sanorex ®), Orlistat (Xenical ®), Sibutramine hydrochloride monohydrate (Meridia ®, Reductil ®), Rimonabant (Acomplia ®), Amfepramone, Chromium picolinate, RM-493, TZP-301; combination such as Phentermine/Topiramate, Bupropion/Naltrexone, Sibutramine/Metformin, Bupropion SR/Zonisamide SR, Salmeterol, xinafoate/fluticasone propionate; Lorcaserin hydrochloride, Phentermine/topiramate, Bupropion/naltrexone, Cetilistat, Exenatide, KI-0803, Liraglutide, Metformin hydrochloride, Sibutramine/Metformin, 876167, ALS-L-1023, Bupropion SR/Zonisamide SR, CORT-108297, Canagliflozin, Chromium picolinate, GSK-1521498, LY-377604, Metreleptin, Obinepitide, P-57AS3, PSN-821, Salmeterol xinafoate/fluticasone propionate, Sodium tungstate, Somatropin (recombinant), TM-30339, TTP-435, Tesamorelin, Tesofensine, Velneperit, Zonisamide, BMS-830216, ALB-127158, AP-1030, ATHX-105, AZD-2820, AZD-8329, Beloranib hemioxalate, CP-404, HPP-404, ISIS-FGFR4Rx, Insulinotropin, KD-3010PF, 05212389, PP-1420, PSN-842, Peptide YY3-36, Resveratrol, S-234462; S-234462, Sobetirome, TM-38837, Tetrahydrocannabivarin, ZYO-1, beta-Lapachone;
(32) Angiotensin receptor blockers such as Losartan, Valsartan, Candesartan cilexetil, Eprosaran, Irbesartan, Telmisartan, Olmesartran medoxomil, Azilsartan medoxomil;
(33) Renin inhibitors such as Aliskiren hemifumirate;
(34) Centrally acting alpha-2-adrenoceptor agonists such as Methyldopa, Clonidine, Guanfacine;
(35) Adrenergic neuron blockers such as Guanethidine, Guanadrel;
(36) Imidazoline 1-1 receptor agonists such as Rimenidine dihydrogen phosphate and Moxonidine hydrochloride hydrate;
(37) Aldosterone antagonists such as Spironolactone and Eplerenone
(38) Potassium channel activators such as Pinacidil
(39) Dopamine D1 agonists such as Fenoldopam mesilate; Other dopamine agonists such as Ibopamine, Dopexamine and Docarpamine;
(40) 5-HT2 antagonists such as Ketanserin;
(41) Drugs that are currently being developed for the treatment of arterial hypertension:

| **Drugs in development for the treatment of hypertension** | | | |
|---|---|---|---|
| Azilsartan | Takeda | Angiotensin AT1 Antagonists/ Angiotensin AT2 Antagonists/ Insulin Sensitizers | Registered |
| Amlodipine besylate/irbesartan | Dainippon Sumitomo Pharma | Angiotensin AT1 Antagonists/ Calcium Channel Blockers | Pre-Registered |
| Azilsartan/amlodipine besilate | Takeda | Angiotensin AT1 Antagonists/ Insulin Sensitizers/ Calcium Channel Blockers | Phase III |
| Cilnidipine/valsartan | Ajinomoto/ Mochida | Angiotensin AT1 Antagonists/ Calcium Channel Blockers | Phase III |
| Fimasartan | Boryung | Angiotensin AT1 Antagonists | Phase III |
| Irbesartan/atorvastatin | Hanmi | Angiotensin AT1 Antagonists/ Dipeptidyl Peptidase IV (CD26; DPP-IV; DP-IV) Inhibitors/ HMG-CoA Reductase Inhibitors/ TNFSF6 Expression Inhibitors | Phase III |
| Irbesartan/trichlormethiazide | Shionogi | Angiotensin AT1 Antagonists | Phase III |
| Losartan potassium/hydrochlorothiazide/amlodipine besylate | Merck & Co. | Angiotensin AT1 Antagonists/ Calcium Channel Blockers | Phase III |
| Pratosartan | Boryung | Angiotensin AT1 Antagonists | Phase III |
| ACT-280778 | Actelion | | Phase II |
| Amiloride hydrochloride/spironolactone | Hemodynamic Therapeutics | Mineralocorticoid Receptor (MR) Antagonists/ Na+/H+ Exchanger (NHE) Inhibitors/ Epithelial Sodium Channels (ENaC) Blockers/ K(V)1.5 Channel Blockers/ K(V)4.3 Channel Blockers | Phase II |
| Angiotensin vaccine/CoVaccine HT | BTG | | Phase II |
| CYT006-AngQb | Cytos Biotechnology | Anti-Angiotensin II | Phase II |
| Cholecalciferol | Emory University | | Phase II |
| Cobiprostone | Sucampo Pharmaceuticals | CIC-2 Channel Activators | Phase II |
| INT-001 | IntelGenx | | Phase II |
| LCZ-696 | Novartis | Angiotensin AT1 Antagonists/ Neprilysin (Enkephalinase, Neutral Endopeptidase, NEP) Inhibitors | Phase II |
| LFF-269 | Novartis | | Phase II |
| Octreotide acetate | Chiasma | Growth Hormone Release Inhibitors/ Somatostatin Agonists | Phase II |
| PL-3994 | Palatin Technologies | Atrial Natriuretic Peptide A (NPR1; Guanylate Cyclase A) Receptor Agonists | Phase II |
| Rostafuroxine | Sigma-Tau | | Phase II |
| SLx-2101 | NT Life Sciences | Phosphodiesterase V (PDE5A) Inhibitors | Phase II |
| TBC-3711 | Encysive Pharmaceuticals | Endothelin ETA Receptor Antagonists | Phase II |
| Udenafil | Dong-A/ Falk Pharma | Phosphodiesterase V (PDE5A) Inhibitors | Phase II |
| Atorvastatin calcium/losartan potassium | HanAll BioPharma | Angiotensin AT1 Antagonists/ Dipeptidyl Peptidase IV (CD26; DPP-IV; DP-IV) Inhibitors/ HMG-CoA Reductase Inhibitors/ TNFSF6 Expression Inhibitors | Phase I |
| BIA-5-1058 | BIAL | Dopamine beta-monooxygenase Inhibitors | Phase I |
| CS-3150 | Daiichi Sankyo | | Phase I |
| DSP-9599 | Dainippon Sumitomo Pharma | Renin Inhibitors | Phase I |
| MK-1597 | Actelion/ Merck & Co. | Renin Inhibitors | Phase I |
| MK-4618 | Merck & Co. | | Phase I |
| MK-5478 | Merck & Co. | | Phase I |
| MK-7145 | Merck & Co. | | Phase I |
| MK-8266 | Merck & Co. | | Phase I |
| MK-8457 | Merck & Co. | | Phase I |
| MP-157 | Mitsubishi Tanabe Pharma | Angiotensin AT2 Agonists | Phase I |
| MT-3995 | Mitsubishi Tanabe Pharma | Mineralocorticoid Receptor (MR) Antagonists | Phase I |
| Mirodenafil hydrochloride | SK Chemicals | Phosphodiesterase V (PDE5A) Inhibitors | Phase I |
| NV-04 | Novogen | Antioxidants | Phase I |
| Nifedipine/Candesartan cilexetil | Bayer | Angiotensin AT1 Antagonists/ Calcium Channel Blockers/ Antioxidants | Phase I |
| QGC-001 | Quantum Genomics | Glutamyl Aminopeptidase (Aminopeptidase A) Inhibitors | Phase I |
| RDX-5791 | Ardelyx | Na+/H+ Exchanger type 3 (NHE-3) Inhibitors | Phase I |
| TAK-272 | Takeda | Renin Inhibitors | Phase I |
| TAK-591 | Takeda | Angiotensin AT2 Antagonists | Phase I |
| VTP-27999 | Vitae Pharmaceuticals | Renin Inhibitors | Phase I |
| Vasomera | PhaseBio | VPAC2 (VIP2) Agonists | Phase I |

(42) Vasopressin antagonists such as Tolvaptan;
(43) Calcium channel sensitizers such as Levosimendan or activators such as Nicorandil;
(44) PDE-3 inhibitors such as Amrinone, Milrinone, Enoximone, Vesnarinone, Pimobendan, Olprinone;
(45) Adenylate cyclase activators such as Colforsin dapropate hydrochloride;
(46) Positive inotropic agents such as Digoxin and Metildigoxin; metabolic cardiotonic agents such as Ubidecarenone; brain naturetic peptides such as Nesiritide;
(47) Drugs that are currently in development for the treatment of heart failure:

| **Drugs in development for the treatment of heart failure** | | | |
|---|---|---|---|
| Bucindolol hydrochloride | ARCA | beta-Adrenoceptor Antagonists | Pre-Registered |
| Aliskiren hemifumarate | Novartis | Renin Inhibitors | Phase III |
| Ferric carboxymaltose | Vifor | | Phase III |
| LCZ-696 | Novartis | Angiotensin AT1 Antagonists/ Neprilysin (Enkephalinase, Neutral Endopeptidase, NEP) Inhibitors | Phase III |
| Neuregulin-1 | Zensun | | Phase III |
| Olmesartan medoxomil | Tohoku University | Angiotensin AT1 Antagonists | Phase III |
| C3BS-CQR-1 | Cardio3 BioSciences | | Phase II/III |
| MyoCell | Bioheart | | Phase II/III |
| Serelaxin | Novartis | | Phase II/III |
| AAV1/SERCA2a | AmpliPhi Biosciences/ Celladon/ Mount Sinai School of Medicine | | Phase II |
| Albiglutide | GlaxoSmithKline | GLP-1 Receptor Agonists | Phase II |
| Allogeneic mesenchymal precursor cells | Mesoblast | | Phase II |
| AlsterMACS | Miltenyi Biotec | | Phase II |
| BAY-94-8862 | Bayer | Mineralocorticoid Receptor (MR) Antagonists | Phase II |
| COR-1 | Corimmun | | Phase II |
| CXL-1020 | Cardioxyl Pharmaceuticals | Nitric Oxide Donors | Phase II |
| Cenderitide | Nile Therapeutics | Guanylate Cyclase Activators | Phase II |
| Endometrial regenerative cells | ERCell/ Medistem | | Phase II |
| JNJ-39588146 | Johnson & Johnson | | Phase II |
| Omecamtiv mecarbil | Amgen/ Cytokinetics | Cardiac Myosin Activators | Phase II |
| PL-3994 | Palatin Technologies | Atrial Natriuretic Peptide A (NPR1; Guanylate Cyclase A) Receptor Agonists | Phase II |
| Remestemcel-L | Osiris | | Phase II |
| TRV-120027 | Trevena | Angiotensin AT1 Receptor Ligands | Phase II |
| Urocortin 2 | Neurocrine Biosciences | CRF2 Agonists | Phase II |
| AAV6-CMV-SERCA2a | Imperial College | | Phase I/II |
| Anakinra | National Institutes of Health (NIH) | IL-1 Receptor Antagonists | Phase I/II |
| LipiCell | Bioheart/ Instituto de Medicina Regenerativa | | Phase I/II |
| ALD-201 | Cytomedix/ Texas Heart Institute | | Phase I |
| BAY-1021189/Vericiguat | Bayer | | Phase II |
| BAY-1067197 | Bayer | Adenine Receptor Agonists | Phase I |
| BAY-86-8050 | Bayer | Drugs Acting on Vasopressin (AVP) Receptors | Phase I |
| BIA-5-1058 | BIAL | Dopamine beta-monooxygenase Inhibitors | Phase I |
| CSCs | University of Louisville | | Phase I |
| Calcitonin gene related peptide | VasoGenix | | Phase I |
| JVS-100 | Juventas Therapeutics | | Phase I |
| MyoCell SDF-1 | Bioheart | | Phase I |
| Myoblast | Advanced Cell Technology (ACT) | | Phase I |
| RO-1160367 | Serodus | 5-HT4 Antagonists | Phase I |
| Recombinant human glial growth factor 2 | Acorda/ Vanderbilt University | | Phase I |
| [18F]LMI-1195 | Lantheus Medical Imaging | | Phase I |
| 677950 | Kyoto Prefectural University of Medicine | | Phase I |

(48) Drugs currently in development for the treatment of pulmonary hypertension:

| **Drugs in development for the treatment of pulmonary hypertension** | | | |
|---|---|---|---|
| Imatinib mesylate | Novartis | Breast Cancer-Resistant Protein (BCRP; ABCG2) Inhibitors/ Abl Kinase Inhibitors/ Angiogenesis Inhibitors/ Bcr-Abl Kinase Inhibitors/ CSF1R (c-FMS) Inhibitors/ KIT (C-KIT) Inhibitors/ Apoptosis Inducers/ PDGFRalpha Inhibitors/ PDGFRbeta Inhibitors/ Inhibitors of Signal Transduction Pathways | Pre-Registered |
| Treprostinil diethanolamine | United Therapeutics | Prostacyclin Analogs | Pre-Registered |
| GSK-1325760A | GlaxoSmithKline | | Phase III |
| Macitentan | Actelion | Endothelin ETA Receptor Antagonists/ Endothelin ETB Receptor Antagonists | Phase III |
| Riociguat/Adempas | Bayer | Guanylate Cyclase Activators | Approved 2013 |
| Selexipag | Actelion/ Nippon Shinyaku | Prostanoid IP Agonists | Phase III |
| Udenafil | Dong-A | Phosphodiesterase V (PDE5A) Inhibitors | Phase III |
| L-Citrulline | Nat Heart, Lung, and Blood Institute/ Vanderbilt University | | Phase II/III |
| BQ-123 | Brigham & Women's Hospital | Endothelin ETA Receptor Antagonists | Phase II |
| Cicletanine | Gilead | | Phase II |
| Fasudil hydrochloride | Asahi Kasei | Rho Kinase Inhibitors/ Calcium Sensitizers | Phase II |
| Nilotinib hydrochloride monohydrate | Novartis | Bcr-Abl Kinase Inhibitors/ Apoptosis Inducers/ Inhibitors of Signal Transduction Pathways | Phase II |
| PRX-08066 | Clinical Data | 5-HT2B Antagonists | Phase II |
| Terguride | ErgoNex Pharma | 5-HT2A Antagonists/ 5-HT2B Antagonists/ Dopamine Autoreceptor Agonists/ Dopamine D2 Receptor Partial Agonists/ Prolactin Secretion Inhibitors | Phase II |
| Tezosentan disodium | Actelion | Endothelin ETA Receptor Antagonists/ Endothelin ETB Receptor Antagonists | Phase II |
| Anakinra | Virginia Commonwealth University (VCU) | IL-1 Receptor Antagonists | Phase I/II |
| Simvastatin | Imperial College | HDL-Cholesterol Increasing Agents/ HMG-CoA Reductase Inhibitors | Phase I/II |
| 99mTC-PulmoBind | Montreal Heart Institute (MHI) | | Phase I |
| APD-811 | Arena | Prostanoid IP Agonists | Phase I |
| Sorafenib | Bayer | Raf kinase B Inhibitors/ Raf kinase C Inhibitors/ Angiogenesis Inhibitors/ Flt3 (FLK2/STK1) Inhibitors/ VEGFR-1 (Flt-1) Inhibitors/ KIT (C-KIT) Inhibitors/ VEGFR-2 (FLK-1/KDR) Inhibitors/ VEGFR-3 (FLT4) Inhibitors/ PDGFRbeta Inhibitors/ RET Inhibitors/ Inhibitors of Signal Transduction Pathways | Phase I |
| Triplelastat | Proteo Biotech | Elastase Inhibitors | Phase I |

(49) Drugs in current development for the treatment of female sexual dysfunction:

| **Drugs in active development for the treatment of female sexual dysfunction** | | | |
|---|---|---|---|
| Alprostadil | Apricus Biosciences/ VIVUS | | Phase III |
| Prasterone | EndoCeutics/ Monash University | HSD11B1 Expression Inhibitors | Phase III |
| Testosterone transdermal gel | BioSante | Androgen Receptor Agonists | Phase III |
| Bremelanotide | Palatin Technologies | Melanocortin MC3 Receptor Agonists/ Melanocortin MC4 Receptor Agonists | Phase II |
| Pill-Plus | Pantarhei Bioscience | | Phase II |
| Testosterone MDTS | Acrux | Androgen Receptor Agonists | Phase II |
| Estradiol/testosterone | BioSante | Estrogen Receptor (ER) Agonists/ Androgen Receptor Agonists | Phase I |
| LGD-2941 | Ligand | Selective Androgen Receptor Modulators (SARM) | Phase I |
| Lidocaine/heparin | Urigen | | Phase I |
| OnabotulinumtoxinA | Allergan | | Phase I |

(50) Drugs used for the treatment of erectile dysfunction such as Alprostadil, Aviptadil, Phentolamine mesilate, Weige, Alprostadil;
(51) Drugs currently in development for the treatment of male sexual dysfunction:

| **Drugs in active development for the treatment of erectile dysfunction** | | | |
|---|---|---|---|
| Fluvastatin sodium | Novartis | Apoptosis Inducers/ HMG-CoA Reductase Inhibitors | Phase III |
| Lodenafil carbonate | Cristalia | Phosphodiesterase V (PDE5A) Inhibitors | Phase III |
| EFLA-400 | Chonbuk National University Hospital | | Phase II/III |
| Apomorphine hydrochloride | Vectura | Dopamine D2 Agonists | Phase II |
| LY-900010 | Lilly | Phosphodiesterase V (PDE5A) Inhibitors/ Selective Androgen Receptor Modulators (SARM) | Phase II |
| Nitroglycerin | Futura Medical | | Phase II |
| RX-10100 | Rexahn | Drugs Acting on Dopaminergic Transmission/ Drugs Acting on Serotonergic Transmission | Phase II |
| YHD-1023 | Yuhan | | Phase II |
| INT-007 | IntelGenx | | Phase I |
| LY-2452473 | Lilly | Selective Androgen Receptor Modulators (SARM) | Phase I |
| hMaxi-K | Albert Einstein College of Medicine/ Ion Channel Innovations/ Mount Sinai School of Medicine | | Phase I |
| KH-204 | KMSI | | Clinical |

(51) Drugs in development for the treatment of sleep apnea:

| **Drugs in development for the treatment of sleep apnea** | | | |
|---|---|---|---|
| CX-1739 | Cortex | AMPA Receptor Modulators | Phase II |
| Phentermine/topiramate | VIVUS | AMPA Antagonists/ Kainate Antagonists/ Sodium Channel Blockers/ Carbonic Anhydrase Type II Inhibitors | Phase II |
| AVE-0118 | Sanofi | Potassium Channel Blockers | Phase I |
| Suvorexant | Merck & Co. | Orexin Receptor Antagonists | Phase I |

(52) Drugs currently in development for the treatment of metabolic syndrome:

| **Antihyperlipidemic drugs under active development for the treatment of patients with metabolic syndrome** | | | |
|---|---|---|---|
| GFT-505 | Genfit | PPARalpha Agonists/ PPARdelta Agonists | Phase II |
| MBX-8025 | Metabolex | PPARdelta Agonists | Phase II |
| Pitavastatin calcium | Kowa | APOA1 Expression Enhancers/ HMG-CoA Reductase Inhibitors/ SPP1 (Osteopontin) Expression Inhibitors | Phase I |

(53) Antiobesity drugs:

| **Drugs marketed for the treatment of obesity** | | | |
|---|---|---|---|
| Methamphetamine hydrochloride (Desoxyn) | Abbott | Noradrenergic, alpha- and beta-adrenoceptor agonist | 1943 (U.S.) |
| Amfepramone hydrochloride (Tenuate) | Sanofi | Noradrenergic release stimulant | 1959 (U.S.) |
| Phentermine (lonamin) | UCB Celltech | Noradrenergic release stimulant | 1959 (U.S.) |
| Benzfetamine hydrochloride (Didrex) | Pfizer | Noradrenergic release stimulant | 1960 (U.S.) |
| Phendimetrazine tartrate (Bontril, Prelu-2, Plegine) | Pfizer | Noradrenergic release stimulant | 1961 (U.S.) |
| Mazindol (Sanorex) | Novartis | Noradrenergic reuptake inhibitor | 1973 (U.S.) |
| Orlistat (Xenical) | Roche | Pancreatic lipase inhibitor | 1998 (New Zealand) |

(54) Drugs used for the treatment of Alzheimer's disease: e.g., cholinesterase inhibitors prescribed for mild to moderate Alzheimer's disease, including Razadyne® (galantamine), Exelon® (rivastigmine), and Aricept® (donepezil), Cognex® (tacrine); Namenda® (memantine), an N-methyl D-aspartate (NMDA) antagonist, and Aricept®, prescribed to treat moderate to severe Alzheimer's disease; vitamin E (an anti-oxidant).
(55) Antidepressants: tricyclic antidepressants such as amitriptyline (Elavil®), desipramine (Norpramin®), imipramine (Tofranil®), amoxapine (Asendin®), nortriptyline; the selective serotonin reuptake inhibitors (SSRI's) such as paroxetine (Paxil®), fluoxetine (Prozac®), sertraline (Zoloft®), and citralopram (Celexa®); and others such as doxepin (Sinequan®) and trazodone (Desyrel®); SNRIs (e.g., venlafaxine and reboxetine); dopaminergic antidepressants (e.g., bupropion and amineptine).
(56) Neuroprotective agents: e.g., memantine, L-dopa, bromocriptine, pergolide, talipexol, pramipexol, cabergoline, neuroprotective agents currently under investigation including anti-apoptotic drugs (CEP 1347 and CTCT346), lazaroids, bioenergetics, antiglutamatergic agents and dopamine receptors. Other clinically evaluated neuroprotective agents are, e.g., the monoamine oxidase B inhibitors selegiline and rasagiline, dopamine agonists, and the complex I mitochondrial fortifier coenzyme Q10.
(57) Antipsychotic medications: e.g., ziprasidone (Geodon™), risperidone (Risperdal™), and olanzapine (Zyprexa™).
(58) NEP inhibitors such as Sacubitril, Omapatrilat.
(59) Methylene Blue (MB).

### Kits

The compounds and pharmaceutical formulations described herein may be contained in a kit. The kit may include single or multiple doses of two or more agents, each packaged or formulated individually, or single or multiple doses of two or more agents packaged or formulated in combination. Thus, one or more agents can be present in first container, and the kit can optionally include one or more agents in a second container. The container or containers are placed within a package, and the package can optionally include administration or dosage instructions. A kit can include additional components such as syringes or other means for administering the agents as well as diluents or other means for formulation. Thus, the kits can comprise: a) a pharmaceutical composition comprising a compound described herein and a pharmaceutically acceptable carrier, vehicle or diluent; and b) a container or packaging. The kits may optionally comprise instructions describing a method of using the pharmaceutical compositions in one or more of the methods described herein (e.g. preventing or treating one or more of the diseases and disorders described herein). The kit may optionally comprise a second pharmaceutical composition comprising one or more additional agents described herein for co therapy use, a pharmaceutically acceptable carrier, vehicle or diluent. The pharmaceutical composition comprising the compound described herein and the second pharmaceutical composition contained in the kit may be optionally combined in the same pharmaceutical composition.

A kit includes a container or packaging for containing the pharmaceutical compositions and may also include divided containers such as a divided bottle or a divided foil packet. The container can be, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container can be used together in a single package to market a single dosage form. For example, tablets may be contained in a bottle which is in turn contained within a box.

An example of a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide written memory aid containing information and/or instructions for the physician, pharmacist or subject regarding when the medication is to be taken. A "daily dose" can be a single tablet or capsule or several tablets or capsules to be taken on a given day. When the kit contains separate compositions, a daily dose of one or more compositions of the kit can consist of one tablet or capsule while a daily dose of another or more compositions of the kit can consist of several tablets or capsules. A kit can take the form of a dispenser designed to dispense the daily doses one at a time in the order of their intended use. The dispenser can be equipped with a memory-aid, so as to further facilitate compliance with the regimen. An example of such a memory-aid is a mechanical counter which indicates the number of daily doses that have been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

### EXAMPLES

As used herein, all abbreviations, symbols and conventions are consistent with those used in the contemporary scientific literature. See, e.g. Janet S. Dodd, ed., The ACS Style Guide: A Manual for Authors and Editors, 2nd Ed., Washington, D.C.: American Chemical Society, 1997.

### Example 1: Syntheses of the Compounds of Table IA or Table IB

### General Procedure A

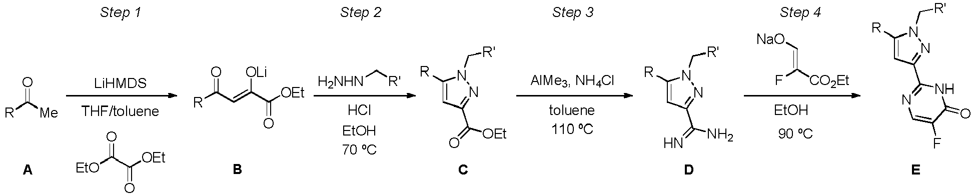

### Step 1:

*Dione enolate formation:* To a solution of ketone **A** in THF cooled to -78 °C, LiHMDS (e.g., 0.9 equiv, 1.0 M in toluene) was added dropwise via syringe. The reaction was allowed to warm to 0 °C, then charged with diethyl oxalate (1.2 equiv). At this time, the reaction was warmed to room temperature and stirred at that temperature until judged complete (e.g., using either TLC or LC/MS analysis). Once the reaction was complete (reaction time was typically 45 minutes), the product dione enolate **B** was used "as-is" in Step 2, i.e., the cyclization step, without any further purification.

### Step 2:

*Pyrazole formation:* Dione enolate **B** was diluted with ethanol and consecutively charged with HCl (e.g., 3 equiv, 1.25 M solution in ethanol) and arylhydrazine hydrate (e.g., 1.15 equiv). The reaction mixture was heated to 70 °C and stirred at this temperature until cyclization was deemed complete (e.g., by LC/MS analysis, typically 30 minutes). Once complete, the reaction mixture was treated carefully with solid sodium bicarbonate (e.g., 4 equiv) and diluted with dichloromethane and water. Layers were separated, and aqueous layer was futher diluted with water before extraction with dichloromethane (3x). The combined organics were washed with brine, dried over MgSO₄, filtered, and concentrated in vacuo. The resulting pyrazole **C** was then purified by SiO2 chromatography using an appropriate gradient of EtOAc in hexanes.

### Step 3:

*Amidine formation:* To a suspension of NH4Cl (e.g., 5 equiv) in toluene cooled to 0 °C was added AlMe₃ (e.g., 5 equiv, 2.0M solution in toluene) dropwise via syringe. The reaction was allowed to warm to room temperature, and stirred at this temperature until no more bubbling was observed. Pyrazole **C** was added in 1 portion to the reaction mixture, heated to 110 °C, and stirred at this temperature until judged complete (e.g., using either TLC or LC/MS analysis). Once complete, the reaction was cooled, treated with excess methanol, and stirred vigorously for 1 hour at room temperature. The thick slurry was filtered, and the resulting solid cake was washed with methanol. The filtrate was concentrated in vacuo, and the resulting solids were re-suspended in an ethyl acetate : isopropyl alcohol = 5:1 solvent mixture. The reaction was further treated with saturated sodium carbonate solution, and stirred for 10 minutes before the layers are separated. The aqueous layer was extracted with the ethyl acetate : isopropyl alcohol = 5:1 solvent mixture (3x), and the combined organics were washed with brine. The organics were further dried over MgSO₄, filtered, and the solvent removed in vacuo. The product amidine **D** was used as-is in subsequent steps without further purification.

### Step 4:

*Pyrimidone formation:* Amidine **D** was suspended in ethanol, and stirred vigorously at 23 °C to encourage full solvation. The reaction was further treated with sodium 3-ethoxy-2-fluoro-3-oxoprop-1-en-1-olate (e.g., 3 equiv.), and the flask was equipped with a reflux condenser. The reaction was placed into a pre-heated oil bath maintained at 90 °C and stirred until full consumption of starting material was observed on the LC/MS (reaction times were typically 1 h). The contents were cooled to 23 °C, and the reaction mixture acidified with HCl (e.g., 3 equiv., 1.25M solution in EtOH). The mixture was stirred for 30 minutes, and the majority of the solvent was removed in vacuo. Contents were re-suspended in ether and water (1:1 mixture), and the resulting slurry was stirred for 20 min. The suspension was vacuum filtered, and the solid cake was rinsed with additional water and ether and dried on high vacuum overnight. The resulting pyrimidone E was used as-is in subsequent steps without further purification.

### General procedure B

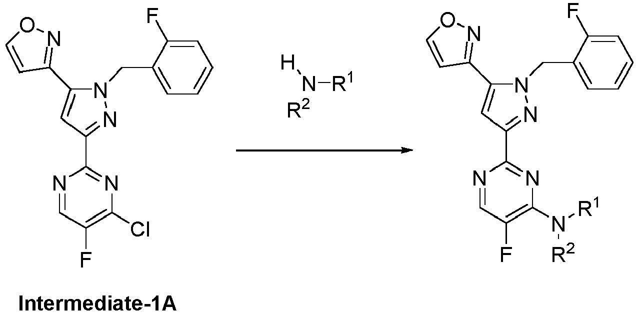

A solution of amino nucleophile (3 equiv.), triethylamine (10 equiv.), and **Intermediate-1A** (prepared as described later in this section, 1 equiv.) was stirred in dioxane and water (2:1 ratio) at 90 °C until complete consumption of starting material was observed by LC/MS. The solution was diluted with aqueous 1N hydrochloric acid and dichloromethane. The layers were then separated and the aqueous layer was extracted with dichloromethane. The organics were combined, dried over magnesium sulfate, filtered, and the solvent was removed in vacuo. Purification yielded the desired product.

### General procedure C

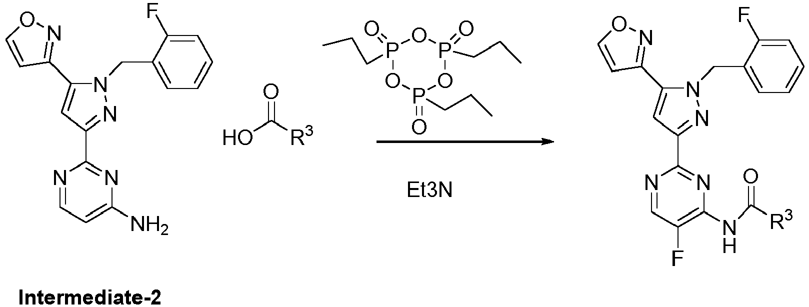

A mixture of **Intermediate-2** (this intermediate was described in patent application publication WO2012/3405 A1; 1 equivalent) and carboxylic acid (1.1 equivalent) in *N,N*-dimethylformamide was treated with triethylamine (4 equivalent) followed by a 50% in ethyl acetate solution of propylphosphonic anhydride (T3P, 1.4 equivalent). The reaction was heated at 80 °C for 24 h, after which the reaction was diluted with water and IN hydrochloric acid solution. Contents were extracted with dichloromethane, then ethyl acetate. The combined organic layers were dried over sodium sulfate, filtered, and concentrated in vacuo. Purification yielded the desired product.

### Synthesis of Intermediate-1A

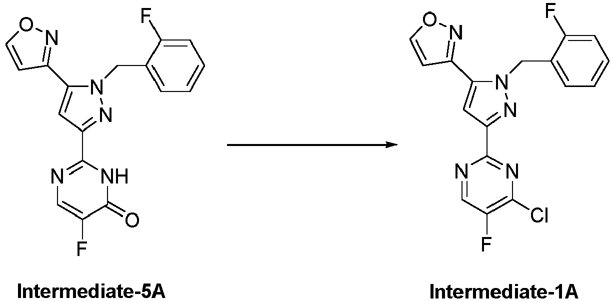

A suspension of 5-fluoro-2-(1-(2-fluorobenzyl)-5-(isoxazol-3-yl)-1H-pyrazol-3-yl)--pyrimidin-4-ol (**Intermediate-5A;** generated via general procedure A, using 1-(isoxazol-3-yl)ethanone in step 1 and 2-fluorobenzylhydrazine in step 2, 11.5 g, 32.4 mmol, 1 equiv.) in phosphoryl trichloride (60.3 mL, 647 mmol, 20 equiv.) was heated at 60 °C for 3 h. The solution was cooled to 23 °C, and poured portionwise over the course of 15 min into ice water (800 mL) with stirring. After completion of addition, contents were stirred for an additional 15 min, and diluted with dichloromethane (500 mL). The layers were separated and the aqueous layer was extracted with dichloromethane (2 x 200 mL). The organics were dried over magnesium sulfate, filtered, and the solvent was removed in vacuo to yield **Intermediate-1A** (12.5 g, 103 % yield) as a tan solid. ¹H NMR (500 MHz, DMSO-*d*₆) δ 9.11 (d, 1 H), 9.04 (s, 1 H), 7.71-7.68 (m, 1 H), 7.37-7.30 (m, 2H), 7.25-7.20 (m, 1 H), 7.12 (t, 1 H), 6.92 (td, 1 H), 5.95 (s, 2 H).

### Synthesis of Intermediate-9

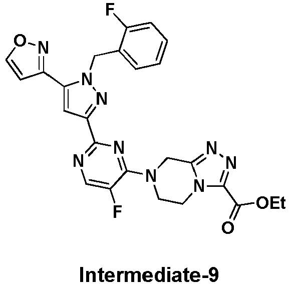

The title compound was prepared following general procedure B from **Intermediate-1A,** except ethyl 5,6,7,8-tetrahydro-[1,2,4]triazolo[4,3-a]pyrazine-3-carboxylate (4 equiv.) was the amine reactant, and the reaction was run in THF. The workup was carried out in dichloromethane and brine. The crude material was purified via silica gel chromatography utilizing a 0-10% methanol/dichloromethane gradient to deliver the desired **Intermediate-9** (42 mg, 37% yield) as a solid. ¹H-NMR (400 MHz, CDCl3) δ 8.47 (d, 1H), 8.35 (d, 1H), 7.40 (s, 1H), 7.21-7.16 (m, 1H), 7.01 (t, 1H), 6.95 (t, 1H), 6.84 (t, 1H), 6.65 (d, 1H), 5.98 (s, 2H), 5.35 (s, 2H), 4.59 (t, 2H), 4.48 (q, 2H), 4.30 (t, 2H), 1.44 (t, 3H).

### Synthesis of Intermediate-8

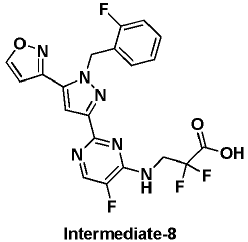

The title compound was prepared following general procedure B, from **Intermediate-1A,** except 3-amino-2,2-difluoropropanoic acid was the amine reactant, and contents were heated at 110 °C for 18 h as a solution in dioxane/water (10:1). Reaction was concentrated in vacuo, methanol was added, and the crude material was purified via reverse phase HPLC to deliver the desired **Intermediate-8** (20 mg, 22% yield). ¹H NMR (500 MHz, CD₃OD) δ ppm 8.78 (d, 1 H), 8.22 (d, 1 H), 7.61 (s, 1 H), 7.25 - 7.31(m, 1 H), 7.07 - 7.12 (m, 1 H), 7.05 (t, 1 H), 6.96 (d, 1 H), 6.89 (t, 1 H), 6.00 (s, 2 H), 4.35 (t, 2 H).

### Synthesis of Intermediate-13

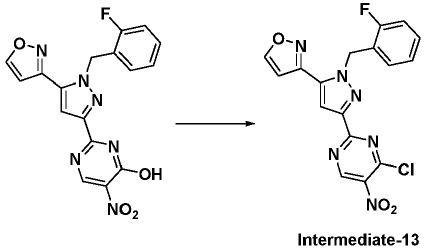

2-(1-(2-fluorobenzyl)-5-(isoxazol-3-yl)-1H-pyrazol-3-yl)-5-nitropyrimidin-4-ol (1 equiv.) (this starting material was described in a previously published patent application: WO2012/3405 A1) (25 mg, 1 equiv.) was treated with POCl₃ (457 µl, 75 equiv.) and stirred at reflux for 1.5 h. Contents were concentrated in vacuo, and residue was azeotroped with toluene (x2). The residue was re-dissolved in THF (0.7 mL) and treated with morpholine (171 µl, 30 equiv.). The contents were heated to 40 °C, and reaction stirred for 1.5 h at this temperature. The residue was transferred to a 1:1 mixture of ethyl acetate and water. The layers were separated, and the aqueous layer was extracted with ethyl acetate (x3). The organic portions were combined and washed with brine. The mixture was dried over MgSO₄, filtered, and concentrated in vacuo to deliver the desired compound (30 mg, 97%) as a pale yellow solid.
¹H-NMR (400 MHz, CDCl₃) δ 8.47 (d, 1 H), 8.36 (d, 1 H), 8.09 - 8.16 (m, 1 H), 7.69 (dd, 1 H), 7.41 (d, 1 H), 7.20 (t, 1 H), 6.66 - 6.70 (m, 1 H), 6.45 (d, 1 H), 6.06 (s, 2 H), 3.79 - 3.86 (m, 4 H), 3.74 (m, 4 H).

### Synthesis of Intermediate-3

The title compound was synthesized in 3 steps:

### Step 1: Synthesis of 2-(trifluoromethyl)oxirane-2-carboxamide

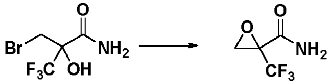

To a solution of 2-(bromomethyl)-3,3,3-trifluoro-2-hydroxypropanamide (1 equiv.) in acetone was added potassium carbonate (2 equiv.). The mixture was heated at reflux for 2 h. The mixture was concentrated under in vacuo. The resulting residue was diluted with water and extracted with ethyl acetate. The organic layer was dried, filtered and evaporated to give 2-(trifluoromethyl)oxirane-2-carboxamide (1.44 g 76 % yield) as a yellow gum.
¹H NMR (500 MHz, CD₃OD) δ ppm 3.17 (dd, 2 H).

### Step 2: Synthesis of 2-(aminomethyl)-3,3,3-trifluoro-2-hydroxypropanamide

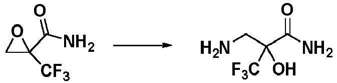

A mixture of ammonia [7M in methanol] (10 equiv.) and 2-(trifluoromethyl)oxirane-2-carboxamide (1 equiv.) was stirred in a sealed vial at 80 °C for 24 h. The mixture was concentrated in vacuo to give 2-(aminomethyl)-3,3,3-trifluoro-2-hydroxypropanamide (1.3 g, 84% yield) as a brown gum.
¹H NMR (500 MHz, DMSO-*d*₆) δ 3.01 - 3.11 (m, 1 H), 2.84 (d, 1 H).

### Step 3: Synthesis of Intermediate-3

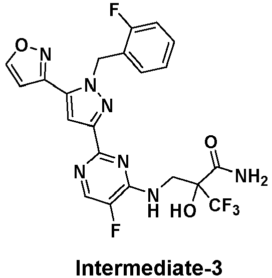

The title compound was prepared following general procedure B from **Intermediate-1A,** except 2-(aminomethyl)-3,3,3-trifluoro-2-hydroxypropanamide (4 equiv.) was the amine reactant, 4 equivalents of triethylamine was used, and contents were heated at 90 °C for 24 h as a solution in dioxane/water (3:1). The mixture was diluted in ethyl acetate and washed with water. The organic layer was dried, filtered and evaporated to give a solid. The solid was purified via silica gel chromatography (0 to 80% ethyl acetate in hexanes gradient) to deliver the desired **Intermediate-3** (262 mg, 40% yield) as a white solid.
¹H NMR (500 MHz, DMSO-*d*₆) δ ppm 9.08 - 9.13 (m, 1 H), 8.33 (d, 1 H), 7.49 - 7.55 (m, 1 H), 7.28 - 7.37 (m, 1 H), 7.17 - 7.25 (m, 2 H), 7.10 (t, 1 H), 6.98 (t, 1 H), 5.86 - 5.92 (m, 2 H), 3.92 - 4.04 (m, 2 H).

### Synthesis of Intermediate-5D

The title compound was synthesized in 5 steps:

### Step 1: Synthesis of ethyl 1-(2-fluorobenzyl)-5-methyl-1H-pyrazole-3-carboxylate

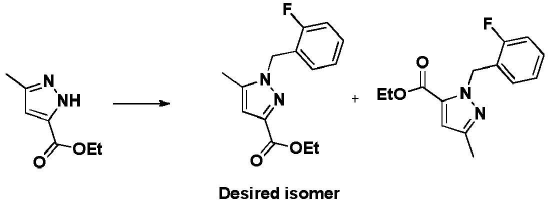

To ethyl 3-methyl-1*H*-pyrazole-5-carboxylate in DMF was added sodium hydride (60 wt% in mineral oil, 1.2 equiv.). After 10 min, 2-fluorobenzyl bromide (1.2 equiv.) was added and the reaction was stirred for 20 h. Water was added and the resulting mixture was extracted with ethyl acetate. The combined organic phases were washed with water and brine, dried over sodium sulfate, filtered, and the solvent was removed in vacuo. Purification via silica gel chromatography (10-40% ethyl acetate/hexanes gradient) yielded ethyl 1-(2-fluorobenzyl)-5-methyl-1*H*-pyrazole-3-carboxylate (79% yield) and ethyl 1-(2-fluorobenzyl)-3-methyl-1*H*-pyrazole-5-carboxylate (9% yield).

### Step 2: Synthesis of 1-(2-fluorobenzyl)-5-methyl-1H-pyrazole-3-carboxylic acid

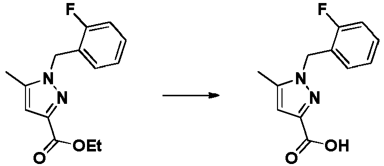

To a solution of ethyl 1-(2-fluorobenzyl)-5-methyl-1*H*-pyrazole-3-carboxylate in THF/MeOH/Water (3:1:1 ratio) was added lithium hydroxide hydrate (1.5 equiv.). After 23 h, the volatile organics were removed in vacuo and the resultant mixture was acidified to pH 3 with 1N HCl. 1-(2-fluorobenzyl)-5-methyl-1*H*-pyrazole-3-carboxylic acid was collected by vacuum filtration (92% yield).

### Step 3: Synthesis of 1-(2-fluorobenzyl)-5-methyl-1H-pyrazole-3-carbonitrile

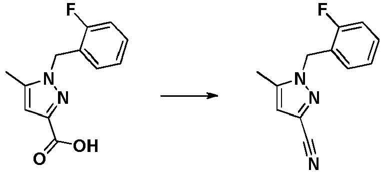

To a suspension of 1-(2-fluorobenzyl)-5-methyl-1*H*-pyrazole-3-carboxylic acid, 2-methylpropan-2-amine (3 equiv.), and triethylamine (2 equiv.) in ethyl acetate was added *n*-propylphosphonic anhydride (T3P, 50 wt% solution in ethyl acetate, 3 equiv.). The resultant yellow solution was heated at 65°C for 2.5 h. The solvent was removed in vacuo. Phosphoryl trichloride (12 equiv.) was added and the resulting mixture was stirred at 70 °C for 1 hour 40 min. The reaction was quenched by carefully pouring into a mixture of water and ice, neutralized to pH 7 by addition of saturated sodium bicarbonate solution and extracted with dichloromethane. The combined organic phases were dried over sodium sulfate, filtered, and the solvent was removed in vacuo. Purification by silica gel chromatography (10% ethyl acetate/hexanes gradient) yielded 1-(2-fluorobenzyl)-5-methyl-1*H*-pyrazole-3-carbonitrile (49% yield).

### Step 4: Synthesis of 1-(2-fluorobenzyl)-5-methyl-1H-pyrazole-3-carboximidamide

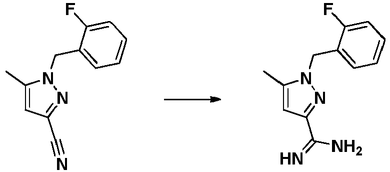

A solution of 1-(2-fluorobenzyl)-5-methyl-1*H*-pyrazole-3-carbonitrile in methanol was treated sodium methoxide (25 wt% solution in MeOH, 5 equiv.) and stirred for 24 h. Ammonium chloride (10 equivalents) was added. After 26 hours, the reaction mixture was concentrated in vacuo and partitioned between half-saturated sodium bicarbonate and ethyl acetate. The organic phases were dried over sodium sulfate, filtered, and the solvent was removed in vacuo. The crude product was contaminated with starting material due to incomplete reaction. This material was re-subjected to similar conditions to afford 1-(2-fluorobenzyl)-5-methyl-1*H*-pyrazole-3-carboximidamide (92% yield). Step 5: Synthesis of **Intermediate-5D**

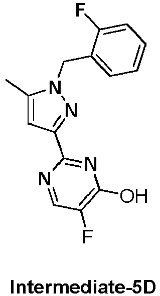

A suspension of 1-(2-fluorobenzyl)-5-methyl-1*H*-pyrazole-3-carboximidamide was treated with sodium (Z)-3-ethoxy-2-fluoro-3-oxoprop-1-en-1-olate (see also general procedure A, Step 4, 3.0 equiv.) and heated at 90 °C for 1 hour. After cooling to ambient temperature, the reaction mixture was neutralized by addition of HCl (1.25 M solution in EtOH). The resultant tan suspension was concentrated in vacuo. The residue was partitioned between dichloromethane and water and the aqueous layer was back-extracted with dichloromethane. The combined organic phases were dried over sodium sulfate, filtered, and the solvent was removed in vacuo. Trituration with dichloromethane yielded the titled compound (206 mg, 62% yield) as a white solid.
¹H-NMR (500 MHz, DMSO-d₆) δ 12.9 (br s, 1 H), 8.07 (br s, 1 H), 7.38 (app. q, 1 H), 7.25 (m, 1 H), 7.18 (app. t, 1 H), 7.11 (m, 1 H), 6.72 (s, 1 H), 5.44 (s, 2 H), 2.30 (s, 3 H).

### Synthesis of Intermediate-12

The title compound was prepared in 2 steps:

### Step 1: Synthesis of diethyl 2-(dicyanomethyl)-2-methylmalonate.

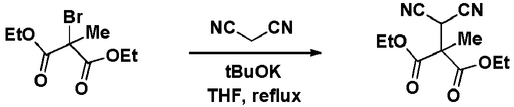

A mixture of diethyl 2-bromo-2-methylmalonate (1 equiv.), malononitrile (1 equiv.) and potassium t-butoxide (1 equiv.) in THF was heated to reflux for 15 h. The mixture was diluted with ethyl acetate and saturated aqueous ammonium chloride solution and the phases were separated. The aqueous phase was extracted twice with ethyl acetate. The combined organic phase was washed with brine, dried over anhydrous sodium sulfate, filtered and concentrated to give an oil. The oil was purified by silica gel chromatography (10-15% ethyl acetate in hexane gradient) to give diethyl 2-(dicyanomethyl)-2-methylmalonate (5.76 g, 32 % yield) as a colorless oil.
¹H NMR (500 MHz, CDCl₃) δ ppm 4.53 (s, 1 H), 4.27 - 4.39 (m, 4 H), 1.81 (s, 3 H), 1.33 (t, 6 H). Step 2: Synthesis of **Intermediate-12**

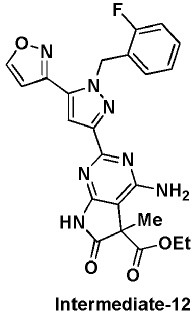

A mixture of 1-(2-fluorobenzyl)-5-(isoxazol-3-yl)-1H-pyrazole-3-carboximidamide hydrochloride (generated in step 3 of general procedure A, by using 1-(isoxazol-3-yl)ethanone in step 1 and 2-fluorobenzylhydrazine in step 2) (1 equiv.), diethyl 2-(dicyanomethyl)-2-methylmalonate (1.15 equiv.) and potassium bicarbonate (2 equiv.) in t-BuOH was heated to reflux for 5 h. After cooling, the reaction mixture was added with water and stirred for 30 min. The precipitate was filtered, washed with a minimum amount of water and diethyl ether and dried overnight under high vacuum to give **Intermediate-12** (385 mg, 52% yield) as a white solid. ¹H NMR (500 MHz, DMSO-*d*₆) δ ppm 11.30 (s, 1 H), 9.10 (d, 1 H), 7.38 (s, 1 H), 7.29 - 7.36 (m, 1 H), 7.18 - 7.26 (m, 2 H), 7.08 - 7.14 (m, 1 H), 6.81 - 6.90 (m, 1 H), 6.65 (br. s., 2 H), 5.88 (s, 2 H), 4.04 - 4.16 (m, 2 H), 1.59 (s, 3 H), 1.11 (t, 3 H).

### Synthesis of Intermediate-11

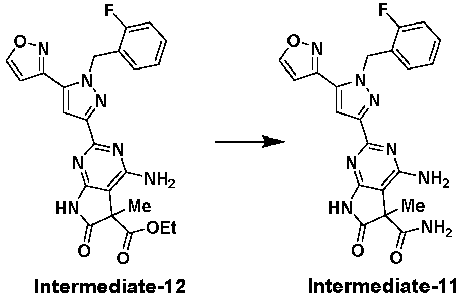

Ammonia (7.0 M in MeOH) (200 equiv.) was added to **Intermediate-12** (1 equiv.). The reaction mixture was heated at 50 °C for 16 h. The resultant solution was then concentrated in vacuo, and the residue was purified via reverse phase HPLC (5-60% acetonitrile in water with 1% TFA) to deliver the desired **Intermediate-11** (24 mg, 63% yield) as a white solid. ¹H NMR (400 MHz, DMSO-*d*₆) δ ppm 11.35 (br. s., 1 H), 9.08 - 9.13 (m, 1 H), 7.47 (s, 1 H), 7.43 (s, 1 H), 7.28 - 7.38 (m, 1 H), 7.23 - 7.27 (m, 1 H), 7.17 - 7.23 (m, 2 H), 7.06 - 7.14 (m, 1 H), 6.77 - 7.00 (m, 3 H), 5.91 (s, 2 H), 1.56 (s, 3 H).

### Synthesis of Intermediate-5B

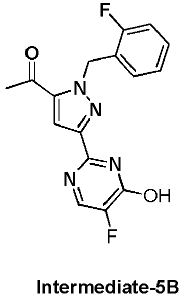

A suspension of **Intermediate-5A** and sodium methoxide in methanol (0.5 M solution, 4 equiv.) was heated in a microwave vessel at 130 °C for 4 h. The reaction was quenched with IN HCl solution to pH 2, and the resulting residue was filtered. The solids were washed with methanol and dried in vacuo to deliver the desired compound (1.45 g, 68%) as a white solid. ¹H NMR (500 MHz, CD₃OD) δ ppm 8.04 (d, 1 H), 7.71 (s, 1 H), 7.23 - 7.36 (m, 1 H), 7.00 - 7.18 (m, 2 H), 6.90 (t, 1 H), 5.94 (s, 2 H), 2.56 (s, 3 H)

### Synthesis of Intermediate-1B

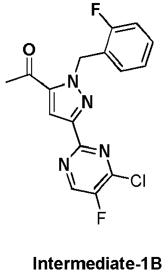

**Intermediate-5B** was charged with phosphoryl trichloride (60 equiv.) and the resulting mixture was stirred at 45 °C until the reaction was judged complete by LC/MS. The reaction was then carefully poured over ice, extracted with 4:1 dichloromethane/isopropanol and the layers were separated. The organic portions were combined, dried with sodium sulfate, filtered, and concentrated in vacuo. The material was carried forward into the next step without further purification.

### Synthesis of Intermediate-6

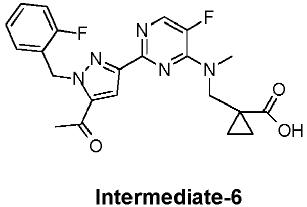

The title compound was prepared following general procedure B, with 1-((methylamino)methyl)cyclopropanecarboxylic acid as the amine reactant, **Intermediate-1B** was used in place of **Intermediate-1A,** and contents were heated at 100 °C for 36 h as a solution in dioxane. The crude material was purified via reverse phase HPLC to deliver the desired **Intermediate-6** (50 mg, 69% yield) as a tan solid. ¹H NMR (500 MHz, DMSO-*d*6) δ ppm 12.53 (br. s, 1 H), 8.19 (d, 1 H), 7.65 (s, 1 H), 7.33 (d, 1 H), 7.17 - 7.26 (m, 1 H), 7.11 (t, 1 H), 6.86 (t, 1 H), 5.81 (s, 2 H), 4.00 (s, 2 H), 3.24 (d, 3 H), 2.57 (s, 3 H), 1.03 (d, 2 H), 0.74 - 0.91 (m, 2 H).

### Synthesis of Intermediate-4

The title compound was synthesized in 3 steps:

### Step 1: Synthesis of 2-(bromomethyl)-3,3,3-trifluoro-2-hydroxypropanoic acid.

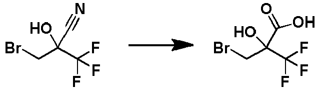

A mixture of 2-(bromomethyl)-3,3,3-trifluoro-2-hydroxypropanenitrile (1 equiv.), water (1 equiv.) and concentrated sulfuric acid (4 equiv.) was heated to 110 °C in a sealed vial for 1 h. The mixture was poured over ice and extracted with diethyl ether. The organic layer was dried over MgSO₄, filtered, and concentrated in vacuo to deliver 2-(bromomethyl)-3,3,3-trifluoro-2-hydroxypropanoic acid (1.3 g, 33% yield) as a clear oil.
¹H NMR (500 MHz, CDCl₃) δ ppm 3.89 (d, 1 H), 3.63 - 3.69 (m, 1 H).

### Step 2: Synthesis of 2-(aminomethyl)-3,3,3-trifluoro-2-hydroxypropanoic acid.

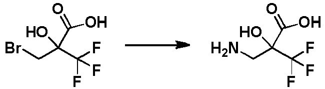

A mixture of ammonium hydroxide [28% solution in water] (10 equiv.) and 2-(bromomethyl)-3,3,3-trifluoro-2-hydroxypropanoic acid (1 equiv.) was stirred at 23 °C for 24 h. The mixture was concentrated in vacuo. The resulting solid was treated with a minimal amount of ethanol. The precipitate was collected by filtration and dried under vacuum to deliver 2-(aminomethyl)-3,3,3-trifluoro-2-hydroxypropanoic acid (412 mg, 43% yield) as a white solid.
¹H NMR (500 MHz, DMSO-*d*₆) δ ppm 2.86 - 3.27 (m, 2 H).

### Step 3: Synthesis of Intermediate-4

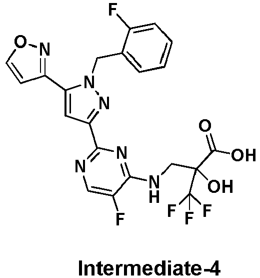

The title compound was prepared following general procedure B, except 2-(aminomethyl)-3,3,3-trifluoro-2-hydroxypropanoic acid (4 equiv.) was the amine reactant, 6 equivalents of triethylamine was used, and contents were heated to 85 °C as a solution in 1,4-dioxane/water (4:1) for 24 h. The mixture was cooled to 23 °C and diluted with ethyl acetate. The organic layer was washed with saturated solution of ammonium chloride, dried over MgSO₄, filtered, and concentrated in vacuo to yield a crude solid. The crude material was purified via silica gel chromatography utilizing a 0-100% ethyl acetate/hexanes gradient to deliver the desired **Intermediate-4** (50 mg, 7% yield for step 3) as a white solid. ¹H NMR (500 MHz, DMSO-*d*₆) δ ppm 8.28 (d, 1 H), 7.59 (t, 1 H), 7.46 (s, 1 H), 7.30 - 7.36 (m, 1 H), 7.16 - 7.24 (m, 2 H), 7.10 (t, 1 H), 6.91 (t, 1 H), 5.88 (s, 2 H), 4.24 (dd, 1 H), 3.84 (dd, 1 H).

### Synthesis of Intermediate-7

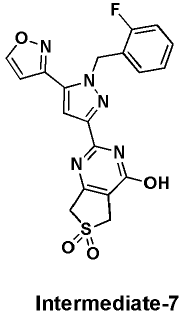

A solution of 1-(2-fluorobenzyl)-5-(isoxazol-3-yl)-1H-pyrazole-3-carboximidamide hydrochloride (generated in step 3 of general procedure A, 1 equiv.), methyl 4-oxotetrahydrothiophene-3-carboxylate (3 equiv.), and 1,8-diazabicyclo[5.4.0]undec-7-ene (1 equiv.) in pyridine was heated to 80 °C for 12 h. The reaction was concentrated in vacuo, slurried in methanol, concentrated in vacuo, and slurried again in methanol. The precipitate was filtered and dried to provide the desired cyclic sulfide intermediate (190 mg, 45% yield) as a light-tan solid. To a solution of this sulfide intermediate (1 equiv.) in dichloromethane was added peracetic acid (2.3 equiv.). After 30 min, the reaction was concentrated in vacuo, slurried in water, and filtered to deliver the desired **Intermediate-7** (148.8 mg, 73% yield) as an off-white solid. ¹H-NMR (500 MHz, CDCl₃) δ 10.2 (br. s, 1 H), 8.56 (s, 1 H), 7.31-7.34 (m, 1 H), 7.30 (s, 1 H), 7.07-7.12 (m, 3 H), 6.64 (m, 1 H), 5.93 (s, 2 H), 4.36 (s, 2 H), 4.35 (s, 2 H).

### Synthesis of Intermediate-10

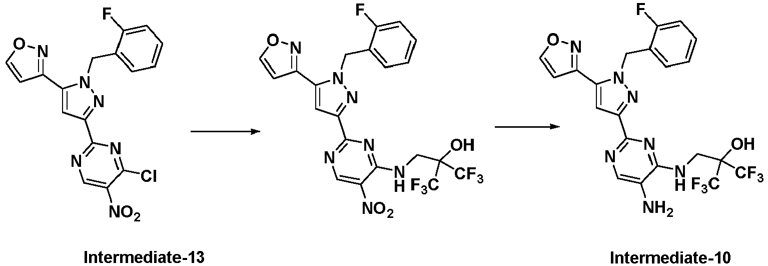

The title compound was synthesized in 2 steps:

### Step 1:

Following general procedure B, **Intermediate-13** was used in place of **Intermediate-1A,** 2-(aminomethyl)-1,1,1,3,3,3-hexafluoropropan-2-ol (1.5 equiv.) was the amine reactant, 3 equivalents of triethylamine was used, and contents were heated at 30 °C for 1 h as a solution in dioxane:water (3:1). The reaction was cooled and diluted with ethyl acetate. The organic layer was washed with water (2x) and brine, then dried over sodium sulfate, filtered, and concentrated in vacuo. The crude material was purified via silica gel chromatography utilizing a 0-100% ethyl acetate/hexanes gradient to deliver the desired intermediate (77 mg, 73% yield) as a white solid. ¹H-NMR (400 MHz, CDCl₃) δ ppm 9.36 (s, 1 H), 8.59 (m, 1 H), 8.55 (d, 1 H), 7.64 (br s, 1 H), 7.42 (s, 1 H), 7.28 (m, 1 H), 7.08 (m, 1 H), 7.06 (m, 1 H), 6.64 (d, 1 H), 5.98 (s, 2 H), 4.27, (d, 2 H).

### Step2: Synthesis of Intermediate-10

A solution of the intermediate obtained in Step 1 (1 equiv.) in methanol at 23 °C was treated with 10% palladium on carbon (0.2 equiv.), then placed under an atmosphere of H₂ delivered via a balloon filled with hydrogen attached to a needle. The mixture was stirred for 1 h under positive H₂ pressure, and filtered through celite. The filter cake was rinsed with methanol, and the combined washes were concentrated in vacuo. The resulting crude residue was purified via silica gel chromatography utilizing a ethyl acetate in hexanes gradient to deliver the desired **Intermediate-10** (53 mg, 66% yield) as a white solid. ¹H-NMR (400 MHz, CDCl₃) δ ppm 9.39 (s, 1H), 7.92 (br s, 1H), 7.19 (m, 1H), 7.13 (m, 2H), 7.98 (m, 1H), 6.92 (m, 2H), 6.52 (s, 1H), 5.85 (s, 2H), 4.01, (s, 2H).

### Synthesis of Intermediate-5C

The title compound was synthesized in 4 steps:

### Step 1: Synthesis of (3,3,3-trifluoropropyl)hydrazine hydrochloride

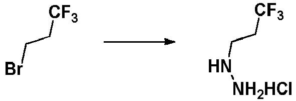

3-Bromo-1,1,1-trifluoropropane (1 equiv.) and hydrazine hydrate (10 equiv.) were dissolved in absolute ethanol and heated at 80 °C for 18 h. The solution was cooled to 23 °C and concentrated under vacuum at 15 °C. The thick oil was diluted with water and dichloromethane, then solid potassium carbonate was added to saturate the aqueous layer. The phases were mixed and separated, then the aqueous phase was extracted with additional dichloromethane (2x). The combined organic phases were dried over sodium sulfate, filtered, and concentrated under vacuum to give a colorless oil. A small portion of the neutral hydrazine product was removed for characterization by NMR. The remainder was taken up in diethyl ether and treated with hydrochloric acid (2.5 M solution in ethanol), and the resulting mixture was concentrated in vacuo to deliver the desired intermediate (3,3,3-trifluoropropyl)hydrazine hydrochloride (2.02 g, 43% yield) as a white solid. ¹H-NMR (400 MHz, CDCl₃) δ ppm 3.18 (br s, 4 H), 3.02 (m, 2 H), 2.36 (m, 2 H).

### Step 2: Synthesis of ethyl 3-(isoxazol-3-yl)-1-(3,3,3-trifluoropropyl)--1H-pyrazole-5-carboxylate

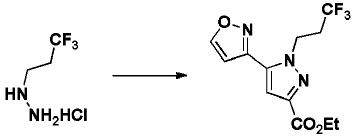

A solution of (3,3,3-Trifluoropropyl)hydrazine hydrochloride (1 equiv.) in a mixture of ethanol and water (9:1) at 23 °C was treated with potassium carbonate (0.6 equiv.) followed by ethyl 4-(isoxazol-3-yl)-2-(methoxy(methyl)amino)-4-oxobut-2-enoate (2 equiv., generated in step 1 of general procedure A, by using 1-(isoxazol-3-yl)ethanone in step 1). The solution was stirred 2 d at 23 °C, then 6N hydrochloric acid (1.5 equiv.) was added drop wise to the reaction. Solvents were removed in vacuo, and the residue was taken up in ethyl acetate. The organics were washed with water (5x), brine, dried over sodium sulfate, filtered, and concentrated in vacuo. The crude residue was purified via silica gel chromatography utilizing a ethyl acetate in dichloromethane gradient to yield the desired pyrazole ester, ethyl 3-(isoxazol-3-yl)-1-(3,3,3-trifluoropropyl)-1H-pyrazole-5-carboxylate (1.34 g, 36% yield) as a light yellow solid. ¹H-NMR (400 MHz, CDCl₃) δ ppm 8.55 (d, 1 H), 7.15 (s, 1 H), 6.63 (d, 1 H), 4.95 (m, 2 H), 4.46 (q, 2 H), 2.85 (m, 2 H), 1.44 (t, 3 H).

### Step 3: Synthesis of 5-(isoxazol-3-yl)-1-(3,3,3-trifluoropropyl)-1H-pyrazole-3 -carboximidamide

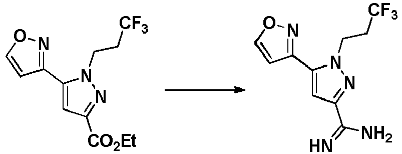

The desired amidine intermediate was generated according to the procedure described in step 3 of general procedure A, with the exception of using ethyl 3-(isoxazol-3-yl)-1-(3,3,3-trifluoropropyl)-1H-pyrazole-5-carboxylate as the starting ester, and the mixture was heated 4 h at 110°C. The reaction mixture was cooled in ice, then methanol (14 equiv.) and aqueous hydrochloric acid (17 equiv.) were added in succession over 5 min. This mixture was heated 30 min at 80 °C, then cooled in ice and filtered. The filter cake was washed with toluene (2x) and air dried to yield the crude amidine hydrochloride salt. This material was stirred in saturated aqueous sodium carbonate, and was extracted with ethyl acetate/isopropyl alcohol (5:1 mix). The organic phase was washed with water and brine, dried over sodium sulfate, filtered, and concentrated in vacuo to deliver the desired neutral amidine
5-(isoxazol-3-yl)-1-(3,3,3-trifluoropropyl)-1H-pyrazole-3-carboximidamide as a light yellow solid. ¹H-NMR (400 MHz, CDCl₃) δ ppm 8.45 (d, 1 H), 6.99 (s, 1 H), 6.55 (d, 1 H), 5.61 (br. s., 3 H), 4.83-4.74 (m, 2 H), 2.81-2.65 (m, 2 H).

### Step 4: Synthesis of Intermediate-5C

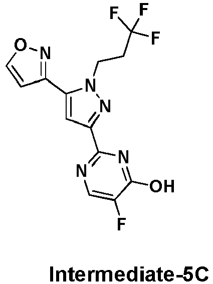

The title product was prepared following step 4 of general procedure A, except 5-(isoxazol-3-yl)-1-(3,3,3-trifluoropropyl)-1H-pyrazole-3-carboximidamide was the starting amidine, 2.5 equivalents of sodium (Z)-3-ethoxy-2-fluoro-3-oxoprop-1-en-1-olate was used, and the mixture was heated for 2 h at 90 °C. The reaction was cooled to 23 °C and the solvent was removed in vacuo. The residue was redissolved in dichloromethane and treated with hydrochloric acid (2.5M in ethanol, 3 equiv.). The resulting solids were filtered, washed with dichloromethane (2x), and air dried to deliver the desired compound (0.43 g, 110% yield) as a white solid. ¹H-NMR (400 MHz, CD₃OD) δ ppm 8.84 (d, 1 H), 8.03 (d, 1 H), 7.40 (s, 1 H), 6.95 (d, 1 H), 4.96 (t, 2 H), 2.92 (m, 2 H).

### Synthesis of Intermediate-16

The title compound was prepared in 2 steps:

### Step 1: Synthesis of (E)-2-(1-(2-fluorobenzyl)-5-(isoxazol-3-yl)-1H-pyrazol-3-yl)-5-(phenyldiazenyl)pyrimidine-4,6-diamine.

A mixture of 1-(2-fluorobenzyl)-5-(isoxazol-3-yl)-1H-pyrazole-3-carboximidamide (generated in step 3, General Scheme A, towards the synthesis of **Intermediate-IA** (1 equiv.), (*E*)-2-(phenyldiazenyl)malononitrile (1.2 equiv.) and potassium bicarbonate (2 equiv.) in *t*-BuOH was heated to reflux for 18 h. After cooling, the reaction mixture was concentrated in vacuo, and carried forward to the next step without any further purification.

### Step 2: Synthesis of Intermediate-16

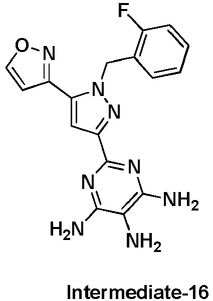

A mixture of (*E*)-2-(1-(2-fluorobenzyl)-5-(isoxazol-3-yl)-1H-pyrazol-3-yl)--5-(phenyldiazenyl) pyrimidine-4,6-diamine (1 equiv.) and 20% palladium on carbon (0.5 equiv.) in DMF was stirred under a hydrogen atmosphere at 23 °C for 18 h. The reaction mixture was then filtered through celite and the residue was washed with DMF followed by a small portion of methanol. The filtrate was concentrated in vacuo, and the residue was suspended in ethyl acetate and a drop of methanol and stirred vigorously. The precipitate was filtered, washed with ethyl acetate, and dried under vacuum to deliver the desired triaminopyrimidine intermediate, 2-(1-(2-fluorobenzyl)-5-(isoxazol-3-yl)-1H-pyrazol-3-yl)--pyrimidine-4,5,6-triamine (278 mg, 46% yield over 2 steps) as a dark yellow solid.

### Synthesis of Intermediate-14

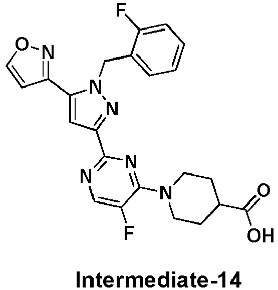

A solution of piperidine-4-carboxylic acid (3 equivalents), triethylamine (10 equivalents), and **Intermediate-1A** was stirred in tetrahydrofuran and water (1:1 ratio) at 100 °C until complete consumption of starting material by LC/MS, following general procedure B. The solution was diluted with aqueous IN hydrochloric acid and ethyl acetate. The layers were separated and the aqueous layer was extracted with ethyl acetate and 5:1 dichloromethane/isopropyl alcohol. The organics were combined, dried over magnesium sulfate, filtered, and the solvent was removed in vacuo. Purification by reverse-phase HPLC (5-75% acetonitrile in water with 0.1% trifluoroacetic acid, 20 minute gradient) yielded **Intermediate-14** (11 mg, 44% yield) as a white solid. ¹H-NMR (400 MHz, CD₃OD) δ 8.79 (m, 1H), 8.23 (d, 1H), 7.57 (m, 1H), 7.31-7.26 (m, 1H), 7.12-7.03 (m, 2H), 6.96 (m, 1H), 6.90 (t, 1H), 5.99 (s, 2H), 4.70 (d, 2H), 3.51-3.45 (m, 2H), 2.79-2.74 (m, 1H), 2.15-2.11 (m, 2H), 1.90-1.80 (m, 2H).

### Reference Compound 25

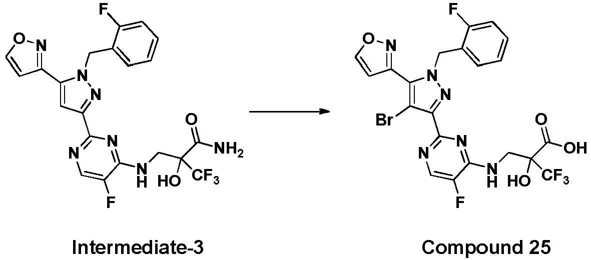

A mixture of **Intermediate-3** (1 equiv.) and NBS (1.2 equiv.) in DMF was stirred at 23 °C for 24 h. The mixture was diluted in ethyl acetate and washed with water. The organic layer was dried, filtered and evaporated to give a crude oil. The oil was purified by column chromatography to deliver the desired compound (27 mg, 4% yield) as a white solid. ¹H NMR (500 MHz, DMSO-*d*₆) δ ppm 9.24 (d, 1 H), 8.37 (d, 1 H), 7.86 (br. s., 1 H), 7.31 - 7.37 (m, 1 H), 7.09 - 7.20 (m, 3 H), 5.68 (s, 2 H), 4.04 (d, 2 H).

### Reference Compound 52

The title compound was synthesized in 3 steps:

### Step 1: Synthesis of 3,3,3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropanehydrazide.

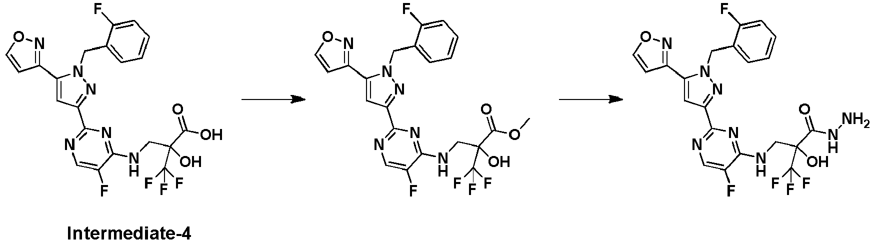

A mixture containing (diazomethyl)trimethylsilane (2 equiv.) and **Intermediate-4** (1 equiv.) in THF (3.0 ml) was heated at 80 °C for 4 h. The mixture was cooled to 23 °C and concentrated in vacuo to give the desired intermediate ester. The intermediate (1 equiv.) was combined with water (11 equiv.), anhydrous hydrazine (130 equiv.) and methanol, and heated to 50 °C for 2 h. Upon the completion of the reaction, the excess hydrazine was removed using methanol and benzene as azeotropes. The resulting residue was further dried in vacuo to deliver the desired intermediate,3,3,3-trifluoro-2-(((5-fluoro-2-(1-(2-fluorobenzyl)-5-(isoxazol-3-yl)-1H-pyrazol-3-yl)pyr imidin-4-yl)amino)methyl)-2-hydroxypropanehydrazide (257 mg, 64% yield) as a light yellow solid.
¹H NMR (500 MHz, DMSO-*d*₆) δ ppm 9.11 (d, 1 H), 8.33 (d, 1 H), 7.52 (s, 1 H), 7.27 - 7.41 (m, 1 H), 7.18 - 7.26 (m, 2 H), 7.11 (t, 1 H), 6.96 (t, 1 H), 5.90 (s, 2 H), 3.98 (br. s., 2 H).

### Step 2: Synthesis of N'-acetyl-3,3,3-trifluoro-2-(((5-fluoro-2-(1-(2-fluorobenzyl)--5-(isoxazol-3-yl)-1H-pyrazol-3-yl)pyrimidin-4-yl)amino)methyl)-2-hydroxypropanehydrazi-de.

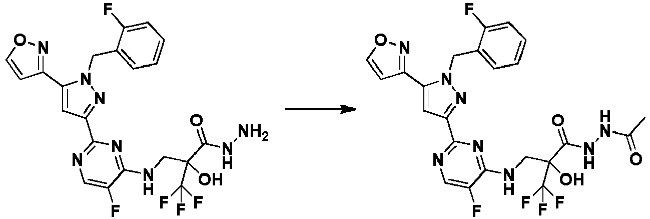

To a mixture containing potassium carbonate (5 equiv.) and 3,3,3-trifluoro-2-(((5-fluoro-2-(1-(2-fluorobenzyl)-5-(isoxazol-3-yl)-1H-pyrazol-3-yl)pyrimidin-4-yl)a mino)methyl)-2-hydroxypropanehydrazide (1 equiv.) in a 1:1 mixture of THF and water was added acetyl chloride (1.5 equiv.). The mixture was stirred at 23 °C for 1 h. The mixture was diluted in ethyl acetate and washed with water. The organic layer was dried, filtered, and concentrated in vacuo to give a crude solid. The solid was purified via silica gel chromatography to deliver the desired intermediate, *N'*-acetyl-3,3,3-trifluoro-2-((5-fluoro-2-(1-(2-fluorobenzyl)-5-(isoxazol-3-yl)-1H-pyrazol-3-yl)pyrimidin-4-yl)amino)methyl)-2-hydroxypropa nehydrazide (190 mg, 64% yield) as a light yellow solid. ¹H NMR (500 MHz, DMSO-*d*₆) δ ppm 9.08 - 9.12 (m, 1H), 8.06 (s, 1 H), 7.50 - 7.56 (m, 1 H), 7.32 (d, 1 H), 7.17 - 7.26 (m, 2 H), 7.10 (t, 1 H), 6.89 - 6.99 (m, 1 H), 5.84 - 5.96 (m, 2 H), 3.91 - 4.17 (m, 2 H), 1.85 (s, 3 H).

### Step 3: Synthesis of Compound 52

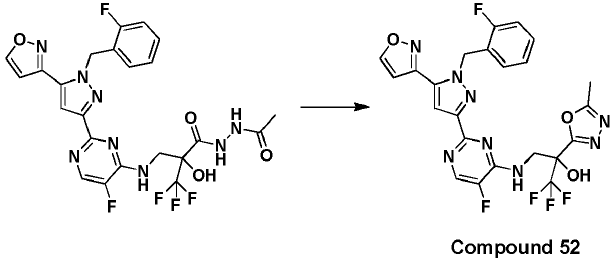

To a cooled solution of *N*'-acetyl-3,3,3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropanehydrazide (1 equiv.) in pyridine at 0 °C was added triflic anhydride (5 equiv.). The mixture was removed from the ice bath and stirred at 23 °C for 24 h. The mixture was diluted in ethyl acetate and washed with water. The organic layer was dried, filtered and evaporated to give a crude oil which was purified via silica gel chromatography. The material was further rinsed with a minimal amount of methanol and dichloromethane, collected by filtration, and dried in vacuo to deliver the desired compound (48 mg, 26% yield) as a tan colored solid. ¹H NMR (500 MHz, DMSO-*d*₆) δ ppm 9.11 (d, 1 H), 8.26 (d, 1 H), 7.40 (s, 1 H), 7.31 - 7.37 (m, 1 H), 7.22 (d, 1 H), 7.19 (d, 1 H), 7.12 (t, 1 H), 6.94 (t, 1 H), 5.84 - 5.92 (m, 2 H), 4.29 (dd, 1 H), 4.17 (dd, 1 H), 2.25 (s, 3 H).

### Reference Compound 69

The title compound was synthesized in 2 steps:

### Step 1: Synthesis of N'-(3,3,3-trifluoro-2-(((5-fluoro-2-(1-(2-fluorobenzyl)-5--(isoxazol-3-yl)-1H-pyrazol-3-yl)pyrimidin-4-yl)amino)methyl)-2-hydroxypropanoyl)cyclopropaneca rbohydrazide.

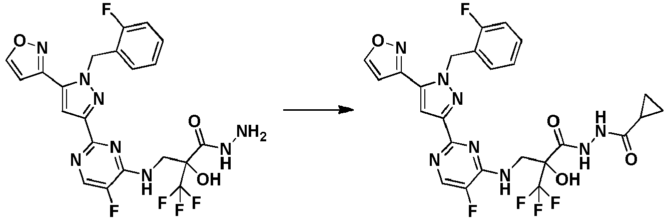

To a mixture containing potassium bicarbonate (5 equiv.) and 3,3,3-trifluoro-2-(((5-fluoro-2-(1-(2-fluorobenzyl)-5-(isoxazol-3-yl)-1H-pyrazol-3-yl)pyrimidin-4-yl)a mino)methyl)-2-hydroxypropanehydrazide (as described in synthesis of **Compound 52,** step 1) (1 equiv.) in a 1:1 mixture of THF and water was added cyclopropanecarboxylic acid chloride (5 equiv.). The mixture was stirred at 23 °C for 1 h. The mixture was taken up in ethyl acetate and washed with water. The organic layer was dried, filtered and evaporated to deliver the desired intermediate, *N'*-(3,3,3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropanoyl)cyclopropanecarbohydrazide (206 mg, 51% yield) as a white solid. ¹H NMR (500 MHz, DMSO-*d*₆) δ ppm 9.10 (d, 1 H), 8.32 (d, 1 H), 7.52 (s, 1 H), 7.33 (s, 1 H), 7.16 - 7.26 (m, 2 H), 7.09 (t, 1 H), 6.93 (s, 1 H), 5.90 (s, 2 H), 4.07 - 4.14 (m, 2 H), 1.59 (d, 1 H), 0.66 - 0.76 (m, 4 H).

### Step 2: Synthesis of 2-(5-cyclopropyl-1,3,4-oxadiazol-2-yl)-1,1,1-trifluoro-3--((5-fluoro-2-(1-(2-fluorobenzyl)-5-(isoxazol-3-yl)-1H-pyrazol-3-yl)pyrimidin-4-yl)amino)propan-2-ol.

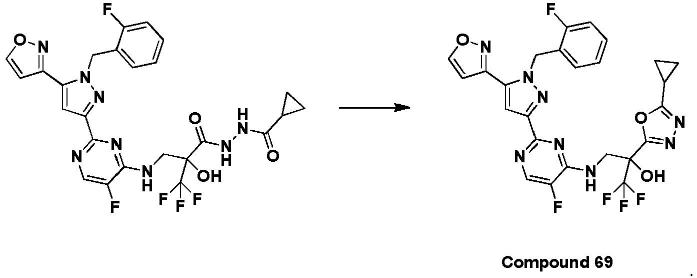

To a cold solution of *N*'-(3,3,3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropanoyl)cyclopropane carbohydrazide (1 equiv.) in pyridine was added triflic anhydride (4 equiv.). Upon completion of the reaction, the mixture was diluted with ethyl acetate and washed with a IN HCl solution. The organic layer was dried, filtered and evaporated to give a crude oil. The oil was purified via silica gel chromatography to deliver the desired compound (9 mg, 9% yield) as a white solid. ¹H NMR (500 MHz, DMSO-*d*₆) δ ppm 9.08 - 9.13 (m, 1 H), 8.27 (d, 1 H), 7.37 (s, 1 H), 7.28 - 7.36 (m, 1 H), 7.19 - 7.25 (m, 1 H), 7.17 (d, 1 H), 7.11 (t, 1 H), 6.93 (t, 1 H), 5.87 (s, 2 H), 4.08 - 4.16 (m, 2 H), 1.97 - 2.08 (m, 1 H), 0.93 (dd, 2 H), 0.74 (dd, 2 H).

### Reference Compound 61

The title compound was synthesized in 3 steps:

### Step 1: Synthesis of 1-(2-fluorobenzyl)-5-(isoxazol-3-yl)-1H-pyrazole-3-carboximidhydrazide.

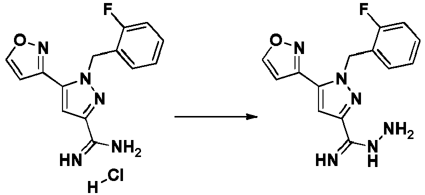

To a suspension of 1-(2-fluorobenzyl)-5-(isoxazol-3-yl)-1H-pyrazole-3-carboximidamide hydrochloride (generated in step 3 of general procedure A, by using 1-(isoxazol-3-yl)ethanone in step 1 and 2-fluorobenzylhydrazine in step 2, 1 equiv.) in ethanol was added triethylamine (4 equiv.). To this mixture was added hydrazine monohydrate (1 equiv.). The mixture was stirred at 23 °C for 24 h and concentrated in vacuo. The resulting residue was diluted with ethyl acetate and washed with brine. The organic layer was dried, filtered and evaporated to deliver the desired intermediate, 1-(2-fluorobenzyl)-5-(isoxazol-3-yl)-1H-pyrazole-3-carboximidhydrazide (461 mg, 99% yield) as a light yellow solid. ¹H NMR (500 MHz, CD₃OD) δ ppm 8.75 (d, 1 H), 7.18 - 7.40 (m, 1 H), 6.97 - 7.15 (m, 3 H), 6.79 - 6.92 (m, 2 H), 5.82 - 5.97 (m, 2 H).

### Step 2: Synthesis of ethyl 2-(3-(1-(2-fluorobenzyl)-5-(isoxazol-3-yl)-1H-pyrazol-3-yl)-5-hydroxyl--1,2,4-triazin-6-yl)-2-methylpropanoate (Compound 110)

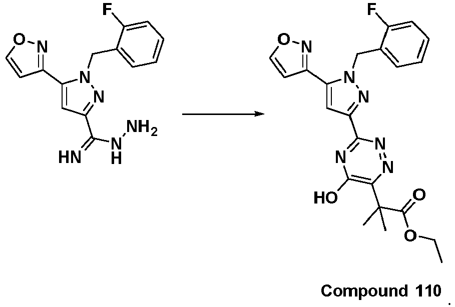

A mixture containing 1-(2-fluorobenzyl)-5-(isoxazol-3-yl)-1H-pyrazole-3-carbohydrazonamide (1 equiv.), potassium bicarbonate (1.2 equiv.), and diethyl 2,2-dimethyl-3-oxosuccinate (1.2 equiv.) in ethanol was heated at 80 °C for 24 h and concentrated in vacuo. The resulting residue was diluted in ethyl acetate and washed with brine. The organic layer was dried, filtered and evaporated to give a crude oil. The oil was purified by column chromatography to deliver the desired intermediate, ethyl 2-(3-(1-(2-fluorobenzyl)-5-(isoxazol-3-yl)-1H-pyrazol-3-yl)-5-hydroxy-1,2,4-triazin-6-yl)-2-methylpr opanoate (400 mg, 34% yield) as a light yellow solid. ¹H NMR (500 MHz, CD₃OD) δ ppm 8.80 (d, 1 H), 8.77 (d, 1 H), 7.53 (s, 1 H), 7.36 (s, 1 H), 7.26 - 7.33 (m, 3 H), 6.03 (s, 2 H), 4.11 - 4.17 (m, 2 H), 1.53 (s, 6 H), 1.22 - 1.27 (m, 3 H).

### Step 3: Synthesis of Compound 61

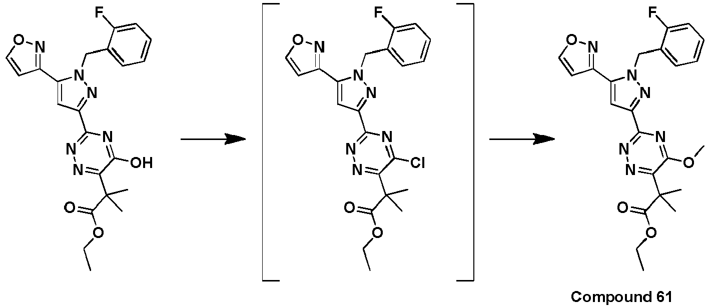

A mixture containing ethyl 2-(3-(1-(2-fluorobenzyl)-5-(isoxazol-3-yl)-1H-pyrazol-3-yl)-5-hydroxy-1,2,4-triazin-6-yl)-2-methylpropanoate **(Compound 110)** and phosphorous oxychloride (10 equiv.) was stirred at 23 °C for 2 h. The mixture was concentrated under vacuum. To this mixture were added a solution of 7 N ammonia in methanol (4 equiv.) and additional methanol. The reaction was stirred at 23 °C for 30 min. The precipitate formed was isolated by filtration to deliver the desired compound (8.3 mg, 8% yield) as a white solid. ¹H NMR (500 MHz, CD₃OD) δ ppm 8.78 - 8.80 (m, 1 H), 7.64 (s, 1 H), 7.26 - 7.32 (m, 1 H), 7.08 - 7.14 (m, 1 H), 7.05 (t, 1 H), 6.95 - 6.98 (m, 1 H), 6.87 (t, 1 H), 6.00 - 6.05 (m, 2 H), 4.17 (s, 3 H), 4.15 (q, 2 H), 1.66 (s, 6 H), 1.15 - 1.21 (m, 3 H).

### Compound 70

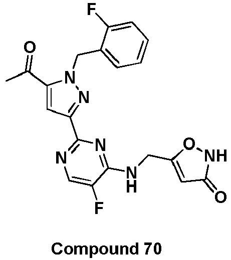

A mixture containing muscimol (1.5 equiv.), trimethylamine (1.5 equiv.), and **Intermediate-1B** (1 equiv.) in a 3:1 mixture of 1,4-dioxane and water was heated to 70 °C for 24 h. The mixture was diluted in ethyl acetate and washed with water. The organic layer was dried, filtered and evaporated to give a crude oil. The oil was purified via silica gel chromatography (0 to 100% ethyl acetate in hexanes) to deliver the desired compound (13 mg, 21% yield) as a white solid. ¹H NMR (500 MHz, DMSO-*d*₆) δ ppm 8.26 (d, 1 H), 7.68 (s, 1 H), 7.29 - 7.36 (m, 1 H), 7.18 - 7.26 (m, 2 H), 7.11 (t, 1 H), 6.85 (t, 1 H), 5.82 (s, 2 H), 3.31 (s, 2 H), 2.57 (d, 3 H).

### Reference Compound 71

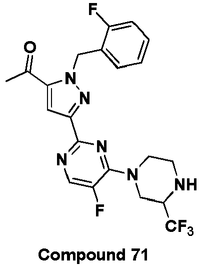

A mixture containing 2-(trifluoromethyl)piperazine (3 equiv.), triethylamine (3 equiv.) and **Intermediate-1B** (1 equiv.) in a 3:1 mixture of 1,4-dioxane and water was heated at 80 °C for 1 h. The mixture was diluted in ethyl acetate. The organic layer was washed with water, dried, filtered and evaporated to give a crude oil. The oil was purified via silica gel chromatography (0 to 100% ethyl acetate in hexanes) to deliver the desired compound (40 mg, 60% yield) as a white solid. ¹H NMR (500 MHz, CD₃OD) δ ppm 8.24 (d, 1 H), 7.66 (s, 1 H), 7.20 - 7.39 (m, 1 H), 7.03 - 7.15 (m, 2 H), 6.83 (t, 1 H), 5.84 (s, 2 H), 4.67 (d, 1 H), 4.42 (d, 1 H), 3.58 (t, 1 H), 3.37 - 3.48 (m, 2 H), 3.15 (d, 1 H), 2.87 - 3.02 (m, 1 H), 2.58 (s, 3 H).

### Reference Compound 72

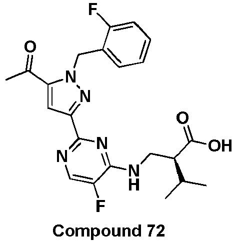

A mixture containing (*S*)-2-(aminomethyl)-3-methylbutyric acid hydrochloride (4 equiv.), triethylamine (2 equiv.) and 1-(3-(4-chloro-5-fluoropyrimidin-2-yl)-1-(2-fluorobenzyl)-1H-pyrazol-5-yl)ethanone (**Intermediate-1B,** 1 equiv.) in 1,4-dioxane and water was heated to 70 °C for 24 h. The mixture was diluted in ethyl acetate. The organic layer was washed with IN HCl, dried, filtered and evaporated to give a solid. The solid was purified via silica gel chromatography (0 to 10% methanol in dichloromethane) to deliver the desired compound (14 mg, 29% yield) as a white solid. ¹H NMR (500 MHz, CD₃OD) δ ppm 8.04 (d, 1 H), 7.63 - 7.69 (m, 1 H), 7.27 (q, 1 H), 7.00 - 7.15 (m, 2 H), 6.83 (t, 1 H), 5.86 - 5.95 (m, 2 H), 3.70 - 3.93 (m, 2 H), 2.63 - 2.74 (m, 1 H), 2.56 (s, 3 H), 1.93 - 2.07 (m, 1H), 1.02 - 1.15 (m, 6 H).

### Reference Compound 73 and Reference Compound 74

The title compounds were synthesized in 3 steps:

### Step 1:

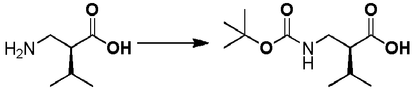

Synthesis of (*S*)-2-(((tert-butoxycarbonyl)amino)methyl)-3-methylbutanoic acid A mixture containing di-*tert*-butyl dicarbonate (2 equiv.), triethylamine (1 equiv.) and (*S*)-2-(aminomethyl)-3-methylbutanoic acid (1 equiv.) in methanol was stirred at 23 °C for 24 h. The mixture was concentrated under vacuum. The resulting residue was diluted in ethyl acetate and washed with IN HCl solution. The organic layer was dried, filtered and evaporated to deliver the desired intermediate, (*S*)-2-(((tert-butoxycarbonyl)- -amino)methyl)-3-methylbutanoic acid (730 mg, 100% yield) as a white solid. ¹H NMR (500 MHz, CDCl₃) δ ppm 3.39 - 3.55 (m, 1 H) 3.06 - 3.31 (m, 1 H) 2.40 - 2.58 (m, 1 H) 1.86 - 2.10 (m, 1 H) 1.38 - 1.52 (m, 9 H) 0.94 - 1.05 (m, 6 H).

### Step 2:

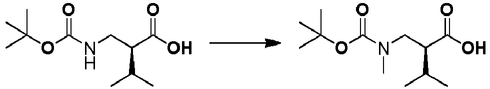

Synthesis of (*S*)-2-(((*tert*-butoxycarbonyl)amino)methyl)-3-methylbutanoic acid. To a cold solution of (*S*)-2-(((*tert*-butoxycarbonyl)amino)methyl)-3-methylbutanoic acid (1 equiv.) in THF at 0 °C, was added sodium hydride [60% dispersion in mineral oil] (10 equiv.). The mixture was stirred at 0 °C for 15 min. To this mixture, was added iodomethane (10 equiv.). The mixture was removed from the ice bath and stirred at 23 °C for 24 h. The mixture was diluted in ethyl acetate and washed with IN HCl solution. The organic layer was dried, filtered and evaporated to give a crude oil. The oil was purified by column chromatography (0 to 30% ethyl acetate in hexanes) to deliver the desired intermediate, (*S*)-2-(((tert-butoxycarbonyl)(methyl)amino)methyl)-3-methylbutanoic acid (186 mg, 25% yield) as a yellow oil. ¹H NMR (500 MHz, CDCl₃) δ ppm 3.50 - 3.61 (m, 1 H) 3.29 - 3.41 (m, 1 H) 2.87 (s, 3 H) 2.47 - 2.68 (m, 1 H) 1.91 (d, 1 H) 1.46 (s, 9 H) 0.96 - 1.06 (m, 6 H).

### Step 3: Preparation of Compound 73 and Compound 74

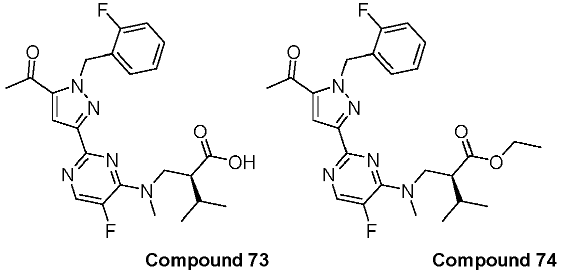

A mixture containing a 1.25M solution of HCl in ethanol (10 equiv.) and (*S*)-2-(((*tert*-butoxycarbonyl)(methyl)amino)methyl)-3-methylbutanoic acid (1 equiv.) was stirred at 23 °C for 24 h. The mixture was concentrated under vacuum. To this mixture were added triethylamine (3 equiv.), 1-(3-(4-chloro-5-fluoropyrimidin-2-yl)-1-(2-fluorobenzyl)-1H-pyrazol-5-yl)ethanone (**Intermediate-1B,** 1 equiv.), 1,4-dioxane and water (3:1). The mixture was heated to 40 °C for 4 h. The mixture was diluted in ethyl acetate and washed with IN HCl solution. The organic layer was dried, filtered and evaporated to give a crude oil. The oil was purified by column chromatography (0 to 100% ethyl acetate in hexanes) to deliver two products, **Compound 73** (5 mg, 4% yield) as a white solid and **Compound 74** (44 mg, 36% yield) as a light yellow oil.
¹H NMR for **Compound 73** (500 MHz, CD₃OD) δ ppm 8.12 (d, 1 H) 7.65 (s, 1 H), 7.24 - 7.32 (m, 1 H), 7.10 (s, 1 H), 7.04 (t, 1 H), 6.82 (t, 1 H), 5.85 - 5.99 (m, 2 H), 4.17 (dd, 1 H), 3.84 (dd, 1 H), 3.36 (d, 3 H), 2.66 - 2.74 (m, 1 H), 2.55 (s, 3 H), 1.90 - 1.99 (m, 1 H), 0.99 - 1.15 (m, 6 H).
¹H NMR for **Compound 74** (500 MHz, CD₃OD) δ ppm 8.12 (d, 1 H), 7.64 (s, 1 H), 7.23 - 7.32 (m, 1 H), 7.08 - 7.14 (m, 1 H), 7.04 (t, 1 H), 6.83 (t, 1 H), 5.90 (s, 2 H), 3.99 - 4.10 (m, 3 H), 3.91 (dd, 1 H), 3.31 (d, 3 H), 2.68 - 2.77 (m, 1 H), 2.56 (s, 3 H), 1.90 - 2.03 (m, 1 H), 1.07 - 1.16 (m, 6H), 0.99 (d, 3 H).

### Reference Compound 4

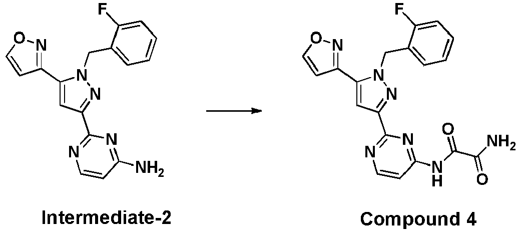

To a 23 °C suspension of **Intermediate-2** (1 equiv.) in dichloromethane was added a 2M in dichloromethane solution of oxalyl chloride (3 equiv.) followed by triethylamine (3 equiv.). After 10 min, the reaction mixture was concentrated in vacuo, reconstituted in tetrahydrofuran, and treated with saturated ammonium hydroxide solution. The reaction mixture turned gold in color, after which the reaction was acidified with IN hydrochloric acid solution and extracted with ethyl acetate. The combined organic layers were washed with IN hydrochloric acid solution, dried over sodium sulfate, filtered and concentrated in vacuo. The crude material was purified via reverse phase HPLC utilizing a 5-95% acetonitrile in water gradient to deliver the desired compound, **Compound 4** (2.3 mg, 4 % yield) as a white solid. ¹H-NMR (500 MHz, DMSO-*d*₆) δ ppm 10.19 (s, 1 H), 9.07 (d, 1 H), 8.82 (d, 1 H), 8.52 (s, 1 H), 8.17 (s, 1 H), 7.95 (d, 1 H), 7.69 (s, 1 H), 7.28-7.32 (m, 1 H), 7.24 (d, 1 H), 7.18-7.22 (m, 1 H), 7.07-7.10 (m, 1 H), 6.85-6.88 (m, 1 H), 5.91 (s, 2 H).

### Reference Compound 17

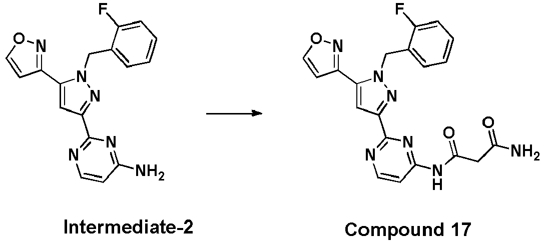

To a room temperature solution of **Intermediate-2** (1 equiv.) in dichloromethane was added methyl 3-chloro-3-oxopropanoate (1.15 equiv.) followed by triethylamine (1.5 equiv.). After stirring for 1 h at 23 °C, the reaction mixture was poured into IN hydrochloric acid solution and extracted with dichloromethane, followed by ethyl acetate. The combined organic layers were dried over sodium sulfate, filtered and concentrated in vacuo to afford a solid. The crude material was purified via silica gel chromatography using a 1-8 % methanol in dichloromethane gradient to deliver the desired intermediate, methyl 3-((2-(1-(2-fluorobenzyl)-5-(isoxazol-3-yl)-1H-pyrazol-3-yl)pyrimidin-4-yl)amino)-3-oxopropanoate (173 mg, 65 % yield) as an off-white solid.
To a suspension of methyl 3-((2-(1-(2-fluorobenzyl)-5-(isoxazol-3-yl)-1H-pyrazol-3-yl) pyrimidin-4-yl)amino)-3-oxopropanoate (1 equiv.) in methanol was added a 7M in methanol solution of ammonia (7 equiv.). The reaction mixture was stirred at room temperature for 12 h, after which the reaction was diluted in water then filtered to afford an off-white solid. The resulting solid was dissolved in ethyl acetate and washed with IN hydrochloric acid solution. The organic layer was dried over sodium sulfate, filtered, and concentrated in vacuo. The crude material was purified via reverse phase HPLC utilizing a 5-95 % acetonitrile in water gradient to deliver the desired compound (4.1 mg, 2 % yield) as a white solid. ¹H-NMR (500 MHz, CD₃OD) δ ppm 8.78 (d, 1 H), 8.69 (d, 1 H), 8.13 (d, 1 H), 7.54 (s, 1 H), 7.26-7.31 (m, 1 H), 7.06-7.12 (m, 1 H), 7.03-7.06 (m, 1 H), 6.87-6.92 (m, 2H, 2 overlapping shifts), 5.98 (s, 2 H), 3.31 (s, 2 H, isochronous with CD₃OD peak).

### Reference Compound 27

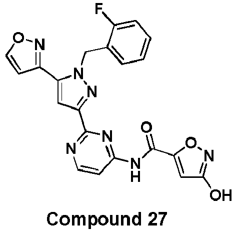

The title compound was prepared from **Intermediate-2** following general procedure C, except 3-hydroxyisoxazole-5-carboxylic acid (1.3 equiv.) was the acid reactant, 2.5 equivalents of T3P was used, and ethyl acetate was used for extraction during workup. The crude material was purified via reverse phase HPLC utilizing a 5-95% acetonitrile in water gradient to deliver the desired compound (5.8 mg, 5% yield) as a white solid. ¹H-NMR (500 MHz, DMSO-*d*₆) δ ppm 11.90 (br. s, 1 H), 11.65 (s, 1 H), 9.12 (d, 1 H), 8.84 (d, 1 H), 8.09 (d, 1 H), 7.71 (s, 1 H), 7.32-7.36 (m, 1 H), 7.28 (d, 1 H), 7.22-7.26 (m, 1 H), 7.21 (s, 1 H), 7.10-7.13 (m, 1 H), 6.86-6.90 (m, 1 H), 5.94 (s, 2 H).

### Reference Compound 45

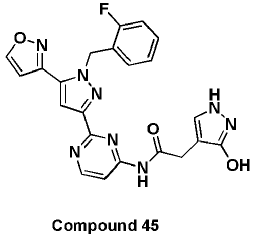

To a room temperature suspension of 2-(3-hydroxy-1H-pyrazol-4-yl)acetic acid (1 equiv.) in dichloromethane was added acetic anhydride (2 equiv.) followed by triethylamine (2 equiv.). The reaction mixture was stirred for 1 h, after which **Intermediate-2** (1 equiv.), additional triethylamine (2 equiv.), and a 50% in ethyl acetate solution of 2,4,6-tripropyl-1,3,5,2,4,6-trioxatriphosphinane 2,4,6-trioxide (1.15 equiv.) were added. The reaction was stirred at 50 °C for 6 h, then stirred at room temperature for an additional 12 h, after which the reaction was diluted in IN hydrochloric acid solution, and extracted with dichloromethane. The combined organic layers were dried over sodium sulfate, filtered and concentrated in vacuo. The crude material was purified via silica gel chromatography utilizing a 3-30% gradient of a 7:1 acetonitrile/methanol solution in dichloromethane, followed by a switch to a 15% methanol in dichloromethane gradient to deliver a mixture of the desired intermediate, 4-(2-((2-(1-(2-fluorobenzyl)-5-(isoxazol-3-yl)-1H-pyrazol-3-yl) pyrimidin-4-yl)amino)-2-oxoethyl)-1H-pyrazol-3-yl acetate, and a close running impurity, 1-acetyl-4-(2-((2-(1-(2-fluorobenzyl)-5-(isoxazol-3-yl)-1H-pyrazol-3-yl)pyrimidin-4-yl)amino)-2-oxo ethyl)-1H-pyrazol-3-yl acetate (32.1 mg, 33 % yield) as an off-white solid.
To this crude mixture of acetates (1 equiv.) in tetrahydrofuran and water was added a 1M aqueous solution of sodium hydroxide (2 equiv.). The reaction mixture was stirred for 24 h at 23 °C, after which additional sodium hydroxide (2 equiv.) was added. After 36 h of stirring, additional sodium hydroxide solution (2 equiv.) was added. After 40 h, the reaction mixture was concentrated in vacuo to remove the tetrahydrofuran and acidified by the addition of 1M hydrochloric acid solution, leading to the formation of a precipitate. This solid was filtered and dried to deliver the desired compound (12.9 mg, 44 % yield) as an off-white solid. ¹H-NMR (500 MHz, CD₃OD) δ ppm 8.78 (s, 1 H), 8.67 (d, 1 H), 8.12 (d, 1 H), 7.54 (s, 1 H), 7.45 (s, 1 H), 7.26-7.30 (m, 1 H), 7.08-7.12 (m, 1 H), 7.03-7.07 (m, 1 H), 6.86-6.90 (m, 2 H, 2 overlapping shifts), 5.97 (s, 2 H), 3.58 (s, 2 H).

### Reference Compound 48

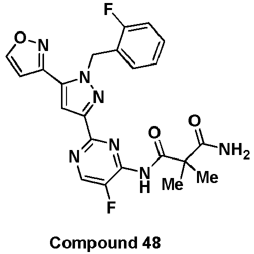

To a suspension of 2,2-dimethylmalonamide (1 equiv.) in tetrahydrofuran was added a 1M in tetrahydrofuran solution of potassium bis(trimethylsilyl)amide (1 equiv.). The reaction was stirred for 30 min then concentrated in vacuo to afford a tan solid. To this solid was added a solution of **Intermediate-1A** in dimethyl sulfoxide, and the reaction was stirred at 23 °C. After 10 min, the reaction was diluted in 3M hydrochloric acid solution, filtered, and dried to afford a solid. The crude material was purified via reverse phase HPLC using a 10-95% acetonitrile in water gradient to afford a mixture of two compounds. This mixture was further purified via silica gel chromatography utilizing a 3-8% methanol in dichloromethane gradient to deliver the desired compound (2.2 mg, 4 % yield) as a white solid. ¹H-NMR (500 MHz, CDCl₃) δ ppm 10.35 (br. s, 1 H), 8.55 (d, 1 H), 7.94 (d, 1 H), 7.30-7.34 (m, 1 H), 7.26 (s, 1 H), 7.08-7.11 (m, 3 H), 6.63 (d, 1 H), 5.91 (s, 2 H), 1.26 (s, 6 H); 2 N-H protons not observed.

### Reference Compound 66

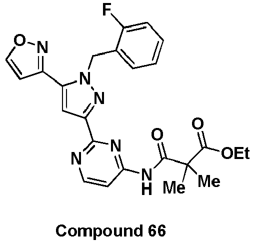

The title compound was prepared from **Intermediate-2** following general procedure C, except 3-ethoxy-2,2-dimethyl-3-oxopropanoic acid (1 equiv.) was the acid reactant, 1.5 equivalents of T3P was used, and dichloromethane was for extraction during workup. The crude material via silica gel chromatography utilizing a 3-10% methanol in dichloromethane gradient to deliver the desired compound (54.0 mg, 42 % yield) as a gummy solid. ¹H-NMR (400 MHz, CDCl₃) δ ppm 8.92 (br. s, 1 H), 8.74 (d, 1 H), 8.47 (s, 1 H), 8.13 (d, 1 H), 7.47 (s, 1 H), 7.27 (s, 1 H), 7.18-7.24 (m, 1 H), 7.02-7.06 (m, 1 H), 6.96-7.01 (m, 1 H), 6.80-6.85 (m, 1 H), 6.04 (s, 2 H), 4.26 (q, 2 H), 1.58 (s, 6 H), 1.30 (t, 3 H).

### Reference Compound 49

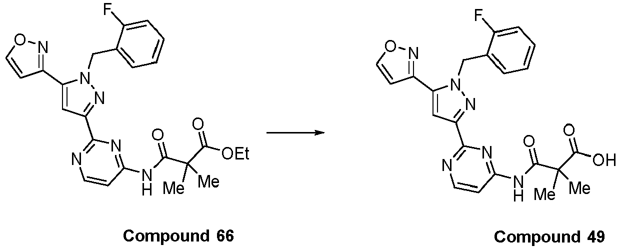

To a solution of **Compound 66** (1 equiv.) in tetrahydrofuran and water was added a 1M aqueous solution of sodium hydroxide (1.08 equiv.). The reaction was stirred at 23 °C for 1.5 h, after which the reaction mixture was concentrated to remove the tetrahydrofuran, then acidified by the addition of 1M aqueous hydrochloric acid solution. The resulting precipitate was filtered and dried to deliver the desired compound (36.2 mg, 75 % yield) as a light-tan solid. ¹H-NMR (500 MHz, DMSO-*d₆*) δ ppm 10.84 (s, 1 H), 9.11 (d, 1 H), 8.74 (d, 1 H), 8.01 (d, 1 H), 7.67 (s, 1 H), 7.31-7.36 (m, 1 H), 7.27 (d, 1 H), 7.21-7.25 (m, 1 H), 7.10-7.13 (m, 1 H), 6.83-6.86 (m, 1 H), 5.93 (s, 2 H), 1.44 (s, 6 H); 1 N-H proton not observed.

### Reference Compound 51

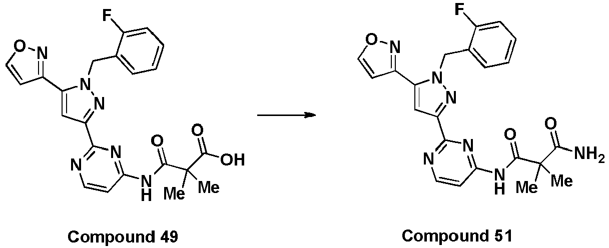

To a 0 °C solution of **Compound 49** (1 equiv.) in dichloromethane was added a 2M in dichloromethane solution of oxalyl chloride (2.5 equiv.). The reaction was stirred at 0 °C for 15 min, then warmed to 23 °C. After 1 h, the reaction mixture was concentrated in vacuo, reconstituted in tetrahydrofuran, and treated with a saturated ammonium hydroxide solution. After 2 h, the reaction was diluted in water, extracted with ethyl acetate, dried over sodium sulfate, filtered and concentrated in vacuo. The crude material was purified via reverse phase HPLC utilizing a 5-95% acetonitrile in water gradient to deliver the desired compound (9.2 mg, 29 % yield) as an off-white solid. ¹H-NMR (500 MHz, CDCl₃) δ ppm 9.63 (br. s, 1 H), 8.79 (d, 1 H), 8.49 (s, 1 H), 8.13 (s, 1 H), 7.49 (s, 1 H), 7.20-7.25 (m, 1 H), 7.02-7.06 (m, 1 H), 6.98-7.02 (m, 1 H), 6.88-6.93 (m, 1 H), 6.64 (d, 1 H), 6.21 (br. s, 1 H), 6.03 (s, 2 H), 5.78 (br. s, 1 H), 1.26 (s, 6 H).

### Reference Compound 35

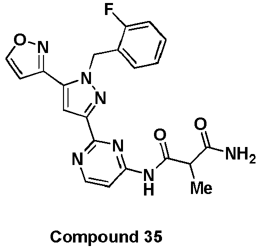

To a 0 °C suspension of 2-methylmalonic acid (1.0 equiv.) in dichloromethane was added oxalyl chloride (2.9 equiv.) followed by 3 drops of *N*,*N*-dimethylformamide. The reaction was stirred for 1 h at 0 °C, after which the reaction was warmed to 23 °C and stirred for 30 min. The reaction mixture was concentrated in vacuo to afford a brown oil which was used in the subsequent step without purification.

To a suspension of **Intermediate-2** (1 equiv.) in dichloromethane was added 2-methylmalonyl dichloride (1.5 equiv.), followed by triethylamine (1.1 equiv.). The reaction was allowed to stir for 2 h, after which the reaction mixture was quenched by the addition of saturated ammonium hydroxide. After stirring for an additional 2 h, the reaction mixture was diluted in 1M hydrochloric acid solution, extracted with ethyl acetate, dried over sodium sulfate, filtered, and concentrated in vacuo. The crude material was purified via reverse phase HPLC utilizing a 10-90% acetonitrile in water gradient to deliver the desired compound (3.3 mg, 3 % yield) as a white solid. ¹H-NMR (500 MHz, CDCl₃) δ ppm 9.66 (br. s, 1 H), 8.77 (d, 1 H), 8.50 (d, 1 H), 8.11 (d, 1 H), 7.48 (s, 1 H), 7.21-7.26 (m, 1 H), 7.03-7.08 (m, 1 H), 6.99-7.03 (m, 1 H), 6.90-6.93 (m, 1 H), 6.64 (d, 1 H), 6.36 (br. s, 1 H), 6.13 (br. s, 1 H), 6.02 (s, 2 H), 3.44 (q, 1 H), 1.61 (d, 3 H).

### Reference Compound 36

The title compound was synthesized in 3 steps:

### Step 1: Synthesis of methyl 3-((2-(1-(2-fluorobenzyl)-5-(isoxazol-3-yl)-1H-pyrazol-3-yl) pyrimidin-4-yl)amino)-2-methyl-3-oxopropanoate

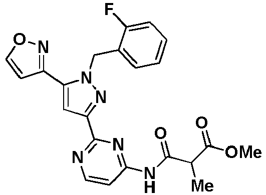

To a solution of **Intermediate-2** (1 equiv.) and 3-methoxy-2-methyl-3-oxopropanoic acid (1.3 equiv.) in *N*,*N*-dimethylformamide was added triethylamine (4 equiv.) followed by a 50% in ethyl acetate solution of 1-propanephosphonic acid cyclic anhydride (2.5 equiv.). After 24 h, the reaction mixture was diluted in water leading to the formation of a tan precipitate. The solid was filtered and dried to afford the desired intermediate, methyl 3-((2-(1-(2-fluorobenzyl)-5-(isoxazol-3-yl)-1H-pyrazol-3-yl)pyrimidin-4-yl)amino)-2-methyl-3-oxopropanoate (246 mg, 93 % yield) as a tan solid. ¹H-NMR (500 MHz, CDCl₃) δ ppm 8.91 (br. s, 1 H), 8.75 (d, 1 H), 8.48 (d, 1 H), 8.10 (d, 1 H), 7.47 (s, 1 H), 7.19-7.23 (m, 1 H), 7.03-7.07 (m, 1 H), 6.96-7.00 (m, 1 H), 6.81-6.84 (m, 1 H), 6.62 (d, 1 H), 6.04 (s, 2 H), 3.81 (s, 3 H), 3.52 (q, 1 H), 1.56 (d, 3 H).

### Step 2: Synthesis of 1-((2-(1-(2-fluorobenzyl)-5-(isoxazol-3-yl)-1H-pyrazol-3-yl) pyrimidin-4-yl)amino)-3-methoxy-2-methyl-1,3-dioxopropan-2-yl benzoate

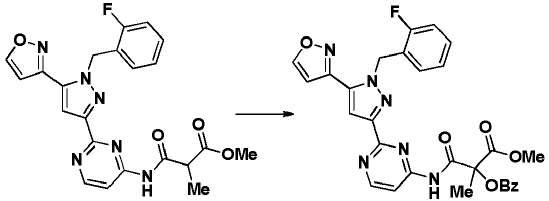

To a 0°C solution of methyl 3-((2-(1-(2-fluorobenzyl)-5-(isoxazol-3-yl)-1H-pyrazol-3-yl)--pyrimidin-4-yl)amino)-2-methyl-3-oxopropanoate (1 equiv.) in tetrahydrofuran was added a 1 M in tetrahydrofuran solution of sodium bis(trimethylsilyl)amide (1 equiv.). After 20 min of stirring at 0 °C, a solution of benzoyl peroxide (1.05 equiv.) in tetrahydrofuran was added. The reaction mixture was allowed to warm to 23 °C and stirred for 12 h. The reaction mixture was then diluted in saturated ammonium chloride solution, extracted with ethyl acetate, dried over sodium sulfate, filtered and concentrated in vacuo. The crude material was purified via reverse phase HPLC utilizing a 5-95% acetonitrile in water gradient to deliver the desired intermediate, 1-((2-(1-(2-fluorobenzyl)-5-(isoxazol-3-yl)-1H-pyrazol-3-yl)pyrimidin-4-yl)amino)-3-methoxy-2-methyl-1,3-dioxopropan-2-yl benzoate (48.3 mg, 73 % yield) as an off-white solid. ¹H-NMR (500 MHz, CDCl₃) δ ppm 11.23 (br. s, 1 H), 10.59 (s, 1 H), 8.84 (d, 1 H), 8.51 (d, 1 H), 8.21 (d, 1 H), 8.13 (d, 1 H), 7.64-7.68 (m, 1 H), 7.50-7.54 (m, 3 H), 7.21-7.26 (m, 1 H), 7.01-7.06 (m, 3 H), 6.65 (m, 1 H), 6.00 (s, 2 H), 3.87 (s, 3 H), 2.01 (s, 3 H).

### Step 3: Synthesis of Compound 36

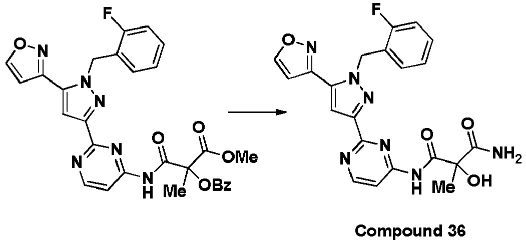

A solution of 1-((2-(1-(2-fluorobenzyl)-5-(isoxazol-3-yl)-1H-pyrazol-3-yl) pyrimidin-4-yl)amino)-3-methoxy-2-methyl-1,3-dioxopropan-2-yl benzoate (1 equiv.) in 1:1 tetrahydrofuran / saturated ammonium hydroxide solution was stirred at 23 °C. After 3 h, the reaction mixture was diluted with water, acidified by the addition of IN hydrochloric acid solution, extracted with ethyl acetate, dried over sodium sulfate, filtered, and concentrated in vacuo. The crude material was purified via reverse phase HPLC utilizing a 5-95% acetonitrile in water gradient to deliver the desired compound (13 mg, 45 % yield) as a white solid. ¹H-NMR (500 MHz, CD₃OD) δ ppm 8.79 (s, 1 H), 8.74 (d, 1 H), 8.17 (d, 1 H), 7.57 (s, 1 H), 7.27-7.31 (m, 1 H), 7.09-7.12 (m, 1 H), 7.04-7.07 (m, 1 H), 6.92 (s, 1 H), 6.89-6.92 (m, 1 H), 6.00 (s, 2 H), 1.70 (s, 3 H).

### Reference Compound 38 and Reference Compound 39

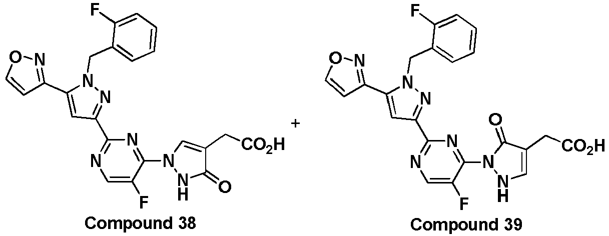

To a mixture of **Intermediate-IA** (1 equiv.) and 2-(3-oxo-2,3-dihydro-1H-pyrazol-4-yl)acetic acid (1.2 equiv.) in 1,4-dioxane was added triethylamine (4 equiv.). The reaction mixture was heated to 70 °C for 88 h, after which the reaction mixture was diluted with water and IN hydrochloric acid solution, and extracted with ethyl acetate. The combined organic layers were dried over sodium sulfate, filtered, and concentrated in vacuo. The crude material was purified via reverse phase HPLC utilizing a 5-95% acetonitrile in water gradient to deliver the desired compounds, **Compound 38** (20.5 mg, 25 % yield), and **Compound 39** (6.1 mg, 8% yield) as white solids.
¹H-NMR for **Compound 38** (500 MHz, DMSO-*d₆*) δ ppm 12.43 (s, 1 H), 11.24 (s, 1 H), 9.12 (d, 1 H), 8.89 (d, 1 H), 8.51 (s, 1 H), 7.74 (s, 1 H), 7.32-7.36 (m, 1 H), 7.27 (d, 1 H), 7.21-7.26 (m, 1 H), 7.10-7.14 (m, 1 H), 6.86-6.90 (m, 1 H), 5.95 (s, 2 H), 3.42 (s, 2 H).
¹H-NMR for **Compound 39** (500 MHz, CD₃OD) δ ppm 8.74 (d, 1 H), 8.68 (d, 1 H), 7.72 (s, 1 H), 7.24-7.28 (m, 1 H), 7.21 (s, 1 H), 7.06-7.10 (m, 1 H), 7.01-7.06 (m, 1 H), 6.83-6.86 (m, 2 H, 2 overlapping shifts), 5.92 (s, 2 H), 3.44 (s, 2 H).

### Reference Compound 40

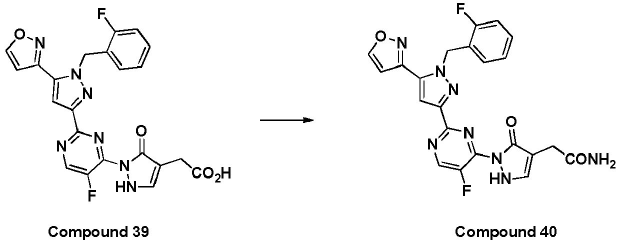

To a suspension of **Compound 39** (1 equiv.) in dichloromethane was added HATU (1.2 equiv.), *N*-ethyl-*N*-isopropylpropan-2-amine (3 equiv.), and a 0.5 M in dioxane solution of ammonia (6 equiv.). After 2 h, the reaction mixture was concentrated in vacuo. The crude material was purified via reverse phase HPLC utilizing a 5-95% acetonitrile in water gradient to deliver the desired compound, **Compound 40** (0.7 mg, 8 % yield) as a white solid. ¹H-NMR (500 MHz, CD₃OD) δ ppm 8.74 (s, 1 H), 8.69 (br. s, 1 H), 7.72 (s, 1 H), 7.26-7.29 (m, 1 H), 7.25 (s, 1 H), 7.05-7.10 (m, 1 H), 7.01-7.05 (m, 1 H), 6.87 (s, 1 H), 6.83-6.86 (m, 1 H), 5.92 (s, 2 H), 3.34 (s, 2 H).

### Reference Compound 56 and Reference Compound 57

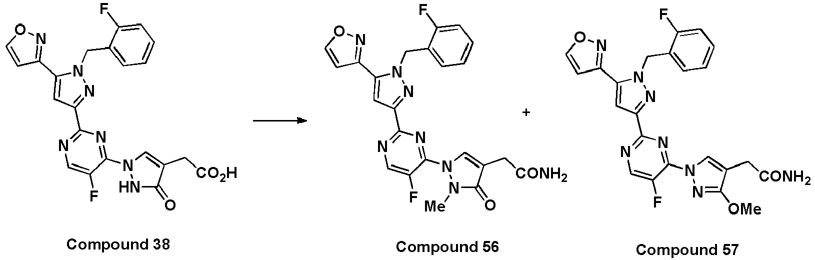

To a suspension of **Compound 38** (1 equiv.) in 1:1 diethyl ether/methanol was added a 2M in diethyl ether solution of trimethylsilyldiazomethane (1.5 equiv.). The reaction mixture was concentrated in vacuo to afford a mixture of esters (19.2 mg, 0.038 mmol) as a yellow residue, which was used in the next step without purification.
To this crude mixture of esters (1 equiv.) was added a 7M in methanol solution of ammonia (222 equiv.). The reaction mixture was stirred at room temperature for 12 h, after which the reaction mixture was concentrated in vacuo. The crude material was purified via reverse phase HPLC utilizing a 5-95% acetonitrile in water gradient to deliver two desired compounds, **Compound 56** (2.9 mg, 16 % yield) and **Compound 57** (0.9 mg, 5 % yield) as white solids.
¹H-NMR for **Compound 56** (500 MHz, CD₃OD) δ ppm 8.79 (m, 2 H, 2 shifts overlapping), 8.28 (s, 1 H), 7.53 (s, 1 H), 7.26-7.30 (m, 1 H), 7.08-7.11 (m, 1 H), 7.03-7.06 (m, 1 H), 6.96 (s, 1 H), 6.90-6.94 (m, 1 H), 5.98 (s, 2 H), 3.72 (s, 3 H), 3.34 (s, 2 H).
¹H-NMR for **Compound 57** (500 MHz, CDCl₃) δ ppm 8.71 (d, 1 H), 8.56 (s, 1 H), 8.51 (s, 1 H), 7.44 (s, 1 H), 7.24-7.27 (m, 1 H), 7.05-7.08 (m, 1 H), 7.00-7.05 (m, 1 H), 6.88-6.93 (m, 1 H), 6.67 (s, 1 H), 6.04 (s, 2 H), 5.83 (br. s, 1 H), 5.43 (br. s, 1 H), 4.12 (s, 3 H), 3.47 (s, 2 H).

### Reference Compound 41

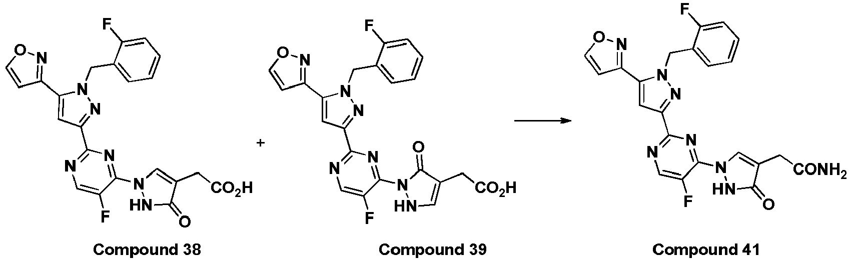

To a mixture of **Compound 38** and **Compound 39** (1 equiv.) in 1:1 dichloromethane/acetonitrile was added HATU (1.2 equiv.), *N*-ethyl-*N*-isopropylpropan-2-amine (3 equiv.), 4-dimethylaminopyridine (0.1 equiv.), and a 0.5 M in dioxane solution of ammonia (6 equiv.). After 12 h, the reaction mixture was diluted in water and 1M hydrochloric acid solution, extracted with ethyl acetate, dried over sodium sulfate, filtered, and concentrated in vacuo. Addition of methanol to this mixture afforded an off-white precipitate. The solid was further purified via reverse phase HPLC utilizing a 5-95% acetonitrile in water gradient to deliver the desired compound (4.1 mg, 3 % yield) as a white solid. ¹H-NMR (500 MHz, DMSO-*d₆*) δ ppm 11.28 (s, 1 H), 9.12 (s, 1 H), 8.88 (d, 1 H), 8.47 (s, 1 H), 7.73 (s, 1 H), 7.48 (br. s, 1 H), 7.30-7.37 (m, 1 H), 7.27 (s, 1 H), 7.20-7.25 (m, 1 H), 7.09-7.14 (m, 1 H), 7.02 (br. s, 1 H), 6.85-6.91 (m, 1 H), 5.95 (s, 2 H), 3.26 (s, 2 H).

### Reference Compound 59

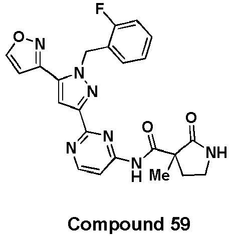

The title compound was prepared from **Intermediate-2** following general procedure C, except 3-methyl-2-oxopyrrolidine-3-carboxylic acid (1.05 equiv.) was the acid reactant, 1.5 equivalents of T3P was used, contents were stirred at 50 °C for 12 h then 80 °C for 36 h, and ethyl acetate was used for extraction during workup. The crude material was purified via reverse phase HPLC utilizing a 10-95 % acetonitrile in water gradient to deliver the desired compound (1.6 mg, 2 % yield) as an off-white solid. ¹H-NMR (500 MHz, CD₃OD) δ ppm 8.78 (d, 1 H), 8.71 (d, 1 H), 8.12 (d, 1 H), 7.56 (s, 1 H), 7.26-7.30 (m, 1 H), 7.08-7.12 (m, 1 H), 7.03-7.07 (m, 1 H), 6.93 (d, 1 H), 6.86-6.90 (m, 1 H), 5.99 (s, 2 H), 3.36-3.46 (m, 2 H), 2.72-2.78 (m, 1 H), 2.10-2.15 (m, 1 H), 1.58 (s, 3 H).

### Reference Compound 3

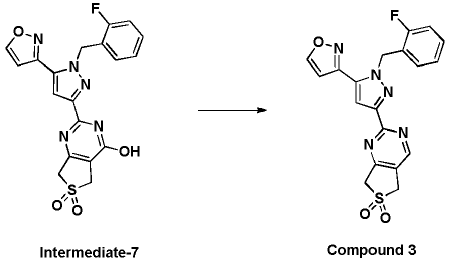

A suspension of **Intermediate-7** (1 equiv.) in phosphorus oxychloride (62 equiv.) was heated at 90 °C for 2 h, after which the reaction mixture was concentrated in vacuo to afford the desired chloro intermediate, 4-chloro-2-(1-(2-fluorobenzyl)-5-(isoxazol-3-yl)-1H-pyrazol-3-yl)-5,7-dihydrothieno[3,4-d]pyrimidine 6,6-dioxide (155 mg, 100% yield) as a tan solid. To a solution of this chloro intermediate (1 equiv.) in tetrahydrofuran was added acetic acid (2 equiv.) and zinc dust (2 equiv.). The resulting suspension was heated at 70 °C for 24 h, after which the reaction was filtered through celite, diluted in saturated ammonium chloride solution, and extracted with ethyl acetate. The combined organic layers were washed with water, dried over sodium sulfate, filtered, and concentrated in vacuo. The crude material was purified via reverse phase HPLC utilizing a 5-95% acetonitrile in water gradient to deliver the desired compound, **Compound 3** (4.0 mg, 4 % yield) as an off-white solid. ¹H-NMR (500 MHz, CDCl₃) δ ppm 8.80 (s, 1 H), 8.49 (d, 1 H), 7.49 (s, 1 H), 7.20-7.25 (m, 1 H), 7.03-7.07 (m, 1 H), 6.97-7.00 (m, 1 H), 6.83-6.86 (m, 1 H), 6.61 (d, 1 H), 6.05 (s, 2 H), 4.56 (s, 2 H), 4.51 (s, 2 H).

### Reference Compound 75

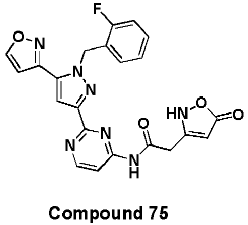

To a solution of 2-(5-hydroxyisoxazol-3-yl)acetic acid (2.0 equiv.) in acetonitrile was added acetic anhydride (2.0 equiv.) followed by triethylamine (2.0 equiv.). After 30 min, **Intermediate-2** (1.0 equiv.) was added, followed by additional triethylamine (2.0 equiv.), and a 50% in ethyl acetate solution of 1-propanephosphonic acid cyclic anhydride (2.0 equiv.). The reaction was stirred at room temperature for 24 h, after which the reaction mixture was diluted with water and extracted with ethyl acetate. The combined organic layers were dried over magnesium sulfate, filtered, and concentrated in vacuo. The crude material was purified via silica gel chromatography utilizing a 3-10% methanol in dichloromethane gradient to deliver the desired intermediate, 3-(2-((2-(1-(2-fluorobenzyl)-5-(isoxazol-3-yl)-1H-pyrazol-3-yl)pyrimidin-4-yl)amino)-2-oxoethyl)isoxazol-5-yl acetate (34.1 mg, 9 % yield) as a cream-colored solid.

To a solution of 3-(2-((2-(1-(2-fluorobenzyl)-5-(isoxazol-3-yl)-1H-pyrazol-3-yl) pyrimidin-4-yl)amino)-2-oxoethyl)isoxazol-5-yl acetate (1.0 equiv.) in tetrahydrofuran and water was added a 1M aqueous solution of sodium hydroxide (3.0 equiv.). After 5 min the reaction mixture was quenched by the addition of 1M hydrochloric acid solution and concentrated in vacuo. The crude material was purified via reverse phase HPLC utilizing a 10-95% acetonitrile in water gradient to deliver the desired compound (2.1 mg, 7 % yield) as a white solid. ¹H-NMR (500 MHz, Acetone-*d₆*) δ ppm 10.29 (s, 1 H), 8.92 (s, 1 H), 8.75 (d, 1 H), 8.03 (d, 1 H), 7.55 (d, 1 H), 7.32-7.37 (m, 1 H), 7.16-7.20 (m, 1 H), 7.09-7.13 (m, 2 H, 2 overlapping shifts), 6.94-6.98 (m, 1 H), 6.01 (s, 2 H), 4.00 (s, 2 H), 3.88 (s, 1H), 2.53 (s, 1H).

### Reference Compound 11

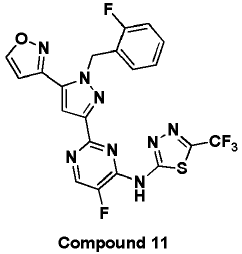

To a stirred solution of 5-(trifluoromethyl)-1,3,4-thiadiazol-2-amine (1 equiv.) and **Intermediate-1A** (1 equiv.) in DMF was added Cs₂CO₃ (3 equiv.), and the mixture was stirred at 90 °C for 24 h. Contents cooled to 23 °C and diluted with ethyl acetate. The mixture was washed with water and brine, concentrated in vacuo, and purified via reverse phase HPLC to deliver the desired compound, **Compound 11** (8 mg, 13 % yield) as a white solid. ¹H NMR (500 MHz, DMSO-*d*₆) δ ppm 9.15 - 9.17 (m, 1 H), 8.76 (d, 1 H), 7.64 (s, 1 H), 7.32 - 7.40 (m, 2 H), 7.30 (d, 1 H), 7.17 - 7.25 (m, 2 H), 7.09 - 7.15 (m, 1H), 5.95 (s, 2 H).

### Reference Compound 12

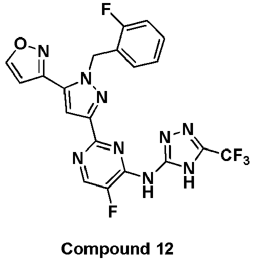

To a stirred solution of **Intermediate-IA** (1 equiv.) in DMF and Water (2:3) was added Cs₂CO₃ (2 equiv.), and the resulting mixture was stirred at 80 °C for 2 h. Contents cooled to 23 °C, and the solids were filtered and dried under high vacuum. The residue was then purified via silica gel chromatography (ethyl acetate in hexanes, 5-35% gradient), to deliver the desired compound (15 mg, 21 % yield) as a white solid. ¹H NMR (500 MHz, DMSO-*d*₆) δ ppm 9.11 - 9.16 (m, 2 H), 8.29 (s, 2 H), 7.73 (s, 1 H), 7.33 - 7.39 (m, 1 H), 7.32 (d, 1 H), 7.22 - 7.28 (m, 1 H), 7.15 (t, 1 H), 7.03 (t, 1 H), 5.94 (s, 2 H).

### Reference Compound 13

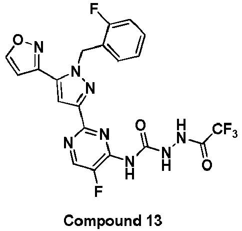

To a stirred solution of 5-(trifluoromethyl)-1,3,4-oxadiazol-2-amine (2 equiv.) in THF was added LiHMDS (2 equiv.), and the resulting mixture was stirred at 0 °C for 10 min. **Intermediate-1A** (1 equiv.) in THF was then introduced to the flask, and the mixture was stirred overnight. The solvent was removed in vacuo, and the resulting residue was purified via reverse phase HPLC to deliver the desired compound (5 mg, 7 % yield) as a white solid. ¹H NMR (500 MHz, CDCl₃) δ ppm 8.85 (br. s., 1 H), 8.56 (d, 1 H), 8.53 (d, 1 H), 7.25 - 7.34 (m, 4 H), 7.03 - 7.13 (m, 3 H), 6.63 (d, 1 H), 5.93 (s, 2 H).

### Reference Compound 14

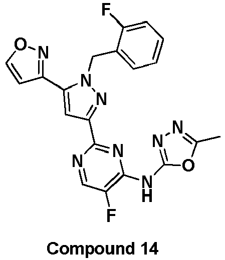

To a stirred solution of 5-methyl-1,3,4-oxadiazol-2-amine (2 equiv.) and **Intermediate-1A** (1 equiv.) in DMF was added Cs₂CO₃ (3 equiv.). The mixture was stirred at 80 °C for 4 h, then filtered. The filtrate was concentrated in vacuo, and the residue was purified via reverse phase HPLC to deliver the desired compound (22 mg, 36 % yield) as a brown solid. ¹H NMR (500 MHz, DMSO-*d*₆) δ ppm 9.12 (d, 1 H), 8.62 (br. s., 1 H), 7.56 (br. s., 1 H), 7.31 -7.38 (m, 1 H), 7.19 - 7.27 (m, 2 H), 7.12 (t, 1 H), 6.96 (t, 1 H), 5.92 (s, 2 H), 2.48 (s, 3 H).

### Reference Compound 15

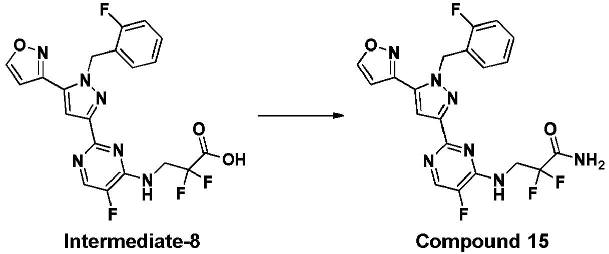

To a stirred solution of **Intermediate-8** (1 equiv.) in dichloromethane was added HATU (1.1 equiv.) and N-ethyl-N-isopropylpropan-2-amine (3 equiv.). The mixture was stirred for 15 min at 0 °C, then warmed to 23 °C and stirred for an additional 1 h. Ammonia (5 equiv.) was added to the reaction, and the mixture was stirred at 23 °C for 4 h. The reaction was diluted with saturated NH₄Cl solution and water, and extracted with ethyl acetate (3x). The organic layer was washed with brine (2x), dried over sodium sulfate, filtered and concentrated in vacuo. The resulting residue was purified via reverse phase HPLC to deliver the desired compound (16 mg, 30 % yield) as a white solid. ¹H NMR (500 MHz, CD₃OD) δ ppm 8.80 (d, 1 H), 8.31 (d, 1 H), 7.61 - 7.67 (m, 1 H), 7.24 - 7.34 (m, 1H), 7.02 - 7.14 (m, 2 H), 6.97 (d, 1 H), 6.92 (t, 1 H), 6.01 (s, 2 H), 4.42 (t, 2 H).

### Reference Compound 16

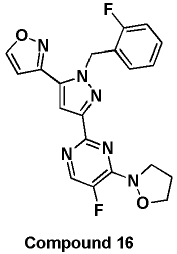

The title compound was prepared from **Intermediate-1A** following general procedure B, except isoxazolidine hydrochloride was the amine reactant, and the contents were heated at 110 °C for 24 h as a solution in dioxane/water (10:1). The reaction was cooled to 23 °C, the solvent was removed in vacuo, and the resulting residue was purified via reverse phase HPLC to deliver the desired compound (38 mg, 69 % yield) as a white solid. ¹H NMR (500 MHz, DMSO-*d*₆) δ ppm 9.03 (d, 1 H), 8.44 (d, 1 H), 7.54 (s, 1 H), 7.23 - 7.29 (m, 1 H), 7.20 (d, 1 H), 7.13 - 7.19 (m, 1 H), 7.04 (t, 1 H), 6.77 (t, 1 H), 5.86 (s, 2 H), 3.95 (t, 2 H), 3.83 -3.89 (m, 2 H), 2.23 (q, 2 H).

### Reference Compound 34

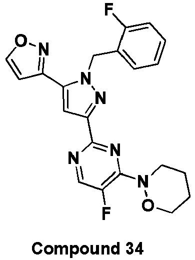

The title compound was prepared following general procedure B, except 1,2-oxazinane hydrochloride was the amine reactant, and the contents were heated at 110 °C for 24 h as a solution in dioxane/water (10:1). The contents were cooled to 23 °C, the solvent was removed in vacuo, and the resulting residue was purified via reverse phase HPLC to deliver the desired compound (36 mg, 63 % yield) as a white solid. ¹H NMR (400 MHz, DMSO-*d*₆) δ ppm 9.03 (d, 1 H), 8.41 (d, 1 H), 7.56 (s, 1 H), 7.22 - 7.30 (m, 1 H), 7.20 (d, 1 H), 7.15 (dd, 1 H), 7.00 - 7.07 (m, 1H), 6.77 (t, 1 H), 5.86 (s, 2 H), 4.02 (br. s., 2 H), 3.90 (br. s., 2 H), 1.73 (br. s., 4 H).

### Reference Compound 42

### Step 1: Synthesis of methyl 3,3,3-trifluoro-2-(((5-fluoro-2-(1-(2-fluorobenzyl)-5-(isoxazol-3-yl)-1H-pyrazol-3-yl) pyrimidin-4-yl) amino) methyl)-2-hydroxypropanoate

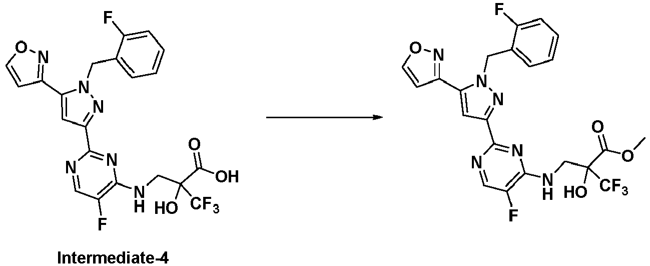

To a stirred suspension of **Intermediate-4** (1 equiv.) in THF was added (diazomethyl)trimethylsilane (2 equiv.), and the mixture was stirred at 80 °C for 4 h. Contents were cooled to 23 °C, and the solvent was removed in vacuo to deliver the desired intermediate, methyl 3,3,3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropa noate (80 mg, 97 % yield) as a brown solid.

### Step 2: Synthesis of 3,3,3-trifluoro-2-(((5-fluoro-2-(1-(2-fluorobenzyl)-5-(isoxazol-3-yl) -1H-pyrazol-3-yl)pyrimidin-4-yl)amino)methyl)-2-hydroxypropanehydrazide.

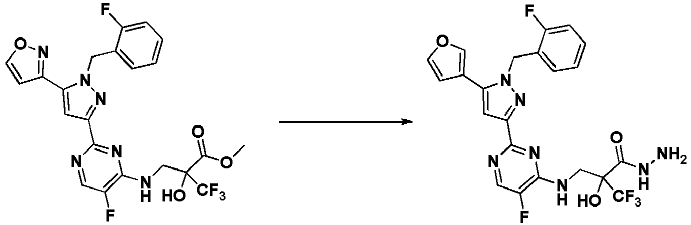

To a solution of methyl 3,3,3-trifluoro-2-(((5-fluoro-2-(1-(2-fluorobenzyl)-5-(isoxazol-3-yl)-1H-pyrazol-3-yl) pyrimidin-4-yl) amino) methyl)-2-hydroxypropanoate (1 equiv.) in ethanol was added hydrazine hydrate (15 equiv.), and the mixture was stirred overnight. Solvent was removed in vacuo, and the residue was triturated with hexane and filtered. The resulting solids were dried under high vacuum to deliver the desired intermediate 3,3,3-trifluoro-2-(((5-fluoro-2-(1-(2-fluorobenzyl)-5-(isoxazol-3-yl)-1H-pyrazol-3-yl)pyrimidin-4-yl)a mino)methyl)-2-hydroxypropanehydrazide (80 mg, 100 % yield) as a white solid.

### Step 3: Synthesis of 2-(3, 3, 3-trifluoro-2-(((5-fluoro-2-(1-(2-fluorobenzyl)-5-(isoxazol-3-yl)-1H-pyrazol-3-yl)pyrimidin-4-yl)amino)methyl)-2-hydroxypropanoyl)hydrazinecarbothioamide.

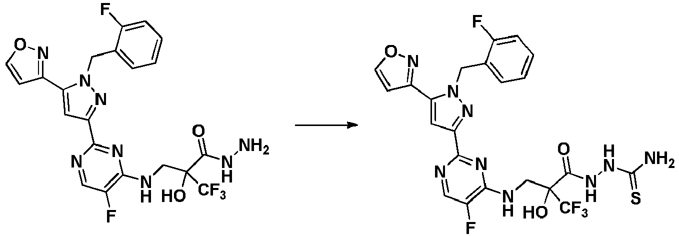

A solution of 3,3,3-trifluoro-2-(((5-fluoro-2-(1-(2-fluorobenzyl)-5-(isoxazol-3-yl)-1H-pyrazol-3-yl) pyrimidin-4-yl) amino) methyl)-2-hydroxypropanehydrazide (1 equiv.) in isopronaol was treated with isothiocyanatotrimethylsilane (2 equiv.). The mixture was heated at 90 °C for 3 h, cooled to 23 °C and filtered. The solid was collected and dried under high vacuum to deliver the desired intermediate, 2-(3,3,3-trifluoro-2-(((5-fluoro-2-(1-(2-fluorobenzyl)-5-(isoxazol-3-yl)-1H-pyrazol-3-yl)pyrimidin-4-yl)amino)-methyl)-2 -hydroxypropanoyl)hydrazinecarbothioamide (80 mg, 90 % yield) as a white solid.

### Step 4: Synthesis of Compound 42

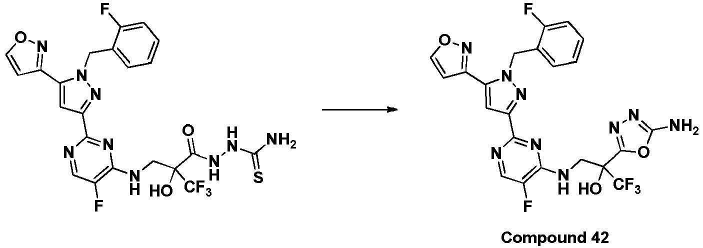

To a stirred solution of 2-(3, 3, 3-trifluoro-2-(((5-fluoro-2-(1-(2-fluorobenzyl)-5-(isoxazol-3-yl)-1H-pyrazol-3-yl)pyrimidin-4-yl)amino)methyl)-2-hy--droxypropanoyl) hydrazinecarbothioamide (1 equiv.) in THF was added 4-methylbenzene-1-sulfonyl chloride (1.5 equiv.) and pyridine (2 equiv.). The mixture was heated via microwave at 150 °C for 30 min. Solvent was removed, and the residue was purified via reverse phase HPLC to deliver the desired compound (5.5 mg, 28% yield) as a white solid. ¹H NMR (500 MHz, CD₃OD) δ ppm 8.71 (s, 1 H), 8.20 (d, 1 H), 7.35 (s, 1 H), 7.16 - 7.24 (m, 1 H), 6.93 - 7.05 (m, 2 H), 6.81 - 6.89 (m, 2 H), 5.90 (s, 2 H), 4.40 (d, 1 H), 4.21 (d, 1 H).

### Compound 43

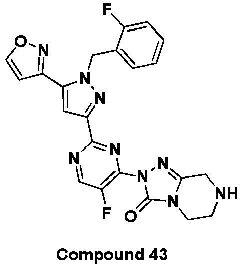

To a stirred solution of **Intermediate-IA** (1 equiv.) and 5,6,7,8-tetrahydro-[1,2,4]triazolo[4,3-a]pyrazin-3-ol 1 eq) in DMF was added Cs₂CO₃ (2 equiv.), and the mixture was stirred at 80 °C for 2 h. Contents cooled to 23 °C, filtered, and the filtrate was concentrated in vacuo. The residue was purified via reverse phase HPLC to deliver the desired compound (5 mg, 7 % yield) as a white solid. ¹H NMR (500 MHz, CD₃OD) δ ppm 8.95 (d, 1 H), 8.77 - 8.80 (m, 1 H), 7.58 (s, 1 H), 7.24 - 7.31 (m, 1 H), 7.07 - 7.13 (m, 1 H), 7.01 - 7.07 (m, 1 H), 6.85 - 6.91 (m, 2 H), 5.98 (s, 2 H), 4.48 (s, 2 H), 3.97 (t, 2 H), 3.69 - 3.75 (m, 2 H).

### Compound 44

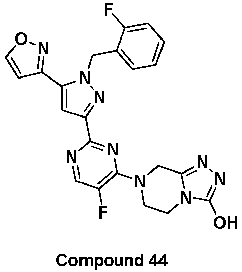

To a stirred solution of **Intermediate-IA** (1 equiv.) and 5,6,7,8-tetrahydro-[1,2,4]triazolo[4,3-a]pyrazin-3-ol (1 equiv.) in DMF was added Cs₂CO₃ (2 equiv.), and the mixture was stirred at 80 °C for 2 h. Contents cooled to 23 °C, filtered, and the filtrate was concentrated in vacuo. The residue was purified via reverse phase HPLC to deliver the desired compound (6 mg, 8 % yield) as a white solid. ¹H NMR (500 MHz, DMSO-*d*₆) δ ppm 11.64 (s, 1 H), 9.08 - 9.12 (m, 1 H), 8.43 (d, 1 H), 7.63 (s, 1 H), 7.30 - 7.36 (m, 1 H), 7.26 (d, 1 H), 7.19 - 7.25 (m, 1 H), 7.11 (t, 1 H), 6.84 (t, 1 H), 5.85 - 5.97 (m, 2 H), 4.90 (s, 2 H), 4.17 (t, 2 H), 3.68 (t, 2 H).

### Reference Compound 65

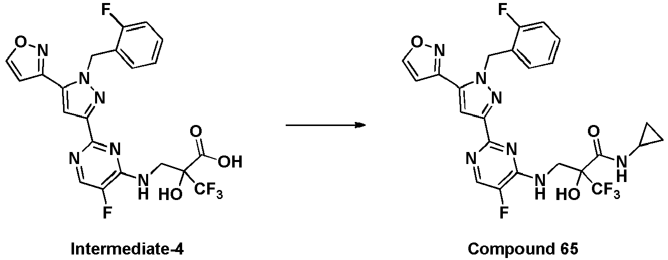

To a stirred solution of **Intermediate-4** (1 equiv.) in dichloromethane was added PyAOP (2 equiv.) and N-ethyl-N-isopropylpropan-2-amine (2 equiv.), and the mixture was stirred for 30 min. Cyclopropanamine (1.5 equiv.) was added to the reaction, and contents were stirred for another 24 h. The solvent was removed in vacuo, and the residue was purified by HPLC to deliver the desired compound (5 mg, 22% yield) as a white solid. ¹H NMR (500 MHz, CD₃OD) δ ppm 8.80 (d, 1 H), 8.25 (d, 1 H), 7.54 (s, 1 H), 7.26 - 7.33 (m, 1 H), 7.02 - 7.13 (m, 2 H), 6.92 - 6.97 (m, 2 H), 5.99 (s, 2 H), 4.35 (d, 1 H), 4.07 (d, 1 H), 2.61 (br, 1 H), 0.51 - 0.72 (m, 2 H), 0.29 - 0.49 (m, 2 H).

### Reference Compound 76

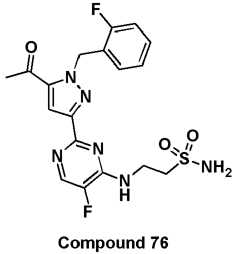

The title compound was prepared following general procedure B, except using **Intermediate-1B** (1 equiv) in place of **Intermediate-1A,** 2-aminoethanesulfonamide (1.5 equiv.) was the amine reactant, and contents were heated at 90 °C for 12 h as a solution in dioxane. The resulting crude material was purified via reverse phase HPLC to deliver the desired compound (12 mg, 48 % yield) as a white solid. ¹H NMR (500 MHz, DMSO-*d*₆) δ ppm 8.25 (d, 1 H), 7.99 (br. s., 1 H), 7.73 (s, 1 H), 7.30 - 7.37 (m, 1 H), 7.20 - 7.26 (m, 1 H), 7.11 (t, 1 H), 7.03 (s, 2 H), 6.84 (t, 1 H), 5.83 (s, 2 H), 3.84 - 3.91 (m, 2 H), 3.34 (t, 2 H), 2.56 (s, 3 H).

### Reference Compound 77

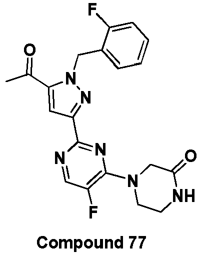

The title compound was prepared following general procedure B, except **Intermediate-1B,** (1 equiv.) was used in place of **Intermediate-1A,** piperazin-2-one (1.5 equiv.) was the amine reactant, and contents were heated at 90 °C for 12 h as a solution in dioxane. The resulting crude material was purified via reverse phase HPLC to deliver the desired compound (13 mg, 55 % yield) as a white solid. ¹H NMR (500 MHz, DMSO-*d*₆) δ ppm 8.36 (d, 1 H), 8.22 (br. s., 1 H), 7.75 (s, 1 H), 7.33 (q, 1 H), 7.19 - 7.26 (m, 1 H), 7.11 (t, 1 H), 6.78 - 6.84 (m, 1 H), 5.83 (s, 2 H), 4.33 (s, 2 H), 3.98 (t, 2 H), 3.35 (br. s, 2 H), 2.58 (s, 3 H).

### Reference Compound 78

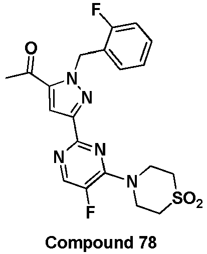

The title compound was prepared following general procedure B, except **Intermediate-1B** (1 equiv.) was used in place of **Intermediate-1A,** thiomorpholine 1,1-dioxide (1.5 equiv.) was the amine reactant, and contents were heated at 90 °C for 12 h as a solution in dioxane. The resulting crude material was purified via reverse phase HPLC to deliver the desired compound (11 mg, 43 % yield) as a white solid. ¹H NMR (500 MHz, DMSO-*d*₆) δ ppm 8.42 (d, 1 H), 7.76 (s, 1 H), 7.33 (q, 1 H), 7.19 - 7.26 (m, 1H), 7.11 (t, 1 H), 6.81 (t, 1 H), 5.83 (s, 2 H), 4.21 (br. s., 4 H), 3.33 (br. s., 4 H), 2.58 (s, 3 H).

### Reference Compound 79

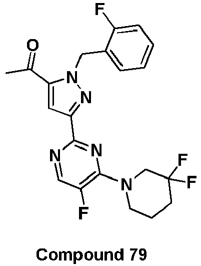

The title compound was prepared following general procedure B, except **Intermediate-1B** (1 equiv.) was used in place of **Intermediate-1A,** 3,3-difluoropiperidine (1.5 equiv.) was the amine reactant, and contents were heated at 90 °C for 12 h as a solution in dioxane. The resulting crude material was purified via reverse phase HPLC to deliver the desired compound (14 mg, 56 % yield) as a white solid. ¹H NMR (500 MHz, DMSO-*d*₆) δ ppm 8.38 (d, 1 H), 7.75 (s, 1 H), 7.33 (q, 1 H), 7.20 - 7.26 (m, 1H), 7.11 (t, 1 H), 6.82 (t, 1 H), 5.83 (s, 2 H), 4.14 (t, 2 H), 3.82 - 3.88 (m, 2 H), 2.58 (s, 3 H), 2.10 - 2.21 (m, 2 H), 1.81 (br. s., 2H).

### Reference Compound 80

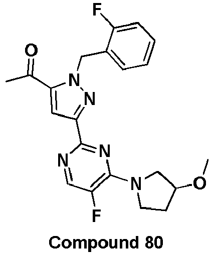

The title compound was prepared following general procedure B, except **Intermediate-1B** (1 equiv.) was used in place of **Intermediate-1A,** 3-methoxypyrrolidine (1.5 equiv.) was the amine reactant, and contents were heated at 90 °C for 12 h as a solution in dioxane. The resulting crude material was purified via reverse phase HPLC to deliver the desired compound (12 mg, 51 % yield) as a white solid. ¹H NMR (500 MHz, DMSO-*d*₆) δ ppm 8.27 (d, 1 H), 7.73 (s, 1 H), 7.30 - 7.37 (m, 1 H), 7.20 - 7.26 (m, 1 H), 7.09 - 7.14 (m, 1 H), 6.82 (t, 1 H), 5.83 (s, 2 H), 4.09 (br. s., 1 H), 3.64 - 3.91 (m, 4 H), 3.28 (s, 3 H), 2.58 (s, 3 H), 1.97 - 2.15 (m, 2 H).

### Reference Compound 1

The title compound was prepared in 5 steps:

### Step 1: Synthesis of ethyl 5-((bis-(tert-butoxycarbonyl))amino)-1-(2-fluorobenzyl)-1H-pyrazole-3-carboxylate

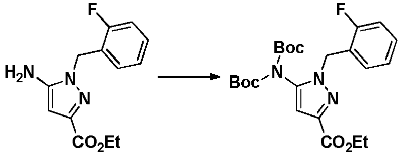

A solution of 4-dimethylaminopyridine (0.05 equiv.), ethyl 5-amino-1-(2-fluorobenzyl)-1H-pyrazole-3-carboxylate (1 equiv.), and triethylamine (2 equiv.) in tetrahydrofuran was treated with Boc anhydride (1.5 equiv.). After stirring for 96 h, the solution was poured into ethyl acetate and water. The layers were separated and the organic layer was washed with 5% potassium hydrogensulfate solution (3x), saturated sodium bicarbonate solution, and saturated aqueous sodium chloride. The solution was dried over magnesium sulfate, filtered, and the solvent was removed in vacuo. The crude material was combined with the crude product from a previous reaction and purification by silica gel chromatography (0-50% ethyl acetate in hexanes) to provide the desired intermediate, ethyl 5-((*bis*-(tert-butoxycarbonyl))amino)-1-(2-fluorobenzyl)-1H-pyrazole-3-carboxylate (236 mg, 63% combined yield) as an oil.

### Step 2: Synthesis of ethyl 5-((tert-butoxycarbonyl)amino)-1-(2-fluorobenzyl)-1H-pyrazole-3-carboxylate

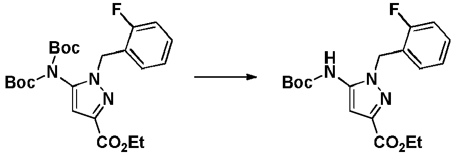

A suspension of potassium carbonate (3 equiv.) and ethyl 5-((*bis*-(tert-butoxycarbonyl))amino)- 1-(2-fluorobenzyl)-1H-pyrazole-3-carboxylate (1 equiv.) was heated at 60 °C in ethanol for 3 h. The solvent was removed in vacuo and the residue was partitioned between dichloromethane and saturated aqueous ammonium chloride(1:1). The layers were separated and the aqueous layer was extracted with dichloromethane (2x). The organics were dried over magnesium sulfate, filtered, and the solvent was removed in vacuo. Purification via silica gel chromatography (0-35% ethyl acetate in hexanes) delivered the desired intermediate, ethyl 5-((tert-butoxycarbonyl)amino)-1-(2-fluorobenzyl)-1H-pyrazole-3-carboxylate (137 mg, 79% yield) as a clear oil.

### Step 3: Synthesis of ethyl 5-((tert-butoxycarbonyl)(methyl)amino)-1-(2-fluorobenzyl)-1H-pyrazole-3-carboxylate

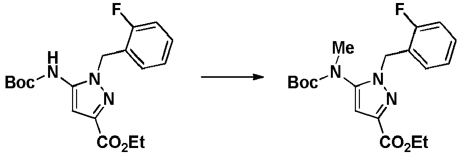

To a solution of ethyl 5-((tert-butoxycarbonyl)amino)-1-(2-fluorobenzyl)-1H--pyrazole-3-carboxylate (1 equiv.) in *N,N-*dimethylformamide at 0 °C was added sodium hydride (1.5 equiv.) as a 60% dispersion in mineral oil. After stirring for 15 min at 0 °C, iodomethane (2.5 equiv.) was added in a single portion. The solution was immediately warmed to 23 °C. After 30 min, the solution was cooled to 0 °C, and saturated aqueous ammonium chloride was added. The solution was then warmed to 23 °C and diluted with ethyl acetate and water (1:1). The layers were separated and the aqueous layer was extracted with ethyl acetate (2x). The organics were washed with water (3x) and saturated aqueous sodium chloride, dried over magnesium sulfate, filtered, and the solvent was removed *in vacuo.* The crude product was combined with the crude product from a previous reaction and purification by silica gel chromatography (ethyl acetate in hexanes) provided the desired intermediate, ethyl 5-((tert-butoxycarbonyl)(methyl)amino)-1-(2-fluorobenzyl)-1H-pyrazole-3-carboxylate (105 mg, 76% combined yield) as a clear oil.

### Step 4:

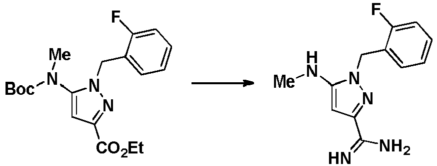

Synthesis of 1-(2-fluorobenzyl)-5-(methylamino)-1H-pyrazole-3-carboximidamide To a suspension of ammonium chloride (5.5 equiv.) in toluene was added trimethylaluminum (5 equiv.) as a 2 M solution in heptanes dropwise over 5 min. After stirring for 30 min, the bubbling had diminished and the aluminum reagent was added to ethyl 5-((tert-butoxycarbonyl)(methyl)amino)-1-(2-fluorobenzyl)-1H-pyrazole-3-carboxylate (1 equiv.) in toluene. The solution was heated to 90 °C and maintained at that temperature for 20 h. The solution was then cooled to 0 °C and methanol (10 equiv.) was added. The solution was immediately removed from the ice bath and warmed to 23 °C. After stirring for 30 min, the suspension was filtered through celite and washed with methanol to deliver the desired intermediate, 1-(2-fluorobenzyl)-5-(methylamino)-1H-pyrazole-3-carboximidamide (43 mg, 55% yield) as a white solid.

### Step 5: Synthesis of Compound 1

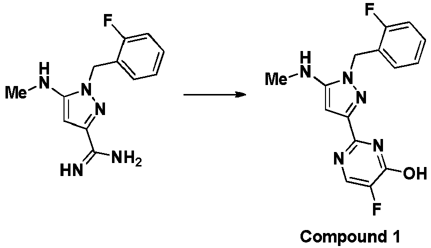

To a suspension of 1-(2-fluorobenzyl)-5-(methylamino)-1H-pyrazole-3-carboximidamide (1 equiv.) in ethanol was added sodium 3-ethoxy-2-fluoro-3-oxoprop-1-en-1-olate (3 equiv.). The solution was stirred at 90 °C in sealed vial for 16 h. 1,8-Diazabicyclo[5.4.0]undec-7-ene (200 µL) was then added, and the resulting solution was stirred for 17 h at 90 °C. The solvent was removed in vacuo and purification by silica gel chromatography (0-10% methanol in dichloromethane) delivered the desire compound, **Compound 1** (1.2 mg, 2% yield) as a yellow film. ¹H-NMR (500 MHz, CD₃OD) δ ppm 7.99 (br s, 1H), 7.35-7.31 (m, 1H), 7.16-7.11 (m, 2 H), 6.94-6.91 (m, 1 H), 6.10 (s, 1 H), 5.35 (s, 2 H), 2.84 (s, 3 H).

### Reference Compound 81

The title compound was synthesized in 3 steps:

### Step 1: Synthesis of ethyl 5-(dimethylamino)-1-(2-fluorobenzyl)-1H-pyrazole-3-carboxylate

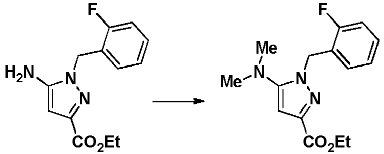

To a 0 °C solution of ethyl 5-amino-1-(2-fluorobenzyl)-1H-pyrazole-3-carboxylate (1 equiv.) in *N,N*-dimethylformamide was added sodium hydride (2.5 equiv.) as a 60% dispersion in mineral oil. The resulting suspension was stirred at 0 °C for 20 min and then iodomethane (3 equiv.) was added and the solution was warmed immediately to 23 °C. After stirring for 1.25 h, the solution was cooled to 0 °C and saturated ammonium chloride was added. After warming to 23 °C, the suspension was diluted with ethyl acetate and water (1:1). The layers were separated and the aqueous layer was extracted with ethyl acetate. The organics were washed with water (3x) and brine, dried over magnesium sulfate, filtered, and the solvent was removed in vacuo. The residue was purified via silica gel chromatography (0-20% ethyl acetate in hexanes) to deliver the desired intermediate, ethyl 5-(dimethylamino)-1-(2-fluorobenzyl)-1H-pyrazole--3-carboxylate (69 mg, 62% yield) as a clear oil.

### Step 2: Synthesis of 5-(dimethylamino)-1-(2-fluorobenzyl)-1H-pyrazole-3-carboximidamide

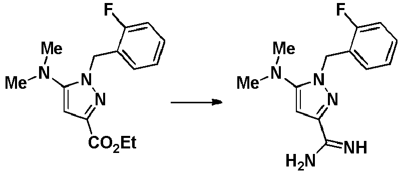

A suspension of ammonium chloride (5.5 equiv.) in toluene was treated with trimethylaluminum (5 equiv.) as a 2 M solution in heptane dropwise over 2 min. After stirring for 30 min ethyl 5-(dimethylamino)-1-(2-fluorobenzyl)-1H-pyrazole-3-carboxylate (1 equiv.) in toluene was added. The resulting solution was heated at 80 °C for 15 h and then cooled to 0 °C. Methanol (10 equiv.) was added and the reaction mixture was warmed to room temperature and stirred for 30 min. Additional methanol was added and the suspension was filtered through celite. The solvent was removed in vacuo to deliver the desired intermediate, 5-(dimethylamino)-1-(2-fluorobenzyl)-1H-pyrazole-3-carboximidamide (51 mg, 82 % yield) as a yellow solid.

### Step 3: Synthesis of Compound 81

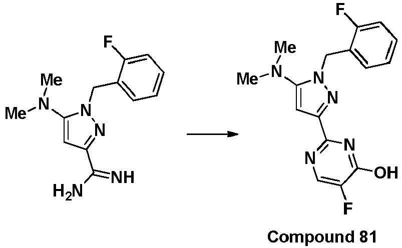

A suspension of sodium 3-ethoxy-2-fluoro-3-oxoprop-1-en-1-olate (3 equiv.), 5-(dimethylamino)-1-(2-fluorobenzyl)-1H-pyrazole-3-carboximidamide (1 equiv.), and 1,8-Diazabicyclo[5.4.0]undec-7-ene (1 equiv.) in ethanol was heated at 90 °C for 2.5 h. The solvent was then removed in vacuo and the resulting residue brought up in dichloromethane and filtered. The resulting solid was suspended in dichloromethane and re-filtered. The solute from both filtrations was combined and purified via column chromatography (methanol in dichloromethane) to deliver the desired compound (25 mg, 39% yield) as a yellow solid. ¹H-NMR (500 MHz, CD₃OD) δ ppm 8.00 (d, 1 H), 7.36-7.32 (m, 1 H), 7.16-7.09 (m, 3 H), 6.62 (s, 1 H), 5.45 (s, 2 H), 2.70 (s, 6 H).

### Reference Compound 50

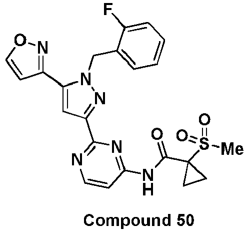

The title compound was prepared from **Intermediate-2** following general procedure C, except 1-(methylsulfonyl)cyclopropanecarboxylic acid (2 equiv.) was the acid reactant, 6 equivalents of triethylamine and 4 equivalents of propylphosphonic anhydride (T3P) was used, and the solution was heated to 65 °C for 2 h. The solution was poured into aqueous 1 N hydrochloric acid and dichloromethane (1:1). The layers were separated and the aqueous layer was extracted with dichloromethane (2x). The organics were dried over magnesium sulfate, filtered, and the solvent was removed *in vacuo.* Purification via silica gel chromatography (20-100% ethyl acetate in hexanes) delivered the desired compound (30 mg, 28% yield) as a white solid. ¹H-NMR (500 MHz, CDCl₃) δ ppm 8.81 (d, 1 H), 8.50 (d, 1 H), 8.05 (d, 1 H), 7.54 (s, 1 H), 7.25-7.21 (m, 1 H), 7.07-7.04 (m, 1 H), 7.01-6.98 (s, 1H), 6.90-6.87 (m, 1 H), 6.66 (d, 1 H), 6.05 (s, 2 H), 3.17 (s, 3 H), 1.89-1.83 (m, 4 H).

### Compound 54

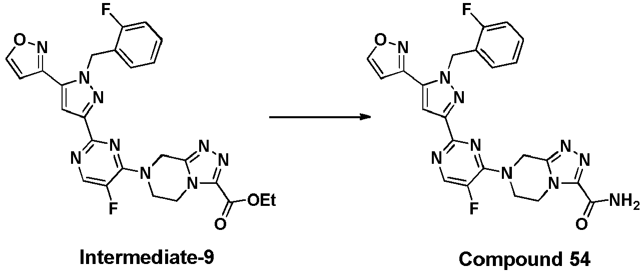

A solution of **Intermediate-9** (1 equiv.) in methanol was treated with ammonia (100 equiv.) as a 7 N solution in methanol and heated at 50 °C for 1.5 h. After storing overnight at 0 °C, additional 7 N methanolic ammonia (150 equiv.) was added and the solution was heated to 50 °C for 1 h. An additional 125 equiv. of methanolic ammonia was added and the resulting solution was heated at 50 °C for 2 h. After storing in the freezer for a second night, the suspension was filtered and washed with dichloromethane to deliver the desired compound (8 mg, 47% yield) as a white solid. ¹H-NMR (500 MHz, DMSO-*d*₆) δ ppm 9.11 (s, 1 H), 8.44 (d, 1 H), 8.26 (s, 1 H), 7.82 (s, 1 H), 7.66 (s, 1 H), 7.35-7.31 (m, 1 H), 7.28 (m, 1 H), 7.24-7.21 (m, 1 H), 7.11 (t, 1 H), 6.85 (t, 1 H), 5.92 (s, 2 H), 5.23 (s, 2 H), 4.48-4.46 (m, 2 H), 4.23-4.21 (m, 2 H).

### Reference Compound 19

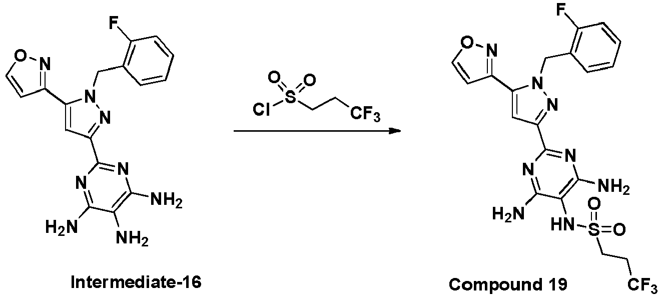

A mixture of **Intermediate-16** (1 equiv.) and 3,3,3-trifluoropropane-1-sulfonyl chloride (1.1 equiv.) in pyridine and dichloromethane (1 : 2) was stirred at 23 °C for 5 h. The reaction mixture was then added with 1 N NaOH and continued to stir for an additional 1.5 h, after which it was diluted with dichloromethane and water, then acidified to pH 3 with 1 N HCl solution. The phases were separated and the aqueous phase was extracted twice with dichloromethane. The combined organic phase was dried over anhydrous magnesium sulfate, filtered and concentrated. The crude material was purified via reverse phase HPLC (C18 column, 30 % to 60 % acetonitrile in water with 0.1 % TFA over 20 mins) to deliver the desired compound (11 mg, 24 % yield) as a yellow solid. ¹H NMR (500 MHz, DMSO-*d*₆) δ ppm 9.13 (d, 1 H), 8.83 - 8.93 (m, 1 H), 7.47 (s, 1 H), 7.32 - 7.38 (m, 1 H), 7.21 - 7.27 (m, 2 H), 7.11 (t, 1 H), 6.84 (t, 1 H), 5.94 (s, 2 H), 3.52 - 3.56 (m, 2 H), 2.71 - 2.79 (m, 2 H).

### Reference Compound 29

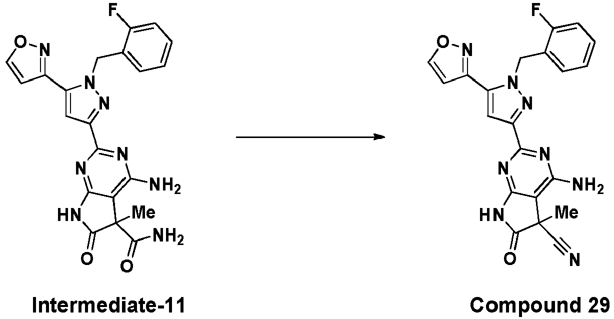

Into a solution of **Intermediate-11** (1 equiv.), in pyridine at 0 °C was added phosphoryl trichloride (8 equiv.) dropwise via syringe. The reaction mixture was slowly warmed to 23 °C with continuous stirring over 2 h. Volatiles were removed under vacuum, and the residue was dissolved in ethyl acetate, washed with water, brine, dried over anhydrous sodium sulfate, filtered and concentrated. The crude solid was then purified by reverse phase HPLC (C18 column, 20 % to 60 % acetonitrile in water with 0.1 % TFA over 20 mins) to deliver the desired (10 mg, 9 % yield) as a white solid. ¹H NMR (500 MHz, DMSO-*d*₆) δ ppm 11.78 (s, 1 H) 9.10 (d, 1 H) 7.40 (s, 1 H) 7.30 - 7.37 (m, 1 H) 7.25 - 7.30 (m, 2 H) 7.23 (d, 2 H) 7.08 - 7.13 (m, 1 H) 6.84 - 6.89 (m, 1 H) 5.89 (s, 2 H) 1.80 (s, 3 H).

### Reference Compound 64

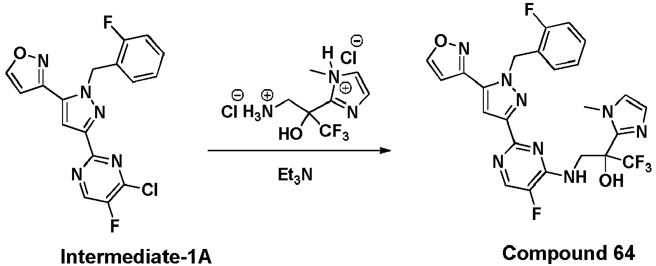

The title compound was prepared from **Intermediate-1A** following general procedure B, 2-(3-ammonio-1,1,1-trifluoro-2-hydroxypropan-2-yl)-1-methyl-1H-imidazol-1-ium chloride (1.5 equiv.) was the amine reactant, 5 equivalents of triethylamine was used, and the contents were heated to 90 °C for 15 h as a solution in dioxane/water (3:1). The contents were cooled to 23 °C, and volatiles were removed under a stream of nitrogen. The resulting crude residue was purified via reverse phase HPLC (C18 column, 5 % to 95 % acetonitrile in water with 0.1% TFA over 20 mins) to deliver the desired compound (32 mg, 83 % yield) as a white powder. ¹H NMR (500 MHz, DMSO-*d*₆) δ ppm 9.11 (s, 1 H), 8.31 (d, 1 H), 7.95 (br. s., 1 H), 7.52 (s, 1 H), 7.29 - 7.38 (m, 2 H), 7.19 - 7.26 (m, 2 H), 7.11 (t, 2 H), 6.94 (t, 1 H), 5.92 (s, 2 H), 4.25 - 4.40 (m, 2 H), 3.88 (s, 3 H), 2.54 (s, 1 H).

### Reference Compound 32

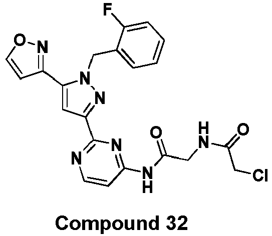

A solution of **Intermediate-2** (1 equiv.) and triethylamine (5 equiv.) in dichloromethane at 0 °C was treated with chloroacetyl chloride (3 equiv.), then allowed to warm to 23 °C, over 20 min. The reaction was quenched by addition of saturated NaHCO₃ and 1:1 Ethyl acetate/THF. The contents were filtered to deliver the desired compound (28 mg, 89%) as a tan solid. ¹H NMR (500 MHz, DMSO-*d*₆) δ ppm 11.21 - 11.36 (m, 1 H), 9.11 (d, 1 H), 8.74 (d, 1 H), 8.56 (t, 1 H), 7.97 (d, 1 H), 7.66 (s, 1 H), 7.30 - 7.38 (m, 1H), 7.27 (d, 1 H), 7.19 - 7.25 (m, 1 H), 7.12 (t, 1 H), 6.89 (t, 1 H), 5.93 (s, 2 H), 4.18 (s, 2 H), 4.01 - 4.10 (m, 2 H).

### Intermediate-15

The title compound was synthesized in 3 steps:

### Step 1: Synthesis of (3-(trifluoromethyl)-[1,2,4]triazolo[4,3-a]pyrazin-6-yl)methanol

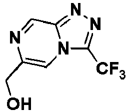

Trifluoroacetic anhydride (10 equiv.) was added to (5-hydrazinylpyrazin-2-yl)methanol (1 equiv.) at 0°C. After complete addition, the reaction was warmed 23 °C, stirred for 20 min and the solvents removed in vacuo. Polyphosphoric acid (excess) was then added to the reaction, and the contents were heated at 100 °C for 2 h. The hot suspension was poured over ice and basified with ammonium hydroxide till pH 11. The mixture was extracted with ethyl acetate, concentrated and purified via silica gel chromatography to deliver the desired intermediate, (3-(trifluoromethyl)-[1,2,4]triazolo[4,3-a]pyrazin-6-yl)methanol (2.035 g, 43%) as a yellow solid. ¹H NMR (500 MHz, DMSO-*d*₆) δ ppm 9.53 - 9.73 (m, 1 H), 8.32 (d, 1 H), 5.82 (t, 1 H), 4.70 (dd, 2 H). Step 2: Synthesis of 6-(((tert-butyldiphenylsilyl)oxy)methyl)-3-(trifluoromethyl)-[1,2,4]triazolo[4,3-a]pyrazine.

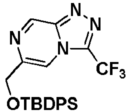

*t*-Butyldiphenylsilyl chloride (2 equiv.) was added to a solution of (3-(trifluoromethyl)-[1,2,4]triazolo[4,3-a]pyrazin-6-yl)methanol (1 equiv.) and imidazole (3 equiv.) in dichloromethane. The reaction was stirred at 23 °C for 20 min, quenched by the addition of water, extracted with ethyl acetate, concentrated, and purified via silica gel chromatography to deliver the desired intermediate, 6-(((tert-butyldiphenylsilyl)oxy)methyl)-3-(trifluoromethyl)-5,6,7,8-tetrahydro-[1,2,4]triazolo[4,3-a]pyrazine (0.414 g, 94%) as a yellow oil. ¹H NMR (500 MHz, DMSO-*d*₆) δ ppm 9.58 - 9.71 (m, 1 H), 8.44 (s, 1 H), 7.61 - 7.76 (m, 4 H), 7.34 - 7.53 (m, 6 H), 4.93 (d, 2 H), 1.00 - 1.12 (m, 9 H).

### Step 3: Synthesis of 6-(((tert-butyldiphenylsilyl)oxy)methyl)-3-(trifluoromethyl)-5,6,7,8-tetrahydro-[1,2,4]triazolo[4,3-a]pyrazine

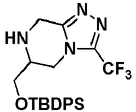

6-(((tert-butyldiphenylsilyl)oxy)methyl)-3-(trifluoromethyl)-[1,2,4]triazolo[4,3-a]pyrazine (1 equiv.) was placed in ethanol along with 10% Pd/C (0.05 equiv.) and hydrogenated under balloon pressure for 40 h at 23 °C. The reaction mixture was filtered, concentrated, and purified via silica gel chromatography to deliver the desired intermediate, 6-(((tert-butyldiphenylsilyl)oxy)methyl)-3-(trifluoromethyl)-5,6,7,8-tetrahydro-[1,2,4]triazolo[4,3-a]pyrazine (0.16 g, 38%) as a tan solid. ¹H NMR (500 MHz, DMSO-*d*₆) δ ppm 7.63 (ddd, 4 H), 7.39 - 7.52 (m, 6 H), 4.18 - 4.28 (m, 2 H), 3.95 - 4.02 (m, 1 H), 3.84 - 3.91 (m, 1 H), 3.71 - 3.83 (m, 2 H), 3.23 - 3.32 (m, 1 H), 2.79 (td, 1 H), 1.00 (s, 9 H).

### Step 4: Synthesis of Intermediate-15

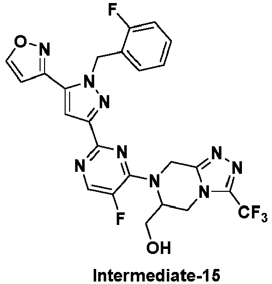

Tetrabutylammonium fluoride (2 equiv.) was added to a suspension of **Intermediate-IA** (1 equiv.), 6-(((tert-butyldiphenylsilyl)oxy)methyl)-3-(trifluoromethyl)--5,6,7,8-tetrahydro-[1,2,4]triazolo[4,3-a]pyrazine (2 equiv.), and triethylamine (2 equiv) in dioxane/water (10:1), and the reaction was heated at 100 °C for 48 h. The reaction mixture was then cooled to 23 °C, concentrated in vacuo and purified via reverse phase HPLC to deliver the desired compound **Intermediate-15** (6 mg, 36%) as a white solid. ¹H NMR (500 MHz, DMSO-*d*₆) δ ppm 9.11 (d, 1 H), 8.46 (d, 1 H), 7.67 (s, 1 H), 7.30 - 7.38 (m, 1 H), 7.28 (d, 1 H), 7.19 - 7.25 (m, 1 H), 7.11 (t, 1 H), 6.85 (t, 1 H), 5.88 - 5.98 (m, 2 H), 5.61 (d, 1 H), 5.20 (t, 1 H), 5.13 (br. s., 1 H), 4.82 (d, 1 H), 4.38 - 4.51 (m, 2 H), 3.64 (dt, 1 H), 3.50 - 3.59 (m, 1 H).

### Compound 62

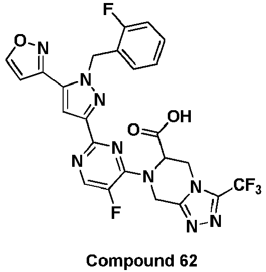

Tetrapropylammonium Perruthenate (0.1 equiv.) was added to a suspension of **Intermediate-15** (1 equiv.) and NMO (10 equiv.) in acetonitrile and water (10 equiv.) at 23 °C. The reaction was monitored for completion by LCMS, filtered, and purified via reverse phase HPLC to deliver the desired compound (9 mg, 12%) as a white solid. ¹H NMR (500 MHz, CD₃OD) δ ppm 8.72 - 8.85 (m, 1 H), 8.50 (d, 1 H), 7.54 - 7.70 (m, 1 H), 7.20 - 7.36 (m, 1 H), 7.07 - 7.15 (m, 1 H), 7.04 (t, 1 H), 6.94 (d, 1 H), 6.86 (t, 1 H), 6.28 (br. s., 1 H), 6.00 (s, 2 H), 5.70 (d, 1 H), 5.08 - 5.21 (m, 1 H), 5.01 (d, 1 H), 4.60 (dd, 1 H). COOH proton exchanged.

### Compound 83

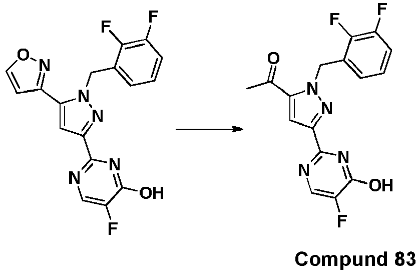

A suspension of 2-(1-(2,3-difluorobenzyl)-5-(isoxazol-3-yl)-1H-pyrazol-3-yl)-5-fluoropyrimidin-4-ol (described in previous patent: WO2013/101830 A1) (1 equiv.) and sodium methoxide in methanol (2.2 equiv.) in diglyme was heated in a pressure tube at 150 °C for 1 h. The reaction was filtered, the residue washed with methanol, and the filtrate was purified via silica gel chromatography to deliver the desired compound (355 mg, 75%) as a white solid. ¹H-NMR (500 MHz, CD₃OD) δ ppm 8.04 (d, 1 H), 7.67 - 7.79 (m, 1 H), 7.14 - 7.25 (m, 1 H), 7.01 - 7.11 (m, 1 H), 6.66 - 6.77 (m, 1 H), 5.96 (s, 2 H), 2.56 (s, 3 H). OH protons exchanged.

### Reference Compound 84

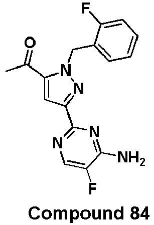

The title compound was prepared following general procedure B, using **Intermediate-1B** (1 equiv.) in place of **Intermediate-1A,** ammonium hydroxide as the amine reactant, and contents were heated at 90 °C for 65 h as a solution in dioxane. The resulting crude material was purified via reverse phase HPLC to deliver the desired compound (4.2 mg, 13.5%) as a white solid. ¹H-NMR (500 MHz, CD₃OD) δ ppm 8.12 (d, 1 H), 7.65 (s, 1 H), 7.23 - 7.32 (m, 1 H), 7.02 - 7.14 (m, 2 H), 6.77 - 6.86 (m, 1 H), 5.90 (s, 2 H), 2.55 (s, 3 H). NH₂ protons exchanged.

### Compound 85

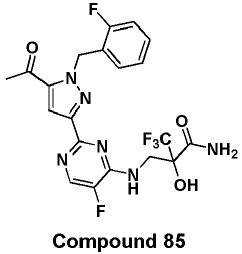

The title compound was prepared following general procedure B, except **Intermediate-1B** (1 equiv.) was used in place of **Intermediate-1A,** 2-(aminomethyl)-3,3,3-trifluoro-2-hydroxypropanamide was the amine reactant, and contents were heated at 90 °C for 65 h as a solution in dioxane. The resulting crude material was purified via reverse phase HPLC to deliver the desired compound (3.5 mg, 7.6%) as a white solid. ¹H NMR (500 MHz, CD₃OD) δ ppm 8.12 (d, 1 H), 7.68 (s, 1 H), 7.23 - 7.35 (m, 1 H), 7.02 - 7.15 (m, 2 H), 6.93 (t, 1 H), 5.91 (s, 2 H), 4.16 - 4.26 (m, 1 H), 4.04 - 4.12 (m, 1 H), 2.58 (s, 3 H). NH, OH and NH₂ protons exchanged.

### Reference Compound 86

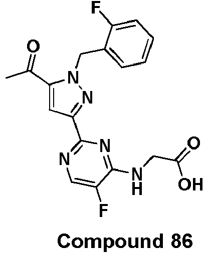

The title compound was prepared following general procedure B, except **Intermediate-1B** (1 equiv.) was used in place of **Intermediate-1A,** glycine was the amine reactant, and contents were heated at 90 °C for 65 h as a solution in dioxane. The resulting crude material was purified via reverse phase HPLC to deliver the desired compound (1 mg, 2.7%) as a white solid. ¹H NMR (500 MHz, CD₃OD) δ ppm 8.27 (d, 1 H), 7.74 (s, 1 H), 7.23 - 7.35 (m, 1 H), 7.01 - 7.17 (m, 2 H), 6.88 (t, 1 H), 5.94 (s, 2 H), 4.43 (s, 2 H), 2.58 (s, 3 H). NH and COOH protons exchanged.

### Reference Compound 87

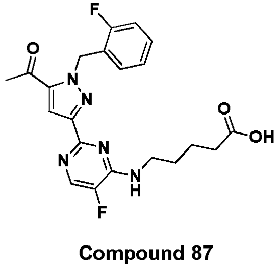

The title compound was prepared following general procedure B, except **Intermediate-1B** (1 equiv.) was used in place of **Intermediate-1A,** 5-aminopentanoic acid was the amine reactant, and contents were heated at 90 °C for 65 h as a solution in dioxane. The resulting crude material was purified via reverse phase HPLC to deliver the desired compound (43 mg, 10.6%) as a tan solid. ¹H NMR (500 MHz, CD₃OD) δ ppm 8.25 (d, 1 H), 7.90 (s, 1 H), 7.27 - 7.36 (m, 1 H), 7.04 - 7.16 (m, 2 H), 6.96 (t, 1 H), 5.97 (s, 2 H), 3.80 (t, 2 H), 2.62 (s, 3 H), 2.43 (t, 2 H), 1.68 - 1.87 (m, 4 H). NH and COOH protons exchanged.

### Reference Compound 88

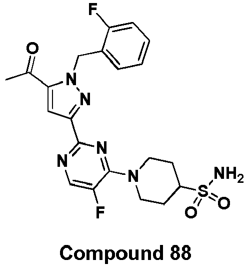

The title compound was prepared following general procedure B, except using **Intermediate-1B** (1 equiv.) in place of **Intermediate-1A,** piperidine-4-sulfonamide was the amine reactant, and contents were heated at 90 °C for 65 h as a solution in dioxane. The resulting crude material was purified via reverse phase HPLC to deliver the desired compound (19 mg, 42%) as a white solid. ¹H NMR (500 MHz, CD₃OD) δ ppm 8.32 (d, 1 H), 7.87 (s, 1 H), 7.31 (q, 1 H), 7.02 - 7.16 (m, 2 H), 6.92 (t, 1 H), 5.96 (s, 2 H), 5.02 (d, 2 H), 3.36 - 3.45 (m, 3 H), 2.61 (s, 3 H), 2.37 (d, 2 H), 1.88 - 2.02 (m, 2 H). NH₂ protons exchanged.

### Reference Compound 89

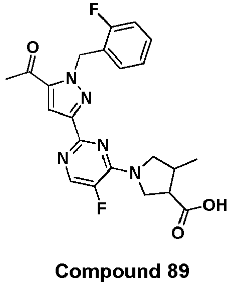

The title compound was prepared following general procedure B, except **Intermediate-1B** (1 equiv.) was used in place of **Intermediate-1A**, 4-methylpyrrolidine-3-carboxylic acid was the amine reactant, and contents were heated at 90 °C for 65 h as a solution in dioxane. The resulting crude material was purified via reverse phase HPLC to deliver the desired compound (10 mg, 24%) as a yellow solid. ¹H NMR (500 MHz, CD₃OD) δ ppm 8.32 (d, 1 H), 7.88 (s, 1 H), 7.27 - 7.40 (m, 1 H), 7.05 - 7.20 (m, 2 H), 6.90 - 7.03 (m, 1 H), 5.99 (s, 2 H), 4.32 - 4.48 (m, 2 H), 4.07 - 4.30 (m, 1 H), 3.53 - 3.74 (m, 1 H), 2.83 - 3.08 (m, 1 H), 2.67 - 2.79 (m, 1 H), 2.63 (s, 3 H), 1.23 - 1.40 (m, 3 H). COOH proton exchanged.

### Reference Compound 90

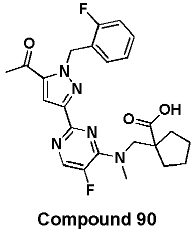

The title compound was prepared following general procedure B, except **Intermediate-1B** (1 equiv.) was used in place of **Intermediate-1A,** 1-((methylamino)methyl)cyclopentanecarboxylic acid (as the HBr salt) was the amine reactant, and contents were heated at 90 °C for 65 h as a solution in dioxane. The resulting crude material was purified via reverse phase HPLC to deliver the desired compound as a tan solid (9.5 mg, 21%). ¹H NMR (500 MHz, CD₃OD) δ ppm 8.31 (d, 1 H), 7.80 (s, 1 H), 7.27 - 7.36 (m, 1 H), 7.02 - 7.16 (m, 2 H), 6.90 - 7.00 (m, 1 H), 5.96 (s, 2 H), 4.30 (s, 2 H), 3.53 (d, 3 H), 2.60 (s, 3 H), 2.18 - 2.35 (m, 2 H), 1.74 - 1.86 (m, 2 H), 1.64 - 1.74 (m, 4 H). COOH proton exchanged.

### Reference Compound 91

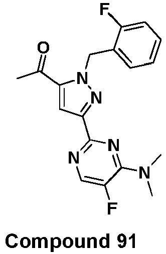

The title compound was prepared following general procedure B, except **Intermediate-1B** (1 equiv.) was used in place of **Intermediate-1A,** dimethylamine (60 equiv.) was the amine reactant, no triethylamine was used, and contents were heated at 90 °C for 2 h as a solution in dioxane. The reaction mixture was cooled to 23 °C, diluted with dicloromethane and washed successively with IN HCl solution, water and brine. The organic layer was then dried over sodium sulfate, filtered and concentrated in vacuo to deliver the desired compound (13 mg, 80%) as a tan solid. ¹H NMR (500 MHz, DMSO-*d*₆) δ ppm 8.22 (d, 1 H), 7.69 (s, 1 H), 7.28 - 7.38 (m, 1 H), 7.18 - 7.26 (m, 1 H), 7.11 (t, 1 H), 6.81 (t, 1 H), 5.81 (s, 2 H), 3.24 (d, 6 H), 2.57 (s, 3 H).

### Reference Compound 92

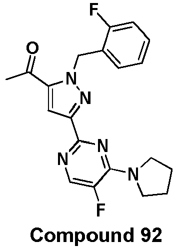

The title compound was prepared following general procedure B, except **Intermediate-1B** (1 equiv.) was used in place of **Intermediate-1A**, pyrrolidine (60 equiv.) was the amine reactant, no triethylamine was used, and contents were heated to 90 °C for 2 h as a solution in dioxane. The reaction mixture was cooled to 23 °C, diluted with dicloromethane and washed successively with IN HCl solution, water and brine. The organic layer was then dried over sodium sulfate, filtered and concentrated in vacuo and purified via reverse phase HPLC to deliver the desired compound (9 mg, 49%) as a tan solid. ¹H NMR (500 MHz, CD₃OD) δ ppm 8.25 (d, 1 H), 7.83 (s, 1 H), 7.25 - 7.41 (m, 1 H), 7.02 - 7.18 (m, 2 H), 6.90 - 7.00 (m, 1 H), 4.02 (d, 4 H), 5.97 (s, 2 H), 2.61 (s, 3 H), 1.99 - 2.24 (m, 4H).

### Reference Compound 93

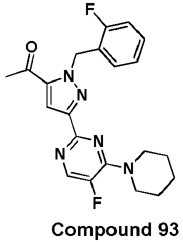

The title compound was prepared following general procedure B, except **Intermediate-1B** (1 equiv.) was used in place of **Intermediate-1A**, piperidine (60 equiv.) was the amine reactant, no triethylamine was used, and contents were heated at 90 °C for 2 h as a solution in dioxane. The reaction mixture was cooled to 23 °C, diluted with dicloromethane and washed successively with 1N HCl solution, water and brine. The organic layer was then dried over sodium sulfate, filtered and concentrated in vacuo to deliver the desired compound (0.012 g, 63%) as a tan solid. ¹H NMR (500 MHz, DMSO-*d*₆) δ ppm 8.26 (d, 1 H), 7.69 (s, 1 H), 7.33 (q, 1 H), 7.19 - 7.27 (m, 1 H), 7.11 (t, 1 H), 6.80 (t, 1 H), 5.82 (s, 2 H), 3.72 - 3.83 (m, 4 H), 2.92 - 3.06 (m, 2 H), 2.57 (s, 3 H), 1.65 - 1.70 (m, 2 H), 1.52 - 1.57 (m, 1 H), 1.42 - 1.50 (m, 1 H).

### Reference Compound 94

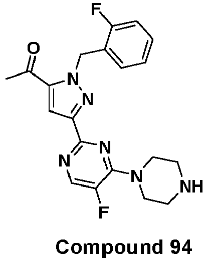

The title compound was prepared following general procedure B, except **Intermediate-1B** (1 equiv.) was used in place of **Intermediate-1A,** piperazine (60 equiv.) was the amine reactant, no triethylamine was used, and contents were heated at 90 °C for 2 h as a solution in dioxane. The reaction mixture was cooled to 23 °C, diluted with dicloromethane and washed successively with IN HCl solution, water and brine. The organic layer was then dried over sodium sulfate, filtered and concentrated in vacuo to deliver the desired compound (10 mg, 55%) as a tan solid. ¹H NMR (500 MHz, DMSO-*d*₆) δ ppm 8.27 (d, 1 H), 7.70 (s, 1 H), 7.33 (q, 1 H), 7.16 - 7.26 (m, 1 H), 7.10 (t, 1 H), 6.79 (t, 1 H), 5.82 (s, 2 H), 3.67 - 3.76 (m, 4 H), 2.75 - 2.86 (m, 4 H), 2.57 (s, 3 H). NH proton exchanged.

### Reference Compound 95

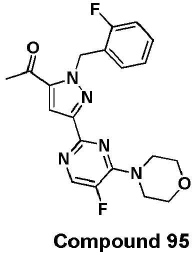

The title compound was prepared following general procedure B, except **Intermediate-1B** (1 equiv.) was used in place of **Intermediate-1A,** morpholine (60 equiv.) was the amine reactant, no triethylamine was used, and contents were heated at 90 °C for 2 h as a solution in dioxane. The reaction mixture was cooled to 23 °C, diluted with dicloromethane and washed successively with IN HCl solution, water and brine. The organic layer was then dried over sodium sulfate, filtered and concentrated in vacuo to deliver the desired compound (13 mg, 72%) as a tan solid. ¹H NMR (500 MHz, DMSO-*d*₆) δ ppm 8.33 (d, 1 H), 7.73 (s, 1 H), 7.33 (q, 1 H), 7.16 - 7.27 (m, 1 H), 7.10 (t, 1 H), 6.79 (t, 1 H), 5.82 (s, 2 H), 3.77 - 3.85 (m, 4 H), 3.69 - 3.75 (m, 4 H), 2.57 (s, 3 H).

### Reference Compound 96

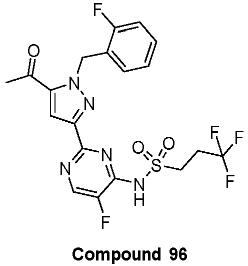

The title compound was prepared following general procedure B, except **Intermediate-1B** (1 equiv.) was used in place of **Intermediate-1A,** 3,3,3-trifluoropropane-1-sulfonamide (4 equiv.) was the amine reactant, potassium carbonate (4 equiv.) was used in place of triethylamine, and contents were heated via microwave to 150 °C for 15 min as a solution in DMSO. The reaction mixture was cooled to 23 °C, filtered and purified by reverse phase HPLC to deliver the desired compound (0.004 g, 9.7%) as a tan solid. 1H NMR (500 MHz, CD₃OD) δ ppm 8.40 - 8.47 (m, 1 H), 7.70 (s, 1 H), 7.25 - 7.34 (m, 1 H), 7.03 - 7.15 (m, 2 H), 6.89 (t, 1 H), 5.91 (s, 2 H), 3.93 - 4.02 (m, 2 H), 2.79 - 2.92 (m, 2 H), 2.57 (s, 3 H). NH proton exchanged.

### Reference Compound 97

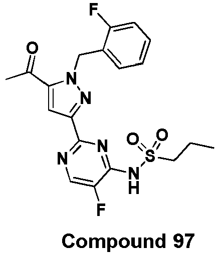

The title compound was prepared following general procedure B, except **Intermediate-1B** (1 equiv.) was used in place of **Intermediate-1A,** propane-1-sulfonamide (4 equiv.) was the amine reactant, potassium carbonate (4 equiv.) was used in place of triethylamine, and contents were heated via microwave to 150 °C for 15 min as a solution in DMSO. The reaction mixture was cooled to 23 °C, filtered and purified by reverse phase HPLC to deliver the desired compound (5 mg, 13.7%) as a tan solid. ¹H NMR (500 MHz, CD₃OD) δ ppm 8.44 - 8.54 (m, 1 H), 7.63 - 7.73 (m, 1 H), 7.26 - 7.35 (m, 1 H), 7.04 - 7.14 (m, 2 H), 6.91 (t, 1 H), 5.92 (s, 2 H), 3.69 - 3.78 (m, 2 H), 2.58 (s, 3 H), 1.85 - 1.96 (m, 2 H), 1.08 (t, 3 H). NH proton exchanged.

### Reference Compound 98

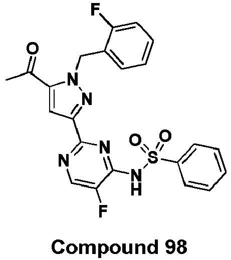

The title compound was prepared following general procedure B, except **Intermediate-1B** (1 equiv.) was used in place of **Intermediate-1A,** benzenesulfonamide (4 equiv.) was the amine reactant, potassium carbonate (4 equiv.) was used in place of triethylamine, and contents were heated via microwave at 150 °C for 15 min as a solution in DMSO. The reaction mixture was cooled to 23 °C, filtered and purified by reverse phase HPLC to deliver the desired compound (4 mg, 9.6%) as a tan solid. ¹H NMR (500 MHz, CD₃OD) δ ppm 8.36 - 8.46 (m, 1 H), 8.27 (d, 2 H), 7.58 - 7.63 (m, 1 H), 7.45 - 7.53 (m, 3 H), 7.29 - 7.36 (m, 1 H), 7.06 - 7.18 (m, 2 H), 6.90 (t, 1 H), 5.92 - 5.99 (m, 2 H), 2.59 - 2.65 (m, 3 H). NH proton exchanged.

### Reference Compound 99

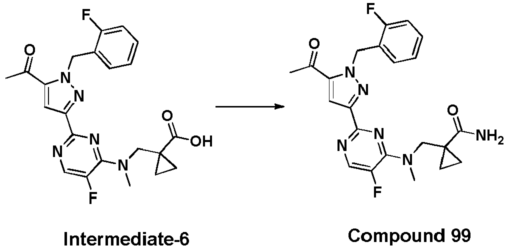

A solution of Intermediate-6 (1 equiv.) in dichloromethane at 0 °C was treated with DAST (2.2 equiv.), and allowed to warm to 23 °C over 2 h. The reaction was then diluted with 7M NH₃ in methanol and stirred for 30 min. The reaction mixture was concentrated and the residue diluted with methanol and filtered to deliver the desired compound (11 mg, 47%) as a white solid. ¹H NMR (500 MHz, DMSO-*d*₆) δ ppm 8.24 (d, 1 H), 7.69 (s, 1 H), 7.27 - 7.41 (m, 1 H), 7.15 - 7.27 (m, 2 H), 7.11 (t, 1 H), 6.80 - 6.96 (m, 2 H), 5.81 (s, 2 H), 4.00 (s, 2 H), 3.24 (d, 3 H), 2.57 (s, 3 H), 1.01 - 1.06 (m, 2 H), 0.83 - 0.89 (m, 2 H).

### Compound 28

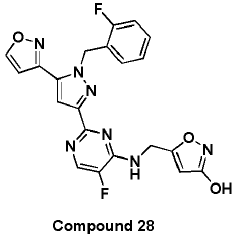

The title compound was prepared following general procedure B, except 5-(aminomethyl)isoxazol-3-ol was the amine reactant, and the contents were heated at 100 °C for 16 h. The contents were cooled to ambient temperature, diluted with water and acidified to pH 3 with IN HCl solution. The resulting precipitate was filtered and dried in vacuo to deliver the desired compound (68 mg, 94% yield, 1:1 solvate with dioxane) as a white solid. ¹H-NMR (500 MHz, DMSO-*d*₆) δ ppm 11.2 (s, 1 H), 9.10 (d, 1 H), 8.34 (br. t, 1 H), 8.27 (d, 1 H), 7.51 (s, 1 H), 7.33 (m, 1 H), 7.24-7.18 (m, 2 H), 7.10 (app. t, 1 H), 6.87 (app. t, 1 H), 6.00 (s, 1 H), 5.89 (s, 2 H), 4.68 (d, 2 H), 3.57 (s, 8 H, dioxane).

### Compound 26

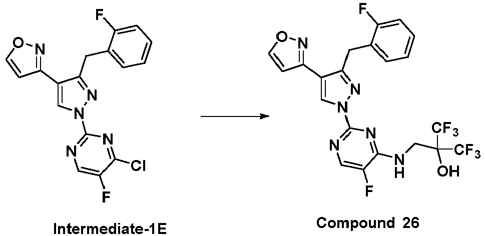

The title compound was prepared following general procedure B, except Intermediate-IE (described in patent application publication WO2014/047325) was used in place of **Intermediate-1A,** 2-(aminomethyl)-1,1,1,3,3,3-hexafluoropropan-2-ol was the amine reactant, and the contents were heated to 100 °C for 15 h. The contents were cooled to 23 °C, diluted with water, acidified to pH 4 with IN HCl solution and extracted with dichloromethane. The organic phases were dried over sodium sulfate, filtered and the solvent was removed in vacuo. The crude material was purified via silica gel chromatography utilizing 15-30% ethyl acetate/hexanes gradient to deliver the desired compound (45 mg, 66% yield) as a white solid. ¹H-NMR (500 MHz, DMSO-*d*₆) δ ppm 9.07 (s, 1 H), 8.97 (d, 1 H), 8.34 (d, 1 H), 8.29 (br. t, 1 H), 8.22 (s, 1 H), 7.27-7.21 (m, 2 H), 7.14 (m, 1 H), 7.06 (m, 1 H), 7.04 (d, 1 H), 4.32 (s, 2 H), 4.13 (d, 2 H).

### Compound 30

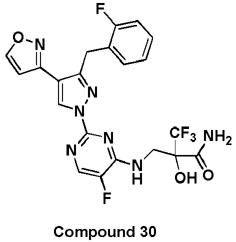

The title compound was prepared following general procedure B, except **Intermediate-IE** was used in place of **Intermediate-1A,** 2-(aminomethyl)-3,3,3-trifluoro-2-hydroxypropanamide was the amine reactant, and the contents were heated at 100 °C for 16 h. The contents were cooled to 23 °C, diluted with water, acidified to pH 4 with IN HCl solution and extracted with dichloromethane. The organic phases were dried over sodium sulfate, filtered and the solvent was removed in vacuo. The crude material was purified via silica gel chromatography utilizing 5-15% acetonitrile-methanol (7:1) in dichloromethane gradient to deliver the desired compound (47 mg, 67% yield) as a white solid. ¹H-NMR (500 MHz, DMSO-*d*₆) δ ppm 9.14 (s, 1 H), 8.96 (d, 1 H), 8.28 (d, 1 H), 8.01 (br. t, 1 H), 7.76 (br. s, 1 H), 7.61 (br. s, 1 H), 7.25 (s, 1 H), 7.27-7.19 (m, 2 H), 7.15 (m, 1 H), 7.06 (app. t, 1 H), 7.03 (d, 1 H), 4.34 (s, 2 H), 4.11 (dd, 1 H), 3.89 (dd, 1 H).

### Compound 102

The title compound was synthesized in 2 steps:

### Step 1: Synthesis of Intermediate-IF

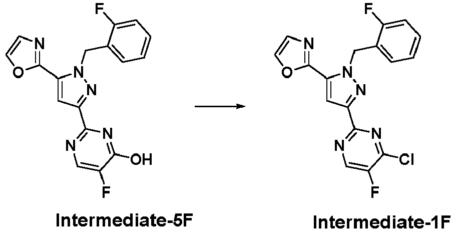

A suspension of 5-fluoro-2-(1-(2-fluorobenzyl)-5-(oxazol-2-yl)-1*H*-pyrazol-3-yl)-pyrimidin-4-ol (**Intermediate-5F**, described in patent application publication WO2012/3405 A1) in phosphoryl trichloride (67 equiv.) as solvent was heated at 65 °C for 2 h. The reaction mixture was cooled to 23 °C, dried under a stream of nitrogen and then concentrated twice from toluene. The resultant light yellow solid was dried in vacuo and used in the next step without further manipulation. Step 2: Synthesis of **Compound 102**

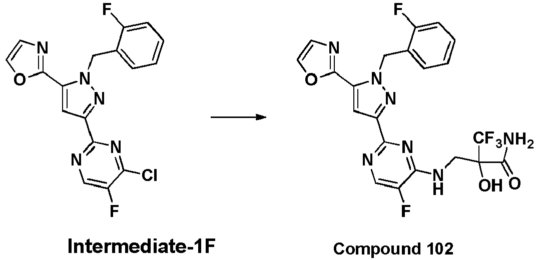

The title compound was prepared following general procedure B, except **Intermediate-IF** was used in place of **Intermediate-1A,** 2-(aminomethyl)-3,3,3-trifluoro-2-hydroxypropanamide was the amine reactant, and the contents were heated at 100 °C for 2 d. The contents were cooled to 23 °C, diluted with water, then acidified to pH 6 with IN HCl solution. The resulting precipitate was filtered and dried in vacuo to deliver the desired compound (59 mg, 91% yield) as an off-white solid. ¹H-NMR (500 MHz, DMSO-*d*₆) δ ppm 8.32 (m, 2 H), 7.84 (br. t, 1 H), 7.75 (br. s, 1 H), 7.72 (s, 1 H), 7.62 (br. s, 1 H), 7.49 (s, 1 H), 7.42 (s, 1 H), 7.34 (m, 1 H), 7.21 (m, 1 H), 7.11 (app. t, 1 H), 7.02 (app. t, 1 H), 6.04 (s, 2 H), 4.01 (m, 2 H).

### Compound 103

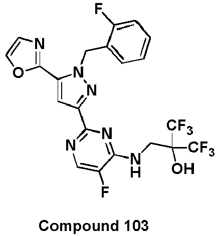

The title compound was prepared following general procedure B, except **Intermediate-1F** was used in place of **Intermediate-1A,** 2-(aminomethyl)-1,1,1,3,3,3-hexafluoropropan-2-ol was the amine reactant, and the contents were heated to 90-100 °C for 5 d. The contents were cooled to 23 °C, diluted with water, acidified to pH 4 with IN HCl solution and extracted with dichloromethane. The organic phases were dried over sodium sulfate, filtered and the solvent was removed in vacuo. The crude material was purified via silica gel chromatography utilizing 15-50% ethyl acetate/hexanes gradient to deliver the desired compound (38 mg, 55% yield) as a white solid. ¹H-NMR (500 MHz, CDCl₃) δ ppm 8.27 (m, 2 H), 7.73 (s, 1 H), 7.45 (s, 1 H), 7.23 (m, 1 H), 7.14 (app. t, 1 H), 7.05-7.00 (m, 2 H), 6.10 (s, 2 H), 5.72 (br. s, 1 H), 4.15 (d, 2 H). The exchangeable OH proton was not observed.

### Reference Compound 109

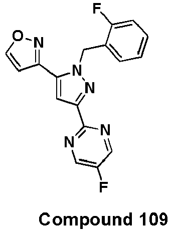

Acetic acid (5.5 equiv.) was added to a suspension of **Intermediate-1A** (1 equiv.) and zinc dust (3.4 equiv.) in THF, and contents were heated at 75 °C for 72 h. After the reaction was complete, the reaction was quenched by addition of IN HCl solution and extracted with ethyl acetate. The organic layers were dried and concentrated to deliver the desired compound (47 mg, 82 % yield) as a brown solid. ¹H-NMR (500 MHz, DMSO-*d*₆) δ ppm 9.05 - 9.18 (m, 1 H), 8.96 (s, 2 H), 7.60 - 7.71 (m, 1 H), 7.31 - 7.38 (m, 1 H), 7.28 (d, 1 H), 7.19 - 7.26 (m, 1 H), 7.12 (t, 1 H), 6.91 - 6.99 (m, 1 H), 5.93 (s, 2 H).

### Reference Compound 37

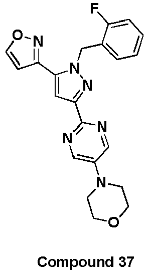

A solution of **Compound 109** (1.0 equiv.), morpholine (7.0 equiv.) in anhydrous DMSO was heated to 120 °C for 18 h. The contents were cooled to 23 °C, diluted with water and extracted with ethyl acetate. The organic phases were dried over sodium sulfate, filtered and the solvent was removed in vacuo. The crude material was purified via silica gel chromatography utilizing 30-50% acetonitrile-methanol (7:1) in dichloromethane gradient to deliver the desired compound (34 mg, 57% yield) as an off-white solid. ¹H-NMR (500 MHz, DMSO-*d*₆) δ ppm 9.09 (d, 1 H), 8.54 (s, 2 H), 7.55 (s, 1 H), 7.34 (m, 1 H), 7.27 (d, 1 H), 7.22 (m, 1 H), 7.11 (app. t, 1 H), 6.90 (app. t, 1 H), 5.89 (s, 2 H), 3.77 (m, 4 H), 3.29 (m, 4 H).

### Reference Compound 8

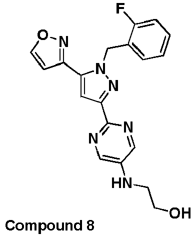

A solution of **Compound 109** (1.0 equiv.), 2-aminoethanol (7.0 equiv.) in anhydrous DMSO was heated to 120 °C for 22 h. The contents were cooled to 23 °C, diluted with water and extracted with ethyl acetate. The organic phases were dried over sodium sulfate, filtered and the solvent was removed in vacuo. The crude material was purified via silica gel chromatography utilizing 25-50% acetonitrile-methanol (7:1) in dichloromethane gradient to deliver the desired compound (36 mg, 65% yield) as a pale yellow solid. ¹H-NMR (500 MHz, DMSO-*d*₆) δ ppm 9.08 (d, 1 H), 8.21 (s, 2 H), 7.46 (s, 1 H), 7.33 (m, 1 H), 7.25 (d, 1 H), 7.22 (m, 1 H), 7.11 (app. t, 1 H), 6.88 (app. t, 1 H), 6.27 (t, 1 H), 5.87 (s, 2 H), 4.80 (t, 1 H), 3.59 (dt, 2 H), 3.21 (dt, 2 H).

### Reference Compound 9

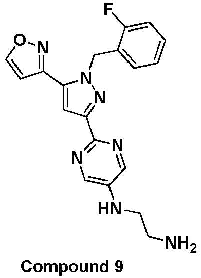

A solution of **Compound 109** (1.0 equiv.), ethane-1,2-diamine (7.0 equiv.) in anhydrous DMSO was heated to 120 °C for 8 h. The contents were cooled to 23 °C, poured into saturated sodium bicarbonate solution and extracted with ethyl acetate. The organic phases were dried over sodium sulfate, filtered and the solvent was removed in vacuo. The crude material was purified via preparative HPLC utilizing a 30-80% acetonitrile/water gradient (with 0.1% TFA) to deliver the desired compound (33 mg, 51% yield, TFA salt) as a light yellow solid. ¹H-NMR (500 MHz, DMSO-*d*₆) δ ppm 9.10 (d, 1 H), 8.24 (s, 2 H), 7.79 (br. s, 2 H), 7.47 (s, 1 H), 7.34 (m, 1 H), 7.25 (d, 1 H), 7.22 (m, 1 H), 7.11 (app. t, 1 H), 6.90 (app. t, 1 H), 6.37 (br. s, 1 H), 5.88 (s, 2 H), 3.40 (m, 2 H), 3.01 (m, 2 H).

### Reference Compound 6

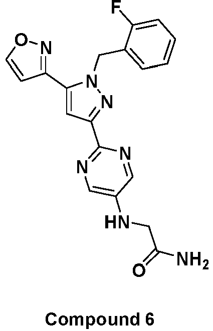

A suspension of **Compound 109** (1.0 equiv.), glycinamide hydrochloride (7.0 equiv.) and sodium bicarbonate (7.0 equiv.) in anhydrous DMSO was heated at 120-130 °C for 2 d. Additional amounts of glycinamide hydrochloride (7.0 equiv.) and sodium bicarbonate (7.0 equiv.) were added and the reaction was heated at 130 °C for another 2 d. The contents were cooled to 23 °C, poured into half-saturated sodium bicarbonate solution and extracted with ethyl acetate. The organic phases were dried over sodium sulfate, filtered and the solvent was removed in vacuo. The crude material was purified via silica gel chromatography utilizing 20-60% acetonitrile-methanol (7:1) in dichloromethane gradient followed by reverse phase HPLC utilizing a 30-80% acetonitrile/water gradient (with 0.1% TFA) to deliver the desired compound (2.0 mg, 3.4% yield) as a white solid. ¹H-NMR (500 MHz, CD₃OD) δ ppm 8.77 (d, 1 H), 8.26 (s, 2 H), 7.41 (s, 1 H), 7.27 (m, 1 H), 7.09 (m, 1 H), 7.03 (app. t, 1 H), 6.89 (d, 1 H), 6.87 (app. t, 1 H), 5.96 (s, 2 H), 3.94 (s, 2 H).

### Reference Compound 21

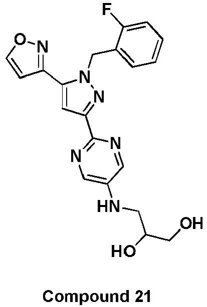

A solution of **Compound 109** (1.0 equiv.), 3-aminopropane-1,2-diol (7.0 equiv.) in anhydrous DMSO was heated to 120 °C for 18 h. The contents were cooled to 23 °C, diluted with water and extracted with ethyl acetate. The organic phases were dried over sodium sulfate, filtered, and the solvent was removed in vacuo. The crude material was purified via silica gel chromatography utilizing 30-50% acetonitrile-methanol (7:1) in dichloromethane gradient to deliver the desired compound (34 mg, 57% yield) as an off-white solid. ¹H-NMR (500 MHz, DMSO-*d*₆) δ ppm 9.08 (d, 1 H), 8.22 (s, 2 H), 7.46 (s, 1 H), 7.33 (m, 1 H), 7.24 (d, 1 H), 7.22 (m, 1 H), 7.11 (app. t, 1 H), 6.88 (app. t, 1 H), 6.22 (t, 1 H), 5.87 (s, 2 H), 4.91 (d, 1 H), 4.68 (t, 1 H), 3.65 (m, 1 H), 3.39 (m, 2 H), 3.27 (m, 1 H), 3.03 (m, 1 H).

### Reference Compound 22

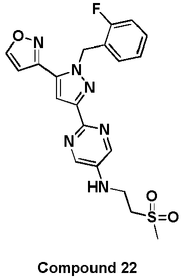

A solution of **Compound 109** (1.0 equiv.), 2-(methylsulfonyl)ethanamine hydrochloride (7.0 equiv.) and triethylamine (7.0 equiv.) in anhydrous DMSO was heated at 120 °C for 4 d. Additional amounts of 2-(methylsulfonyl)ethanamine hydrochloride (7.0 equiv.) and triethylamine (7.0 equiv.) were added and the reaction was heated at 120-130 °C for another 7 d. The contents were cooled to 23 °C, diluted with water and extracted with ethyl acetate. The organic phases were dried over sodium sulfate, filtered and the solvent was removed in vacuo. The crude material was purified via silica gel chromatography utilizing 70-100% ethyl acetate/hexanes gradient to deliver the desired compound (23 mg, 33% yield) as an off-white solid. ¹H-NMR (500 MHz, DMSO-*d*₆) δ ppm 9.08 (d, 1 H), 8.24 (s, 2 H), 7.49 (s, 1 H), 7.34 (m, 1 H), 7.25 (d, 1 H), 7.22 (m, 1 H), 7.11 (app. t, 1 H), 6.88 (app. t, 1 H), 6.47 (t, 1 H), 5.88 (s, 2 H), 3.61 (dt, 2 H), 3.41 (t, 2 H), 3.05 (s, 3 H).

### Reference Compound 24

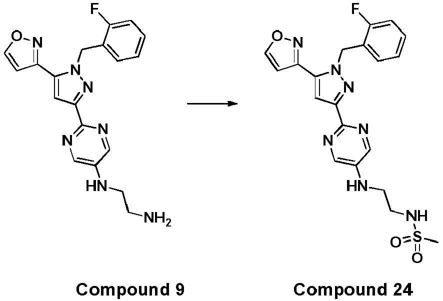

A solution of **Compound 9** (1.0 equiv., as the TFA salt) in dichloromethane at 0 °C was treated with triethylamine (5.0 equiv.) followed by methanesulfonyl chloride (1.1 equiv.). The reaction mixture was warmed to 23 °C and stirred for 3 h at this temperature. The contents were poured into half-saturated sodium bicarbonate solution and extracted with dichloromethane and ethyl acetate. The organic phases were dried over sodium sulfate, filtered and the solvent was removed in vacuo. The crude material was purified via silica gel chromatography utilizing 25% acetonitrile-methanol (7:1) in dichloromethane to deliver the desired compound (9.5 mg, 75% yield) as a light yellow solid. ¹H-NMR (500 MHz, CDCl₃) δ ppm 8.45 (d, 1 H), 8.14 (s, 2 H), 7.30 (s, 1 H), 7.18 (m, 1 H), 7.01 (m, 1 H), 6.95 (app. t, 1 H), 6.84 (app. t, 1 H), 6.60 (d, 1 H), 5.97 (s, 2 H), 5.53 (br. s, 1 H), 3.43-3.35 (m, 4 H), 2.98 (s, 3 H). The exchangeable sulfonamide NH proton was not observed.

### Reference Compound 7

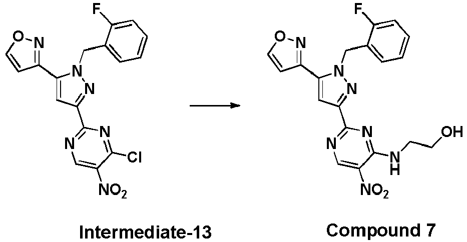

To a solution of **Intermediate-13** (1.0 equiv.) in anhydrous dioxane was added 2-aminoethanol (4.0 equiv.). The reaction mixture became an orange suspension. After 20 h, water was added and the solid was filtered and dried in vacuo to deliver the desired compound (160 mg, 89% yield) as a yellowish tan solid. ¹H-NMR (500 MHz, DMSO-*d*₆) δ ppm 9.19 (s, 1 H), 9.12 (d, 1 H), 8.83 (t, 1 H), 7.73 (s, 1 H), 7.34 (m, 1 H), 7.28 (d, 1 H), 7.23 (m, 1 H), 7.11 (app. t, 1 H), 6.87 (app. t, 1 H), 5.95 (s, 2 H), 4.96 (t, 1 H), 3.80 (dt, 2 H), 3.67 (dt, 2 H).

### Reference Compound 82

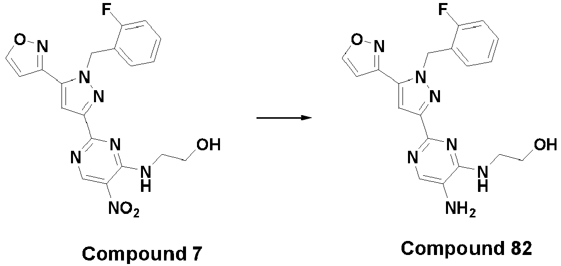

A suspension of **Compound 7** in MeOH/ethyl acetate (1:1) under a nitrogen atmosphere was treated with 10 % palladium on carbon (0.2 equiv.). Hydrogen was introduced (using a balloon) and the resultant mixture was stirred for 1 h 40 min. The reaction vessel was then flushed with nitrogen and the contents were filtered through Celite. The solvent was removed in vacuo to deliver the desired intermediate, 2-((5-amino-2-(1-(2-fluorobenzyl)-5--(isoxazol-3-yl)-1*H*-pyrazol-3-yl)pyrimidin-4-yl)amino)ethanol **(Compound 82),** which was used in the next step without further manipulation.

### Reference Compound 10

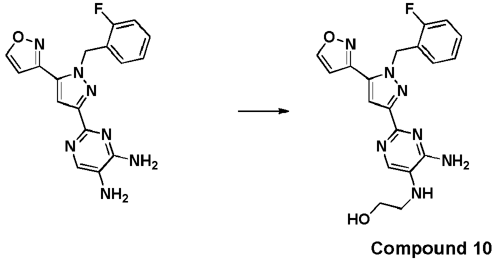

A suspension of 2-(1-(2-fluorobenzyl)-5-(isoxazol-3-yl)-1*H*-pyrazol-3-yl)pyrimidine--4,5-diamine (described in previous patents: WO2012/3405 A1, WO2013/101830 A1) (1.0 equiv.) in anhydrous THF was treated with triethylamine (2.0 equiv.) followed by 1,3,2-dioxathiolane-2,2-dioxide (1.2 equiv.). After 18 h, additional amount of 1,3,2-dioxathiolane-2,2-dioxide (0.30 equiv.) was added and the contents were stirred for 5 h. The reaction mixture was then concentrated in vacuo, re-suspended in aqueous 6 N HCl/THF (3:1 v/v) and heated at 60 °C for 18 h. After cooling to 23 °C, the contents were poured into half-saturated sodium bicarbonate solution and extracted with dichloromethane/iPrOH (4:1). The organic layers were dried over sodium sulfate, filtered and the solvent was removed in vacuo. The crude material was purified via silica gel chromatography utilizing 70-100% acetonitrile-methanol (7:1) in dichloromethane to deliver the desired compound (31 mg, 62% yield) as a beige-colored solid. ¹H-NMR (500 MHz, DMSO-*d*₆) δ ppm 9.19 (d, 1 H), 8.83 (br. s, 1 H), 8.10 (br. s, 1 H), 7.58 (s, 1 H), 7.46 (s, 1 H), 7.39 (m, 1 H), 7.26 (d, 1 H), 7.24 (m, 1 H), 7.19-7.10 (m, 2 H), 5.99 (s, 1 H), 5.89 (s, 2 H), 5.07 (t, 1 H), 4.50 (m, 2 H), 3.62 (m, 2 H).

### Reference Compound 60

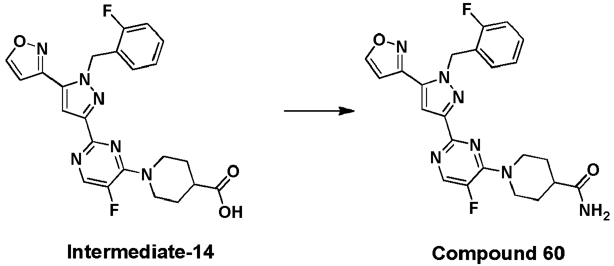

To a suspension of **Intermediate-14** (1.0 equiv.) in dichloromethane at 0 °C was added DAST (2.2 equiv.) in one portion. The mixture was warmed to 23 °C and stirred for 24 h. The solvent was removed in vacuo and the residue dissolved in ammonium hydroxide (conc.), heated to 100 °C for 24 h, and solvent removed under a stream of nitrogen. The crude material was purified via reverse phase HPLC to deliver the desired compound (1.6 mg, 3% yield) as a white solid. ¹H-NMR (500 MHz, CD₃OD) δ ppm 8.83 (m, 1 H), 8.32 (m, 1 H), 7.67 (m, 1 H), 7.32 (m, 1 H), 7.11 (m, 2 H), 6.98 (m, 2 H), 6.04 (m, 2 H), 4.96 (m, 2 H), 3.47 (m, 2 H), 2.75 (m, 1 H), 2.09 (m, 2 H), 1.91 (m, 2 H).

### Compound 105

This compound was prepared in 5 steps:

### Step 1: Synthesis of 3-(ethoxycarbonyl)-1-(2-fluorobenzyl)-1H-pyrazole-5-carboxylic acid.

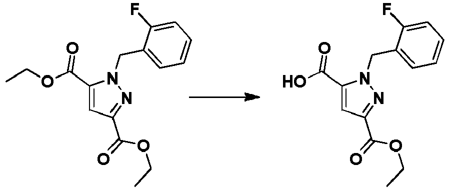

To a suspension of diethyl 1-(2-fluorobenzyl)-1H-pyrazole-3,5-dicarboxylate (previously described in the literature) (1 equiv.) in ethanol was added potassium hydroxide slowly over the course of 1.5 h, as not all of the starting material fully went into solution. After stirring for 15 h at 23 °C, LCMS indicated starting material still present. Added additional 20 mol% of potassium hydroxide, continued to stir at 23 °C for 1.5 h, then added additional 30 mol% and stirred for another 2 h. The solution was poured into saturated NH₄Cl solution and extracted with dichloromethane (6x). The combined organics were dried over magnesium sulfate, filtered, and the solvent was removed in vacuo to deliver the desired intermediate 3-(ethoxycarbonyl)-1-(2-fluorobenzyl)-1H-pyrazole-5-carboxylic acid (2.98 g, 108 % yield) as a white solid. The crude material was carried on to the next step without further purification.

### Step 2: Synthesis of 1-(2-fluorobenzyl)-5-(hydroxymethyl)-1H-pyrazole-3-carboxylic acid.

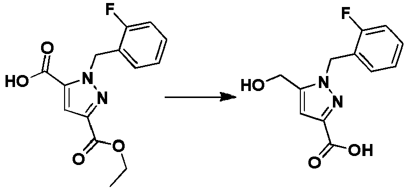

To a solution of 3-(ethoxycarbonyl)-1-(2-fluorobenzyl)-1H-pyrazole-5-carboxylic acid (1 equiv.) in THF at 0°C was added 10M borane-methyl sulfide complex (3 equiv.) dropwise. After gas evolution had ceased (15 min), the solution was slowly warmed to 23°C and then stirred at 65°C for 4 h. The reaction was cooled to 23°C and quenched with IN HCl (aq) and stirred for 1 h. The mixture was diluted with ethyl acetate and washed with water. The organic layer was dried, filtered and evaporated to give the desired intermediate 1-(2-fluorobenzyl)-5-(hydroxymethyl)-1H-pyrazole-3-carboxylic acid (0.59 g, 74% yield) as a colorless oil. ¹H-NMR (500 MHz, DMSO-*d*₆) δ ppm 12.67 (m, 1 H), 7.37 (m, 1 H), 7.24 (m, 1 H), 7.16 (m, 1 H), 7.03 (m, 1 H), 6.65 (m, 1 H), 5.46 (s, 2 H), 4.52(m, 2 H).

### Step 3: Synthesis of methyl

### 1-(2-fluorobenzyl)-5-(methoxymethyl)-1H-pyrazole-3-carboxylate

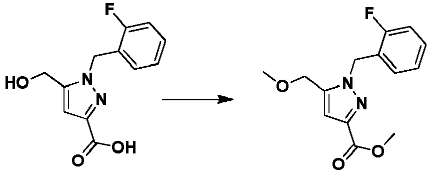

To a solution of 1-(2-fluorobenzyl)-5-(hydroxymethyl)-1H-pyrazole-3-carboxylic acid (1 equiv.) in DMF was added sodium hydride (2.1 equiv.) at 0 °C. The solution was stirred for 30 min at 0 °C and for 30 min at 23 °C. To the solution was added methyl iodide (4.2 equiv.) and stirred for 18 h. The mixture was diluted with ethyl acetate and washed with water. The organic layer was dried, filtered and evaporated to give a crude oil which was purified via silica gel chromatography to deliver the desired intermediate methyl 1-(2-fluorobenzyl)-5-(methoxymethyl)-1H-pyrazole-3-carboxylate (260 mg, 42% yield) as a clear colorless oi1. ¹H-NMR (500 MHz, DMSO-*d*₆) δ ppm 7.34 (m, 1 H), 7.22 (m, 1 H), 7.13 (m, 1 H), 6.90 (m, 2 H), 5.76 (m, 2 H), 4.37 (s, 2 H), 3.81 (m, 3 H), 3.25 (s, 3 H).

### Step 4: Synthesis of 1-(2-fluorobenzyl)-5-(methoxymethyl)-1H-pyrazole-3-carboximidamide (Intermediate-19).

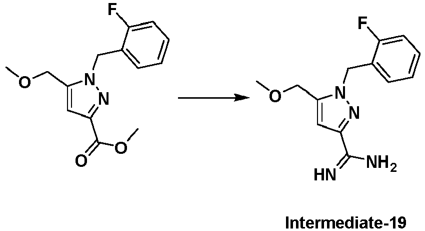

To a suspension of ammonia hydrochloride (5.3 equiv.) in toluene at 0 °C, was added a solution of trimethylaluminum 2M in toluene (5.3 equiv.). The mixture was removed from the ice bath and stirred at 23 °C until bubbling ceased. To this mixture was added a solution of methyl 1-(2-fluorobenzyl)-5-(methoxymethyl)-1H-pyrazole-3-carboxylate (1 equiv.) in toluene and stirred at 80 °C for 24 h. The mixture was cooled in an ice bath and quenched slowly with methanol and the resulting white precipitate was removed by filtration on celite pad. The filtrate was concentrated and dried under vacuum to deliver the desired intermediate 1-(2-fluorobenzyl)-5-(methoxymethyl)-1H-pyrazole-3-carboximidamide (258 mg, 100% yield) as an off-white solid. ¹H-NMR (500 MHz, DMSO-*d*₆) δ ppm 7.29 (m, 6 H), 6.85 (m, 1 H), 5.55 (s, 2 H), 4.36 (s, 2 H), 3.34 (s, 1 H), 3.26 (s, 3 H).

### Step 5: Synthesis of Compound 105

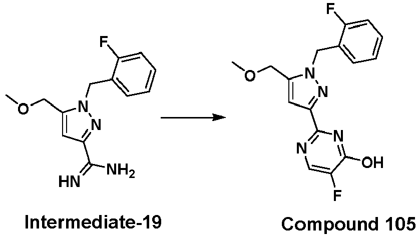

A mixture containing 1-(2-fluorobenzyl)-5-(methoxymethyl)-1H-pyrazole-3-carboximidamide (Intermediate-19, 4 equiv.) in ethanol was stirred at 100°C for 1 h. The mixture was cooled and solvent removed in vacuo. The crude material was purified via reverse phase chromatography to deliver the desired compound (11 mg, 20% yield) as an off white solid. ¹H-NMR (500 MHz, DMSO-*d*₆) δ ppm 8.18 (m, 1 H), 7.31 (d, 1 H), 7.18 (t, 1 H), 7.10 (m, 2 H), 6.93 (t, 1 H), 5.87 (s, 2 H), 4.37 (s, 2 H), 3.27 (s, 3 H).

### Compound 20

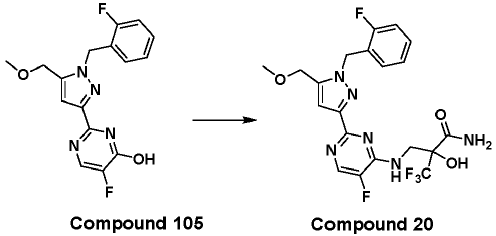

A solution of **Compound 105** (1 equiv.) in phosphoryl trichloride (excess) was heated at 65 °C for 2 h. The solution was cooled and solvent removed under a stream of nitrogen. The resulting residue was dissolved in dioxane and water (3:1), and 2-(aminomethyl)-3,3,3-trifluoro-2-hydroxypropanamide (10 equiv.) and triethylamine (20 equiv.) were added. The solution was stirred for 18 h at 100 °C. The solvent was evaporated and the crude material was purified via reverse phase chromatography to deliver the desired compound (6.7 mg, 47% yield). ¹H-NMR (500 MHz, CDCl₃) δ 8.16 (m, 1 H), 7.22 (m, 1 H), 7.13 (m, 1 H), 7.05 (s, 3 H), 6.80 (m, 1 H), 6.29 (m, 1 H), 6.08 (d, 2 H), 4.58 (s, 2 H), 4.19 (m, 2 H), 3.47 (m, 3 H).

### Reference Compound 5

The title compound was synthesized in 2 steps:

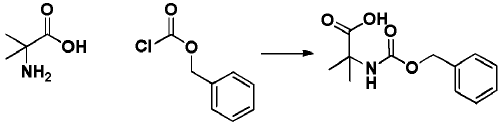

### Step 1: Synthesis of 2-(((benzyloxy)carbonyl)amino)-2-methylpropanoic acid.

A mixture containing sodium carbonate (3 equiv.), 2-amino-2-methylpropanoic acid (1.0 equiv.) and benzyl chloroformate (1.1 equiv.) in water and 1,4-dioxane (2:1) was stirred at 23 °C for 24 h. The mixture was diluted in ethyl acetate and washed with IN HCl solution. The organic layer was dried, filtered and evaporated to deliver the desired intermediate 2-(((benzyloxy)carbonyl)amino)-2-methylpropanoic acid (825 mg, 72% yield) as a clear oil. ¹H NMR (500 MHz, CD₃OD) δ ppm 7.32 - 7.38 (m, 5 H), 5.03 - 5.09 (m, 2 H), 1.43 - 1.50 (m, 6 H).

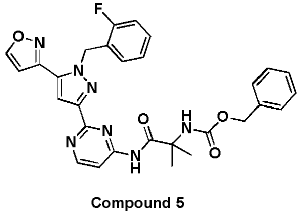

### Step 2: Synthesis of Compound 5

The title compound was prepared following general procedure C, except 2-(((benzyloxy)carbonyl)amino)-2-methylpropanoic acid (1 equiv.) was the acid reactant, 2.5 equivalents of T3P was used, contents were heated at 70 °C for 24 h, and ethyl acetate was used for extraction during workup. The crude material was purified via silica gel chromatography to deliver the desired compound (87 mg, 7% yield) as a brown solid. ¹H NMR (500 MHz, CD₃OD) δ ppm 8.79 (s, 1 H), 8.68 (d, 1 H), 8.09 - 8.20 (m, 1 H), 7.98 (s, 1 H), 7.52 (s, 1 H), 7.21 - 7.39 (m, 4 H), 7.17 (br. s., 1 H), 7.02 - 7.15 (m, 2 H), 6.85 - 6.92 (m, 2 H), 5.99 (s, 2 H), 5.06 (s, 2 H), 1.48 - 1.51 (m, 6 H).

### Reference Compound 33

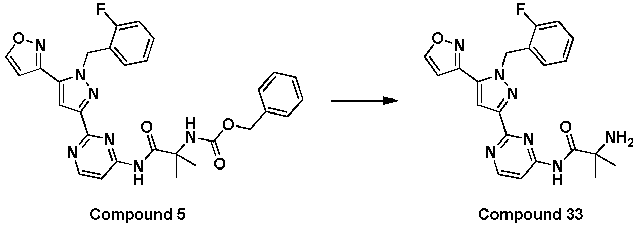

A mixture containing palladium on carbon (0.1 equiv.) and Compound 5 (1 equiv.) in ethanol at ambient temperature was hydrogenated over a balloon of hydrogen for 24 h. The mixture was filtered through an acro disk and the filtrate was concentrated under vacuum to deliver the desired compound (66 mg, 99% yield) as a white solid. ¹H NMR (500 MHz, CDCl₃) δ ppm 8.68 - 8.74 (m, 1 H), 8.46 (s, 1 H), 8.14 - 8.18 (m, 1 H), 7.44 - 7.48 (m, 1 H), 7.19 (q, 1 H), 7.00 - 7.09 (m, 1 H), 6.96 (t, 1 H), 6.81 (t, 1 H), 6.58 - 6.63 (m, 1 H), 6.02 (s, 2 H), 1.44 - 1.48 (m, 6 H).

### Reference Compound 46

The title compound was synthesized in 2 steps:

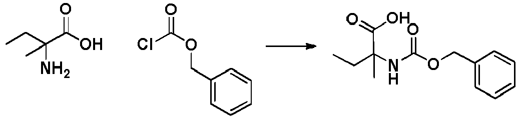

### Step 1: Synthesis of 2-(((benzyloxy)carbonyl)amino)-2-methylbutanoic acid.

A mixture containing 2-amino-2-methylbutyric acid hydrochloride (1 equiv.), sodium carbonate (3 equiv.) and benzyl chloroformate (1.1 equiv.) in 1,4-dioxane and water (2:1) was stirred at 23 °C for 24 h. The mixture was diluted in ethyl acetate and washed with IN HCl solution. The organic layer was dried, filtered and evaporated to deliver the desired intermediate 2-(((benzyloxy)carbonyl)amino)-2-methylbutanoic acid (499 mg, 99% yield) as a clear oil. ¹H NMR (500 MHz, CD₃OD) δ ppm 7.30 - 7.38 (m, 5 H), 5.01 - 5.09 (m, 2 H), 1.85 - 1.95 (m, 2 H), 1.43 - 1.50 (m, 3 H), 0.86 (t, 3 H).

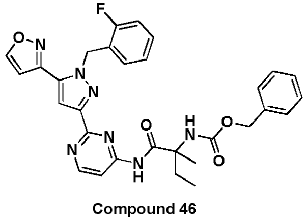

### Step 2: Synthesis of Compound 46

The title compound was prepared following general procedure C, except 2-(((benzyloxy)carbonyl)amino)-2-methylbutanoic acid (1 equiv.) was the acid reactant, 2.5 equivalents of T3P was used, contents were heated at 70 °C for 3 d, and ethyl acetate was used for extraction during workup. The crude material was purified via silica gel chromatography (0 to 100% ethyl acetate in hexanes) to deliver the desired compound (40 mg, 5% yield). ¹H NMR (500 MHz, CD₃OD) δ ppm 8.78 (s, 1 H), 8.67 (d, 1 H), 8.12 (br. s., 1 H), 7.51 (s, 1 H), 7.22 - 7.36 (m, 4 H), 7.17 (d, 1 H), 7.06 - 7.13 (m, 1 H), 7.04 (t, 1 H), 6.82 - 6.96 (m, 3 H), 5.98 (s, 2 H), 5.05 (br. s., 2 H), 1.84 - 2.04 (m, 2 H), 1.46 - 1.51 (m, 3 H), 0.85 - 0.94 (m, 3 H).

### Reference Compound 47

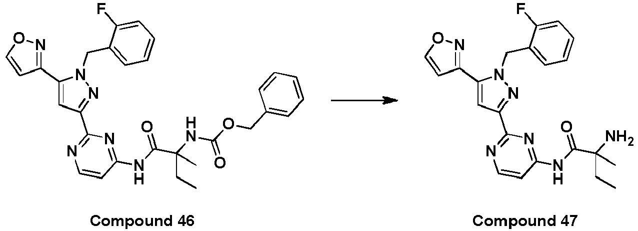

A mixture containing palladium on carbon (0.1 equiv.) and **Compound 46** (1 equiv.) in ethanol at 23 °C was placed under an atmosphere of hydrogen for 24 h. The mixture was filtered through an acro disk and the filtrate was concentrated under vacuum to deliver the desired compound (25 mg, 100% yield) as a clear oil. ¹H NMR (500 MHz, CDCl₃) δ ppm 8.71 (d, 1 H), 8.46 (s, 1 H), 8.18 (d, 1 H), 7.47 (s, 1 H), 7.17 - 7.24 (m, 1 H), 7.04 (d, 1 H), 6.97 (t, 1 H), 6.82 (t, 1 H), 6.61 (s, 1 H), 6.03 (s, 2 H), 1.91 - 2.02 (m, 2 H), 1.44 (s, 3 H), 0.92 - 0.97 (m, 3 H).

### Reference Compound 55

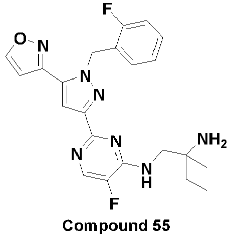

The title compound was prepared following general procedure B, except 2-methylbutane-1,2-diamine (1.1 equiv.) was the amine reactant, 1 equivalent of triethylamine was used, and the contents were stirred as a solution in DMF at 23 °C until complete consumption of starting material by LC/MS. The reaction was diluted with ethyl acetate and water. The organic layer was dried over magnesium sulfate, filtered, and the solvent was removed in vacuo. The residue was purified via silica gel chromatography (0 to 10% methanol in dichlromethane) delivered the desired compound (67 mg, 15% yield) as a white solid. ¹H NMR (500 MHz, CD₃OD) δ ppm 8.67 - 8.74 (m, 1 H), 8.02 (d, 1 H), 7.38 (s, 1 H), 7.21 (q, 1 H), 7.01 - 7.08 (m, 1 H), 6.97 (t, 1 H), 6.84 (s, 1 H), 6.77 (t, 1 H), 5.87 - 5.94 (m, 2 H), 3.25 - 3.29 (m, 2 H), 1.45 - 1.55 (m, 2 H), 1.07 - 1.12 (m, 3 H), 0.90 - 0.97 (m, 3 H).

### Reference Compound 67

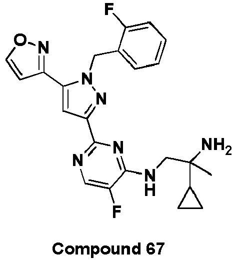

The title compound was prepared following general procedure B, except 2-cyclopropylpropane-1,2-diamine dihydrochloride (2 equiv.) was the amine reactant, 4 equivalents of triethylamine was used, and the contents were stirred as a solution in DMF at 23 °C until complete consumption of starting material by LC/MS. The reaction was diluted with ethyl acetate and water. The organic layer was dried over magnesium sulfate, filtered, and the solvent was removed in vacuo. The residue was purified via silica gel chromatography (0 to 10% methanol in dichlromethane) delivered the desired compound (81 mg, 67% yield) as a clear oil. ¹H NMR (500 MHz, CD₃OD) δ ppm 8.75 (s, 1 H), 8.08 (d, 1 H), 7.44 (s, 1 H), 7.21 - 7.30 (m, 1 H), 7.08 (t, 1 H), 7.01 (t, 1 H), 6.89 (s, 1 H), 6.79 (t, 1 H), 5.95 (s, 2H), 3.35 (s, 2 H), 1.03 - 1.09 (m, 1H), 1.02 (s, 3 H), 0.37 - 0.51 (m, 4 H).

### Reference Compound 68

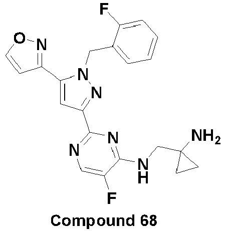

The title compound was prepared following general procedure B, except 1-(aminomethyl)cyclopropanamine (as the 2HCl salt, 2 equiv.) was the amine reactant, 8 equivalents of triethylamine was used, and the contents were stirred as a solution in DMF at 23 °C until complete consumption of starting material by LC/MS. The reaction was diluted with ethyl acetate and water. The organic layer was dried over magnesium sulfate, filtered, and the solvent was removed in vacuo. The residue was purified via silica gel chromatography (0 to 10% methanol in dichlromethane) delivered the desired compound (54 mg, 40% yield) as a white solid. ¹H NMR (500 MHz, CD₃OD) δ ppm 8.75 (s, 1 H), 8.05 (d, 1 H), 7.42 (s, 1 H), 7.17 - 7.33 (m, 1 H), 7.05 - 7.15 (m, 1 H), 7.02 (t, 1 H), 6.89 (s, 1 H), 6.81 (t, 1 H), 5.95 (s, 2 H), 3.69 (s, 2 H), 0.59 - 0.77 (m, 4 H).

### Reference Compound 106

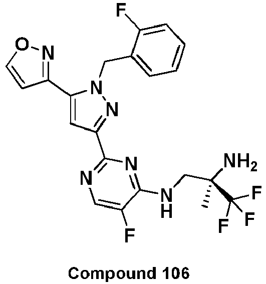

The title compound was prepared following general procedure B, except (*R*)-3,3,3-trifluoro-2-methylpropane-1,2-diamine dihydrochloride was the amine reactant, 6 equivalents of triethylamine was used, and the contents were stirred at 23 °C as a solution in DMF until complete consumption of starting material was observed by LC/MS. The solution was diluted with ethyl acetate and water. The organic layer was dried over magnesium sulfate, filtered, and the solvent was removed in vacuo. Purification of the residue via silica gel chromatography (0 to 10% methanol in dichloromethane) delivered the desired compound (108 mg, 84 % yield) as a white solid. ¹H NMR (500 MHz, CD₃OD) δ ppm 8.76 (d, 1 H), 8.11 (d, 1 H), 7.41 (s, 1 H), 7.19 - 7.36 (m, 1 H), 7.06 - 7.13 (m, 1 H), 7.03 (t, 1 H), 6.89 (d, 1 H), 6.85 (t, 1 H), 5.95 (s, 2 H), 3.80 - 3.98 (m, 2 H), 1.32 (s, 3 H).

### Reference Compound 107

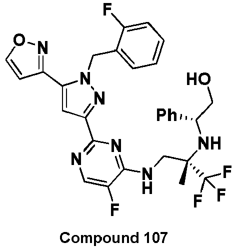

The title compound was prepared following general procedure B, except (*R*)-2-(((*S*)-3-amino-1,1,1-trifluoro-2-methylpropan-2-yl)amino)-2-phenylethanol was the amine reactant, 6 equivalents of triethylamine was used, and the contents were stirred at 23 °C as a solution in DMF until complete consumption of starting material was observed by LC/MS. The solution was diluted with ethyl acetate and water. The organic layer was dried over magnesium sulfate, filtered, and the solvent was removed in vacuo. Purification of the residue via silica gel chromatography (0 to 10% methanol in dichloromethane) delivered the desired compound (72 mg, 70% yield) as a white solid. ¹H-NMR (500 MHz, CD₃OD) δ ppm 8.67 - 8.78 (m, 1 H), 8.12 (d, 1 H), 7.40 (s, 1 H), 7.09 - 7.33 (m, 6 H), 7.01 - 7.07 (m, 1 H), 6.97 (t, 1 H), 6.87 (d, 1 H), 6.82 (t, 1 H), 5.92 (s, 2 H), 4.12 (dd, 1 H), 3.80 - 3.99 (m, 2 H), 3.48 (dd, 1 H), 3.31 (d, 1 H), 1.10 (s, 3 H).

### Reference Compound 108

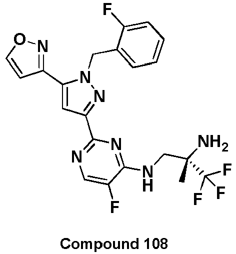

The title compound was prepared following general procedure B, except (*S*)-3,3,3-trifluoro-2-methylpropane-1,2-diamine dihydrochloride was the amine reactant, 6 equivalents of triethylamine was used, and the contents were stirred at 23 °C as a solution in DMF until complete consumption of starting material was observed by LC/MS. The solution was diluted with ethyl acetate and water. The organic layer was dried over magnesium sulfate, filtered, and the solvent was removed in vacuo. Purification of the residue via silica gel chromatography (0 to 10% methanol in dichloromethane) delivered the desired compound (58 mg, 46% yield) as a white solid. ¹H-NMR (500 MHz, CD₃OD) δ ppm 8.75 (s, 1 H), 8.10 (d, 1 H), 7.40 (s, 1 H), 7.26 (q, 1 H), 7.08 (s, 1 H), 7.02 (s, 1 H), 6.87 (d, 1 H), 6.84 (t, 1 H), 5.94 (s, 2 H), 3.88 - 3.99 (m, 1 H), 3.80 - 3.88 (m, 1 H), 1.31 (s, 3 H).**Compound 111** The title compound was prepared over 5 steps:

### Step 1: Synthesis of N-methoxy-N-methylisothiazole-3-carboxamide

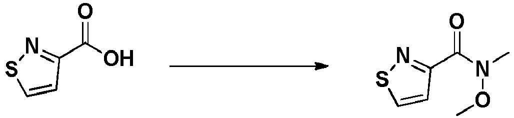

To a suspension of isothiazole-3-carboxylic acid (2 g, 15.49 mmol) in DCM at 0 °C was added oxalyl dichloride (1.3 equiv.) followed by three drops of DMF. Bubbling commenced, and the reaction was warmed to 23 °C after 10 min. The mixture was stirred for 3 h, then cooled to 0 °C. *N*,*O*-dimethylhydroxylamine hydrochloride (1.3 equiv.) was added to the reaction, followed by triethylamine (3.5 equiv.) which was added dropwise via syringe over 10 min. Reactions was warm slowly to 23 °C overnight, and stirred for a total of 15 h. Reaction mixture was diluted with 1N HCl solution and dichloromethane (1:1 ratio). Layers were separated, and the aqueous layer was extracted with DCM (2x). Combined organic layers were dried over magnesium sulfate, filtered, and the solvent was removed in vacuo. Crude reside purified via silica gel chromatography (utilizing a hexane/ethyl acetate mix as eluent) to deliver the desired intermediate, N-methoxy-N-methylisothiazole-3-carboxamide (1 g, 38% yield) as a pale yellow solid. ¹H NMR (400 MHz, CDCl₃) δ ppm 8.67 (d, 1 H), 7.69 (br s, 1 H), 3.80 (s, 3 H), 3.46 (br s, 3 H).

### Step 2: Synthesis of (E)-ethyl 4-(isothiazol-3-yl)-2-(methoxy(methyl)amino)--4-oxobut-2-enoate

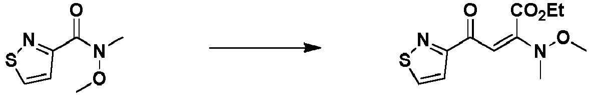

To a solution of N-methoxy-N-methylisothiazole-3-carboxamide (200 mg, 1.161 mmol) and ethyl propiolate (1.5 equiv.) at -55 °C (2:1 ethanol/water w/dry ice) in THF was added sodium bis(trimethylsilyl)amide (1.4 equiv., 1 N solution in THF) over the course of 5 min. The reaction was warmed to -45 °C over 15 min, then to 30 °C over 15 min, then stirred for an additional 15 min. The reaction was treated with IN aqueous HCl solution, stirred at -30 for 3 min, then treated with 10% aqueous citric acid to acidify to pH 2. The mixture was warmed to 23 °C, then partitioned between dichloromethane and water. The layers were separated, and the aqueous layer was extracted with dichloromethane (2x) and ethyl acetate (1x). The combined organic layers were dried over magnesium sulfate, filtered, and concentrated in vacuo. The crude residue was purified via silica gel chromatgrarphy (utilizing a hexane/ethyl acetate mix as eluent) to deliver the desired intermediate, (*E*)-ethyl 4-(isothiazol-3-yl)-2-(methoxy(methyl)amino)-4-oxobut-2-enoate (234 mg, 75% yield) as an oil. ¹H NMR (400 MHz, CDCl₃) δ ppm 8.58 (d, 1 H), 7.78 (d, 1 H), 6.43 (s, 1 H), 4.42 (q, 2 H), 3.70 (s, 3 H), 3.16 (s, 3 H), 1.35 (t, 3 H).

### Step 3: Synthesis of ethyl 1-(2-fluorobenzyl)-5-(isothiazol-3-yl)-1H-pyrazole-3-carboxylate

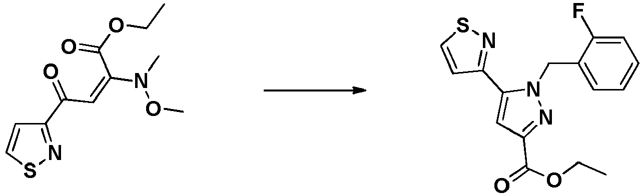

To a solution of (2-fluorobenzyl)hydrazine hydrochloride (1.1 equiv.) in ethanol and water (10:1 ratio) was added potassium carbonate (0.55 equiv.) as a solution in water, followed immediately with (*E*)-ethyl 4-(isothiazol-3-yl)-2-(methoxy(methyl)amino)-4-oxobut-2-enoate (1 equiv.) as a solution in ethanol. The reaction was stirred at 23 °C for 3 h, then diluted with dichloromethane and aqueous IN HCl solution (3:1 ratio). The layers were separated and the aqueous layer was extracted with dichloromethane (2x). The combined organics were dried over magnesium sulfate, filtered, and the solvent was removed in vacuo. The crude reside was purified via silica gel chromatography (utilizing a hexane/ethyl acetate mix as eluent) to deliver the desired intermediate, ethyl 1-(2-fluorobenzyl)-5-(isothiazol-3-yl)-1H-pyrazole-3--carboxylate (166 mg, 57.9 % yield) as a white solid. ¹H NMR (400 MHz, CDCl₃) δ ppm 8.66 (d, 1 H), 7.44 (d, 1 H), 7.18 (s, 1 H), 7.11 - 7.17 (m, 1 H), 6.90 - 7.02 (m, 2 H), 6.76 (td, 1 H), 6.10 (s, 2 H), 4.41 (q, 2H), 1.39 (t, 3 H).

### Step 4: Synthesis of 1-(2-fluorobenzyl)-5-(isothiazol-3-yl)-1H-pyrazole-3-carboximidamide

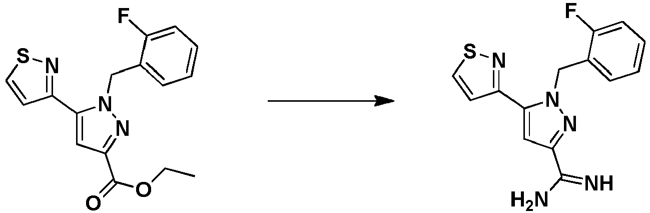

To a suspension of ammonia hydrochloride (5.5 equiv.) in toluene was added over the course of 5 min trimethylaluminum (5 equiv.) as a 2M solution in toluene. After bubbling ceased, the the solution was added directly to ethyl 1-(2-fluorobenzyl)-5-(isothiazol-3-yl)-1H--pyrazole-3-carboxylate (1 equiv.). The reaction mixture was heated to 100 °C in a closed vial (with periodic releasing of pressure as temperature rose) for 3 h. The reaction was cooled to 0 °C, then treated with methanol (10 equiv.). After warming to 23 °C and stirring for 15 min, contents diluted with toluene, and filtered through celite. The solids were washed with 5 mL of methanol, then the filtrate was concentrated in vacuo to deliver the desired intermediate, 1-(2-fluorobenzyl)-5-(isothiazol-3-yl)-1H-pyrazole-3-carboximidamide (130 mg, 86 % yield) as a yellow solid. Contents carried on without further purification. ¹H NMR (400 MHz, DMSO-*d*₆) δ ppm 9.27 (d, 1 H), 7.85 (s, 1 H), 7.74 (d, 1 H), 7.30 - 7.37 (m, 1 H), 7.19 - 7.24 (m, 1 H), 7.11 (td, 1 H), 6.91 (td, 1H), 6.09 (s, 2 H).

### Step 5: Synthesis of Compound 111

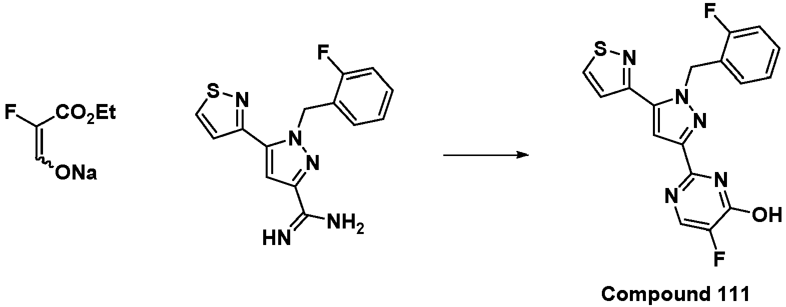

A suspension of sodium 3-ethoxy-2-fluoro-3-oxoprop-1-en-1-olate (3 equiv.) and 1-(2 fluorobenzyl)-5-(isothiazol-3-yl)-1H-pyrazole-3-carboximidamide (1 equiv.) in ethanol was stirred at 90 °C for 1 h 30 min. The solvent was removed in vacuo, and the dark solid was suspended in dichloromethane. The solids were filtered, then purified via silica gel chromatography (utilizing a hexane/ethyl acetate mix as eluent) to deliver the desired compound (53 mg, 33.1 % yield) as a tan solid. ¹H NMR (400 MHz, CD₃OD) δ ppm 9.00 (d, 1 H), 8.04 (d, 1 H), 7.74 (d, 1 H), 7.45 (s, 1 H), 7.22 - 7.30 (m, 1 H), 6.99 - 7.12 (m, 2 H), 6.89 (br t, 1 H), 6.17 (s, 2 H).

### Reference Compound 112

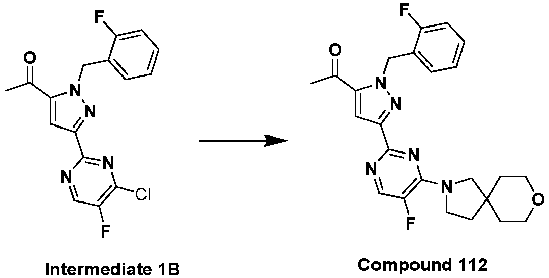

A solution of 8-oxa-2-azaspiro[4.5]decane (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in dioxane was stirred at 130 °C for 3 h. The crude material was purified via reverse phase HPLC to deliver the desired compound (15 mg, 54.8 % yield) as an off-white solid. ¹H NMR (500 MHz, DMSO-*d*₆) δ ppm 8.28 (d, 1 H), 7.74 (s, 1 H), 7.34 (q, 1 H), 7.20 - 7.26 (m, 1 H), 7.11 (t, 1 H), 6.82 (t, 1 H), 5.83 (s, 2 H), 3.83 (br. s., 2 H), 3.58 - 3.66 (m, 6 H), 2.58 (s, 3 H), 1.91 (br. s., 2 H), 1.57 - 1.64 (m, 2 H), 1.50 - 1.57 (m, 2 H).

### Reference Compound 113

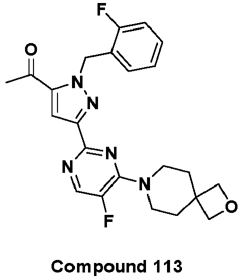

A solution of 2-oxa-7-azaspiro[3.5]nonane (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in dioxane was stirred at 130 °C for 3 h. The crude material was purified via reverse phase HPLC to deliver the desired compound (17 mg, 64.1 % yield) as a white solid. ¹H-NMR (500 MHz, DMSO-*d*₆) δ ppm 8.28 - 8.33 (m, 1 H), 7.71 - 7.75 (m, 1 H), 7.30 - 7.37 (m, 1 H), 7.20 - 7.26 (m, 1H), 7.11 (t, 1H), 6.81 (t, 1 H), 5.82 (s, 2 H), 4.37 (s, 4 H), 3.70 - 3.76 (m, 4 H), 2.58 (s, 3 H), 1.88 - 1.93 (m, 4 H).

### Reference Compound 114

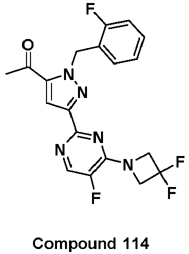

A solution of 3,3-difluoroazetidine (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound 11 mg, 45.0 % yield) as a white solid. ¹H-NMR (500 MHz, DMSO-*d*₆) δ ppm 8.41 (d, 1 H), 7.72 (s, 1 H), 7.30 - 7.36 (m, 1 H), 7.20 - 7.26 (m, 1 H), 7.11 (t, 1 H), 6.79 (t, 1 H), 5.83 (s, 2 H), 4.72 (t, 4 H), 2.57 (s, 3 H).

### Reference Compound 115

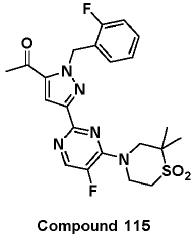

A solution of 2,2-dimethylthiomorpholine 1,1-dioxide (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound (9 mg, 31.4 % yield) as a white solid. ¹H NMR (500 MHz, DMSO-*d*₆) δ ppm 8.42 (d, 1 H), 7.76 (s, 1 H), 7.31 - 7.37 (m, 1 H), 7.19 - 7.25 (m, 1 H), 7.12 (t, 1 H), 6.86 (t, 1 H), 5.82 (s, 2 H), 4.25 (br. s., 2 H), 4.00 (br. s., 2 H), 3.43 (t, 2 H), 2.59 (s, 3 H), 1.30 (s, 6H).

### Reference Compound 116

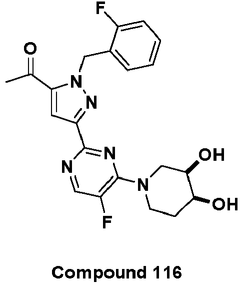

A solution of (3*R*,4*S*)-piperidine-3,4-diol (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound (14 mg, 54.0 % yield) as a white solid. ¹H NMR (500 MHz, DMSO-*d*₆) δ ppm 8.29 (dd, 1 H), 7.73 (d, 1 H), 7.30 - 7.37 (m, 1 H), 7.20 - 7.26 (m, 1 H), 7.11 (t, 1 H), 6.81 (t, 1 H), 5.83 (s, 2 H), 4.06 (br. s., 1 H), 3.86 - 3.95 (m, 1 H), 3.71 - 3.71(m, 1 H), 3.64 - 3.80 (m, 2 H), 3.51 - 3.59 (m, 1 H), 2.58 (s, 3 H), 1.75 - 1.85 (m, 1 H), 1.66 (d, 1 H).

### Reference Compound 117

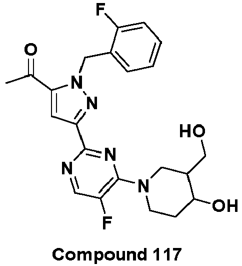

A solution of 4-(hydroxymethyl)piperidin-4-ol (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound (16 mg, 59.8 % yield) as a white solid. ¹H NMR (500 MHz, DMSO-*d*₆) δ ppm 8.30 (t, 1 H), 7.74 - 7.78 (m, 1 H), 7.30 - 7.36 (m, 1 H), 7.20 - 7.26 (m, 1 H), 7.11 (t, 1 H), 6.81 (t, 1 H), 5.83 (s, 2 H), 4.38 (br. s., 2 H), 3.41 (t, 2 H), 3.22 (s, 2 H), 2.58 (s, 3 H), 1.68 (t, 2 H), 1.49 (d, 2 H).

### Reference Compound 118

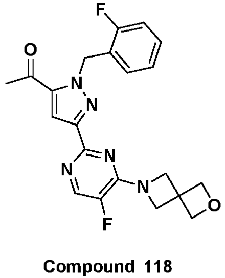

A solution of 2-oxa-6-azaspiro[3.3]heptane (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound (8 mg, 32.2 % yield) as a white solid. ¹H NMR (500 MHz, DMSO-*d*₆) δ ppm 8.27 (d, 1 H), 7.67 (s, 1 H), 7.33 (q, 1 H), 7.20 - 7.26 (m, 1 H), 7.11 (t, 1 H), 6.79 (t, 1 H), 5.83 (s, 2 H), 4.73 (s, 4 H), 4.47 (br. s., 4 H), 2.57 (s, 3 H).

### Reference Compound 119

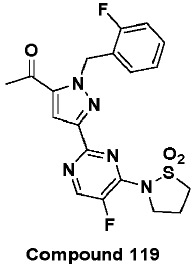

A suspension of isothiazolidine 1,1-dioxide (1.2 equiv.), cesium carbonate (1.5 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 2 h. The solution was diluted with ethyl acetate, and washed with water and brine. The organics were combined, dried over magnesium sulfate, filtered, and the solvent was removed in vacuo. The crude residue was purified via reverse phase HPLC to deliver the desired compound (11 mg, 42.0 % yield) as a white solid. ¹H NMR (500 MHz, DMSO-*d*₆) δ ppm 8.80 (d, 1 H), 7.78 (s, 1 H), 7.31 - 7.37 (m, 1 H), 7.20 - 7.26 (m, 1 H), 7.11 (t, 1 H), 6.84 (t, 1 H), 5.85 (s, 2 H), 4.15 (t, 2 H), 3.64 (t, 2 H), 2.59 (s, 3 H), 2.50 (t, 2H).

### Reference Compound 120

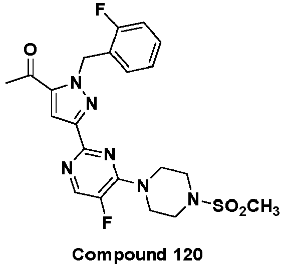

A solution of 1-(methylsulfonyl)-piperazine (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 3 h. The crude material was purified via reverse phase HPLC to deliver the desired compound (14 mg, 48.7 % yield) as a white solid. ¹H NMR (500 MHz, DMSO-*d*₆) δ ppm 8.38 (d, 1 H), 7.76 (s, 1 H), 7.30 - 7.37 (m, 1 H), 7.20 - 7.26 (m, 1 H), 7.11 (t, 1 H), 6.80 (t, 1 H), 5.83 (s, 2 H), 3.93 (br. s., 4 H), 3.28 (d, 4 H), 2.91 (s, 3 H), 2.58 (s, 3 H).

### Reference Compound 121

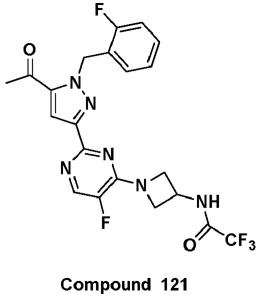

A solution of *tert*-butyl azetidin-3-ylcarbamate (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 1 h. The solution was diluted with ethyl acetate, and washed with aqueous IN HCl solution, water, and brine. The solution was concentrated in vacuo, re-dissolved in DCM, treated with trifluoroacetic anhydride (1 equiv.) and stirred for 2 h at 23 °C. The solvent was removed in vacuo, and the crude material was purified via reverse phase HPLC to deliver the desired compound (12 mg, 27.6 % yield) as a white solid. ¹H NMR (500 MHz, DMSO-*d*₆) δ ppm 10.11 (d, 1 H) 8.31 (d, 1 H) 7.69 (s, 1 H) 7.30 - 7.36 (m, 1H) 7.20 - 7.26 (m, 1 H) 7.11 (t, 1 H) 6.80 (t, 1 H) 5.83 (s, 2 H) 4.77 (dq, 1 H) 4.59 (br. S, 2 H) 4.30 (d, 2 H) 2.57 (s, 3 H).

### Reference Compound 122

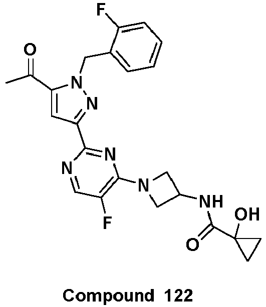

A solution of *N*-(azetidin-3-yl)-1-hydroxycyclopropanecarboxamide (1.5 equiv.), triethylamine (10 equiv.), and 1-(3-(4-chloro-5-fluoropyrimidin-2-yl)-1-(2-fluorobenzyl)--1H-pyrazol-5-yl)ethanone (**Intermediate-1B**, 1 equiv) in DMF was stirred at 130 °C for 2 h. The crude material was purified via reverse phase HPLC to deliver the desired compound (11 mg, 39 % yield) as a white solid. ¹H NMR (500 MHz, DMSO-*d*₆) δ ppm 8.75 (d, 1 H), 8.28 (d, 1 H), 7.69 (s, 1 H), 7.30 - 7.37 (m, 1 H), 7.20 - 7.26 (m, 1 H), 7.11 (t, 1 H), 6.80 (t, 1 H), 5.83 (s, 2 H), 4.73 - 4.81 (m, 1 H), 4.51 (br. s., 2 H), 4.29 (br. s., 2 H), 2.57 (s, 3 H), 1.00 - 1.05 (m, 2 H), 0.82 - 0.88 (m, 2 H).

### Reference Compound 123

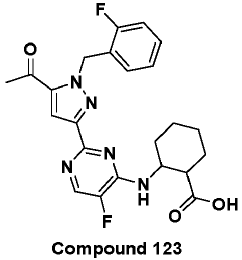

A solution of 2-aminocyclohexanecarboxylic acid (1.2 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 110 °C for 1 h. The crude material was purified via reverse phase HPLC to deliver the desired compound (3.4 mg, 12 % yield) as a white solid. ¹H NMR (500 MHz, DMSO-*d*₆) δ ppm 8.22 (d, 1 H), 7.65 (s, 1 H), 7.30 - 7.37 (m, 1 H), 7.18 - 7.29 (m, 2 H), 7.11 (t, 1 H), 6.84 (t, 1 H), 5.82 (s, 2 H), 4.57 (br. s., 1 H), 2.90 (d, 1 H), 2.58 (s, 3 H), 2.04 (d, 1 H), 1.81 - 1.92 (m, 1 H), 1.61 - 1.74 (m, 2 H), 1.36 - 1.55 (m, 4 H).

### Reference Compound 124

The title compound was prepared in 2 steps:

### Step 1: Synthesis of 1-(3-(4-(3-aminoazetidin-1-yl)-5-fluoropyrimidin-2-yl)-1-(2-fluorobenzyl)-1H-pyrazol-5-yl)ethanone

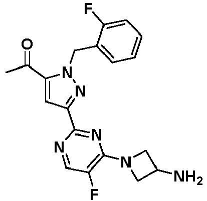

A solution of *tert*-butyl azetidin-3-ylcarbamate (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in DMF was stirred at 130 °C for 1 h. The crude material was purified via reverse phase HPLC to deliver the desired intermediate, 1-(3-(4-(3-aminoazetidin-1-yl)-5-fluoropyrimidin-2-yl)-1-(2-fluorobenzyl)-1H-pyrazol-5-yl)ethanone (33 mg, 99% yield) as a solid. Compound taken onto next step without further purification.

### Step 2: Synthesis of Compound 124

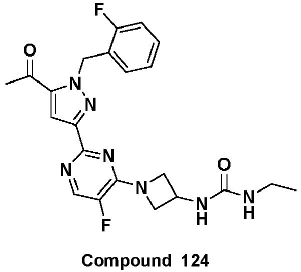

To a stirred solution of isocyanatoethane (1.5 equiv) in toluene was added triethylamine (1 equiv.) and 1-(3-(4-(3-aminoazetidin-1-yl)-5-fluoropyrimidin-2-yl)--1-(2-fluorobenzyl)-1H-pyrazol-5-yl)ethanon. The mixture was stirred and heated at 90 °C for 48 hours, then concentrated in vacuo. The resulting crude residue was purified via reverse phase HPLC to deliver the desired compound (6.0 mg, 15 % yield) as a white solid. 1H NMR (500 MHz, CDCl₃) δ ppm 7.91 (brs, 1 H), 7.30 (br s, 1 H), 7.10 (br t, 1 H), 6.91 - 7.04 (m, 3 H), 5.83 (br d, 2 H), 4.81 (br s, 1 H), 4.42 - 4.58 (m, 1 H), 4.32 (br s, 2 H), 3.14 - 3.22 (m, 2 H), 2.45 - 2.50 (m, 2 H), 2.45 (s, 3 H), 1.12 - 1.19 (m, 1H), 1.08 (br t, 3 H).

### Reference Compound 125

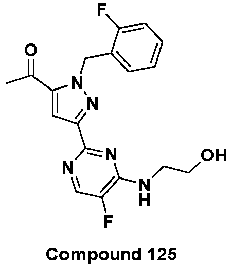

A solution of 2-aminoethanol (10 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in 1,4-dioxane was stirred at 90 °C for 5 h. The crude material was purified via reverse phase HPLC to deliver the desired compound (19.5 mg, 91 % yield) as a white solid. ¹H NMR (500 MHz, DMSO-*d*₆) δ ppm 8.23 (d, 1 H), 7.97 (br. s., 1 H), 7.71 (s, 1 H), 7.30 - 7.36 (m, 1 H), 7.20 - 7.26 (m, 1 H), 7.11 (t, 1 H), 6.82 (t, 1 H), 5.83 (s, 2 H), 3.57 - 3.63 (m, 4 H), 2.58 (s, 3 H).

### Reference Compound 126

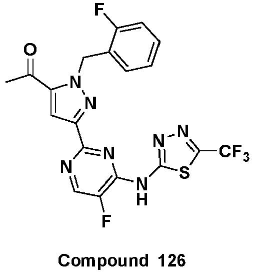

A solution of 5-(trifluoromethyl)-1,3,4-thiadiazol-2-amine (1.5 equiv.), triethylamine (10 equiv.), and 1-(3-(4-chloro-5-fluoropyrimidin-2-yl)-1-(2-fluorobenzyl)-1H-pyrazol-5-yl)ethanone (**Intermediate-1B**, 1 equiv) in 1,4-dioxane was stirred at 90 °C for 5 h. The crude material was purified via reverse phase HPLC to deliver the desired compound (10.4 mg, 38 % yield) as a white solid. ¹H NMR (500 MHz, DMSO-*d*₆) δ ppm 8.76 (br. s., 1 H), 7.80 (s, 1 H), 7.31 - 7.40 (m, 2 H), 7.16 - 7.23 (m, 2 H), 7.10 - 7.15 (m, 1 H), 5.89 (s, 2 H), 2.63 (s, 3 H).

### Reference Compound 193

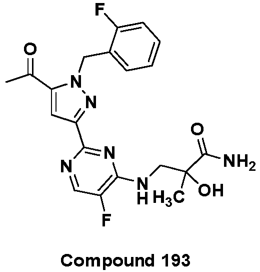

A solution of 3-amino-2-hydroxy-2-methylpropanamide (1.5 equiv.), triethylamine (10 equiv.), and 1-(3-(4-chloro-5-fluoropyrimidin-2-yl)-1-(2-fluorobenzyl)--1H-pyrazol-5-yl)ethanone (**Intermediate-1B**, 1 equiv) in 1,4-dioxane was stirred at 90 °C for 5 h. The crude material was purified via reverse phase HPLC to deliver the desired compound (19 mg, 77 % yield) as a white solid. ¹H NMR (500 MHz, DMSO-*d*₆) δ ppm 8.24 (d, 1 H), 7.69 (s, 1 H), 7.38 (br. s., 1 H), 7.30 - 7.35 (m, 2 H), 7.19 - 7.26 (m, 2 H), 7.11 (t, 1 H), 6.84 (t, 1 H), 5.83 (s, 2 H), 3.76 (dd, 1 H), 3.59 (dd, 1 H), 2.57 (s, 3 H), 1.29 (s, 3 H).

### Reference Compound 128

The title compound was synthesized in 5 steps:

### Step 1: Synthesis of N-methoxy-N-methylisoxazole-3-carboxamide.

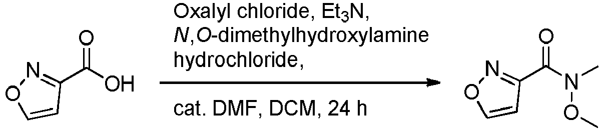

To a cold solution of isoxazole-3-carboxylic acid (2.0 g, 1.0 equiv.) in dichloromethane (80 ml) at 0 °C, was added oxalylchloride (2.0 ml, 1.3 equiv.) followed by two drops of DMF. The mixture was stirred at rt for 1h. To this mixture, was added *N*,*O*-dimethylhydroxylamine hydrochloride (2.2 g, 1.3 equiv.) and then triethylamine (8.6 ml, 3.5 equiv.). The mixture was stirred at rt for 3 h. The mixture was quenched with 1 N HCl (50 mL) and diluted with DCM (50 ml). The layers were separated, and the aqueous layer was extracted with DCM (2 x 50 mL). The organics were combined, washed with water (2 x 50 mL), brine (50 mL), and dried over MgSO₄, and filtered. The solvent was removed *in vacuo* to give the crude product. Purification by silica gel chromatography using an EtOAc/hexanes gradient gave *N*-methoxy-*N*-methylisoxazole-3-carboxamide (3.21 g, 93% yield) as a yellow oil. ¹H NMR (400 MHz, CDCl₃) δ 8.46-8.51 (m, 1H), 6.67-6.76 (m, 1H), 3.80 (br. s., 3H), 3.39 (br. s., 3H).

### Step 2: Synthesis of ethyl 4-(isoxazol-3-yl)-2-(methoxy(methyl)amino)-4-oxobut-2-enoate.

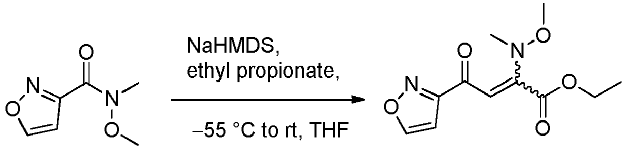

To a cold solution of *N*-methoxy-*N*-methylisoxazole-3-carboxamide (0.31 g, 1.0 equiv.) and ethyl propionate (0.39 g, 2.0 equiv.) in anhydrous THF (10 mL) at -55 °C, was added sodium bis(trimethylsilyl)amide as a 1 M solution in THF (3.8 mL, 1.9 equiv.). The mixture was stirred at -40 °C over 20 min. A dark solution was obtained. The mixture was quenched with 1 N HCl (4 mL) and warmed to rt. The mixture was partitioned between EtOAc (10 mL) and H₂O (6 mL). The organic layer was washed with 15% NaCl and concentrated *in vacuo* to give oil. Purification by column chromatography using a 0 to 100% EtOAc/hexanes gradient gave ethyl 4-(isoxazol-3-yl)-2-(methoxy(methyl)amino)-4-oxobut-2-enoate (769 mg, 43% yield); ¹H NMR (500 MHz, CDCl₃) δ 8.42 (d, 1H), 6.77 (d, 1H), 6.19 (s, 1H), 4.47 (q, 2H), 3.76 (s, 3H), 3.22 (s, 3H), 1.41 (t, 3H).

### Step 3: Synthesis of ethyl 5-(isoxazol-3-yl)-1-(3,3,4,4,4-pentafluorobutyl)-1H-pyrazole--3-carboxylate.

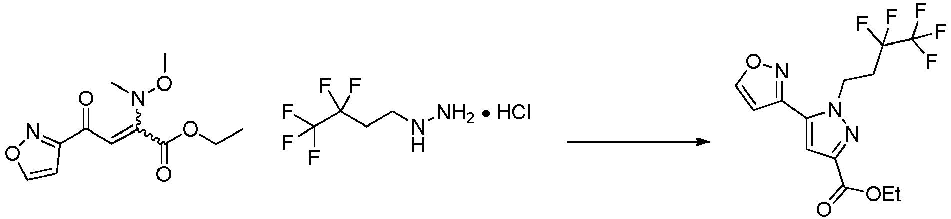

A mixture containing ethyl 4-(isoxazol-3-yl)-2-(methoxy(methyl)amino)-4-oxobut-2-enoate (67 mg, 1.0 equiv.) and (3,3,4,4,4-pentafluorobutyl)hydrazine, hydrochloride (56 mg, 1.0 equiv.) in ethanol (1.3 ml) was stirred at 65 °C for 1 h. The mixture was concentrated *in vacuo.* The residual oil was purified by column chromatography using a 0 to 10% ethyl acetate/hexanes gradient to give ethyl 5-(isoxazol-3-yl)-1-(3,3,4,4,4-pentafluorobutyl)-1H-pyrazole-3-carboxylate (49 mg, 53 % yield) as a light yellow solid. ¹H NMR (500 MHz, CDCl₃) δ 8.54 (d, 1H), 7.27 (s, 1H), 6.62 (d, 1H), 4.96-5.01 (m, 2H), 4.42-4.48 (m, 2H), 2.73-2.86 (m, 2H), 1.41-1.45 (m, 3H).

### Step 4: Synthesis of 5-(isoxazol-3-yl)-1-(3,3,4,4,4-pentafluorobutyl)-1H-pyrazole-3-carboximidamide.

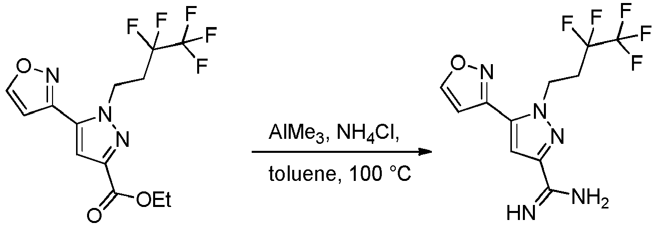

In a 40 ml vial and under a stream of Argon, a suspension of ammonium chloride (218 mg, 6.5 equiv.) in toluene (2.1 ml) was cooled to 0 °C for 30 min. To this mixture, was added trimethylaluminum as a 2.0 M solution in toluene (2.0 ml, 6.5 equiv.). The mixture was removed from the ice bath and stirred at rt until the bubbling ceased. The mixture became clear. To this mixture, was added a solution of ethyl 5-(isoxazol-3-yl)-1-(3,3,4,4,4-pentafluorobutyl)-1H-pyrazole-3-carboxylate (223 mg, 1.0 equiv.) in toluene (2.0 ml). The mixture was heated to 110 °C for 24 h. The gas generated during the reaction was released. The mixture was cooled to 0 °C and diluted with toluene and quenched with methanol. The mixture was stirred vigorously and the precipitate formed was removed by filtration using a Buchner funnel. The filtrate was transferred to a round-bottom flask and concentrated *in vacuo* to give a solid. The solid was then treated with a 5:1 EtOAc:IPA mixture (60 ml) and saturated solution of sodium bicarbonate (40 ml). The aqueous layer was back extracted with a 5:1 EtOAc:IPA mixture (50 ml). The organic layers were combined, dried over magnesium sulfate, filtered and evaporated *in vacuo.* The solid was dried to give 5-(isoxazol-3-yl)-1-(3,3,4,4,4-pentafluorobutyl)-1H-pyrazole-3-carboximidamide (230 mg, quantitative yield) as a tan solid. ¹H NMR (400 MHz, DMSO-*d*₆) δ ppm 9.18 (d, 1 H) 7.54 (s, 1 H) 7.08 (d, 1 H) 4.88 (t, 2 H) 2.76 - 3.04 (m, 2 H).

### Step 5: Synthesis of Compound 128

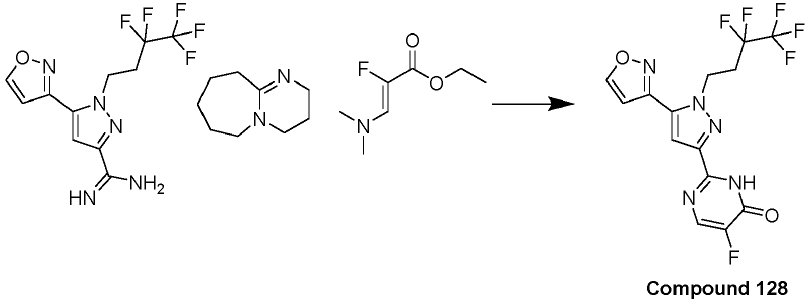

A mixture of 5-(isoxazol-3-yl)-1-(3,3,4,4,4-pentafluorobutyl)-1H-pyrazole--3-carboximidamide (106 mg, 1.0 equiv.), DBU (87 µl, 1.8 equiv.) and ethyl 3-(dimethylamino)-2-fluoroacrylate (133 mg, 2.5 equiv.) in EtOH (1.6 mL) was heated to 70 °C for 24 h. The mixture was concentrated *in vacuo* to give a crude oil. Purification of the crude oil by column chromatography using 0 to 100% ethyl acetate/hexanes gradient gave 5-fluoro-2-(5-(isoxazol-3-yl)-1-(3,3,4,4,4-pentafluorobutyl)-1H-pyrazol-3-yl)pyrimidin-4(3H)-one (4.5 mg, 4 % yield) as a white solid. ¹H NMR (400 MHz, CD₃OD) δ 8.86 (d, 1H), 8.05 (d, 1H), 7.43 (s, 1H), 6.98 (d, 1H), 5.00-5.09 (m, 2H), 2.83-3.01 (m, 2H).

### Compound 129

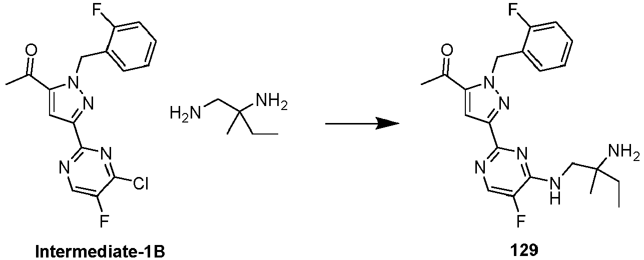

A mixture containing triethylamine (3.0 equiv.), 1-(3-(4-chloro-5-fluoropyrimidin-2-yl)-1-(2-fluorobenzyl)-1H-pyrazol-5-yl)ethanone **(Intermediate-1B,** 32 mg, 1.0 equiv.) and 2-methylbutane-1,2-diamine (3.0 equiv.) in NMP (0.5 ml) was stirred at rt for 24 h. The mixture was diluted in ethyl acetate (50 ml) and washed with water (50 ml). The organic layer was dried, filtered and evaporated to give a crude oil. The oil was purified by column chromatography using a 0 to 10% MeOH/DCM gradient to give 1-(3-(4-((2-amino-2-methylbutyl)amino)-5-fluoropyrimidin-2-yl)--1-(2-fluorobenzyl)-1H-pyrazol-5-yl)ethanone (12 mg, 32 % yield) as a white solid. ¹H NMR (500 MHz, METHANOL-*d*₄) δ ppm 8.24 (d, 1 H) 7.77 (s, 1 H) 7.28 - 7.36 (m, 1 H) 7.25 (d, 1 H) 7.13 - 7.19 (m, 1 H) 7.08 (t, 1 H) 5.94 (s, 2 H) 3.72 (s, 2 H) 2.56 - 2.61 (m, 3 H) 1.72 - 1.83 (m, 2 H) 1.37 (s, 3 H) 1.08 (t, 3 H).

### Reference Compound 130

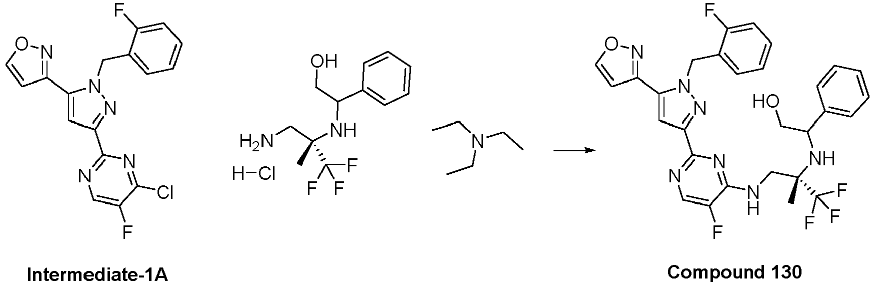

A mixture containing 3-(3-(4-chloro-5-fluoropyrimidin-2-yl)-1-(2-fluorobenzyl)-1H-pyrazol-5-yl)isoxazole **(Intermediate-1A,** 100 mg, 1.0 equiv.), triethylamine (8.0 equiv.) and 2-(((*S*)-3-amino-1,1,1-trifluoro-2-methylpropan-2-yl)amino)-2-phenylethanol hydrochloride (3.0 equiv.) in DMF (1.3 ml) was stirred at rt for 24 h. The mixture was diluted in ethyl acetate (50 ml) and washed with water (50 ml). The organic layer was dried, filtered and evaporated to give a crude oil. The oil was purified by column chromatography using a 0 to 100% EtOAc/hexanes gradient to give 2-phenyl-2-(((*S*)-1,1,1-trifluoro-3-((5-fluoro-2-(1-(2-fluorobenzyl)-5-(isoxazol-3-yl)-1H-pyrazol-3-yl) pyrimidin-4-yl)amino)-2-methylpropan-2-yl)amino)ethanol (132 mg, 82 % yield) as a white solid. ¹H NMR (500 MHz, DMSO-*d*₆) δ ppm 8.25 (d, 1 H) 7.76 (br. s., 1 H) 7.45 (s, 1 H) 7.32 (d, 3 H) 7.17 - 7.24 (m, 4 H) 7.11 - 7.16 (m, 1 H) 7.09 (t, 1 H) 6.88 (t, 1 H) 5.88 (s, 2 H) 5.24 (t, 1 H) 3.85 (dd, 1 H) 3.70 (dd, 1 H) 3.21 (td, 1 H) 2.91 (d, 1 H) 1.04 (s, 3 H).

### Reference Compound 131

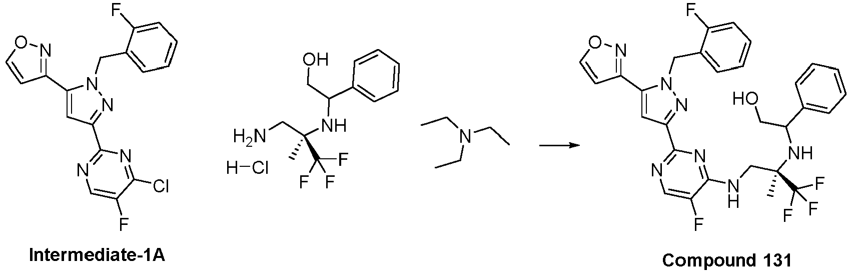

A mixture containing 3-(3-(4-chloro-5-fluoropyrimidin-2-yl)-1-(2-fluorobenzyl)-1H-pyrazol-5-yl)isoxazole **(Intermediate-1A,** 420 mg, 1.0 equiv.), triethylamine (6.0 equiv.) and 2-(((*R*)-3-amino-1,1,1-trifluoro-2-methylpropan-2-yl)amino)-2-phenylethanol hydrochloride (3.0 equiv.) in DMF (5.6 ml) was stirred at rt for 24 h. The mixture was diluted in ethyl acetate (50 ml) and washed with water (50 ml). The organic layer was dried, filtered and evaporated to give a crude oil. The oil was purified by column chromatography using a 0 to 100% EtOAc/hexanes gradient to give2-pheny1-2-(((*R*)-1,1,1-trifluoro-3-((5-fluoro-2-(1-(2-fluorobenzyl)-5-(isoxazol-3-yl)-1H-pyrazol-3-yl)pyrimidin-4-yl)amino)-2-methylpropan-2-yl)amin o)ethanol (348 mg, 52 % yield) as a white solid. ¹H NMR (500 MHz, METHANOL-*d*₄) δ ppm 8.76 (s, 1 H) 8.08 (d, 1 H) 7.32 - 7.46 (m, 3 H) 7.24 (t, 3 H) 7.18 (d, 2 H) 7.06 - 7.12 (m, 1 H) 7.02 (s, 1 H) 6.88 (s, 1 H) 5.96 (s, 2 H) 4.17 (dd, 1 H) 3.82 - 4.09 (m, 2 H) 3.48 - 3.56 (m, 1 H) 3.36 (d, 1 H) 1.08 - 1.18 (m, 3 H).

### Reference Compound 132

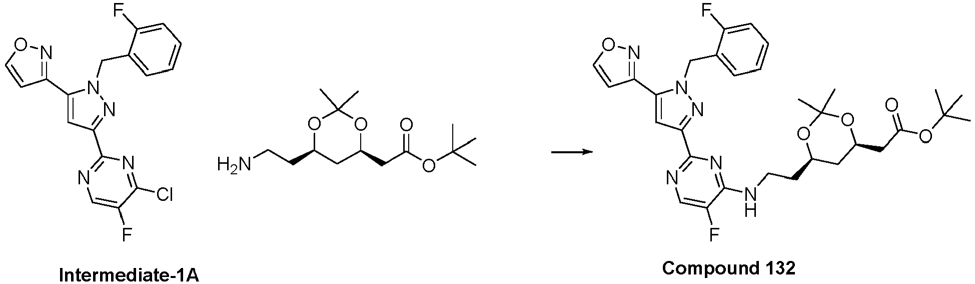

A mixture containing 3-(3-(4-chloro-5-fluoropyrimidin-2-yl)-1-(2-fluorobenzyl)--1H-pyrazol-5-yl)isoxazole **(Intermediate-1A,** 364 mg, 1.0 equiv.), (4*R*, 6*R*)-*tert*-butyl-6-(2-aminoethyl)-2,2-dimethyl-1,3-dioxane-4-acetate (3.0 equiv.) and triethylamine (3.0 equiv.) in 1,4-Dioxane (3.7 ml) and water (1.3 ml) was heated to 60 °C for 2 h. The mixture was diluted in ethyl acetate (50 ml) and washed with water (50 ml). The organic layer was dried, filtered and evaporated *in vacuo* to give a crude oil. The oil was purified by column chromatography using a 0 to 50% ethyl acetate/hexanes gradient to give the desired compound as a white solid (536 mg, 90% yield). ¹H NMR (500 MHz, CHLOROFORM-*d*) δ ppm 8.34 (s, 1 H) 7.97 (s, 1 H) 7.23 (s, 1 H) 7.01 - 7.08 (m, 1 H) 6.86 - 6.91 (m, 1 H) 6.83 (t, 1 H) 6.72 (t, 1 H) 6.46 (s, 1 H) 5.87 (s, 2 H) 4.00 (qd, 2 H) 3.73 - 3.82 (m, 1 H) 3.46 - 3.54 (m, 1 H) 2.29 - 2.36 (m, 1 H) 2.18 - 2.25 (m, 1 H) 1.64 - 1.81 (m, 2 H) 1.43 - 1.52 (m, 2 H) 1.30 - 1.37 (m, 15 H).

### Reference Compound 133

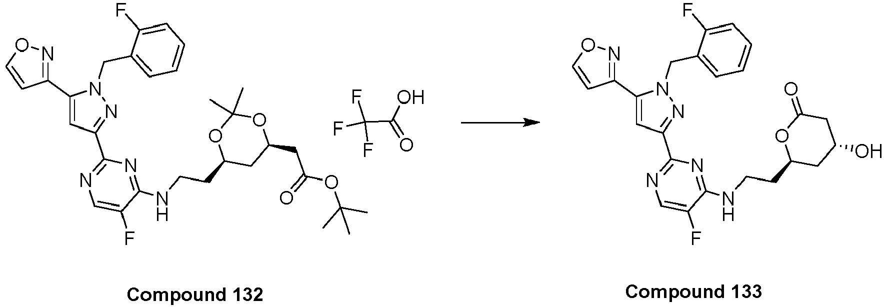

To a solution of **Compound 132** (0.575 g, 1.0 equiv.) in DCM (100 mL), was added TFA (14 mL, 200 equiv.). The mixture was stirred at rt for 1 h. The mixture was concentrated *in vacuo.* The resulting residue was partitioned between DCM (50 ml) and IN sodium bicarbonate (50 ml). The organic layer was dried, filtered and evaporated to give an oil. The oil was purified by column chromatography using a 0 to 100% ethyl acetate/hexanes gradient to give (4*R*,6*R*)-6-(2-((5-fluoro-2-(1-(2-fluorobenzyl)-5-(isoxazol-3-yl)-1H-pyrazol-3-yl)pyrimidin-4-yl)amin o)ethyl)-4-hydroxytetrahydro-2H-pyran-2-one (365 mg, 78% yield) as a white solid. ¹H NMR (500 MHz, CHLOROFORM-*d*) δ ppm 8.45 - 8.48 (m, 1 H) 8.10 (d, 1 H) 7.36 (s, 1 H) 7.17 - 7.24 (m, 1 H) 7.01 - 7.07 (m, 1 H) 6.98 (td, 1 H) 6.88 (td, 1 H) 6.71 (d, 1 H) 5.92 - 6.03 (m, 2 H) 5.59 (br. s., 1 H) 4.86 - 4.93 (m, 1 H) 3.68 - 3.97 (m, 2 H) 2.53 - 2.73 (m, 2 H) 2.32 (dt, 1 H) 2.06 - 2.11 (m, 1 H) 1.97 - 2.04 (m, 1 H) 1.72 (ddd, 1 H).

### Reference Compound 134

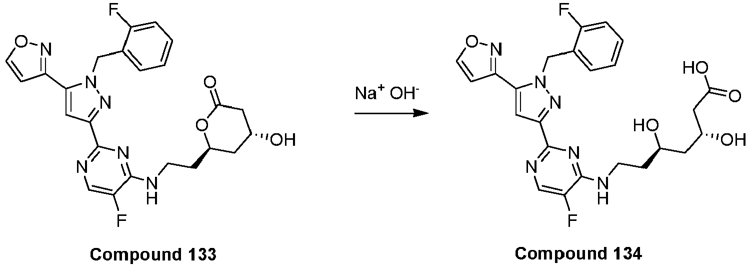

A mixture containing **Compound 133** (173 mg, 1.0 equiv.) and sodium hydroxide (1.0 equiv.) in THF (0.9 ml) and MeOH (0.9 ml) was stirred at rt for 1 h. The mixture was concentrated in vacuo to give a sodium salt of (3*R*,5*R*)-7-((5-fluoro-2-(1-(2-fluorobenzyl)--5-(isoxazol-3-yl)-1H-pyrazol-3-yl)pyrimidin-4-yl)amino)-3,5-dihydroxyheptanoic acid (187 mg, 100 % yield) as a white solid. ¹H NMR (500 MHz, MeOD) δ 8.72 (d, 1H), 7.98 (d, 1H), 7.40 (s, 1H), 7.18-7.27 (m, 1H), 7.01-7.08 (m, 1H), 6.98 (t, 1H), 6.88 (d, 1H), 6.74 (t, 1H), 5.91 (s, 2H), 4.08-4.15 (m, 1H), 3.93 (dt, 1H), 3.64-3.86 (m, 2H), 2.25-2.40 (m, 2H), 1.82-1.97 (m, 2H), 1.62-1.80 (m, 2H).

### Reference Compound 135

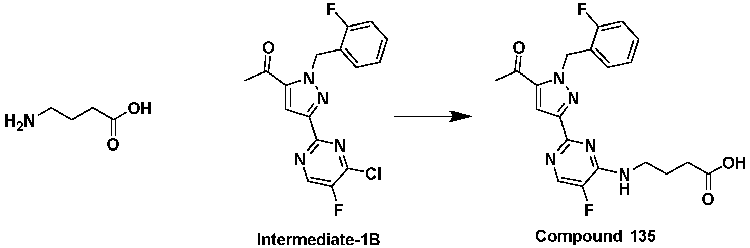

A solution of 1-(3-(4-chloro-5-fluoropyrimidin-2-yl)-1-(2-fluorobenzyl)-1H--pyrazol-5-yl)ethanone **(Intermediate-1B,** 1 equiv.), 4-aminobutanoic acid (2 equiv.) and triethylamine (10 equiv.) in anhydrous dioxane was heated at 90 °C for 1 d. The resultant mixture was concentrated and the crude material was purified via reverse phase HPLC utilizing a 30-80% acetonitrile water 0.1% formic acid gradient to deliver 4-((5-fluoro-2-(1-(2-fluorobenzyl)-5-(isoxazol-3-yl)-1H-pyrazol-3-yl)pyrimidin-4-yl)amino)butanoic acid (2.2 mg, 9% yield) as a white solid. ¹H NMR (500 MHz, METHANOL-*d*₄) δ ppm 8.27 (d, 1 H), 7.96 (s, 1 H), 7.32 (m, 1 H), 7.10 (s, 2 H), 6.97 (s, 1 H), 5.99 (s, 2 H), 3.85 (t, 2 H), 2.65 (m, 3 H), 2.49 (s, 2 H), 2.07 (m, 2 H).

### Reference Compound 136

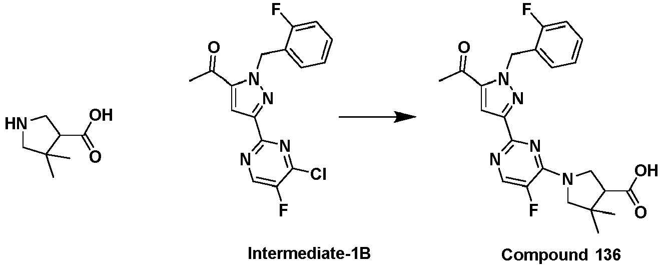

A solution of 1-(3-(4-chloro-5-fluoropyrimidin-2-yl)-1-(2-fluorobenzyl)--1H-pyrazol-5-yl)ethanone **(Intermediate-1B,** 1 equiv.), 4,4-dimethylpyrrolidine-3-carboxylic acid (2 equiv.) and triethylamine (10 equiv.) in anhydrous dioxane was heated at 90 °C for 1 d. The resultant mixture was concentrated and the crude material was purified via reverse phase HPLC utilizing a 30-80% acetonitrile water 0.1% formic acid gradient to deliver 1-(2-(5-acetyl-1-(2-fluorobenzyl)-1H-pyrazol-3-yl)-5--fluoropyrimidin-4-yl)-4,4-dimethylpyrrolidine-3-carboxylic acid (2.7 mg, 10% yield) as a white solid. ¹H-NMR (500 MHz, METHANOL-*d*₄) δ ppm 8.20 (m, 1 H), 7.77 (s, 1 H), 7.31 (m, 1 H), 7.10 (m, 2 H), 6.88 (m, 1 H), 5.95 (s, 2 H), 4.22 (m, 2 H), 3.94 (m, 1 H), 3.72 (m, 1 H), 3.03 (m, 1 H), 2.61 (s, 3 H), 1.38 (s, 3 H), 1.17 (s, 3 H).

### Reference Compound 137

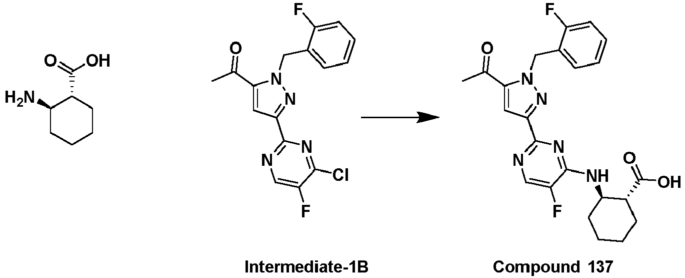

A solution of 1-(3-(4-chloro-5-fluoropyrimidin-2-yl)-1-(2-fluorobenzyl)-1H-pyrazol-5-yl)ethanone (1 equiv.), (1R,2R)-2-aminocyclohexanecarboxylic acid (2 equiv.) and triethylamine (10 equiv.) in anhydrous dioxane was heated at 90 °C for 1 d. The resultant mixture was concentrated and the crude material was purified via reverse phase HPLC utilizing a 30-80% acetonitrile water 0.1% formic acid gradient to deliver (1R,2R)-2-((2-(5-acetyl-1-(2-fluorobenzyl)-1H-pyrazol-3-yl)-5 -fluoropyrimidin-4-yl)amino)cyclohex anecarboxylic acid (2.8 mg, 10% yield) as a white solid. ¹H-NMR (500 MHz, METHANOL-*d*₄) δ ppm 8.00 (m, 1 H), 7.74 (m, 1 H), 7.29 (m, 1 H), 7.08 (m, 2 H), 6.80 (m, 1 H), 5.92 (s, 2 H), 4.54 (m, 1 H), 2.60 (s, 3 H), 2.49 (m, 1 H), 2.10 (m, 2 H), 1.84 (m, 2 H), 1.69 (m, 1 H), 1.56 (m, 1 H), 1.37 (m, 2 H).

### Reference Compound 138

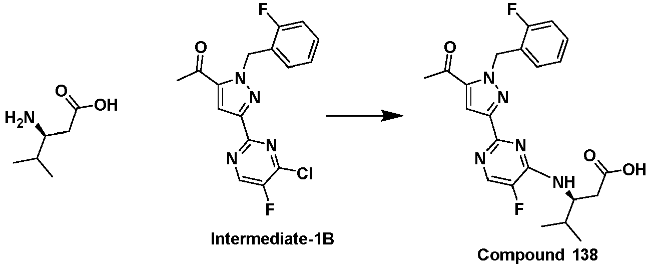

A solution of 1-(3-(4-chloro-5-fluoropyrimidin-2-yl)-1-(2-fluorobenzyl)-1H-pyrazol-5-yl)ethanone (1 equiv.), (S)-3-amino-4-methylpentanoic acid (2 equiv.) and triethylamine (10 equiv.) in anhydrous dioxane was heated at 90 °C for 1 d. The resultant mixture was concentrated and the crude material was purified via reverse phase HPLC utilizing a 30-80% acetonitrile water 0.1% formic acid gradient to deliver (S)-3-((2-(5-acetyl-1-(2-fluorobenzyl)-1H-pyrazol-3-yl)-5-fluoropyrimidin-4-yl)amino)-4-methylpent anoic acid (5.4 mg, 20% yield) as a white solid. ¹H-NMR (500 MHz, METHANOL-*d*₄) δ ppm 8.00 (m, 1 H), 7.72 (m, 1 H), 7.28 (m, 1 H), 7.09 (m, 2 H), 6.81 (m, 1 H), 5.92 (s, 2 H), 4.73 (m, 1 H), 2.59 (s, 5 H), 2.07 (m, 1H), 1.04 (t, 6 H).

### Reference Compound 139

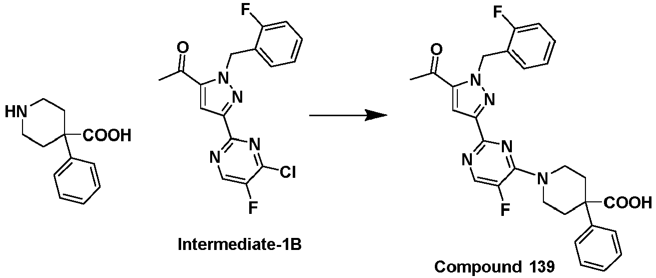

A solution of 1-(3-(4-chloro-5-fluoropyrimidin-2-yl)-1-(2-fluorobenzyl)--1H-pyrazol-5-yl)ethanone (1 equiv.), 4-phenylpiperidine-4-carboxylic acid (3 equiv.) and triethylamine (10 equiv.) in anhydrous dioxane was heated at 90 °C for 1 d. The resultant mixture was concentrated and the crude material was purified via reverse phase HPLC utilizing a 30-80% acetonitrile water 0.1% formic acid gradient to deliver 1-(2-(5-acetyl-1-(2-fluorobenzyl)-1H-pyrazol-3-yl)-5-fluoropyrimidin-4-yl)-4-phenylpiperidine-4-car boxylic acid (7.9 mg, 27% yield) as a white solid. ¹H-NMR (500 MHz, METHANOL-*d*4) δ ppm 8.31 (m, 1 H), 7.92 (m, 1 H), 7.51 (m, 2 H), 7.39 (m, 2 H), 7.32 (m, 2 H), 7.11 (m, 2 H), 6.95 (m, 1H), 5.98 (m, 2 H), 4.83 (m, 2 H), 3.70 (m, 2 H), 2.81 (m, 2 H), 2.64 (s, 3 H), 2.16 (m, 2 H).

### Reference Compound 185

This compound was prepared in 5 steps:

### Step 1: cyano pyrimidine

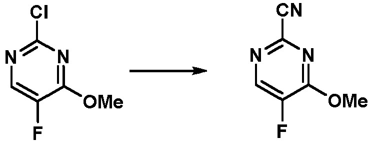

A mixture of zinc(II) cyanide (10.8 g, 92 mmol) and 2-chloro-5-fluoro-4-methoxypyrimidine (15.0 g, 92 mmol) in dimethylformamide (200 mL) was degassed at room temperature by bubbling nitrogen through the solution for 10 min. Tetrakis(triphenylphosphine)palladium(0) (10.0 g, 8.65 mmol) was added, degassing was continued another 10 min and the reaction was heated 2 days at 90°C. The mixture was cooled to room temperature and diluted with ethyl acetate (150 mL), brine (50 mL) and concentrated aqueous ammonium hydroxide (10 mL). After mixing, the layers were separated and the aqueous phase was extracted with another portion of ethyl acetate (150 mL). The combined organic phases were washed with 2 x 20 mL brine then dried over sodium sulfate, filtered and concentrated by rotary evaporation at 60°C. Purification over SiO2 with a hexane/ethyl acetate gradient gave recovered chloropyrimidine starting material and 5.5 g of the desired cyano pyrimidine as a colorless oil. The recovered starting material was reprocessed as above to give another 3.0 g of the depicted intermediate (total yield 8.5 g, 60% yield).
¹H-NMR (500 MHz, CDC13) δ 8.41 (s, 1H), 4.17 (s, 3H) ppm.

### Step 2: pyrimidine ester

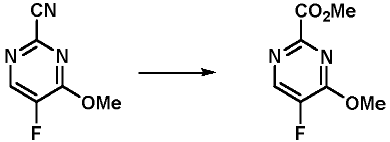

The cyano pyrimidine obtained in Step 1 (8.1 g, 52.9 mmol) was cooled in ice as IN sodium hydroxide(aq) (63.5 mL, 63.5 mmol) was added over 5 min. The mixture was stirred overnight at room temperature, then recooled in ice as 3N hydrochloric acid(aq) was added to pH 3. The mixture was concentrated to dryness, first by rotary evaporation, then high-vacuum to leave 12.8 g crude carboxylic acid as a white solid that was carried on directly to the esterification. The crude solid was stirred in anhydrous methanol (150 mL) at room temperature and concentrated sulfuric acid (1.5 mL, 29.1 mmol) was added. The mixture was stirred overnight, then cooled in ice and 10% aqueous NaHCO3 (100 mL) was added followed by another 1 hr of stirring at room temperature. The solvents were removed under vacuum and the residue was partioned between water (100 mL) and ethyl acetate (300 mL). The organic phase was washed with 3 x 20 mL H2O, then the combined aqueous phases were back-extracted with 200 mL ethyl acetate. The combined organic phases were dried over Na2SO4, filtered and concentrated by rotary evaporation. Purification over SiO2 with a gradient hexane/ethyl acetate as eluant gave the ester pyrimidine intermediateas a white solid (4.5 g, 46% yield from the cyano pyrimidine).
¹H-NMR (500 MHz, CDCl₃) δ 8.45 (s, 1H), 4.20 (s, 3H), 4.03 (s, 3H) ppm.

### Step 3 and Step 4: Intermediate-17

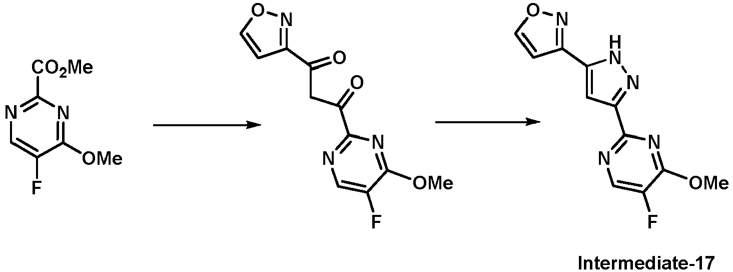

Step 3: A solution of 1-(isoxazol-3-yl)ethanone (4.0 g, 36.3 mmol) in THF (200 mL) was cooled in dry ice/acetone. Lithium bis(trimethylsilyl)amide (1M in toluene, 33.8 mL, 33.8 mmol) was added over 10 min followed by 30 min of stirring at -65° to -70°C. The ester pyrimidine obtained above (4.5 g, 24.2 mmol) in THF (20 mL) was dripped into the enoate solution over 5 min and stirring was continued overnight at room temperature. The solvents were removed *in vacuo,* then the residue was broken up under ether (100 mL) and filtered. The filter cake was washed with ether (20 mL) and air dried to leave 8.2 g crude diketo isoxazole which was carried on directly to the next reaction without further purification. LCMS (m/e) 266 (M+H).

Step 4: Crude diketo isoxazole (theory 6.4 g, 24.2 mmol) was dissolved in methanol (100 mL), then glacial acetic acid (11.4 mL, 199 mmol) and hydrazine hydrate (4.0 mL, 83 mmol) were added and the solution was heated at 60°C for 30 min. The solvents were removed under vacuum, then the residue was covered with ethyl acetate (50 mL) and 10% aqueous NaHCO3 (200 mL) and stirred at room temperature until no more gas evolution was observed. Hexane (80 mL) was added and the biphasic mixture stirred 30 min and filtered. The filter cake was washed with 2 x 50 mL H2O, 1:1 hexane/ethyl acetate (50 mL) and dried under vacuum to leave 2.69 g of **Intermediate-17** as a light tan solid. The organic filtrate was found to contain additional impure product. Chromatography of this mixture over SiO2 using a gradient elution of dichloromethane/ethyl acetate gave an addional 0.39 g of **Intermediate-17**(total yield: 3.1 g, 71% from the ester pyrimidine). ¹H-NMR (500 MHz, CD₃OD) δ 8.76 (s, 1H), 8.49 (s, 1H), 7.40 (s, 1H), 6.94 (s, 1H), 4.23 (s, 3H) ppm. LCMS (m/e) 262 (M+H). Step 5: **Compound 185**

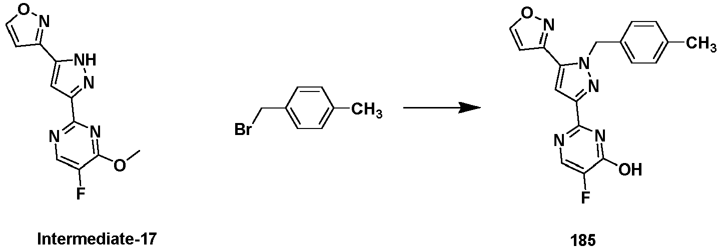

A solution of tert-butyl 3-(3-(5-fluoro-4-methoxypyrimidin-2-yl)-1H-pyrazol-5-yl)isoxazole (**Intermediate-17,** 1 equiv.), and lithium tert-butoxide (2 equiv.) in dimethoxyethane (2 ml) was stirred at 60°C for 5 min. To it was added 1-(bromomethyl)-4-methylbenzene (1.1 equiv.) and reaction stirred at 60°C overnight. After cooling to ambient temperature, solvent was removed under a stream of nitrogen. The resultant solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver **Compound 185** (1.5 mg, 5% yield) as a white solid. ¹H NMR (500 MHz, Methanol-*d*4) δ ppm 8.75 (m, 1 H), 7.95 (m, 1 H), 7.35 (s, 1 H), 7.11 (m, 4 H), 6.85 (m, 1 H), 5.84 (s, 2 H), 2.28 (s, 3 H)

### Reference Compound 187

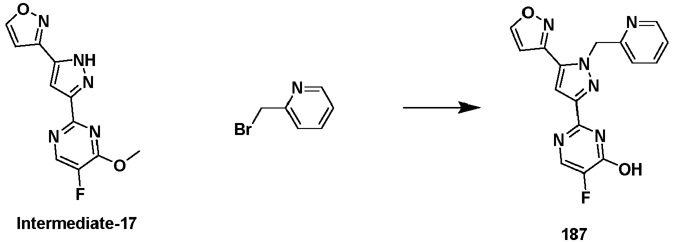

A solution of tert-butyl 3-(3-(5-fluoro-4-methoxypyrimidin-2-yl)-1H-pyrazol-5-yl)isoxazole (**Intermediate-17,** 1 equiv.), and lithium tert-butoxide (3 equiv.) in dimethoxyethane (2 ml) was stirred at 60°C for 5 min. To it was added 2-(bromomethyl)pyridine, HBr (1.1 equiv.) and reaction stirred at 60°C overnight. After cooling to ambient temperature, solvent was removed under a stream of nitrogen. The resultant solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver **Compound 187** (1.5 mg, 5% yield) as a white solid. ¹H NMR (500 MHz, Methanol-*d*4) δ ppm 8.78 (s, 1 H), 8.70 (d, 1 H), 8.19 (t, 1 H), 8.07 (d, 1 H), 7.69 (t, 1 H), 7.56 (s, 1 H), 7.52 (d, 1 H), 6.92 (s, 1 H), 6.20 (s, 2 H).

### Reference Compound 189

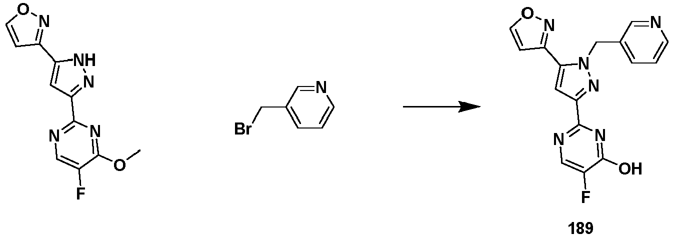

A solution of tert-butyl 3-(3-(5-fluoro-4-methoxypyrimidin-2-yl)-1H-pyrazol-5-yl)isoxazole (**Intermediate-17,** 1 equiv.), and lithium tert-butoxide (3 equiv.) in dimethoxyethane (2 ml) was stirred at 60°C for 5 min. To it was added 3-(bromomethyl)pyridine, HBr (1.1 equiv.) and reaction stirred at 60°C overnight. After cooling to ambient temperature, solvent was removed under a stream of nitrogen. The resultant solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver **Compound 189** (12.9 mg, 40% yield) as a white solid. ¹H NMR (500 MHz, Methanol-*d*4) δ ppm 8.90 (s, 1 H), 8.86 (d, 1 H), 8.77 (d, 1 H), 8.50 (d, 1 H), 8.08 (d, 1 H), 7.96 (br. s., 1 H), 7.52 (s, 1 H), 7.00 (d, 1 H), 6.14 (s, 2 H)

### Reference Compound 190

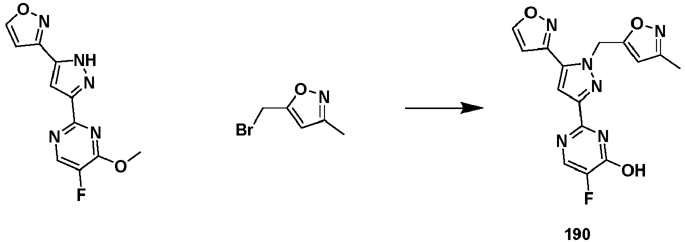

A solution of tert-butyl 3-(3-(5-fluoro-4-methoxypyrimidin-2-yl)-1H-pyrazol-5-yl)isoxazole (**Intermediate-17,** 1 equiv.), and lithium tert-butoxide (2 equiv.) in dimethoxyethane (2 ml) was stirred at 60°C for 5 min. To it was added 5-(bromomethyl)-3-methylisoxazole (1.1 equiv.) and reaction stirred at 60°C overnight. After cooling to ambient temperature, solvent was removed under a stream of nitrogen. The resultant solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver **Compound 190** (13.2 mg, 42% yield) as a white solid. ¹H NMR (500 MHz, Methanol-*d*4) δ ppm 8.84 (d, 1 H), 8.04 (m, 1 H), 7.47 (s, 1 H), 6.97 (d, 1 H), 6.15 (m, 1 H), 6.06 (s, 2 H), 2.22 (m, 3 H).

### Synthesis of Reference Compound 141 and Reference Compound 140

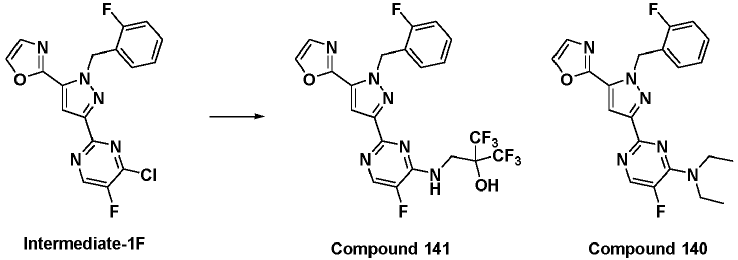

A solution of 2-(3-(4-chloro-5-fluoropyrimidin-2-yl)-1-(2-fluorobenzyl)-1*H*-pyrazol-5-yl)oxazole, 2-(aminomethyl)-1,1,1,3,3,3-hexafluoropropan-2-ol (**Intermediate-IF,** 3.0 equiv.) and triethylamine (10 equiv.) in dioxane-water (2:1) was heated to 90-100 °C for 5 days. The reaction mixture was diluted with water, acidified to pH 4 with IN HCl solution and extracted with dichloromethane. The combined organic phases were dried over sodium sulfate, filtered, and the solvent was removed in vacuo. Purification by silica gel chromatography (10-25% ethyl acetate in hexanes gradient) yielded **Compound 141** (38 mg, 55% yield over 2 steps) as a white solid and **Compound 140** (11mg, 21% over 2 steps) as a white solid
**Reference Compound 141:** ¹H-NMR (500 MHz, CDCl₃) δ ppm 8.27 (m, 2H), 7.73 (s, 1H), 7.45 (s, 1H), 7.23 (m, 1H), 7.14 (app. t, 1H), 7.05-7.00 (m, 2H), 6.10 (s, 2H), 5.72 (br s, 1H), 4.15 (d, 2H). The exchangeable OH proton was not observed.
**Reference Compound 140:** ¹H-NMR (500 MHz, CDCl₃) δ ppm 8.13 (d, 1H), 7.68 (s, 1H), 7.48 (s, 1H), 7.21 (s, 1H), 7.18 (m, 1H), 7.01 (app. t, 1H), 6.95 (app. t, 1H), 6.87 (app. t, 1H), 6.09 (s, 2H), 3.68 (q, 4H), 1.29 (t, 6H).

### Synthesis of Reference Compound 142

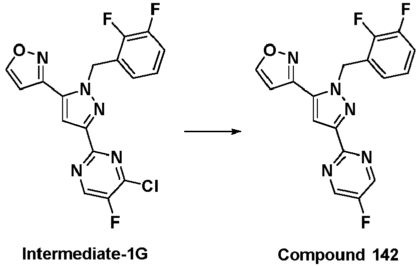

A greyish yellow suspension of 3-(3-(4-chloro-5-fluoropyrimidin-2-yl)-1-(2,3-difluorobenzyl)-1*H*-pyrazol-5-yl)isoxazole **(Intermediate-1G,** this compound was prepared analogously to the preparation of Intermediate IF, but starting from the corresponding 2-(1-(2,3-difluorobenzyl)-5-(isoxazol-3-yl)-1H-pyrazol-3-yl)-5-fluoropyrimidin-4-ol, which had previously been described in patent application publication WO2013/101830) and zinc dust (2.5 equiv.) in THF was treated with acetic acid (2.8 equiv.) and heated at 75 °C for 2 days. After cooling to ambient temperature, the reaction mixture was poured into IN NaOH solution and extracted with ethyl acetate. The organic phases were dried over sodium sulfate, filtered, and the solvent was removed in vacuo. The crude material was purified via silica gel chromatography (10-50% ethyl acetate/hexanes gradient) to afford **Compound 142** (39 mg, 74%) as a white solid. ¹H-NMR (500 MHz, DMSO-*d*₆) δ ppm 9.12 (s, 1H), 8.97 (s, 2H), 7.69 (s, 1H), 7.38 (m, 1H), 7.30 (s, 1H), 7.14 (m, 1H), 6.78 (app. t, 1H), 5.97 (s, 2H).

### Reference Compound 143

The title compound was synthesized in 5 steps:

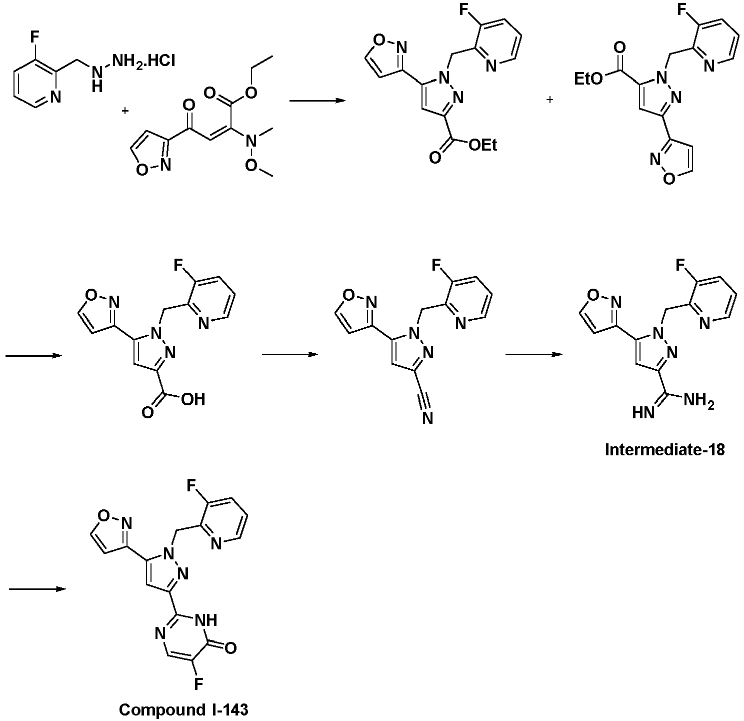

### Step 1: Synthesis of ethyl 1-((3-fluoropyridin-2-yl)methyl)-5-(isoxazol-3-yl)-1H-pyrazole-3-carboxylate and ethyl 1-((3-fluoropyridin-2-yl)methyl)-3-(isoxazol-3-yl)-1H-pyrazole-5-carboxylate.

A suspension of 3-fluoro-2-(hydrazinylmethyl)pyridine hydrochloride (1.0 equiv.) and potassium carbonate (0.5 equiv.) in ethanol/water (10:1) was treated with ethyl 4-(isoxazol-3-yl)-2-(methoxy(methyl)amino)-4-oxobut-2-enoate (1 equiv.). The resultant orange suspension was heated at 60°C for 24 hours. The crude mixture was concentrated in vacuo. Water was added and the aqueous phase was extracted with ethyl acetate. The combined organic phases were dried over sodium sulfate, filtered, and the solvent was removed in vacuo. Purification by silica gel chromatography (10-25% ethyl acetate in hexanes gradient) yielded ethyl 1-((3-fluoropyridin-2-yl)methyl)-5-(isoxazol-3-yl)-1*H*-pyrazole-3-carboxylate (47%) and ethyl 1-((3-fluoropyridin-2-yl)methyl)-3-(isoxazol-3-yl)-1*H*-pyrazole-5-carboxylate (9.7%). Step 2: Synthesis of 1-((3-fluoropyridin-2-yl)methyl)-5-(isoxazol-3-yl)-1*H*-pyrazole-3-carboxylic acid.

To a solution of ethyl 1-((3-fluoropyridin-2-yl)methyl)-5-(isoxazol-3-yl)-1*H*-pyrazole-3-carboxylate in THF/water (3:1 ratio) was added lithium hydroxide (2.0 equiv.). After 5 hours, the reaction mixture was concentrated in vacuo to remove most of the THF. The resultant mixture was diluted with water and acidified to pH 4-5 by addition of IN HCl solution. The product, 1-((3-fluoropyridin-2-yl)methyl)-5-(isoxazol-3-yl)-1*H-*pyrazole-3-carboxylic acid, was collected by vacuum filtration. The filtrate was extracted with ethyl acetate. The combined organic phases were dried over sodium sulfate, filtered, and the solvent was removed in vacuo to give additional product (96%).

### Step 3: Synthesis of 1-((3-fluoropyridin-2-yl)methyl)-5-(isoxazol-3-yl)-1H-pyrazole-3-carbonitrile

To a suspension of 1-((3-fluoropyridin-2-yl)methyl)-5-(isoxazol-3-yl)-1*H-*pyrazole-3-carboxylic acid, 2-methylpropan-2-amine (3.0 equiv.), and triethylamine (2.0 equiv.) in ethyl acetate was added *n*-propylphosphonic anhydride (T3P, 50 wt% solution in ethyl acetate, 3.0 equiv.). The resultant yellow solution was heated at 65°C for 3 hours. The solvent was removed in vacuo. Phosphoryl trichloride (20 equiv.) was added and the resulting mixture was stirred at 70°C for 2 hours. The reaction was quenched by carefully pouring into a mixture of water and ice, neutralized to pH 7 by addition of saturated sodium bicarbonate solution/solid sodium bicarbonate and extracted with ethyl acetate. The combined organic phases were dried over sodium sulfate, filtered, and the solvent was removed in vacuo to afford 1-((3 -fluoropyridin-2-yl)methyl)-5 -(isoxazol-3-yl)-1*H*-pyrazole-3-carbonitrile (>99%).

### Step 4: Synthesis of 1-((3-fluoropyridin-2-yl)methyl)-5-(isoxazol-3-yl)-1H-pyrazole-3-carboximidamide (Intermediate-18)

A solution of 1-((3-fluoropyridin-2-yl)methyl)-5-(isoxazol-3-yl)-1H-pyrazole-3-carbonitrile in methanol was treated sodium methoxide (0.5 N solution in MeOH, 4.0 equiv.) and stirred for 4 hours. Ammonium chloride (10 equiv.) was added. The reaction mixture was stirred at ambient temperature for 36 hours and at 50°C for 6.5 hours. The crude mixture was concentrated in vacuo and partitioned between half-saturated sodium bicarbonate solution and ethyl acetate. The organic phases were dried over sodium sulfate, filtered, and the solvent was removed in vacuo to afford 1-((3-fluoropyridin-2-yl)methyl)-5-(isoxazol-3-yl)-1*H*-pyrazole-3-caiboximidamide (98%) which was used without further manipulation.

### Step 5: Synthesis of Compound 143

A suspension of 1-((3-fluoropyridin-2-yl)methyl)-5-(isoxazo1-3-yl)-1*H-*pyrazole-3-carboximidamide **(Intermediate-18)** in ethanol was treated with sodium (*Z*)-3-ethoxy-2-fluoro-3-oxoprop-1-en-1-olate (4.0 equiv.) and heated at 90°C for 2 hours. After cooling to ambient temperature, the reaction mixture was neutralized by addition of HCl (1.25 M solution in EtOH). The resultant suspension was concentrated in vacuo. The residue was partitioned between dichloromethane/isopropanol (7:1) and water, and the pH was adjusted to 6 by addition of IN NaOH solution. The aqueous layer was back-extracted with dichloromethane/isopropanol (7:1). The combined organic phases were dried over sodium sulfate, filtered, and the solvent was removed in vacuo. Purification by silica gel chromatography (5-20% acetonitrile/methanol (7:1) in dichloromethane gradient) yielded **Compound 143** (220 mg, 60%) as a light tan solid. ¹H-NMR (500 MHz, DMSO-*d*₆) δ ppm 13.2 (br s, 1H), 9.07 (s, 1H), 8.23 (d, 1H), 8.12 (br s, 1H), 7.76 (app. t, 1H), 7.63 (s, 1H), 7.40 (m, 1H), 7.23 (s, 1H), 6.05 (s, 2H).

### Reference Compound 144

The title compound was synthesized in 2 steps:

### Step 1: Synthesis of 3-(3-(4-chloro-5-fluoropyrimidin-2-yl)-1-((3-fluoropyridin-2-yl) methyl)-1H-pyrazol-5-yl)isoxazole

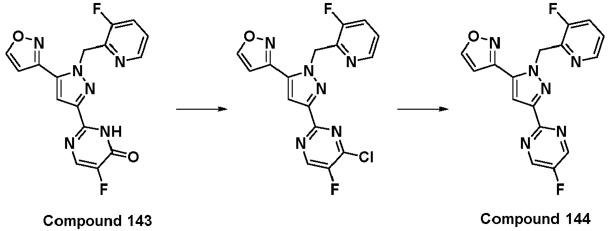

A solution of 5-fluoro-2-(1-((3-fluoropyridin-2-yl)methyl)-5-(isoxazol-3-yl)-1*H*-pyrazol-3-yl)pyrimidin-4(3*H*)-one in phosphoryl trichloride (85 equiv.) as solvent was heated at 65 °C for 2 hours. The reaction mixture was cooled to ambient temperature, blown dried under a stream of nitrogen and then concentrated twice from toluene. The resultant yellowish brown solid, 3-(3-(4-chloro-5-fluoropyrimidin-2-yl)-1-((3-fluoropyridin-2-yl) methyl)-1*H*-pyrazol-5-yl)isoxazole, was dried in vacuo and used in the next step without further manipulation.

### Step 2: Synthesis of Compound 144

A greyish yellow suspension of 3-(3-(4-chloro-5-fluoropyrimidin-2-yl)-1--((3-fluoropyridin-2-yl)methyl)-1*H*-pyrazol-5-yl)isoxazole and zinc dust (1.7 equiv.) in THF was treated with acetic acid (2.8 equiv.) and heated at 75 °C for 3 hours. Additional amounts of zinc dust (2.8 equiv.) and acetic acid (2.8 equiv.) were added and the reaction was heated at 75°C for 20 hours. After cooling to ambient temperature, the reaction mixture was filtered and the filtrate was partitioned between half-saturated sodium bicarbonate solution and ethyl acetate. The organic phases were dried over sodium sulfate, filtered, and the solvent was removed in vacuo. The crude material was purified via silica gel chromatography (30-60% ethyl acetate/hexanes gradient) to afford **Compound 144** (30 mg, 35% over 2 steps) as a pale yellow solid.
¹H-NMR (500 MHz, DMSO-*d*₆) δ ppm 9.06 (s, 1H), 8.95 (s, 2H), 8.25 (d, 1H), 7.76 (app. t, 1H), 7.64 (s, 1H), 7.40 (m, 1H), 7.28 (s, 1H), 6.06 (s, 2H).

### Reference Compound 145

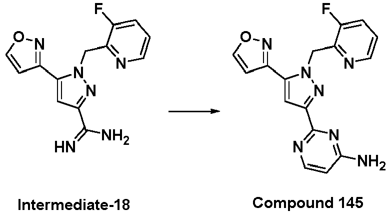

A solution of 1-((3-fluoropyridin-2-yl)methyl)-5-(isoxazol-3-yl)-1*H*-pyrazole-3-carboximidamide **(Intermediate-18)** in pyridine was treated with 1,8-diazabicyclo[5.4.0]undec-7-ene (DBU, 4.0 equiv.) and 3-ethoxyacrylonitrile (2.5 equiv.) and heated at 110 °C for 46 hours. The reaction mixture was cooled to ambient temperature, concentrated in vacuo and partitioned between half-saturated sodium bicarbonate solution and dichloromethane. The organic phases were dried over sodium sulfate, filtered, and the solvent was removed in vacuo. The crude material was purified via silica gel chromatography (10-25% acetonitrile/methanol (7:1) in dichloromethane gradient) followed by preparative HPLC (5-75% acetonitrile/water gradient with 0.1% trifluoroacetic acid) to afford **Compound 1-145** (17 mg, 21%, TFA salt) as a white solid. ¹H-NMR (500 MHz, DMSO-*d*₆) δ ppm 14.1 (br s, 1H, TFA), 9.11 (s, 1H), 8.78 (br s, 2H), 8.24 (d, 1H), 8.10 (d, 1H), 7.78 (app. t, 1H), 7.61 (s, 1H), 7.42 (m, 1H), 7.28 (s, 1H), 6.63 (d, 1H), 6.11 (s, 2H).

### Reference Compound 146

The title compound was synthesized in 2 steps:

### Step 1: Synthesis of 3-(3-(4-chloro-5-fluoropyrimidin-2-yl)-1-((3-fluoropyridin-2-yl)methyl)-1H-pyrazol-5-yl)isoxazole

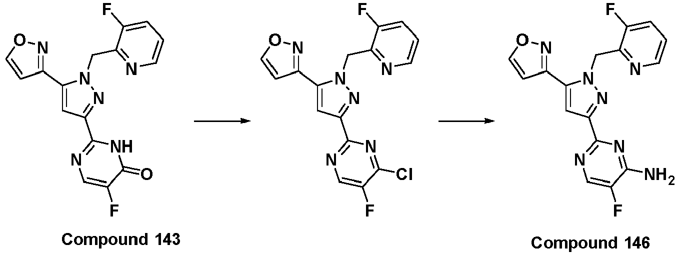

A solution of 5-fluoro-2-(1-((3-fluoropyridin-2-yl)methyl)-5-(isoxazol-3-yl)--1*H*-pyrazol-3-yl)pyrimidin-4(3*H*)-one in phosphoryl trichloride (100 equiv.) as solvent was heated at 65 °C for 3 hours. The reaction mixture was cooled to ambient temperature, blown dried under a stream of nitrogen and then concentrated twice from toluene. The resultant yellowish brown solid, 3-(3-(4-chloro-5-fluoropyrimidin-2-yl)-1-((3-fluoropyridin-2-yl)methyl)-1*H*-pyrazol-5-yl)isoxazole, was dried in vacuo and used in the next step without further manipulation.

### Step 2: Synthesis of Compound 146

A yellow solution of 3-(3-(4-chloro-5-fluoropyrimidin-2-yl)-1-((3-fluoropyridin-2-yl)methyl)-1*H*-pyrazol-5-yl)isoxazole and ammonium hydroxide (35 equiv., 29% solution in water) in dioxane was heated to 60 °C for 22 hours. The resultant yellow suspension was diluted with water. The product was collected by filtration, washed with water and dried in vacuo to afford Compound 146 (37 mg, 98% yield over 2 steps) as a light tan solid. ¹H-NMR (500 MHz, DMSO-*d*₆) δ ppm 9.04 (s, 1H), 8.25 (d, 1H), 8.21 (d, 1H), 7.75 (app. t, 1H), 7.44 (s, 1H), 7.42-7.34 (m, 3H), 7.22 (s, 1H), 6.00 (s, 2H).

### Reference Compound 147

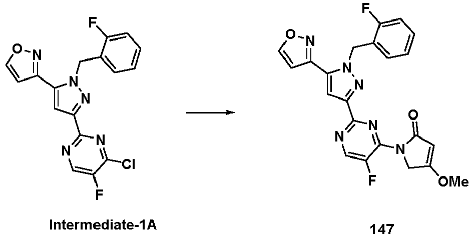

A suspension of Intermediate-IA (50.0 mg, 0.134 mmol), 4-methoxy-1H-pyrrol-2(5H)-one (18.2 mg, 0.161 mmol), and cesium carbonate (65.4 mg, 0.201 mmol) in dioxane (2 mL) was heated to 95 °C for 2 hours, then heated at 70 °C for 12 hours. The reaction mixture was then diluted in water, extracted with dichloromethane (3 x 30 mL), dried (sodium sulfate), filtered and concentrated to afford a residue. Purification was achieved by reverse phase HPLC utilizing a gradient of 5 to 95% acetonitrile in water (spiked with 0.1% trifluoroacetic acid) over 25 minutes to deliver 1-(5-fluoro-2-(1-(2-fluorobenzyl)-5-(isoxazol-3-yl)-1H-pyrazol-3-yl)pyrimidin-4-yl)-4-methoxy-1H-p yrrol-2(5H)-one, **Compound 147** (2.0 mg, 3 % yield), as a white solid. ¹H NMR (500 MHz, CDCl₃) δ (ppm): 8.64 (d, 1H), 8.49 (d, 1H), 7.36 (s, 1H), 7.20 - 7.23 (m, 1H), 7.02 - 7.07 (m, 1H), 6.98 - 7.01 (m, 1H), 6.87 - 6.90 (m, 1H), 6.61 (d, 1H), 5.99 (s, 2H), 5.26 (s, 1H), 4.70 (s, 2H), 3.94 (s, 3H).

### Reference Compound 148

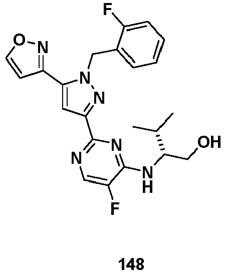

A suspension of **Intermediate-IA** (70.0 mg, 0.187 mmol), (*R*)-2-amino-3-methylbutan-1-ol (0.0250 mL, 0.225 mmol), and triethylamine (0.104 ml, 0.749 mmol) was heated in a mixture of dioxane (1 mL) and water (0.5 mL) at 85 °C for 16 hours. The reaction mixture was then cooled to room temperature and diluted in water, leading to the formation of a white precipitate, which was filtered and dried. The crude product was reconstituted in dichloromethane and washed with water (2 x 30 mL), dried (sodium sulfate), filtered, and concentrated to afford a (*R*)-2-((5-fluoro-2-(1-(2-fluorobenzyl)-5-(isoxazol-3-yl)-1H-pyrazol-3-yl)pyrimidin-4-yl)amino)-3-m ethylbutan-1-ol, **Compound 148** (69.4 mg, 95 % yield), as a white solid. No purification was necessary. ¹H NMR (500 MHz, CD₃OD) δ (ppm): 8.75 (s, 1H), 8.05 (d, 1H), 7.40 (s, 1H), 7.24 - 7.29 (m, 1H), 7.07 - 7.11 (m, 1H), 7.01 - 7.04 (m, 1H), 6.90 (s, 1H), 6.79 - 6.82 (m, 1H), 5.96 (s, 2H), 4.34 - 4.37 (m, 1H), 3.80 (dd, 1H), 3.72 (dd, 1H), 2.03 - 2.09 (m, 1H), 1.05 (d, 3H), 1.00 (d, 3H).

### Reference Compound 149

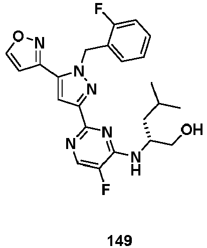

A suspension of Intermediate-IA (70.0 mg, 0.187 mmol), (*R*)-2-amino-4-methylpentan-1-ol (0.0290 mL, 0.225 mmol), and trimethylamine (0.104 mL, 0.749 mmol) was heated in a mixture of dioxane (1 mL) and water (0.5 mL) at 85 °C for 16 hours. The reaction mixture was then cooled to room temperature and diluted in water, leading to the formation of a white precipitate, which was filtered and dried. The crude product was reconstituted in dichloromethane and washed with water (2 x 30 mL), dried (sodium sulfate), filtered, and concentrated to afford (*R*)-2-((5-fluoro-2-(1-(2-fluorobenzyl)-5-(isoxazol-3-yl)-1H-pyrazol-3-yl)pyrimidin-4-yl)amino)-4-m ethylpentan-1-ol, **Compound 149** (67.3 mg, 79 % yield), as a white solid. No purification was necessary. ¹H NMR(500 MHz, DMSO-*d*₆) δ (ppm): 9.09 (s, 1H), 8.14 (d, 1H), 7.43 (s, 1H), 7.28 - 7.34 (m, 2H), 7.19 - 7.23 (m, 2H, 2 overlapping shifts), 7.08 - 7.11 (m, 1H), 6.86 - 6.89 (m, 1H), 5.91 (d, 1H), 5.84 (d, 1H), 4.74 (m, 1H), 4.44 (br. s, 1H), 3.46 - 3.50 (m, 1H), 3.40 - 3.45 (m, 1H), 1.56 - 1.62 (m, 1H), 1.45 - 1.51 (m, 1H), 1.36 - 1.41 (m, 1H), 0.90 (d, 3H), 0.88 (d, 3H).

### Reference Compound 150

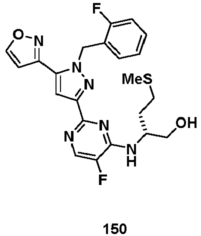

A suspension of **Intermediate-IA** (70.0 mg, 0.187 mmol), (*R*)-(+)-Methionanol (30.4 mg, 0.225 mmol), and triethylamine (0.104 mL, 0.749 mmol) was heated in a mixture of dioxane (1 mL) and water (0.5 mL) at 85 °C for 16 hours. The reaction mixture was then cooled to room temperature and diluted in water, leading to the formation of a yellow precipitate, which was filtered and dried. The crude product was reconstituted in dichloromethane and washed with water (2 x 30 mL), dried (sodium sulfate), filtered, and concentrated to afford (*R*)-2-((5-fluoro-2-(1-(2-fluorobenzyl)-5-(isoxazol-3-yl)-1H-pyrazol-3-yl)pyrimidin-4-yl)amino)-4-(m ethylthio)butan-1-ol, **Compound 150** (47.5 mg, 54 % yield), as pale yellow waxy solid. No purification was necessary. ¹H NMR (500 MHz, DMSO-*d*₆) δ (ppm): 9.09 (d, 1H), 8.17 (d, 1H), 7.49 (s, 1H), 7.39 (d, 1H), 7.31 - 7.34 (m, 1H), 7.23 (s, 1H), 7.19 - 7.23 (m, 1H), 7.08 -7.11 (m, 1H), 6.81 - 6.84 (m, 1H), 5.90 (d, 1H), 5.87 (d, 1H), 4.80 - 4.82 (m, 1H), 4.35 - 4.42 (m, 1H), 3.51 - 3.55 (m, 1H), 3.45 - 3.50 (m, 1H), 2.50 - 2.54 (m, 2H), 2.02 (s, 3H), 1.90 - 1.95 (m, 1H), 1.80 - 1.85 (m, 1H).

### Reference Compound 151

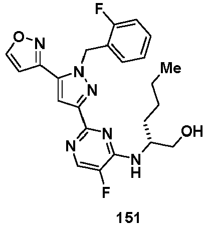

A suspension **Intermediate-IA** (75.0 mg, 0.201 mmol), (*R*)-2-aminohexan-1-ol (28.2 mg, 0.241 mmol), and triethylamine (0.104 mL, 0.749 mmol) was heated in a mixture of dioxane (1 mL) and water (0.5 mL) at 85 °C for 16 hours. The reaction mixture was then cooled to room temperature and diluted in water, leading to the formation of a white precipitate, which was filtered and dried. The crude product was reconstituted in dichloromethane and washed with water (2 x 30 mL), dried (sodium sulfate), filtered, and concentrated to afford (*R*)-2-((5-fluoro-2-(1-(2-fluorobenzyl)-5-(isoxazol-3-yl)-1H-pyrazol-3-yl)pyrimidin-4-yl)amino)hexa n-1-ol, **Compound 151** (83.2 mg, 91 % yield), as an off-white solid. No purification was necessary.
¹H NMR (500 MHz, DMSO-*d*₆) δ (ppm): 9.09 (d, 1H), 8.15 (d, 1H), 7.46 (s, 1H), 7.29 - 7.34 (m, 2H), 7.20 - 7.23 (m, 2H, 2 overlapping shifts), 7.08 - 7.11 (m, 1H), 6.81 - 6.85 (m, 1H), 5.91 (d, 1H), 5.87 (d, 1H), 4.71 - 4.74 (m, 1H), 4.29 - 4.34 (m, 1H), 3.48 - 3.52 (m, 1H), 3.42 - 3.48 (m, 1H), 1.61 - 1.68 (m, 1H), 1.46 - 1.53 (m, 1H), 1.19 - 1.36 (m, 4H), 0.81 (t, 3H).

### Reference Compound 152

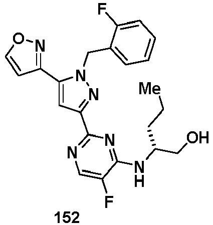

A suspension of **Intermediate-IA** (75.0 mg, 0.201 mmol), (*R*)-2-aminopentan-1-ol (24.8 mg, 0.241 mmol), and triethylamine (0.104 mL, 0.749 mmol) was heated in a mixture of dioxane (1 mL) and water (0.5 mL) at 85 °C for 16 hours. The reaction mixture was then cooled to room temperature and diluted in water, leading to the formation of a white precipitate, which was filtered and dried. The crude product was reconstituted in dichloromethane and washed with water (2 x 30 mL), dried (sodium sulfate), filtered, and concentrated to afford (*R*)-2-((5-fluoro-2-(1-(2-fluorobenzyl)-5-(isoxazol-3-yl)-1H-pyrazol-3-yl)pyrimidin-4-yl)amino)penta n-1-ol, **Compound 152** (47.1 mg, 53 % yield), as an off-white solid. No purification was necessary. ¹H NMR (500 MHz, DMSO-*d*₆) δ (ppm): 9.09 (s, 1H), 8.15 (d, 1H), 7.46 (s, 1H), 7.29 - 7.35 (m, 2H), 7.20 - 7.23 (m, 2H, 2 overlapping shifts), 7.08 - 7.11 (m, 1H), 6.83 - 6.86 (m, 1H), 5.91 (d, 1H), 5.87 (d, 1H), 4.73 (m, 1H), 4.32 - 4.38 (m, 1H), 3.48 - 3.52 (m, 1H), 3.43 - 3.47 (m, 1H), 1.58 - 1.65 (m, 1H), 1.46 - 1.53 (m, 1H), 1.26 - 1.40 (m, 2H), 0.88 (t, 3H).

### Reference Compound 127

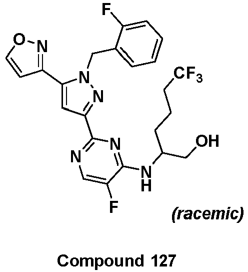

A suspension of **Intermediate-IA** (82.0 mg, 0.219 mmol), 2-amino-6,6,6-trifluorohexan-1-ol (45.0 mg, 0.263 mmol), and triethylamine (0.122 mL, 0.876 mmol) in a mixture of dioxane (1 mL) and water (0.5 mL) was heated at 85 °C for 72 hours. The reaction mixture was then cooled to room temperature and diluted in water, leading to the formation of a white precipitate, which was filtered and dried. The crude product was reconstituted in dichloromethane and washed with water (2 x 30 mL), dried (sodium sulfate), filtered, and concentrated to afford 6,6,6-trifluoro-2-((5-fluoro-2-(1-(2-fluorobenzyl)-5-(isoxazol-3-yl)-1H-pyrazol-3-yl)pyrimidin-4-yl)a mino)hexan-1-ol, **Compound 127** (80.1 mg, 72 % yield), as a white solid. No purification was necessary. ¹H NMR(500 MHz, DMSO-*d*₆) δ (ppm): 9.08 (s, 1H), 8.18 (d, 1H), 7.49 (s, 1H), 7.38 (d, 1H), 7.30 - 7.34 (m, 1H), 7.20 - 7.24 (m, 2H, 2 overlapping shifts), 7.07 - 7.10 (m, 1H), 6.78 - 6.81 (m, 1H), 5.88 (s, 2H), 4.80 (m, 1H), 4.33 - 4.38 (m, 1H), 3.50 - 3.55 (m, 1H), 3.45 - 3.49 (m, 1H), 2.40 - 2.48 (m, 1H), 2.20 - 2.29 (m, 1H), 1.70 - 1.76 (m, 1H), 1.54 - 1.63 (m, 2H), 1.46 - 1.52 (m, 1H).

### Reference Compound 153

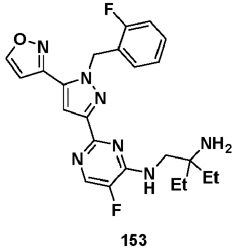

A solution of **Intermediate-IA** (74.5 mg, 0.199 mmol), 2-ethylbutane-1,2-diamine (27.8 mg, 0.239 mmol), and triethylamine (0.111 mL, 0.797 mmol) in a mixture of dioxane (1 mL) and water (0.5 mL) was heated at 85 °C for 16 hours. The reaction mixture was then cooled to room temperature and diluted in water, leading to the formation of a white precipitate, which was filtered and dried in vacuo to afford 2-ethyl-*N1*-(5-fluoro-2-(1-(2-fluorobenzyl)-5-(isoxazol-3-yl)-1H-pyrazol-3-yl)pyrimidin-4-yl)butane-1,2-diamine, **Compound 153** (76.8 mg, 85 % yield), as an off-white solid. No purification was necessary. ¹H NMR (500 MHz, CD₃OD) δ (ppm): 8.77 (s, 1H), 8.15 - 8.19 (m, 1H), 7.46 - 7.49 (m, 1H), 7.26 - 7.30 (m, 1H), 7.10 - 7.13 (m, 1H), 7.02 - 7.06 (m, 1H), 6.90 (s, 1H), 6.76 - 6.80 (m, 1H), 5.98 (s, 2H), 3.66 (s, 2H), 1.59 - 1.72 (m, 4H), 0.96 - 1.03 (m, 6H).

### Reference Compound 154

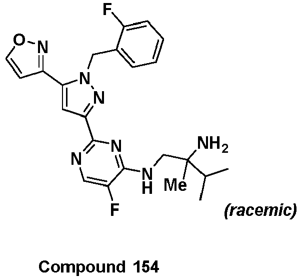

A solution of **Intermediate-IA** (70.0 mg, 0.187 mmol), 2,3-dimethylbutane-1,2-diamine (26.1 mg, 0.225 mmol), and triethylamine (0.104 mL, 0.749 mmol) in a mixture of dioxane (1 mL) and water (0.5 mL) was heated at 85 °C for 16 hours. The reaction mixture was then cooled to room temperature and diluted in water, leading to the formation of a white precipitate, which was filtered and dried in vacuo to afford *N1*-(5-fluoro-2-(1-(2-fluorobenzyl)-5-(isoxazol-3-yl)-1H-pyrazol-3-yl)pyrimidin-4-yl)-2,3-dimethylbu tane-1,2-diamine, **Compound 154** (70.7 mg, 83 % yield), as an off-white solid. No purification was necessary. ¹H NMR(500 MHz, DMSO-*d*₆) δ (ppm): 9.09 (m, 1H), 8.18 (d, 1H), 7.45 (s, 1H), 7.31 - 7.35 (m, 1H), 7.25 - 7.29 (m, 1H), 7.19 - 7.23 (m, 2H), 7.09 - 7.12 (m, 1H), 6.88 - 6.91 (m, 1H), 5.87 (s, 2H), 3.41 - 3.49 (m, 2H), 1.57 - 1.63 (m, 1H), 0.91 (d, 3H), 0.90 (s, 3H), 0.88 (s, 3H), [2 N-H protons not observed].

### Reference Compound 155

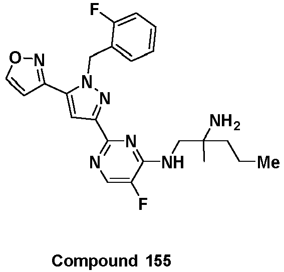

A solution of **Intermediate-IA** (113 mg, 0.303 mmol), 2-methylpentane-1,2-diamine (42.3 mg, 0.364 mmol), and triethylamine (0.169 mL, 1.21 mmol) in a mixture of dioxane (1 mL) and water (0.5 mL) was heated at 85 °C for 16 hours. The reaction mixture was cooled to room temperature, diluted with water, extracted with dichloromethane (3 x 30 mL), dried (sodium sulfate), filtered and concentrated to a viscous oil which solidified upon standing to afford *N1*-(5-fluoro-2-(1-(2-fluorobenzyl)-5-(isoxazol-3-yl)-1H-pyrazol-3-yl)pyrimidin-4-yl)-2-methylpenta ne-1,2-diamine (117 mg, 85 % yield) as a white foamy solid. No purification was necessary. ¹H NMR (500 MHz, CD₃OD) δ (ppm): 8.76 (s, 1H), 8.07 (d, 1H), 7.43 (s, 1H), 7.24 - 7.29 (m, 1H), 7.07 - 7.11 (m, 1H), 7.01 - 7.04 (m, 1H), 6.90 (s, 1H), 6.80 - 6.83 (m, 1H), 5.96 (s, 2H), 3.67 (d, 1H), 3.60 (d, 1H), 1.42 - 1.50 (m, 4H), 1.15 (s, 3H).

### Reference Compound 156

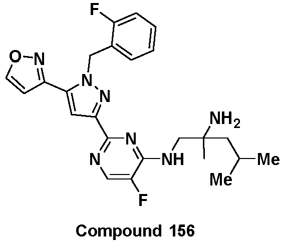

A solution of **Intermediate-IA** (101 mg, 0.271 mmol), 2,4-dimethylpentane-1,2-diamine (42.4 mg, 0.326 mmol), and triethylamine (0.151 mL, 1.21 mmol) in a mixture of dioxane (1 mL) and water (0.5 mL) was heated at 85 °C for 16 hours. The reaction mixture was cooled, diluted in water, extracted with dichloromethane (3 x 30 mL), dried (sodium sulfate), filtered and concentrated in vacuo to afford *N1*-(5-fluoro-2-(1-(2-fluorobenzyl)-5-(isoxazol-3-yl)-1H-pyrazol-3-yl)pyrimidin-4-yl)-2,4-dimethylpe ntane-1,2-diamine, **Compound 156** (109.7 mg, 86 % yield), as an off-white sticky gum. No purification was necessary. ¹H NMR (500 MHz, DMSO-*d*₆) δ (ppm): 9.09 (s, 1H), 8.18 (d, 1H), 7.47 (s, 1H), 7.31 - 7.34 (m, 1H), 7.19 - 7.23 (m, 2H, 2 overlapping shifts), 7.08 - 7.11 (m, 1H), 6.85 - 6.88 (m, 1H), 5.88 (s, 2H), 3.36 - 3.44 (m, 2H), 1.78 - 1.85 (m, 1H), 1.58 (br. s, 2H), 1.23 - 1.31 (m, 2H), 1.01 (s, 3H), 0.92 (d, 3H), 0.88 (d, 3H), [2 N-H protons not observed].

### Reference Compound 157

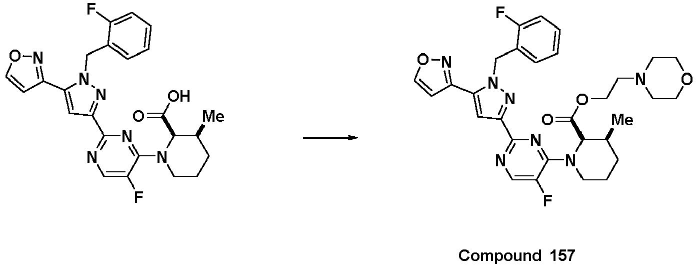

To a suspension of (2R, 3*S*)-1-(5-fluoro-2-(1-(2-fluorobenzyl)-5 -(isoxazol-3-yl)-1H-pyrazol-3-yl)pyrimidin-4-yl)-3 -methy lpiperidine-2-carboxylic acid (this compound was previously described in patent application publication WO2014144100, 25.0 mg, 0.0520 mmol) in dioxane (1 mL) was added 4-(2-bromoethyl)morpholine hydrobromide (15.7 mg, 0.0570 mmol) followed by cesium carbonate (25.4 mg, 0.0780 mmol). The reaction mixture was heated at 90 °C for 2 hours, after which the reaction mixture was diluted in DMSO (1 mL) and water (0.5 mL) and directly purified by reverse phase HPLC utilizing a gradient of 5 to 95% acetonitrile in water (spiked with 0.1% trifluoroacetic acid) over 25 minutes to afford the (2R,3S)-2-morpholinoethyl 1-(5-fluoro-2-(1-(2-fluorobenzyl)-5-(isoxazol-3-yl)-1H-pyrazol-3-yl)pyrimidin-4-yl)-3-methylpiperidi ne-2-carboxylate, **Compound 157** (16.7 mg, 54 % yield), as a white foamy solid. ¹H NMR (500 MHz, CD₃CN) δ (ppm): 8.69 (s, 1H), 8.24 (d, 1H), 7.47 (s, 1H), 7.30 - 7.34 (m, 1H), 7.11 - 7.15 (m, 1H), 7.08 - 7.11 (m, 1H), 6.96 - 6.99 (m, 1H), 6.90 (s, 1H), 5.88 (s, 2H), 5.32 (d, 1H), 4.39 - 4.47 (m, 3H, 2 overlapping shifts), 3.62 - 3.79 (m, 4H), 3.50 - 3.55 (m, 2H), 3.28 - 3.34 (m, 4H), 2.84 - 2.93 (m, 2H), 1.85 - 1.88 (m, 1H), 1.68 - 1.75 (m, 2H), 1.50 - 1.56 (m, 1H), 1.16 (d, 3H).

### Reference Compound 158

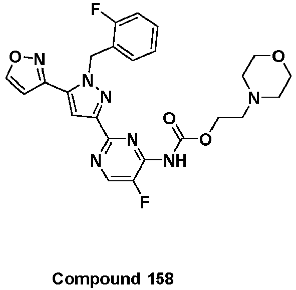

To a solution of 2-morpholinoethyl carbamate (134 mg, 0.769 mmol) in methanol (4 mL) was added potassium tert-butoxide (86.0 mg, 0.769 mmol). The reaction mixture was stirred at room temperature for 1 hour, after which it was concentrated to afford a white solid. The resulting solid was reconstituted in DMSO (4 mL), after which **Intermediate-1A** (287 mg, 0.769 mmol) was added. The reaction mixture was stirred at room temperature for 72 hours, after which the dioxane was removed in vacuo. The crude product mixture was purified by reverse phase HPLC utilizing a gradient of 5 to 95% acetonitrile in water (spiked with 0.1% trifluoroacetic acid) over 25 minutes to afford a mixture of two compounds. Further purification by silica gel chromatography utilizing a gradient of 3 to 15% methanol in dichloromethane over 50 minutes afforded 2-morpholinoethyl (5-fluoro-2-(1-(2-fluorobenzyl)-5-(isoxazol-3-yl)-1H-pyrazol-3-yl)pyrimidin-4-yl)carbamate, **Compound 158** (28.7 mg, 7 % yield), as a white crystalline solid. ¹H NMR (500 MHz, CD₃OD) δ (ppm): 8.78 (s, 1H), 8.64 (d, 1H), 7.53 (s, 1H), 7.26 - 7.30 (m, 1H), 7.08 - 7.12 (m, 1H), 7.02 - 7.06 (m, 1H), 6.89 (s, 1H), 6.85 - 6.89 (m, 1H), 5.97 (s, 2H), 4.51 - 4.56 (m, 2H), 3.78 - 3.86 (m, 4H), 3.33 - 3.37 (m, 2H), [4H not observed, isochronous with CD₃OD].

### Reference Compound 159

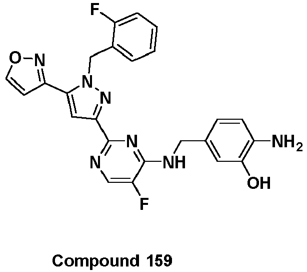

A suspension of **Intermediate-IA** (281 mg, 0.751 mmol), 2-amino-5-(aminomethyl)phenol dihydrochloride (182 mg, 0.864 mmol), and triethylamine (0.524 mL, 3.76 mmol) in a mixture of dioxane (3 mL) and water (1.5 mL) was heated at 90 °C for 16 hours. The reaction mixture was then cooled to room temperature, diluted in IN aqueous hydrochloric acid solution and water, filtered, and dried in vacuo to afford a crude solid. Purification was achieved using silica gel chromatography utilizing a gradient of 3 to 10% methanol in dichloromethane over 60 minutes to afford a mixture of products. Further purification by silica gel chromatography utilizing a gradient of 1 to 8% methanol in dichloromethane over 45 minutes afforded 2-amino-5-(((5-fluoro-2-(1-(2-fluorobenzyl)-5-(isoxazol-3-yl)-1H-pyrazol-3-yl)pyrimidin-4-yl)amino )methyl)phenol, **Compound 159** (135 mg, 38 % yield), as an orange-tan solid. ¹H NMR (500 MHz, DMSO-*d*₆) δ (ppm): 9.09 (s, 1H), 8.93 (br. s, 1H), 8.17 (d, 1H), 8.11 (m, 1H), 7.49 (s, 1H), 7.31 - 7.35 (m, 1H), 7.24 (s, 1H), 7.20 - 7.24 (m, 1H), 7.10 - 7.13 (m, 1H), 6.85 - 6.88 (m, 1H), 6.67 (s, 1H), 6.62 (d, 1H), 6.50 (d, 1H), 5.90 (s, 2H), 4.51 (d, 2H), 4.42 (br. s, 2H).

### Reference Compound 160

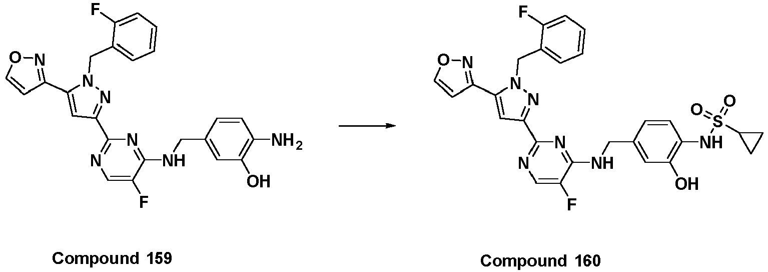

To a solution of **Compound 159** (49.5 mg, 0.104 mmol) in pyridine (1 mL) was added cyclopropanesulfonyl chloride (0.0120 mL, 0.115 mmol). After 16 hours, the reaction mixture was purified directly using silica gel chromatography using a gradient of 1 to 8% methanol in dichloromethane over 60 minutes to afford *N*-(4-(((5-fluoro-2-(1-(2-fluorobenzyl)-5-(isoxazol-3-yl)-1H-pyrazol-3-yl)pyrimidin-4-yl)amino)meth yl)-2-hydroxyphenyl)cyclopropanesulfonamide, **Compound 160** (41.9 mg, 69 % yield), as an orange solid. ¹H NMR (500 MHz, CD₃CN) δ (ppm): 8.65 (s, 1H), 8.10 (d, 1H), 7.49 (br. s, 1H), 7.36 (s, 1H), 7.27 - 7.30 (m, 1H), 7.27 (d, 1H), 7.10 - 7.13 (m, 1H), 7.05 - 7.09 (m, 1H), 7.02 (m, 2H), 6.91 - 6.94 (m, 2H), 6.84 (s, 1H), 6.60 - 6.62 (m, 1H), 5.86 (s, 2H), 4.70 (d, 2H), 2.43 - 2.48 (m, 1H), 0.89 - 0.94 (m, 2H), 0.85 - 0.89 (m, 2H).

### Reference Compound 161

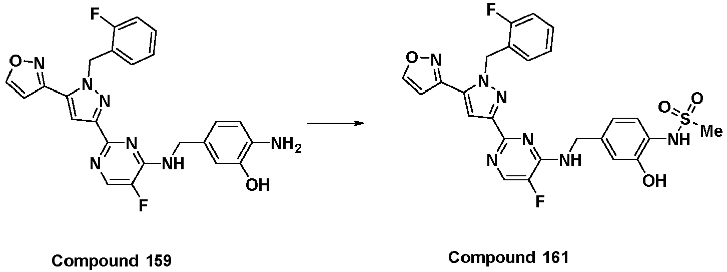

To a solution of **Compound 159** (40.8 mg, 0.086 mmol) in pyridine (1 mL) was added methanesulfonyl chloride (7.36 µL, 0.0940 mmol). After 16 hours, the reaction mixture was concentrated to about 10% of its volume and purified by reverse phase HPLC utilizing a gradient of 5 to 95% acetonitrile in water (spiked with 0.1% trifluoroacetic acid) over 25 minutes to afford *N*-(4-(((5-fluoro-2-(1-(2-fluorobenzyl)-5-(isoxazol-3-yl)-1H-pyrazol-3-yl)pyrimidin-4-yl)amino)meth yl)-2-hydroxyphenyl)methanesulfonamide, **Compound 161** (5.8 mg, 12 % yield), as a tan solid. ¹H NMR (500 MHz, CD₃CN) δ (ppm): 8.69 (s, 1H), 8.10 (d, 1H), 7.61 (m, 1H), 7.49 (s, 1H), 7.26 - 7.30 (m, 1H), 7.26 (d, 1H), 7.16 (br. s, 1H), 7.03 - 7.11 (m, 3H, 3 shifts overlapping), 6.94 - 6.97 (m, 2H), 6.88 (s, 1H), 5.86 (s, 2H), 4.77 (d, 2H), 2.89 (s, 3H), [1 N-H proton not observed].

### Reference Compound 162

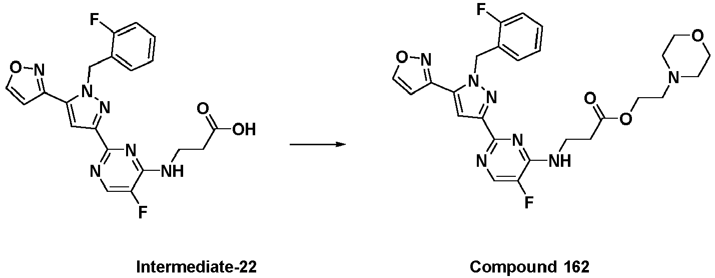

A suspension of 3-((5-fluoro-2-(1-(2-fluorobenzyl)-5-(isoxazol-3-yl)-1H-pyrazol-3-yl)pyrimidin-4-yl)amino)propanoi c acid (**Intermediate-22,** 50.0 mg, 0.117 mmol, this intermediate was described in patent application publication WO2014144100), 4-(2-bromoethyl)morpholine hydrobromide (35.5 mg, 0.129 mmol), and cesium carbonate (57.3 mg, 0.176 mmol) in dioxane (1 mL) was heated at 90 °C for 16 hours. The reaction mixture was allowed to cool to room temperature then purified by silica gel chromatography utilizing a gradient of 3 to 7% methanol in dichloromethane over 45 minutes to afford 2-morpholinoethyl 3-((5-fluoro-2-(1-(2-fluorobenzyl)-5-(isoxazol-3-yl)-1H-pyrazol-3-yl)pyrimidin-4-yl)amino)propanoa te, **Compound 162** (34.8 mg, 55 % yield), as a white solid. ¹H NMR (500 MHz, CD₃OD) δ (ppm): 8.76 (s, 1H), 8.07 (d, 1H), 7.44 (s, 1H), 7.25 - 7.29 (m, 1H), 7.07 - 7.11 (m, 1H), 7.02 - 7.05 (m, 1H), 6.91 (s, 1H), 6.81 - 6.84 (m, 1H), 5.96 (s, 2H), 4.22 (t, 2H), 3.91 (t, 2H), 3.63 (t, 4H), 2.76 (t, 2H), 2.59 (t, 2H), 2.45 - 2.50 (m, 4H).

### Reference Compound 163

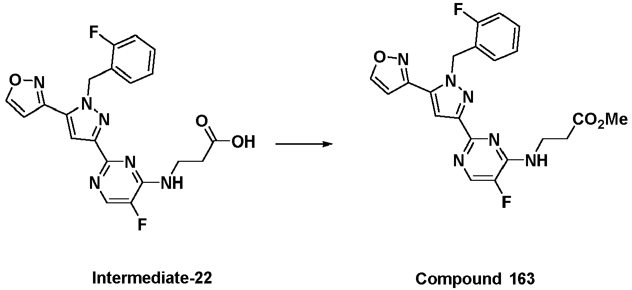

To a suspension of 3-((5-fluoro-2-(1-(2-fluorobenzyl)-5-(isoxazol-3-yl)-1H-pyrazol-3-yl) pyrimidin-4-yl)amino)propanoic acid (**Intermediate-22,** 52.1 mg, 0.122 mmol) in a mixture of diethyl ether (4 mL) and methanol (1 mL) was added a 2M in diethyl ether solution of trimethylsilyldiazomethane (183 µL, 0.367 mmol). After 30 minutes, the reaction mixture was concentrated then purified by silica gel chromatography utilizing a gradient of 1 to 8% methanol in dichloromethane over 45 minutes to afford methyl 3-((5-fluoro-2-(1-(2-fluorobenzyl)-5-(isoxazol-3-yl)-1H-pyrazol-3-yl)pyrimidin-4-yl)amino)propanoa te, **Compound 163** (27.1 mg, 50 % yield), as a white solid. ¹H NMR (500 MHz, CD₃OD) δ (ppm): 8.76 (s, 1H), 8.06 (d, 1H), 7.43 (s, 1H), 7.25 - 7.29 (m, 1H), 7.07 - 7.11 (m, 1H), 7.02 - 7.05 (m, 1H), 6.90 (s, 1H), 6.80 - 6.83 (m, 1H), 5.96 (s, 2H), 3.89 (t, 2H), 3.66 (s, 3H), 2.74 (t, 2H).

### Reference Compound 164

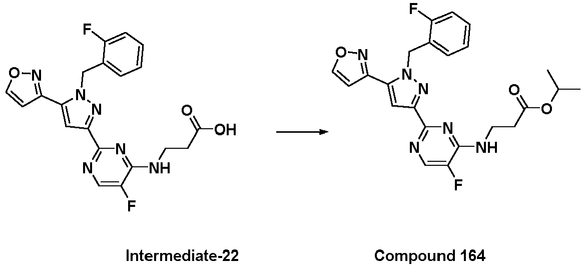

To a 0 °C suspension of 3-((5-fluoro-2-(1-(2-fluorobenzyl)-5-(isoxazol-3-yl)-1H-pyrazol-3-yl)pyrimidin-4-yl)amino)propanoic acid (**Intermediate-22,** 68.6 mg, 0.161 mmol) in a mixture of dichloromethane (4 mL) and acetonitrile (2 mL) was added a 2M in dichloromethane solution of oxalyl chloride (0.201 mL, 0.402 mmol). Three drops of *N,N*-dimethylformamide were added, and the resulting reaction mixture was stirred at 0 °C for 10 minutes then warmed up to room temperature. After 30 minutes, additional oxalyl chloride solution (0.8 mL) was added, after which the reaction mixture was stirred for 15 minutes, then concentrated to dryness to afford 3-((5-fluoro-2-(1-(2-fluorobenzyl)-5-(isoxazol-3-yl)-1H-pyrazol-3-yl)pyrimidin-4-yl)amino)propanoyl chloride (72 mg, 0.162 mmol, 101 % yield) as a waxy off-white solid.
To a solution of 3-((5-fluoro-2-(1-(2-fluorobenzyl)-5-(isoxazol-3-yl)-1H-pyrazol-3-yl)pyrimidin-4-yl)amino)propanoyl chloride (36 mg, 0.081 mmol) in dichloromethane (7.5 mL) was added propan-2-ol (0.616 ml, 8.09 mmol). The reaction mixture was stirred for 15 minutes, after which the solvent was removed in vacuo to afford the crude product. Purification was achieved by silica gel chromatography utilizing a gradient of 1 to 8% methanol in dichloromethane over 45 minutes to afford isopropyl 3-((5-fluoro-2-(1-(2-fluorobenzyl)-5-(isoxazol-3-yl)-1H-pyrazol-3-yl)pyrimidin-4-yl)amino)propanoa te, **Compound 164** (11 mg, 29 % yield) as an off-white waxy solid. ¹H NMR (500 MHz, CDCl₃) δ (ppm): 8.46 (s, 1H), 8.15 (s, 1H), 7.35 (s, 1H), 7.18 - 7.22 (m, 1H), 7.01 - 7.05 (m, 1H), 6.96 - 6.99 (m, 1H), 6.83 - 6.86 (m, 1H), 6.60 (s, 1H), 5.98 (s, 2H), 5.74 (br. s, 1H), 5.08 (m, 1H), 3.93 - 3.96 (m, 2H), **2**.70 (t, 2H), 1.27 (d, 6H).

### Reference Compound 165

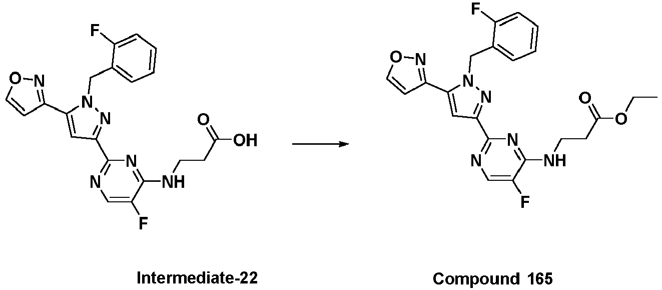

To a 0 °C suspension of 3-((5-fluoro-2-(1-(2-fluorobenzyl)-5-(isoxazol-3-yl)-1H-pyrazol-3-yl) pyrimidin-4-yl)amino)propanoic acid (**Intermediate-22,** 68.6 mg, 0.161 mmol) in a mixture of dichloromethane (4 mL) and acetonitrile (2 mL) was added a 2M in dichloromethane solution of oxalyl chloride (0.201 mL, 0.402 mmol). Three drops of *N,N*-dimethylformamide were added, and the resulting reaction mixture was stirred at 0 °C for 10 minutes then warmed up to room temperature. After 30 minutes, additional oxalyl chloride solution (0.8 mL) was added, after which the reaction mixture was stirred for 15 minutes, then concentrated to dryness to afford 3-((5-fluoro-2-(1-(2-fluorobenzyl)-5-(isoxazol-3-yl)-1H-pyrazol-3-yl) -pyrimidin-4-yl)amino)propanoyl chloride (72 mg, 0.162 mmol, 101 % yield) as a waxy off-white solid.

To a solution of 3-((5-fluoro-2-(1-(2-fluorobenzyl)-5-(isoxazol-3-yl)-1H--pyrazol-3-y1)pyrimidin-4-yl)amino)propanoyl chloride (36 mg, 0.081 mmol) in dichloromethane (7.5 mL) was added absolute ethanol (0.473 mL, 8.09 mmol). The reaction mixture was stirred for 15 minutes, after which the solvent was removed in vacuo to afford the crude product. Purification was achieved by silica gel chromatography utilizing a gradient of 1 to 8% methanol in dichloromethane over 45 minutes to afford ethyl 3-((5-fluoro-2-(1-(2-fluorobenzyl)-5-(isoxazol-3-yl)-1H-pyrazol-3-yl)pyrimidin-4-yl)amino)propanoa te, **Compound 165** (8.0 mg, 22 % yield), as an off-white waxy solid. ¹H NMR (500 MHz, CDCl₃) δ (ppm): 8.46 (s, 1H), 8.16 (s, 1H), 7.39 (br. s, 1H), 7.18 - 7.22 (m, 1H), 7.01 - 7.05 (m, 1H), 6.96 - 6.99 (m, 1H), 6.84 - 6.87 (m, 1H), 6.61 (s, 1H), 5.98 (s, 2H), 4.20 (q, 2H), 3.95 - 3.98 (m, 2H), 2.74 (t, 2H), 1.29 (t, 3H), [1 NH proton not observed].

### Reference Compound 166

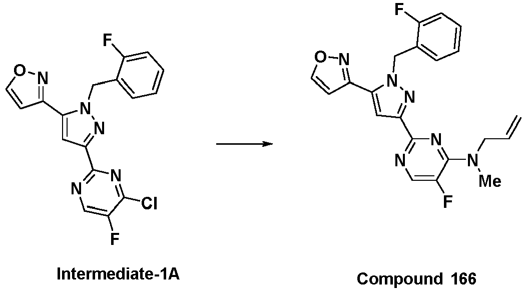

A suspension of **Intermediate-IA** (294 mg, 0.787 mmol), *N*-methylprop-2-en-1-amine (0.187 mL, 1.98 mmol) in a mixture of dioxane (2 mL) and water (1 mL) was heated at 90 °C for 2 hours. The reaction mixture was allowed to cool to room temperature then diluted in IN hydrochloric acid solution and water, leading to the formation of a precipitate. This solid was filtered and dried in vacuo to afford *N*-allyl-5-fluoro-2-(1-(2-fluorobenzyl)-5-(isoxazol-3-yl)-1H-pyrazol-3-yl)-N-methylpyrimidin-4-amin e, **Compound 166** (288 mg, 90 % yield), as a pale yellow solid. ¹H NMR (500 MHz, CDCl₃) δ (ppm): 8.45 (d, 1H), 8.16 (d, 1H), 7.29 (s, 1H), 7.17 - 7.21 (m, 1H), 7.00 - 7.05 (m, 1H), 6.95 - 6.98 (m, 1H), 6.83 - 6.87 (m, 1H), 6.59 (d, 1H), 5.97 (s, 2H), 5.87 - 5.96 (m, 1H), 5.25 - 5.28 (m, 1H), 5.23 (s, 1H), 4.23 (d, 2H), 3.28 (d, 3H).

### Reference Compound 167

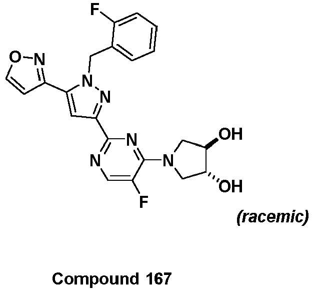

A suspension of 3 -(3 -(4-chloro-5 -fluoropyrimidin-2-yl)-1-(2-fluorobenzyl)-1H-pyrazol-5-yl) isoxazole (**Intermediate 1A**, 195 mg, 0.521 mmol) and trans-pyrrolidine-3,4-diol (113 mg, 1.10 mmol) in a mixture of dioxane (1 mL) and water (0.5 mL) was heated at 90 °C for 2 hours. The reaction mixture was then cooled to room temperature and diluted in IN hydrochloric acid and water, leading to the formation of a white precipitate, which was filtered and dried. The crude product was reconstituted in dichloromethane/isopropanol (5:1) and washed with water (2 x 30 mL), saturated sodium chloride solution (2 x 30 mL), dried (sodium sulfate), filtered, and concentrated to afford trans-1-(5-fluoro-2-(1-(2-fluorobenzyl)-5-(isoxazol-3-yl)-1H-pyrazol-3-yl)pyrimidin-4-yl)pyrrolidine-3,4-diol, **Compound 167** (226 mg, 98 % yield), as an off-white solid. ¹H NMR (500 MHz, DMSO-*d₆*) δ (ppm): 9.08 (d, 1H), 8.22 (d, 1H), 7.52 (s, 1H), 7.31 - 7.35 (m, 1H), 7.24 (d, 1H), 7.20 - 7.23 (m, 1H), 7.09 - 7.12 (m, 1H), 6.83 - 6.86 (m, 1H), 5.90 (s, 2H), 5.21 (d, 2H), 4.05 (br. s, 2H), 3.79 - 3.83 (br. m, 2H), 3.68 (d, 2H).

### Reference Compound 168

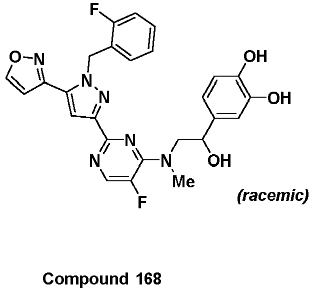

A suspension of **Intermediate-IA** (122 mg, 0.326 mmol), 4-(1-Hydroxy-2-methylamino-ethyl)-benzene-1,2-diol (62.1 mg, 0.339 mmol), and triethylamine (0.182 mL, 1.31 mmol) in a mixture of dioxane (1 mL) and water (0.5 mL) was heated at 90 °C for 2 hours. The reaction mixture was then allowed to cool to room temperature, diluted in IN aqueous hydrochloric acid solution and water, filtered and dried to afford the crude product as tan solid. Purification of this material was achieved by silica gel chromatography utilizing a gradient of 1 to 8% methanol in dichloromethane over 60 minutes to deliver 4-(2-((5-fluoro-2-(1-(2-fluorobenzyl)-5-(isoxazol-3-yl)-1H-pyrazol-3-yl)pyrimidin-4-yl)(methyl)amino)-1-hydroxyethyl)benzene-1,2-diol, **Compound 168** (24.4 mg, 14 % yield), as a tan solid. ¹H NMR (500 MHz, CD₃OD) δ (ppm): 8.75 (d, 1H), 8.07 (d, 1H), 7.38 (s, 1H), 7.24 - 7.28 (m, 1H), 7.06 - 7.10 (m, 1H), 7.01 - 7.04 (m, 1H), 6.90 (d, 1H), 6.87 - 6.89 (m, 1H), 6.84 - 6.86 (m, 1H), 6.73 - 6.75 (m, 1H), 6.72 (d, 1H), 5.96 (s, 2H), 4.89 - 4.93 (m, 1H), 3.82 - 3.93 (m, 2H), 3.30 (d, 3H).

### Reference Compound 169

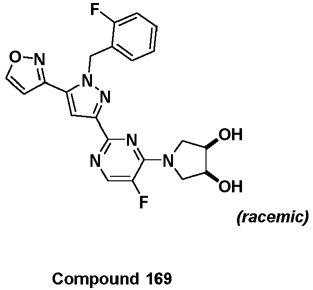

A suspension of **Intermediate-IA** (115 mg, 0.308 mmol), cis-pyrrolidine-3,4-diol (41.2 mg, 0.400 mmol) and triethylamine (0.214 mL, 1.538 mmol) in a mixture of dioxane (1 mL) and water (0.5 mL) was heated at 90 °C for 2 hours. The reaction mixture was then allowed to cool to room temperature, diluted in aqueous IN hydrochloric acid solution and water, filtered and dried in vacuo to afford the crude product cis-1-(5-fluoro-2-(1-(2-fluorobenzyl)-5-(isoxazol-3-yl)-1H-pyrazol-3-yl)pyrimidin-4-yl)pyrrolidine-3, 4-diol, **Compound 169** (104.6 mg, 0.219 mmol, 71.0 % yield), as an off-white solid. No purification was necessary. ¹H NMR (500 MHz, DMSO-*d₆*) δ (ppm): 9.08 (d, 1H), 8.21 (d, 1H), 7.51 (s, 1H), 7.30 - 7.35 (m, 1H), 7.24 (d, 1H), 7.20 - 7.23 (m, 1H), 7.09 - 7.12 (m, 1H), 6.82 - 6.86 (m, 1H), 5.90 (s, 2H), 5.01 (d, 2H), 4.12 - 4.15 (m, 2H), 3.80 - 3.84 (m, 2H), 3.55 - 3.59 (m, 2H).

### Reference Compound 170

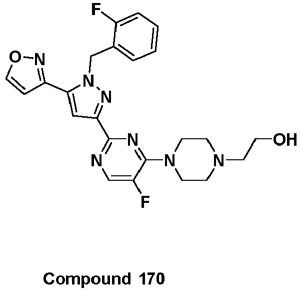

A suspension of **Intermediate-IA** (65.5 mg, 0.175 mmol) and 2-(piperazin-1-yl)ethanol (0.0860 mL, 0.701 mmol) in a mixture of dioxane (2 mL) and water (1 mL) was heated to 85 °C for 12 hours. The reaction mixture was then cooled to room temperature, diluted in aqueous IN hydrochloric acid solution and water, filtered and dried in vacuo to afford 2-(4-(5-fluoro-2-(1-(2-fluorobenzyl)-5-(isoxazol-3-yl)-1H-pyrazol-3-yl)pyrimidin-4-yl)piperazin-1-yl )ethanol, **Compound 170** (40.8 mg, 50 % yield), as a white solid. No purification was necessary. ¹H NMR (500 MHz, CD₃OD) δ (ppm): 8.74 (d, 1H), 8.15 (d, 1H), 7.42 (s, 1H), 7.24 - 7.28 (m, 1H), 7.06 - 7.10 (m, 1H), 7.00 - 7.03 (m, 1H), 6.89 (d, 1H), 6.79 - 6.82 (m, 1H), 5.94 (s, 2H), 3.96 (t, 4H), 3.72 (t, 2H), 2.67 (t, 4H), 2.59 (t, 2H).

### Reference Compound 171

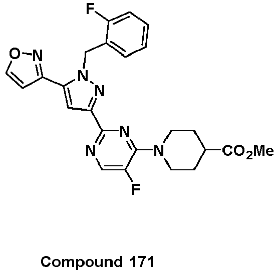

A suspension of **Intermediate-IA** (70.0 mg, 0.187 mmol) and methyl 4-piperidinecarboxylate (0.0760 mL, 0.562 mmol) in a mixture of dioxane (2 mL) and water (1 mL) was heated at 90 °C for 12 hours. The reaction mixture was allowed to cool to room temperature, then diluted with water, extracted with dichloromethane (3 x 30 mL), dried (sodium sulfate), filtered and concentrated to afford the crude product. Purification was achieved by silica gel chromatography utilizing a gradient of 1 to 8% methanol in dichloromethane over 60 minutes to afford methyl 1-(5-fluoro-2-(1-(2-fluorobenzyl)-5-(isoxazol-3-yl)-1H-pyrazol-3-yl)pyrimidin-4-yl)piperidine-4-carb oxylate, Compound **171** (73.8 mg, 82 % yield), as a clear viscous oil which solidified upon standing to a white gum. ¹H NMR (500 MHz, CD₃OD) δ (ppm): 8.75 (d, 1H), 8.14 (d, 1H), 7.42 (s, 1H), 7.24 - 7.29 (m, 1H), 7.08 - 7.10 (m, 1H), 7.01 - 7.04 (m, 1H), 6.90 (d, 1H), 6.80 - 6.83 (m, 1H), 5.95 (s, 2H), 4.58 - 4.61 (m, 2H), 3.70 (s, 3H), 3.27 - 3.31 (m, 2H), 2.72 - 2.78 (m, 1H), 2.03 - 2.06 (m, 2H) 1.74 - 1.82 (m, 2H).

### Reference Compound 172

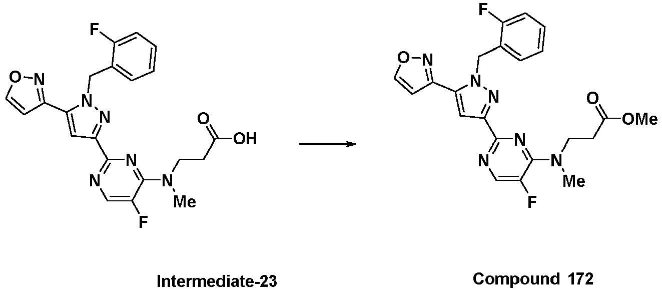

To a suspension of 3-((5-fluoro-2-(1-(2-fluorobenzyl)-5-(isoxazol-3-yl)-1H-pyrazol-3-yl) pyrimidin-4-yl)(methyl)amino)propanoic acid **(Intermediate-23**, 25.6 mg, 0.0580 mmol, this intermediate was described in patent application publication WO2014144100) in a solution of diethyl ether (0.75 mL) and methanol (0.25 mL) was added a 2M in diethyl ether solution of trimethylsilyldiazomethane (0.035 mL, 0.070 mmol). After 1 hour, the reaction was purified by silica gel chromatography utilizing a gradient of 1 to 8% methanol in dichloromethane over 60 minutes to afford methyl 3-((5-fluoro-2-(1-(2-fluorobenzyl)-5-(isoxazol-3-yl)-1H-pyrazol-3-yl)pyrimidin-4-yl)(methyl)amino)p ropanoate, **Compound 172** (7.2 mg, 27 % yield), as a clear oil which solidified upon standing to a white waxy solid. ¹H NMR (500 MHz, CDCl₃) δ (ppm): 8.45 (d, 1H), 8.18 (d, 1H), 7.30 (s, 1H), 7.18 - 7.21 (m, 1H), 7.01 - 7.04 (m, 1H), 6.95 - 6.99 (m, 1H), 6.85 - 6.89 (m, 1H), 6.59 (d, 1H), 5.96 (s, 2H), 4.00 (t, 2H), 3.69 (s, 3H), 3.35 (d, 3H), 2.76 (t, 2H).

### Reference Compound 173

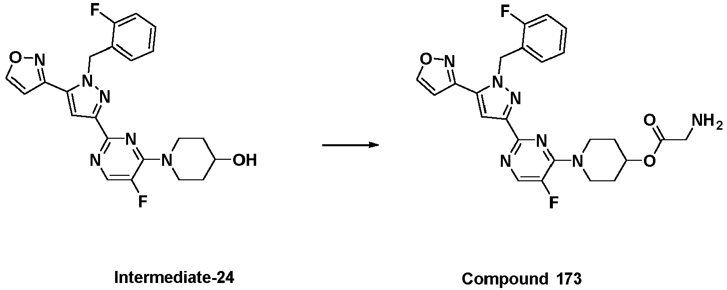

To a solution of 1-(5-fluoro-2-(1-(2-fluorobenzyl)-5-(isoxazol-3-yl)-1H-pyrazol-3-yl )pyrimidin-4-yl)piperidin-4-ol (**Intermediate-24**, 15 mg, 0.034 mmol, this intermediate was described in patent application publication WO2014144100) in dichloromethane (1 mL) was added *N*-alpha-t-Boc-Glycine (7.8 mg, 0.044 mmol) followed by *N,N-*dimethylaminopyridine (2.1 mg, 0.017 mmol) and 1-ethyl-3-(3-dimethylaminopropyl)carbodiimide hydrochloride (8.5 mg, 0.044 mmol). The reaction mixture was allowed to stir at room temperature for 72 hours, after which the reaction mixture was purified by silica gel chromatography utilizing a gradient of 1 to 8% methanol in dichloromethane over 60 minutes to afford 1-(5-fluoro-2-(1-(2-fluorobenzyl)-5-(isoxazol-3-yl)-1H-pyrazol-3-yl)pyrimidin-4-yl)piperidin-4-yl 2-((tert-butoxycarbonyl)amino)acetate (21.0 mg, 103 % yield) as a white solid. To a solution of 1-(5-fluoro-2-(1-(2-fluorobenzyl)-5-(isoxazol-3-yl)-1H-pyrazol-3-yl) -pyrimidin-4-yl)piperidin-4-yl 2-((tert-butoxycarbonyl)amino)acetate (21.0 mg, 0.035 mmol) in dichloromethane (4 mL) was added trifluoroacetic acid (0.30 mL, 3.9 mmol). The reaction mixture was heated at 60 °C for one hour, after which the reaction mixture was cooled to room temperature, neutralized by the addition of saturated sodium bicarbonate solution, extracted with dichloromethane (3 x 30 mL), dried (sodium sulfate), filtered and concentrated to afford 1-(5-fluoro-2-(1-(2-fluorobenzyl)-5-(isoxazol-3-yl)-1H-pyrazol-3-yl)pyrimidin-4-yl)piperidin-4-yl 2-aminoacetate, **Compound 173** (12.7 mg, 73 % yield), as creme-colored solid. ¹H NMR (500 MHz, CD₃OD) δ (ppm): 8.75 (d, 1H), 8.16 (d, 1H), 7.42 (s, 1H), 7.25 - 7.29 (m, 1H), 7.07 - 7.11 (m, 1H), 7.01 - 7.04 (m, 1H), 6.90 (d, 1H), 6.80 - 6.83 (m, 1H), 5.95 (s, 2H), 5.12 - 5.16 (m, 1H), 4.18 - 4.24 (m, 2H), 3.76 - 3.82 (m, 2H), 3.43 (s, 2H), 2.05 - 2.10 (m, 2H) 1.77 - 1.84 (m, 2H).

### Reference Compound 174

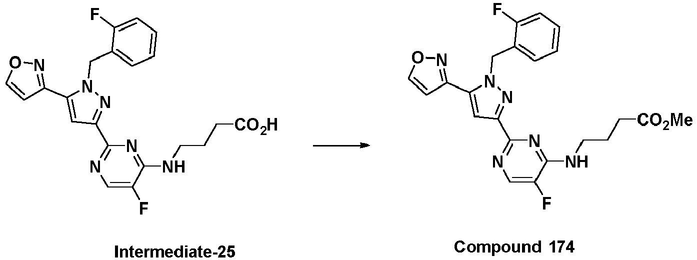

To a suspension of 4-((5-fluoro-2-(1-(2-fluorobenzyl)-5-(isoxazol-3-yl)-1H-pyrazol-3-yl) pyrimidin-4-yl)amino)butanoic acid (Intermediate-24, 8.6 mg, 0.020 mmol, this intermediate was described in patent application publication WO2014144100) in a mixture of diethyl ether (0.50 mL) and methanol (0.167 mL) was added a 2M in diethyl ether solution of trimethylsilyldiazomethane (9.8 µL, 0.020 mmol). After 1 hour, the reaction was concentrated, then purified using reverse phase HPLC utilizing a gradient of 5 to 95% acetonitrile in water (spiked with 0.1% trifluoroacetic acid) over 25 minutes to afford methyl 4-((5-fluoro-2-(1-(2-fluorobenzyl)-5-(isoxazol-3-yl)-1H-pyrazol-3-yl)pyrimidin-4-yl)amino)butanoate, **Compound 174** (2.8 mg, 32 % yield), as a clear colorless oil. ¹H NMR (500 MHz, CDCl₃) δ (ppm): 8.52 (d, 1H), 8.38 (d, 1H), 7.48 (s, 1H), 7.23 - 7.27 (m, 1H), 7.14 - 7.18 (m, 1H), 7.04 - 7.07 (m, 1H), 7.01 - 7.04 (m, 1H), 6.66 (d, 1H), 5.93 (s, 2H), 3.81 (m, 2H), 3.72 (s, 3H), 2.54 (t, 2H), 2.07 - 2.12 (m, 2H), [1 N-H not observed].

### Reference Compound 175

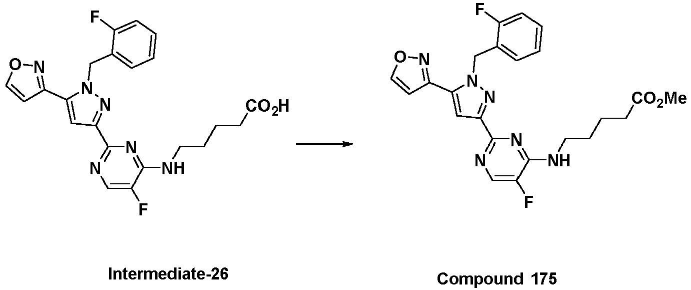

To a suspension of 5-((5-fluoro-2-(1-(2-fluorobenzyl)-5-(isoxazol-3-yl)-1H-pyrazol-3-yl) pyrimidin-4-yl)amino)pentanoic acid (37.2 mg, 0.0820 mmol, this intermediate was previously described in patent application publication WO2014144100) in a mixture of diethyl ether (0.75 mL) and methanol (0.250 mL) was added a 2M diethyl ether solution of trimethylsilyldiazomethane (0.045 mL, 0.090 mmol). After 1 hour, the reaction was purified directly by silica gel chromatography utilizing a gradient of 1 to 8% methanol in dichloromethane over 60 minutes to deliver methyl 5 -((5 -fluoro-2-(1-(2-fluorobenzyl)-5--(isoxazol-3-yl)-1H-pyrazol-3-yl)pyrimidin-4-yl)amino)pentanoate, **Compound 175** (12.8 mg, 33 % yield), as a clear colorless waxy gum. ¹H NMR (500 MHz, CDCl₃) δ (ppm): 8.45 (d, 1H), 8.14 (d, 1H), 7.34 (s, 1H), 7.17 - 7.22 (m, 1H), 7.01 - 7.04 (m, 1H), 6.95 - 6.98 (m, 1H), 6.84 - 6.87 (m, 1H), 6.65 (d, 1H), 5.98 (s, 2H), 5.17 (m, 1H), 3.68 (s, 3H), 3.62 - 3.67 (m, 2H), 2.43 (t, 2H), 1.74 - 1.81 (m, 4H).

### Reference Compound 176

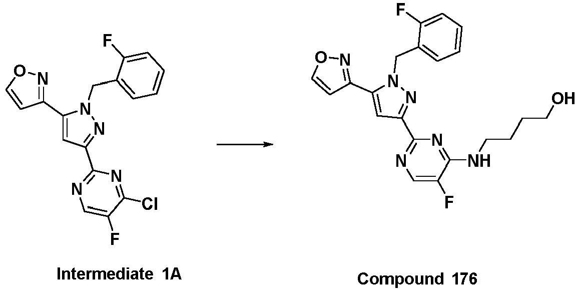

A suspension of **Intermediate-IA** (124 mg, 0.332 mmol) and 4-aminobutan-1-ol (0.122 mL, 1.33 mmol) in a mixture of dioxane (2 mL) and water (1 mL) was heated at 70 °C for 2 hours. The reaction mixture was diluted in water leading to the formation of a white precipitate. This product was filtered and dried in vacuo to afford 4-((5-fluoro-2-(1-(2-fluorobenzyl)-5-(isoxazol-3-yl)-1H-pyrazol-3-yl)pyrimidin-4-yl)amino)butan-1-o 1, **Compound 176** (98.8 mg, 70 % yield), as a white solid. No purification was necessary. ¹H NMR (500 MHz, CD₃OD) δ (ppm): 8.74 (s, 1H), 8.01 (d, 1H), 7.40 (s, 1H), 7.24 - 7.28 (m, 1H), 7.06 - 7.10 (m, 1H), 7.01 - 7.04 (m, 1H), 6.89 (m, 1H), 6.81 - 6.84 (m, 1H), 5.95 (s, 2H), 3.61 - 3.66 (m, 4H), 1.74 - 1.80 (m, 2H) 1.62 - 1.68 (m, 2H).

### Reference Compound 177

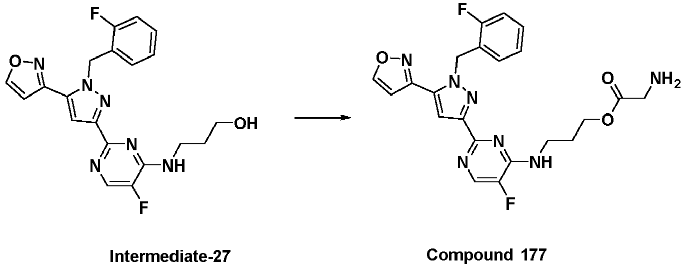

To a solution of 3-((5-fluoro-2-(1-(2-fluorobenzyl)-5-(isoxazol-3-yl)-1H-pyrazol-3-yl) -pyrimidin-4-yl)amino)propan-1-ol (**Intermediate-27**, 98.5 mg, 0.239 mmol, this intermediate was described in patent application publication WO2014144100), *N*-alpha-t-Boc-Glycine (50.2 mg, 0.287 mmol), and *N,N-*dimethylaminopyridine (8.8 mg, 0.072 mmol) in dichloromethane (2 mL) was added 1-ethyl-3-(3-dimethylaminopropyl)carbodiimide hydrochloride (54.9 mg, 0.287 mmol). The reaction was stirred at room temperature for 16 hours, after which the reaction mixture was purified directly by silica gel chromatography utilizing a gradient of 1 to 8% methanol in dichloromethane over 60 minutes to 4-((5-fluoro-2-(1-(2-fluorobenzyl)-5-(isoxazol-3-yl)-1H-pyrazol-3-y1)pyrimidin-4-yl)amino)butyl 2-((tert-butoxycarbonyl)amino)acetate intermediate (98.8 mg).
To a solution of 4-((5-fluoro-2-(1-(2-fluorobenzyl)-5-(isoxazol-3-yl)-1H-pyrazol-3-yl)pynmidin-4-yl)amino)butyl 2-((tert-butoxycarbonyl)amino)acetate in dichloromethane (2 mL) was added trifluoroacetic acid (0.50 mL, 6.5 mmol). The reaction mixture was heated at 60 °C for 30 minutes, after which the deprotection was complete. The reaction mixture was then neutralized by the addition of saturated sodium carbonate solution, extracted with dichloromethane (3 x 30 mL), dried (sodium sulfate), filtered and concentrated in vacuo to afford 3-((5-fluoro-2-(1-(2-fluorobenzyl)-5-(isoxazol-3-yl)-1H-pyrazol-3-yl)pyrimidin-4-yl)amino)propyl 2-aminoacetate, **Compound 177** (68.6 mg, 61 % yield), as a waxy white solid. No purification was necessary. ¹H NMR (500 MHz, CD₃OD) : δ (ppm) 8.75 (s, 1H), 8.04 (d, 1H), 7.41 (s, 1H), 7.24 - 7.29 (m, 1H), 7.07 - 7.11 (m, 1H), 7.02 - 7.05 (m, 1H), 6.90 (m, 1H), 6.82 - 6.85 (m, 1H), 5.95 (s, 2H), 4.28 (t, 2H), 3.72 (t, 2H), 3.36 (s, 2H), 2.02 - 2.09 (m, 2H).

### Reference Compound 178

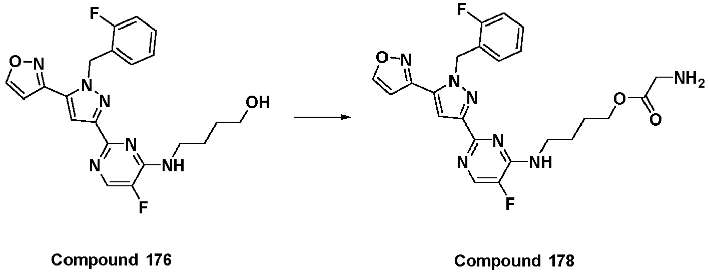

To a solution of 4-((5-fluoro-2-(1-(2-fluorobenzyl)-5-(isoxazol-3-yl)-1H-pyrazol-3-yl) pyrimidin-4-yl)amino)butan-1-ol (**Compound 176**, 69.1 mg, 0.162 mmol), *N*-alpha-t-Boc-Glycine (34.1 mg, 0.194 mmol), and *N,N-*dimethylaminopyridine (5.9 mg, 0.049 mmol) in dichloromethane (2 mL) was added 1-ethyl-3-(3-dimethylaminopropyl)carbodiimide hydrochloride (37.3 mg, 0.194 mmol). The reaction was stirred at room temperature for 16 hours, after which the reaction mixture was purified directly by silica gel chromatography utilizing a gradient of 1 to 8% methanol in dichloromethane over 60 minutes to afford 4-((5-fluoro-2-(1-(2-fluorobenzyl)-5-(isoxazol-3-yl)-1H-pyrazol-3-yl)pyrimidin-4-yl)amino)butyl 2-((tert-butoxycarbonyl)amino)acetate (64.9 mg).
To a solution of 4-((5-fluoro-2-(1-(2-fluorobenzyl)-5-(isoxazol-3-yl)-1H-pyrazol-3-yl) pyrimidin-4-yl)amino)butyl 2-((tert-butoxycarbonyl)amino)acetate (64.9 mg) in dichloromethane (2 mL), was added trifluoroacetic acid (0.50 mL, 6.5 mmol). The reaction mixture was heated to 60 °C for 30 minutes, after which the deprotection was complete. The reaction mixture allowed to cool to room temperature, neutralized by the addition of saturated sodium carbonate solution, extracted with dichloromethane (3 x 30 mL), dried (sodium sulfate), filtered and concentrated to afford 4-((5-fluoro-2-(1-(2-fluorobenzyl)-5-(isoxazol-3-yl)-1H-pyrazol-3-yl)pyrimidin-4-yl)amino)butyl 2-aminoacetate, **Compound 178** (35.4 mg, 45 % yield), as a clear gum. No purification was necessary. ¹H NMR (500 MHz, CD₃OD): δ (ppm) 8.75 (s, 1H), 8.03 (d, 1H), 7.41 (s, 1H), 7.24 - 7.28 (m, 1H), 7.07 - 7.10 (m, 1H), 7.02 - 7.05 (m, 1H), 6.92 (m, 1H), 6.82 - 6.85 (m, 1H), 5.95 (s, 2H), 4.22 - 4.25 (m, 2H), 3.65 - 3.68 (m, 2H), 3.34 (s, 2H), 1.74 - 1.81 (m, 4H).

### Reference Compound 179

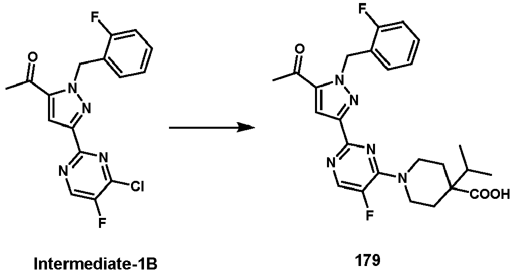

**Compound 179** was obtained by reacting 1-(3-(4-chloro-5-fluoropyrimidin-2-yl)-1-(2-fluorobenzyl)-1H-pyrazol-5-yl)ethanone **(Intermediate-1B**, 0.02, 0.057 mmol) with 4-isopropylpiperidine-4-carboxylic acid (0.054 g, 0.315 mmol), with triethylamine (10 equiv) as the base in dioxane (0.5 mL). The reaction was complete in 15 h after heating at 100 °C. After an aqueous workup, the product was purified using RP-HPLC to obtain the product as a tan solid (0.012 g, 39%). ¹H NMR (500 MHz, DMSO-*d*6) δ ppm 12.62 (br. s., 1 H), 8.29 (d, 1 H), 7.33 (q, 1 H) 7.75 (s, 1 H), 7.18 - 7.27 (m, 1 H), 7.11 (t, 1 H), 6.81 (t, 1 H), 5.82 (s, 2 H), 4.53 (d, 2 H), 3.02 (t, 2 H), 2.58 (s, 3 H), 2.12 (d, 2 H), 1.72 (dt, 1 H), 1.41 - 1.55 (m, 2 H), 0.87 (d, 6 H).

### Reference Compound 180

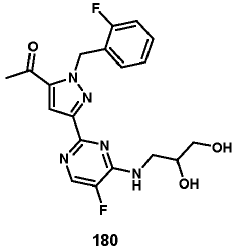

The titled product was obtained by reacting 1-(3-(4-chloro-5-fluoropyrimidin-2-yl)-1-(2-fluorobenzyl)-1H-pyrazol-5-yl) ethanone **(Intermediate-1B**, 0.02, 0.057 mmol) with 3-aminopropane-1,2-diol (0.052 g, 0.57 mmol), with triethylamine (10 equiv) as the base in dioxane (0.5 mL). The reaction was complete in 15 h after heating at 100 °C. After an aqueous workup, the product was purified using RP-HPLC and then flash chromatography eluting with 0-30% 7:1 ACN:MeOH in DCM to obtain the product as a white solid (0.014 g, 54%). ¹H NMR (500 MHz, METHANOL-*d*4) δ ppm 8.08 (d, 1H), 7.27 (q, 1 H) 7.68 (s, 1 H), 7.07 - 7.14 (m, 1 H), 7.04 (t, 1 H), 6.79 (t, 1 H), 5.90 (s, 2 H), 2.56 (s, 3 H) 3.87 (quintet, 1 H), 3.75 - 3.81 (m, 1 H), 3.61 - 3.68 (m, 1 H), 3.58 (d, 2 H), -NH and -OH protons exchanged with solvent.

### Reference Compound 181

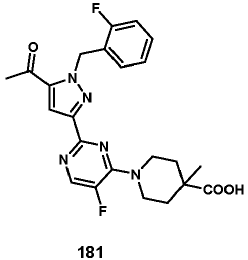

The titled product was obtained by reacting 1-(3-(4-chloro-5-fluoropyrimidin-2-yl)-1-(2-fluorobenzyl)-1H-pyrazol-5-yl) ethanone (**Intermediate-1B**, 0.02, 0.057 mmol) with 4-methylpiperidine-4-carboxylic acid (0.045 g, 0.315 mmol), with triethylamine (10 equiv) as the base in dioxane (0.5 mL). The reaction was complete in 15 h after heating at 100 °C. After an aqueous workup, the product was purified using RP-HPLC to obtain the product as a tan solid (0.025 g, 86%). ¹H NMR (500 MHz, METHANOL-*d*4) δ ppm; ¹H NMR (500 MHz, DMSO-*d*6) δ ppm 12.34 - 12.73 (br. s, 1 H), 8.30 (d, 1 H), 7.74 (s, 1 H), 7.28 - 7.39 (m, 1 H), 7.17 - 7.27 (m, 1 H), 7.06 - 7.15 (m, 1 H), 6.81 (t, 1 H), 5.82 (s, 2 H), 4.20 (d, 2 H), 3.36 (t, 2 H), 2.58 (s, 3 H), 2.07 (d, 2 H), 1.50 (t, 2 H), 1.19 (s, 3 H).

### Intermediate-30

This intermediate was prepared in two steps: 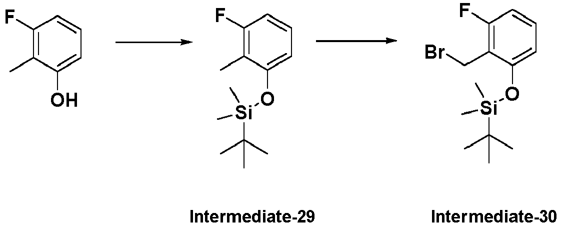
Step 1: A solution of commercially available 3-fluoro-2-methylphenol 2.0 g, 15.9 mmol) and tert-butylchlorodimethylsilane (3.6 g, 23.8 mmol) in dichloromethane (20 mL) was prepared at room temperature. Triethylamine (5.5 mL, 39.6 mmol) and 4-dimethylaminomethylpyridine (0.1 g, 0.8 mmol) were added and stirring was continued overnight at room temperature. The mixture was diluted with ethyl acetate (200 mL) and washed with water (3 x 30 mL), brine, then dried over sodium sulfate, filtered and concentrated by rotary evaporation to furnish the product **Intermediate-29** as a colorless oil (3.85 g, 101% yield). ¹H-NMR (500 MHz, CDCl₃) δ 6.77 (q, 1H), 6.43 (t, 1H), 6.36 (d, 1H), 1.90 (d, 3H), 0.80 (s, 9H), 0.00 (s, 6H) ppm.
Step 2: **Intermediate-29** (3.6 g, 15.0 mmol) and N-bromosuccinimide (2.8 g, 15.7 mmol) were mixed in carbon tetrachloride (20 mL) at room temperature. AIBN (0.2 g, 1.5 mmol) was added and the solution was heated 2 hr at 80°C. The mixture was cooled to room temperature and filtered. The filter cake was washed with CCl₄ and the combined filtrates concentrated by rotary evaporation. The residue was purified by chromatography over SiO₂ using a gradient of hexane/ethyl acetate as eluent to give **Intermediate-30** (4.9 g, 102% yield) as a colorless oil. ¹H-NMR (500 MHz, CDC13) δ 6.96 (q, 1H), 6.50 (t, 1H), 6.46 (d, 1H), 4.38 (s, 2H), 0.88 (s, 9H), 0.12 (s, 6H) ppm.

### Intermediate-31 and Intermediate-32

These were prepared in from a common **Intermediate-17**

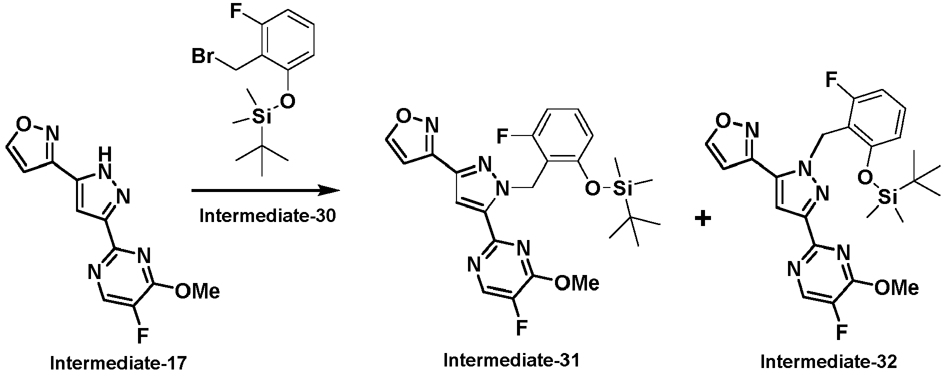

3-(3-(5-Fluoro-4-methoxypyrimidin-2-yl)-1H-pyrazol-5-yl)isoxazole (**Intermediate-17**, 50 mg, 0.19 mmol) was dissolved in dimethoxyethane (3 mL) at room temperature. A solution of potassium tert-butylate in tert-butanol (1.0 M, 0.38 mL, 0.38 mmol) and **Intermediate-30** (92 mg, 0.29 mmol) were added in succession and the mixture was heated at 60°C for 1 hr. The cooled solution was diluted with ethyl acetate, (80 mL) and washed with water (2 x 10 mL), brine, then dried over sodium sulfate, filtered and concentrated by rotary evaporation. Chromatography over SiO2 with hexane/ethyl acetate gave the two products as thick syrups. **Intermediate-31** (21 mg, 22 % yield). ¹H-NMR (500 MHz, CDCl₃) δ 8.38 (d, 1H), 8.35 (d, 1H), 7.53 (s, 1H), 7.14 (q, 1H), 6.6-6.7 (m, 3H), 6.09 (s, 2H), 4.13 (s, 3H), 0.93 (s, 9H), 0.23 (s, 6H) ppm. **Intermediate-32** (24 mg, 25 % yield). ¹H-NMR (500 MHz, CDCl₃) δ 8.47 (d, 1H), 8.35 (d, 1H), 7.28 (s, 1H), 7.10 (q, 1H), 6.55-6.65 (m, 3H), 5.91 (s, 2H), 4.14 (s, 3H), 0.95 (s, 9H), 0.26 (s, 6H) ppm.

### Reference Compound 182

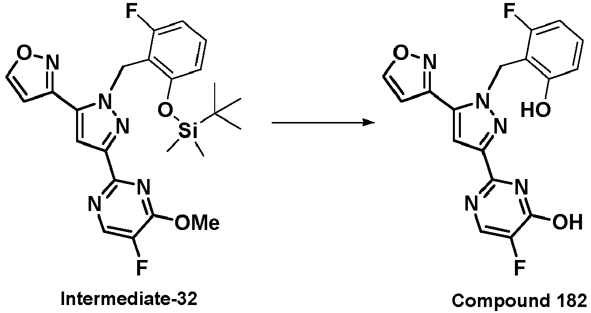

Concentrated aqueous hydrochloric acid (0.3 mL, 3.0 mmol) was added to **Intermediate-32** (21 mg, 0.04 mmol) in methanol (1 mL) and the mixture was heated in a sealed vial overnight at 60°C. After cooling to room temperature, the solvent was removed under vacuum to furnish the product as a white solid (15 mg, 96 % yield). LCMS (m/e) 372 (M+H).

### Reference Compound 183

This compound was prepared in two steps: 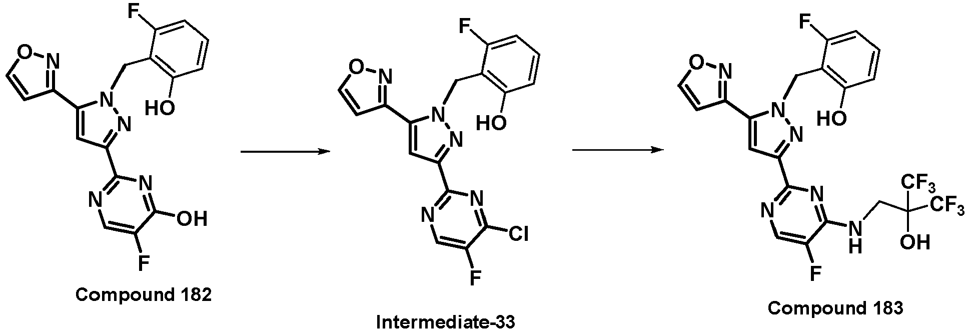
Step 1:**Compound 182** (15 mg, 0.04 mmol) in phosphorous oxychloride (0.50 mL, 5.4 mmol) was heated for 2 hr at 60°C, then the solvent was removed under vacuum. Pure **Intermediate-33** was obtained as a white solid by chromatography over SiO₂ with a gradient of hexane/ethyl acetate as eluant (2 mg, 16 % yield). ¹H-NMR (500 MHz, CDCl₃) δ 10.31 (br s, 1H), 8.66 (s, 1H), 8.60 (d, 1H), 7.32 (s, 1H), 7.19 (q, 1H), 6.85 (d, 1H), 6.70 (d, 1H), 6.62 (t, 1H), 5.97 (s, 2H) ppm.
Step 2: **Intermediate-33** (2 mg, 0.005 mmol and 2-(aminomethyl)-1,1,1,3,3,3-hexafluoropropan-2-ol (10 mg, 0.05 mmol) were dissolved in dimethylsulfoxide (1 mL) in a sealed vial and the solution was heated overnight at 125°C. The reaction mixture was diluted with ethyl acetate (50 mL) and washed with water (3 x 5 mL), brine and dried over sodium sulfate. Filtration and rotary evaporation gave a residue which was subjected to chromatographic purification over SiO2 using hexane/ethyl acetate as eluent. **Compound 183** was obtained as a white solid (2 mg, 64 % yield). ¹H-NMR (500 MHz, CDCl₃) δ 9.59 (br s, 1H), 8.27 (s, 1H), 8.24 (br s, 1H), 7.18 (s, 1H), 7.17 (q, 1H), 6.83 (s, 1H), 6.81 (s, 1H), 6.68 (s, 1H), 6.61 (t, 1H), 5.93 (s, 2H), 5.58 (br t, 1H), 4.18 (d, 2H) ppm.

### Intermediate-34

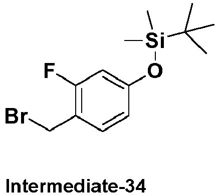

The same conditions described for the synthesis of **Intermediate-30** were used to prepare **Intermediate-34**, a colorless oil, from commercial 3-fluoro-4-methylphenol in 44 % overall yield. ¹H-NMR (500 MHz, CDC13) δ 7.25 (t, 1H), 6.63 (dd, 1H), 6.58 (dd, 1H), 4.53 (s, 2H), 1.00 (s, 9H), 0.24 (s, 6H) ppm.

### Intermediate-36

This intermediate was prepared in 2 steps from **Intermediate-17**; 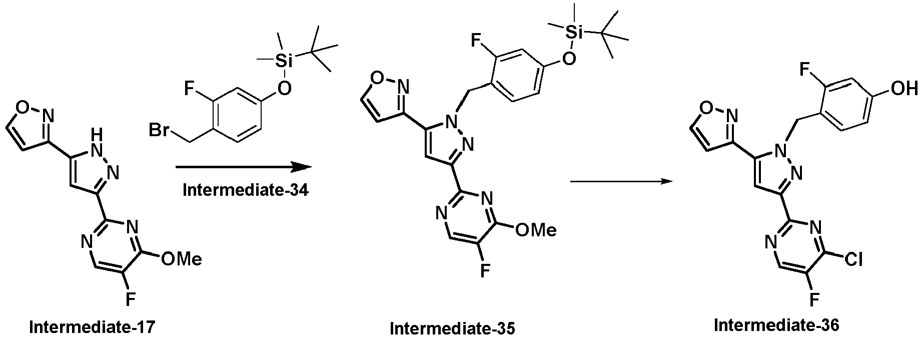
Step 1: 3-(3-(5-Fluoro-4-methoxypyrimidin-2-yl)-1H-pyrazol-5-yl)isoxazole (90 mg, 0.34 mmol) was dissolved in dimethoxyethane (3 mL) at room temperature. A solution of potassium tert-butylate in tert-butanol (1.0 M, 0.69 mL, 0.69 mmol) and **Intermediate-35** (165 mg, 0.52 mmol) were added in succession and the mixture was heated at 60°C for 1 hr. The cooled solution was diluted with ethyl acetate, (80 mL) and washed with water (2 x 10 mL), brine, then dried over sodium sulfate, filtered and concentrated by rotary evaporation. Chromatography over SiO2 with a gradient of hexane/ethyl acetate as eluant gave the product (38 mg, 22 % yield) as a colorless glass. ¹H-NMR (500 MHz, CDCl₃) δ 8.47 (s, 1H), 8.40 (d, 1H), 7.33 (s, 1H), 6.80 (t, 1H), 6.52 (dd, 1H), 6.46 (dd, 1H), 5.88 (s, 2H), 4.18 (s, 3H), 0.94 (s, 9H), 0.15 (s, 6H) ppm.
Step 2: Concentrated aqueous hydrochloric acid (0.3 mL, 3.0 mmol) was added to **Intermediate-35** (60 mg, 0.12 mmol) in methanol (1 mL) and the mixture was heated in a sealed vial overnight at 60°C. The solvents were removed under vacuum and the demethylated residue (44 mg, 99 % yield) carried on directly to next reaction. LCMS (m/e) 370 (M-H). The residue (44 mg, 0.12 mmol) in phosphorous oxychloride (1.0 mL, 10.7 mmol) was heated for 5 hr at 50°C, then the solvent was removed under vacuum. The residue was rubbed with ether/hexane and redried to leave crude **Intermediate-36** as a white solid (14 mg, 20% yield). This was carried on to the next reaction without characterization.

### Reference Compound 184

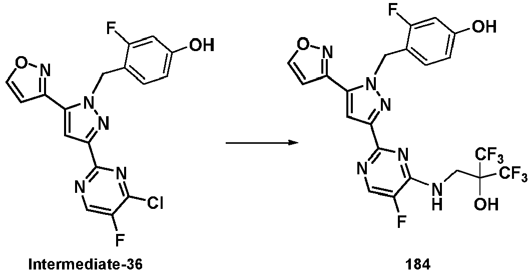

**Intermediate-36** (14 mg, 0.036 mmol and 2-(aminomethyl)-1,1,1,3,3,3-hexafluoropropan-2-ol (28 mg, 0.14 mmol) were dissolved in dimethylsulfoxide (1 mL) in a sealed vial and the solution was heated overnight at 125°C. The reaction mixture was diluted with ethyl acetate (50 mL) and washed with water (3 x 5 mL), brine and dried over sodium sulfate. Filtration and rotary evaporation gave a residue which was purified by preparative reverse phase HPLC using a gradient of water/acetonitrile (0.1% trifluoroacetic acid) as eluant. The product was obtained as a white solid (2 mg, 8 % yield). ¹H-NMR (500 MHz, d6-acetone) δ 9.00 (br s, 1H), 8.93 (s, 1H), 8.33 (d, 1H), 7.80 (brt, 1H), 7.40 (s, 1H), 7.21 (t, 1H), 7.05 (s, 1H), 6.59 (dd, 1H), 6.55 (dd, 1H), 5.83 (s, 2H), 4.18 (d, 2H) ppm.

### Reference Compound 207

The title compound was prepared over 2 steps:

### Step 1: Synthesis of N'-(cyanomethyl)-1-(2-fluorobenzyl)-5-(isoxazol-3-yl)-1H-pyrazole-3-carbohydrazonamide (Intermediate-28)

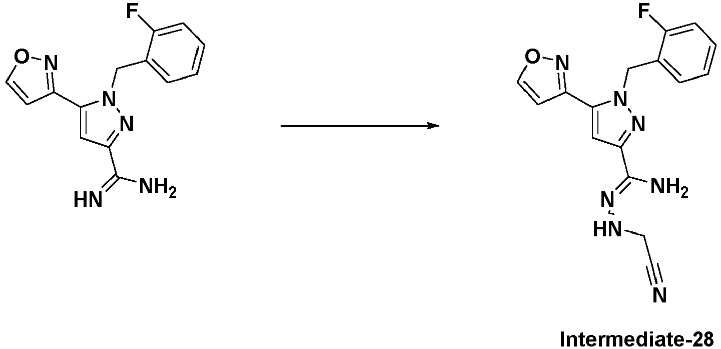

To a suspension of 1-(2-fluorobenzyl)-5-(isoxazol-3-yl)-1H-pyrazole-3-carboximidamide hydrochloride (1 equiv., this intermediate was described in a previous patent application publication WO2013/101830) in DMF was added hydrazine hydrate (1.5 equiv.), and contents were stirred for 1 h at 23 °C. 2-bromoacetonitrile (4 equiv.) and triethylamine (5 equiv.), was subsequently added, and reaction stirred at 23 °C for 2 h. The solution was diluted with a 1:1 mixture of ethyl acetate and water. The layers were separated and the aqueous was extracted with ethyl acetate (2x). The organic layers were combined, washed with water (3x) and brine. The contents were dried over magnesium sulfate, filtered, and the solvent was removed in vacuo. Purification of the resulting residue via silica gel chromatography (utilizing a methanol in DCM gradient) delivered the desired intermediate, N'-(cyanomethyl)-1-(2-fluorobenzyl)-5-(isoxazol-3-yl)-1H-pyrazole-3-carbohydrazonamide (397 mg, 75 % yield) as a yellow foam. ¹H NMR (400 MHz, CDCl₃) δ ppm 8.41 - 8.47 (m, 1 H), 7.17 - 7.28 (m, 1 H), 6.92 - 7.11 (m, 3 H), 6.75 - 6.87 (m, 1 H), 6.50 - 6.63 (m, 1 H), 5.86 (s, 2 H), 5.02 (s, 2 H), 3.90 - 4.06 (m, 3 H).

### Step 2: Synthesis of Compound 207

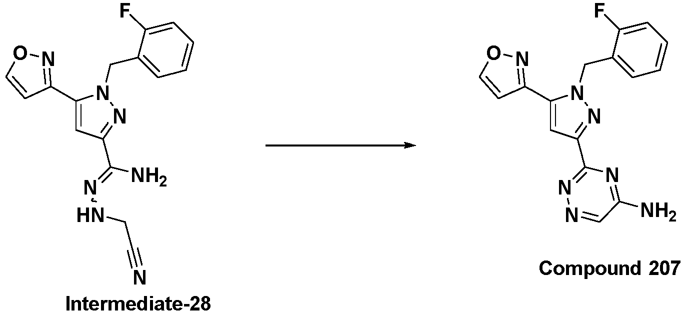

A solution of N'-(cyanomethyl)-1-(2-fluorobenzyl)-5-(isoxazol-3-yl)-1H-pyrazole-3-carbohydrazonamide (1 equiv., **Intermediate-28**) in ethanol was heated to 70 °C for 40 min. The reaction mixture was poured into a 1:1 mixture of ethyl acetate and saturated ammonium chloride. The layers were separated, and the aqueous layer was extracted with ethyl acetate (2x). The organics were combined, dried over magnesium sulfate, filtered, and the solvent was removed in vacuo. Purification of the resulting residue via reverse phase HPLC delivered the desired compound (9 mg, 7% yield) as a white solid. ¹H NMR (400 MHz, CD₃OD) δ ppm 8.82 (d, 1 H), 8.31 (s, 1 H), 7.62 (s, 1 H), 7.23 - 7.35 (m, 1 H), 6.88 - 7.16 (m, 4 H), 5.96 - 6.08 (m, 2 H).

### Reference Compound 198

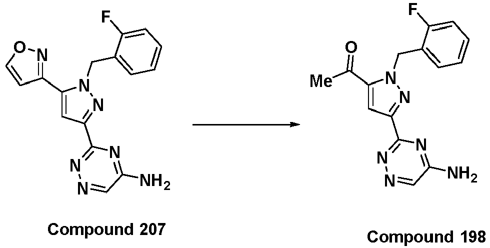

A suspension of sodium ethoxide (7.8 equiv.) and **Compound 207** (1 equiv.,) in methanol was microwaved at 150 °C for 30 min. The solvent was removed in vacuo and the crude residue was distributed between ethyl acetate and saturated aqueous ammonium chloride solution. The layers were separated, and the aqueous layer was extracted with ethyl acetate (2x), dried over magnesium sulfate, filtered, and the solvent was removed in vacuo. Purification via reverse phase HPLC (5-75% acetonitrile in water w/0.1% trifluoroacetic acid, 20 min gradient) delivered the desired compound, **Compound 198** (6 mg, 23% yield) as a tan solid. ¹H-NMR (500 MHz, MeOD) δ ppm 8.36 (s, 1 H), 7.89 (s, 1 H), 7.36-7.32 (m, 1 H), 7.16-7.09 (m, 2 H), 7.00-6.97 (s, 1 H), 6.01 (s, 2 H), 2.61 (s, 3 H).

### Reference Compound 199

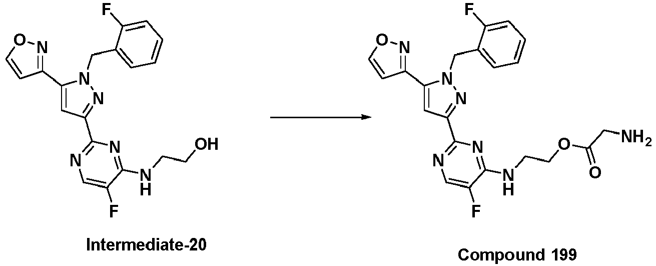

A suspension of **Intermediate-20** (1 equiv., the synthesis of this compound was described in patent application publication WO2014/144100), 4-dimethylaminopyridine (0.1 equiv.), and 2-((tert-butoxycarbonyl)amino)acetic acid (1.5 equiv.) in dichloromethane at 0 °C was treated with 1-ethyl-3-(3-dimethylaminopropyl)carbodiimide hydrochloride (1.9 equiv.). After 10 min the solution was warmed to 23 °C and then stirred for 15 h. The reaction mixture was diluted in dichloromethane and washed with aqueous 1 N hydrochloric acid solution. The organics were dried over magnesium sulfate, filtered and concentrated in vacuo. The residue was brought up in dichloromethane and cooled to 0 °C. Trifluoroacetic acid was added and the resulting solution was warmed to 23 °C over the course of 2 h. The solvent was then removed in vacuo and purification via reverse phase HPLC (5-75% acetonitrile in water w/0.1% trifluoroacetic acid, 20 minute gradient) delivered the desired compound, **Compound 199** (43 mg, 54% yield) as a white solid (in the form of the TFA salt). ¹H-NMR (500 MHz, MeOD) δ ppm 8.84 (s, 1 H), 8.31 (d, 1 H), 7.66 (s, 1 H), 7.34-7.30 (m, 1 H), 7.14-7.06 (m, 2 H), 6.98-6.95 (m, 2 H), 6.03 (s, 2 H), 4.58 (t, 2 H), 4.08 (t, 2 H), 3.90 (s, 2 H).

### Reference Compound I-200

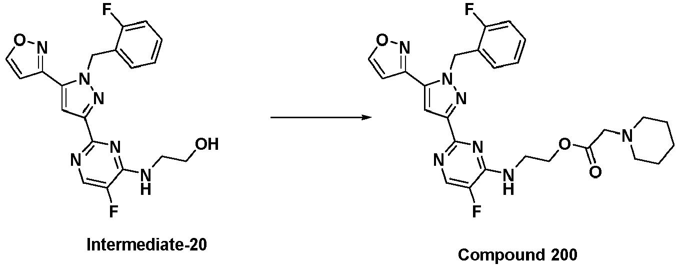

To a solution of **Intermediate-20** (1 equiv., this compound was described in a previous patent application publication WO2014/144100), 2-(piperidin-1-yl)acetic acid (1.5 equiv.), and 4-dimethylaminopyridine (0.1 equiv.) in dichloromethane (13 mL) at 0 °C was added 1-ethyl-3-(3-dimethylaminopropyl)carbodiimide hydrochloride (1.3 equiv.) in a single portion. The solution was immediately warmed to 23 °C and stirred for 15 h. The solvent was removed in vacuo and purification via reverse phase HPLC (5-75% acetonitrile in water w/0.1% trifluoroacetic acid, 20 minute gradient) delivered the desired compound, **Compound I-200** (55 mg, 69% yield) as a white solid (obtained in the form of the TFA salt). ¹H-NMR (500 MHz, MeOD) δ ppm 8.85 (s, 1 H), 8.30 (d, 1 H), 7.64 (s, 1 H), 7.35-7.31 (m, 1 H), 7.15-7.07 (m, 2 H), 7.00-6.98 (m, 2 H), 6.03 (s, 2 H), 4.59 (t, 2 H), 4.14 (s, 2 H), 4.09 (t, 2 H), 4.58 (br s, 2 H), 3.02 (br s, 2 H), 1.95-1.78 (m, 5 H), 1.52 (br s, 1 H).

### Reference Compound 1-201

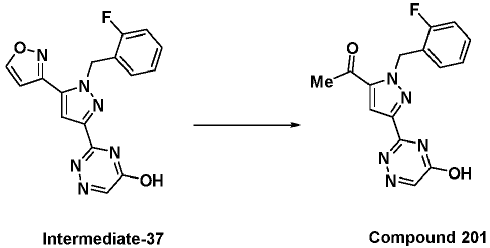

A suspension of **Intermediate-37** (1 equiv., this intermediate was previously described in patent application publication WO2014/144100) and sodium ethoxide (5 equiv.) in methanol with a drop of water was microwaved at 150 °C for 30 min. The solvent was removed in vacuo and the crude residue was brought up in saturated aqueous ammonium chloride solution and ethyl acetate (1:1 ratio). The layers were separated and the aqueous layer was extracted with ethyl acetate (2x). The organics were dried over magnesium sulfate, filtered, and the solvent was removed in vacuo. Purification via reverse phase HPLC (5-75% acetonitrile in water w/0.1% trifluoroacetic acid, 20 minute gradient) delivered the desired compound, **Compound 201**(15 mg, 20% yield) as a yellow solid. ¹H-NMR (500 MHz, MeOD) δ ppm 7.84 (s, 1 H), 7.83 (s, 1 H), 7.35-7.31 (m, 1 H), 7.15-7.08 (m, 1 H), 6.95-6.92 (m, 1 H), 5.98 (s, 2 H), 2.60 (s, 3 H).

### Reference Compound 202

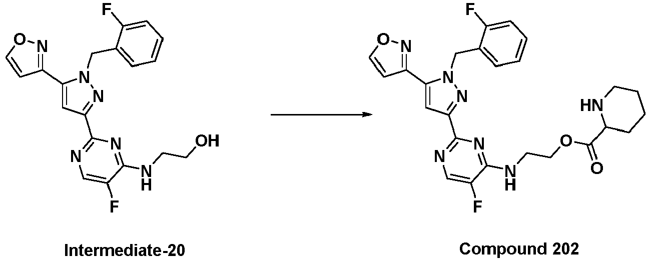

To a 0 °C suspension of **Intermediate-20** (1 equiv., described in a previous patent application WO2014/144100), 4-dimethylaminopyridine (0.1 equiv.), and 1-(tert-butoxycarbonyl)piperidine-2-carboxylic acid (1.5 equiv.) in dichloromethane was added 1-ethyl-3-(3-dimethylaminopropyl)carbodiimide hydrochloride (1.8 equiv.). The solution was immediately warmed to 23 °C and stirred for an additional 24 h. The solution was poured into dichloromethane and aqueous 1 N hydrochloric acid solution (2:1 ratio). The layers were separated and the organic layer was dried over magnesium sulfate, filtered, and the solvent was removed in vacuo. The residue was brought up in dichloromethane, cooled to 0 °C, and treated with trifluoroacetic acid (1/3 volume of DCM). The solution was stirred at 0 °C for 1 h, at which point the solvent was removed in vacuo and purification via reverse phase HPLC (5-75% acetonitrile in water w/0.1% trifluoroacetic acid, 20 minute gradient) delivered the desired compound, **Compound 202** (76 mg, 97% yield) as a white solid (in the form of the TFA salt). ¹H-NMR (500 MHz, MeOD) δ ppm 8.86 (s, 1 H), 8.35 (d, 1 H), 7.68 (s, 1 H), 7.35-7.31 (m, 1 H), 7.15-7.07 (m, 2 H), 7.02-6.99 (m, 2 H), 6.04 (s, 2 H), 4.65-4.60 (m, 1 H), 4.58-4.53 (m, 1 H), 4.16-4.03 (m, 3 H), 3.43-2.99 (m, 1 H), 3.04-2.99 (m, 1 H), 2.29-2.26 (m, 1 H), 1.87-1.81 (m, 2 H), 1.76-1.57 (m, 3 H).

### Reference Compound 204

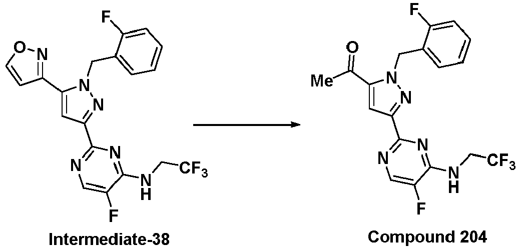

A suspension of sodium ethoxide (5.2 equiv) and **Intermediate-38** (1 equiv, described in previous patent application publication WO2014/144100.) in methanol with a drop of water was microwaved at 150 °C for 40 min. The solvent was removed in vacuo and the resulting residue was brought up in dichloromethane and water (1:1 ratio). The layers were separated and the aqueous layer was extracted with dichloromethane (2x). The organics were dried over magnesium sulfate, filtered, and the solvent was removed in vacuo. Purification via reverse phase HPLC (5-75% acetonitrile in water w/0.1% trifluoroacetic acid, 20 minute gradient) delivered the desired compound, **Compound 204** (6 mg, 7% yield) as a white film. ¹H-NMR (500 MHz, MeOD) δ ppm 8.28 (d, 1 H), 7.78 (s, 1 H), 7.32-7.28 (m, 1 H), 7.14-7.10 (m, 1 H), 7.08-7.05 (t, 1 H), 6.86-6.83 (t, 1 H), 5.94 (s, 2 H), 4.48 (q, 2 H), 2.60 (s, 3 H).

### Reference Compound 205

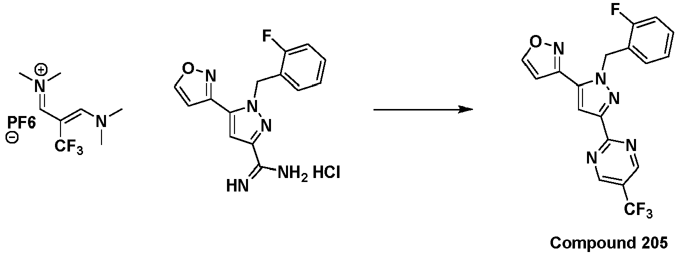

A suspension of *N*-(3-(dimethy1amino)-2-(trifluoromethy1)allylidene)-N-methylmethanaminium hexafluorophosphate (2.4 equiv.), 1-(2-fluorobenzyl)-5-(isoxazol-3-yl)-1H-pyrazole-3-carboximidamide hydrochloride (1 equiv., this intermediate was described in a previous patent application publications WO2013/101830 and WO2014/144100), and triethylamine (2.4 equiv.) in acetonitrile was stirred at 23 °C of 3 h. The reaction mixture was poured into water and dichloromethane (1:1 ratio). The layers were separated and the aqueous layer was extracted with dichloromethane (2x). The organics were dried over magnesium sulfate, filtered, and the solvent was removed in vacuo. Purification via silica gel chromatography (0-50% ethyl acetate in hexanes) delivered the desired compound, **Compound 205** (55 mg, 91% yield) as a white solid. ¹H-NMR (500 MHz, CDCl₃) δ ppm 9.08 (s, 2 H), 8.51 (s, 1 H), 7.55 (s, 1 H), 7.25-7.21 (m, 1 H), 7.08-7.04 (m, 1 H), 7.00 (t, 1 H), 6.91-6.88 (m, 1 H), 6.64 (s, 1 H), 6.06 (s, 2 H).

### Reference Compound 206

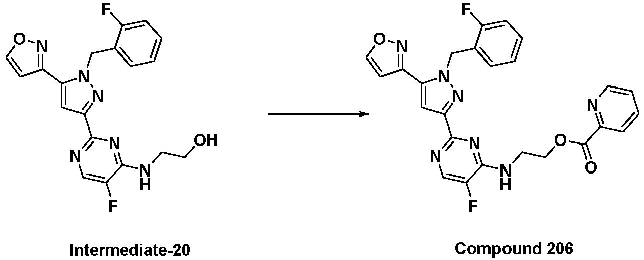

A solution of picolinic acid (1.46 equiv.), 1-ethyl-3-(3-dimethylaminopropyl)carbodiimide hydrochloride (1.3 equiv.), N,N-dimethylaminopyridine (0.1 equiv.), and **Intermediate-20** (1 equiv. described in a previous patent application publication WO2014/144100) in dichloromethane was stirred at room temperature for 20 h. The solvent was removed in vauco and purification via silica gel chromatography (0-10% methanol in dichloromethane) delivered the desired compound, **Compound 206** (59 mg, 93% yield) as a white solid. ¹H-NMR (500 MHz, CDCl₃) δ ppm 9.29 (br s, 1 H), 8.78 (br s, 1 H), 8.50 (s, 1 H), 8.37 (br s, 1 H), 8.24 (br s, 1 H), 7.49 (br s, 1 H), 7.43 (br s, 1 H), 7.24-7.21 (m, 1 H), 7.07-6.99 (m, 2 H), 6.91 (br s, 1 H), 6.66 (s, 1 H), 5.99 (s, 2 H), 4.71 (br s, 2 H), 4.16 (br s, 2 H).

### Reference Compound 217

This compound was made in

### Step 1: Synthesis of 2-(2-fluorophenyl)-N-hydroxyacetimidamide

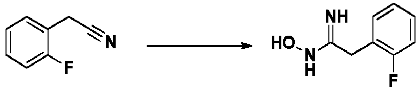

To a soution of hydroxylamine hydrochloride (2.2 equiv.) and 2-(2-fluorophenyl)acetonitrile (1 equiv.) in methanol and water (5: 1 ratio) was added sodium bicarbonate (2.4 equiv.). After stirring at 70 °C for 20 h, the methanol was removed *in vacuo* and the resulting solution was diluted with water and dichloromethane (1:2 ratio). The layers were separated and the organic layer was washed with saturated aqueous sodium chloride. The organic layer was dried over magnesium sulfate, filtered, and the solvent was removed *in vacuo* to deliver the desired intermediate, 2-(2-fluorophenyl)-N-hydroxyacetimidamide (1.13 g, 90% yield) as a white solid.

### Step 2: Synthesis of ethyl 2-(2-fluorobenzyl)-1H-imidazole-5-carboxylate

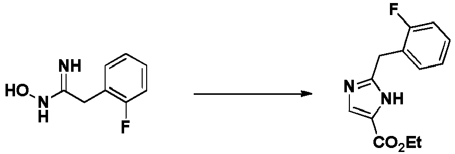

A solution of ethyl propiolate (1.08 equiv.) and 2-(2-fluorophenyl)-N-hydroxyacetimidamide (1 equiv.) in ethanol was heated to 80 °C for 5 h, at which point the solvent was removed *in vacuo.* The resulting residue was brought up in diphenyl ether and heated to 170 °C for 14 h. The black reaction mixture was poured into hexanes (4x volume of diphenyl ether) and stirred for 24 h. The hexane was then decanted to give the crude product. Purification via silica gel chromatography (0-5% methanol in dichloromethane) delivered the desired intermediate, ethyl 2-(2-fluorobenzyl)-1H-imidazole-5-carboxylate (1.8 g, 27% yield) as a black solid.

### Step 3: Synthesis of ethyl 2-(2-fluorobenzyl)-1-methyl-1H-imidazole-4-carboxylate

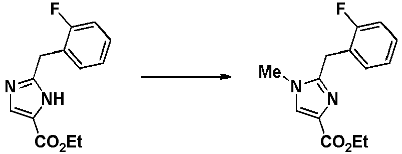

A suspension of ethyl 2-(2-fluorobenzyl)-1H-imidazole-5-carboxylate (1 equiv.) and cesium carbonate (1.2 equiv.) in acetonitrile was treated with iodomethane (1 equiv.). After stirring at room temperature for 2 h, the solvent was removed *in vacuo* and the black residue was diluted with dichloromethane and water (4:3 ratio). The layers were separated and the aqueous layer was extracted with dichloromethane (2x). The organics were dried over magnesium sulfate, filtered, and the solvent was removed *in vacuo.* Purification via silica gel chromatography (0-80% ethyl acetate in hexanes) delivered the desired intermediate, ethyl 2-(2-fluorobenzyl)-1-methyl-1H-imidazole-4-carboxylate (375 mg, 36% yield) as a dark oil.

### Step 4: Synthesis of 2-(2-fluorobenzyl)-1-methyl-1H-imidazole-4-carboximidamide hydrochloride

### (Intermediate-21)

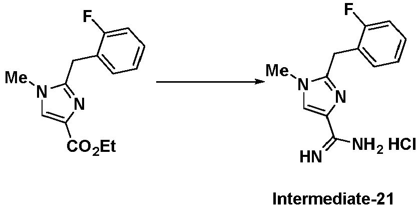

A suspension of ammonium chloride (5.5 equiv.) in toluene was treated with trimethylaluminum (5 equiv., 2N solution in toluene) over the course of 10 min. After stirring for 40 min, the solution was added to ethyl 2-(2-fluorobenzyl)-1-methyl-1H-imidazole-4-carboxylate (1 equiv.) and the resulting suspension was heated to 80 °C for 6 h. The reaction mixture was cooled to 0 °C and methanol was added dropwise. The reaction mixture was warmed to 23 °C and stirred for an additional 1 h. The resulting slurry was filtered through celite and then washed with 4:1 diethyl ether/methanol followed by 1:1 dichloromethane/methanol to deliver the desired intermediate, 2-(2-fluorobenzyl)-1-methyl-1H-imidazole-4-carboximidamide hydrochloride (240 mg, 73%) as a solid.

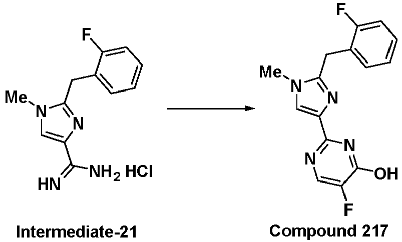

### Step 5: Synthesis of Compound 217

A suspension of 1,8-diazabicycloundec-7-ene (1 equiv.), sodium 3-ethoxy-2-fluoro-3-oxoprop-1-en-1-olate (3.06 equiv.), and 2-(2-fluorobenzyl)-1-methyl-1H-imidazole-4-carboximidamide hydrochloride (1 equiv.) in ethanol was heated to 80 °C in a sealed vial for 15 h. The solvent was removed *in vacuo,* the crude residue was suspended in methanol, the solids were filtered off, and purification of the filtrate via reverse phase HPLC (5-75% acetonitrile in water w/0.1% trifluoroacetic acid, 20 minute gradient) delivered the desired compound (29 mg, 63% yield) as a tan solid. ¹H-NMR (400 MHz, CD₃OD) δ ppm 8.17 (d, 1 H), 8.00 (s, 1 H), 7.42-7.37 (m, 1 H), 7.34-7.30 (m, 1 H), 7.24-7.16 (m, 2 H), 4.42 (s, 2 H), 3.79 (s, 3 H).

### Reference Compound 196

This compound was prepared in 5 steps:

### Step 1: Synthesis of ethyl 1-(2-fluorobenzyl)-5-mercapto-1H-pyrazole-3-carboxylate

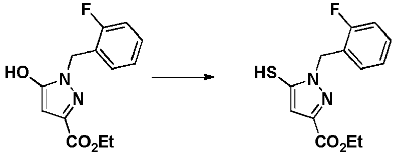

A suspension of ethyl 1-(2-fluorobenzyl)-5-hydroxy-1H-pyrazole-3-carboxylate (1.8 equiv.) and Lawesson's reagent (1 equiv.) in toluene (10 mL) was heated to 120 °C for 2 h. The solvent was removed *in vacuo* and the crude residue was brought up in dichloromethane. The resulting solid was filtered off and the filtrate was purified via silica gel chromatography (0-50% ethyl acetate in hexanes) to deliver the desired intermediate, ethyl 1-(2-fluorobenzyl)-5-mercapto-1H-pyrazole-3-carboxylate (305 mg, 55% yield) as an oil.

### Step 2: Synthesis of ethyl 1-(2-fluorobenzyl)-5-(methylthio)-1H-pyrazole-3-carboxylate

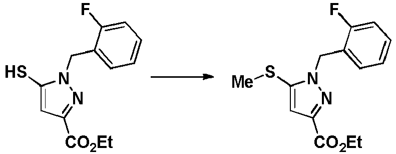

A suspension of potassium carbonate (2 equiv.) and ethyl 1-(2-fluorobenzyl)-5-mercapto-1H-pyrazole-3-carboxylate (1 equiv.) in tetrahydrofuran was treated with iodomethane (1 equiv.). After 1.5 h, the solution was diluted with ethyl acetate and water (1.5:1 ratio). The layers were separated and the aqueous layer was extracted with ethyl acetate (2x). The organics were dried over magnesium sulfate, filtered, and the solvent was removed *in vacuo.* Purification via silica gel chromatography (0-40% ethyl acetate in hexanes) delivered the desired intermediate,ethyl 1-(2-fluorobenzyl)-5-(methylthio)-1H-pyrazole--3-carboxylate (32 mg, 61% yield) as an oil.

### Step 3: ethyl 1-(2-fluorobenzyl)-5-(methylsulfonyl)-1H-pyrazole-3-carboxylate

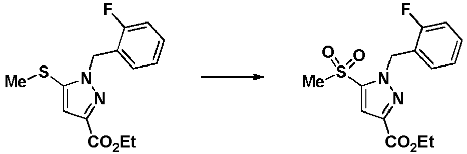

A 0 °C solution of ethyl 1-(2-fluorobenzyl)-5-(methylthio)-1H-pyrazole-3-carboxylate (1 equiv.) in dichloromethane was treated with 70% 3-chlorobenzoperoxoic acid (3 equiv.). The solution was immediately warmed to 23 °C. After 3.5 h, the reaction mixture was poured into ethyl acetate and saturated aqueous sodium bicarbonate. The layers were separated and the organic layer was washed with saturated aqueous sodium thiosulfate. The organic layer was dried over magnesium sulfate, filtered, and the solvent was removed *in vacuo.* Purification via silica gel chromatography (0-40% ethyl acetate in hexanes) delivered the desired intermediate, ethyl 1-(2-fluorobenzyl)-5-(methylsulfonyl)-1H-pyrazole-3-carboxylate (180 mg, 89% yield) as a clear oil that solidified upon standing.

### Step 4: 1-(2-fluorobenzyl)-5-(methylsulfonyl)-1H-pyrazole-3-carboximidamide hydrochloride

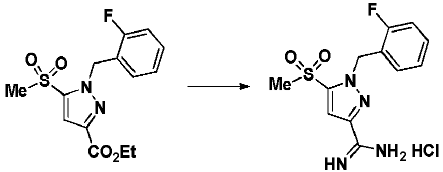

To a suspension of ammonium chloride (5.5 equiv.) in toluene was added trimethylaluminum (5.1 equiv.) as a 2M solution in toluene. After stirring for 30 min the bubbling subsided and the solution was added to ethyl 1-(2-fluorobenzyl)-5-(methylsulfonyl)-1H-pyrazole-3-carboxylate (1 equiv.). The resulting solution stirred for 14 h at 90 °C. The solution was cooled to 0 °C and methanol (5.5 equiv.) was added dropwise over the course of 3 min. The suspension was then immediately warmed to 23 °C and stirred for 1 h. After filtering the suspension through celite, the filter cake was washed with 5 mL of 50:50 methanol/dichloromethane to deliver the desired intermediate, 1-(2-fluorobenzyl)-5-(methylsulfonyl)-1H-pyrazole-3-carboximidamide (52 mg, 28% yield, HCl salt) as a white solid.

### Step 5: Synthesis of compound 196

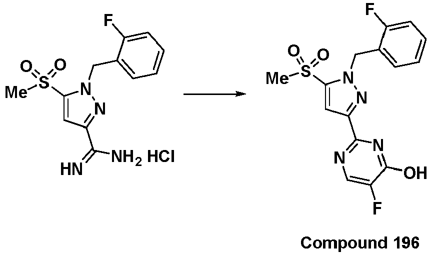

A suspension of 1-(2-fluorobenzyl)-5-(methylsulfonyl)-1H-pyrazole-3-carboximidamide hydrochloride (1 equiv.), sodium 3-ethoxy-2-fluoro-3-oxoprop-1-en-1-olate (3.1 equiv.), and 1,8-diazabicyclo[5.4.0]undec-7-ene (1 equiv.) in ethanol was heated to 90 °C for 4 h. The solvent was removed *in vacuo,* and the resulting residue was brought up in dichloromethane and the solids were filtered off. Purification of the filtrate by silica gel chromatography (0-5% methanol in dichloromethane) provided the desired compound, **Compound 196** (27 mg, 47% yield) as a white solid. ¹H-NMR (500 MHz, DMSO) δ ppm 13.42 (br s, 1 H), 8.15 (br s, 1 H), 7.60 (s, 1 H), 7.43-7.39 (m, 1 H), 7.29-7.25 (m, 1 H), 7.21-7.18 (m, 1 H), 7.14-7.12 (m, 1 H), 5.80 (s, 2 H), 3.41 (s, 3 H).

### Reference Compound 1-188

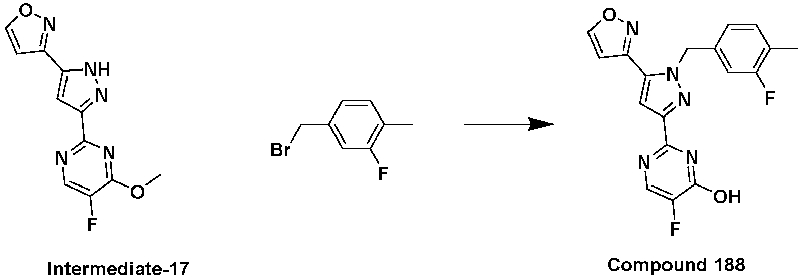

A solution of tert-butyl 3-(3-(5-fluoro-4-methoxypyrimidin-2-yl)-1H-pyrazol-5-yl)isoxazole (1 equiv.), and lithium tert-butoxide (2 equiv.) in dimethoxyethane (2 ml) was stirred at 60°C for 5 min. To it was added 4-(bromomethyl)-2-fluoro-1-methylbenzene (1.1 equiv.) and reaction stirred at 60°C overnight. After cooling to ambient temperature, solvent was removed under a stream of nitrogen. The resultant solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver **Compound 1-188** (2.2 mg, 6% yield) as a white solid. ¹H NMR (500 MHz, Methanol-*d*4) δ ppm 8.81 (m, 1 H), 8.06 (m, 1 H), 7.45 (m, 1 H), 7.17 (m, 1 H), 6.97 (m, 2 H), 6.91 (m, 1 H), 5.89 (s, 2 H), 2.22 (m, 3 H).

### Reference Compound 1-208

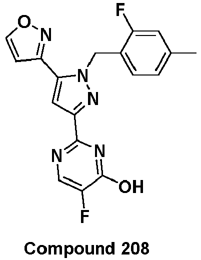

This compound was synthesized using the same procedure as for **Compound 1-188**, except 1-(bromomethyl)-2-fluoro-4-methylbenzene was used as the alkylating agent to deliver the product (7.2 mg, 30% yield) as a white solid. ¹H NMR (500 MHz, Methanol-*d*4) δ ppm 8.79 (s, 1 H), 8.04 (m, 1 H), 7.43 (s, 1 H), 6.90 (d, 4 H), 5.93 (s, 2 H), 2.30 (s, 3 H).

### Reference Compound 1-197

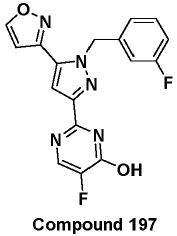

This compound was synthesized using the same procedure as for **Compound 1-188**, except 1-(bromomethyl)-3-fluorobenzene was used as the alkylating agent to give the desired product (3.5 mg, 15% yield) as a white solid. ¹H NMR (500 MHz, Methanol-*d*4) δ ppm 8.81 (d, 1 H), 8.04 (d, 1 H), 7.46 (s, 1 H), 7.32 (d, 1H), 7.09 (d, 1 H), 7.02 (m, 2 H), 6.92 (d, 1 H), 5.94 (s, 2 H)

### Reference Compound 1-213

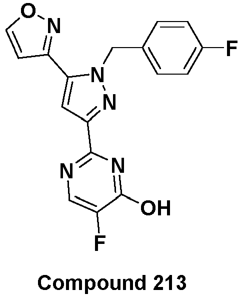

This was synthesized using the same procedure as for **Compound 1-188,** except 1-(bromomethyl)-4-fluorobenzene was used as the alkylating agent to give the product (2 mg, 8% yield) as a white solid. ¹H NMR (500 MHz, Methanol-*d*4) δ ppm 8.81 (d, 1 H), 8.03 (d, 1 H), 7.42 (s, 1 H), 7.34 (m, 2H), 7.03 (s, 2 H), 6.90 (m, 1 H), 5.90 (s, 2 H).

### Reference Compound 1-186

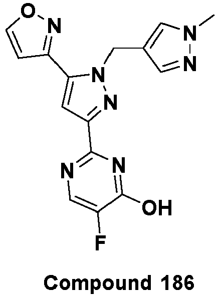

This was synthesized using the same procedure as for **Compound 1-188**, except 4-(bromomethyl)-1-methyl-1H-pyrazole was used as the alkylating agent to give the product (6.5 mg, 20% yield) as a white solid. ¹H NMR (500 MHz, DMSO-*d*6) δ ppm 13.12 (m, 1 H), 9.15 (d, 1 H), 8.12 (br. s., 1 H), 7.70 (s, 1 H), 7.52 (s, 1 H), 7.45 (s, 1 H), 7.22 (d, 1 H), 5.65 (s, 2 H), 3.75 (s, 3 H).

### Reference Compound 1-212

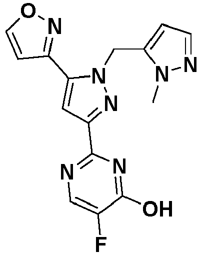

This was synthesized using the same procedure as for **Compound 1-188**, except 5-(bromomethyl)-1-methyl-1H-pyrazole was used as the alkylating agent to give the product (1.8 mg, 6 % yield) as a white solid. ¹H NMR (500 MHz, DMSO-*d*6) δ ppm 13.18 (m, 1 H), 9.15 (d, 1 H), 8.10 (m, 1 H), 7.58 (s, 1H), 7.26 (dd, 2 H), 6.05 (s, 1 H), 5.90 (s, 2 H), 3.96 (s, 3 H).

### Reference Compound 1-211

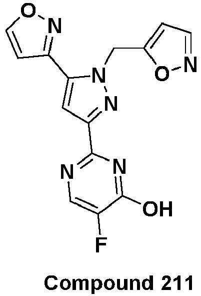

This was synthesized using the same procedure as for **Compound 1-188**, except 5-(bromomethyl)isoxazole was used as the alkylating agent to give the product (5.1 mg, 16 % yield) as a white solid. ¹H NMR (500 MHz, DMSO-*d*6) δ ppm 13.16 (m, 1 H), 9.15 (d, 1 H), 8.53 (d, 1 H), 8.06 (m, 1H), 7.61 (s, 1 H), 7.27 (d, 1 H), 6.41 (s, 1 H), 6.04 (s,2 H).

### Reference Compound 1-214

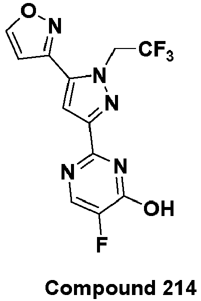

This was synthesized using the same procedure as for **Compound I-188**, except 2,2,2-trifluoroethyl trifluoromethanesulfonate was used as the alkylating agent to give the product (8.5 mg, 27 % yield) as a white solid. ¹H NMR (500 MHz, Methanol-*d*4) δ ppm 8.88 (d, 1 H), 8.07 (d, 1 H), 7.52 (s, 1 H), 7.02 (d, 1 H), 5.60 (d, 2 H).

### Reference Compound 1-216

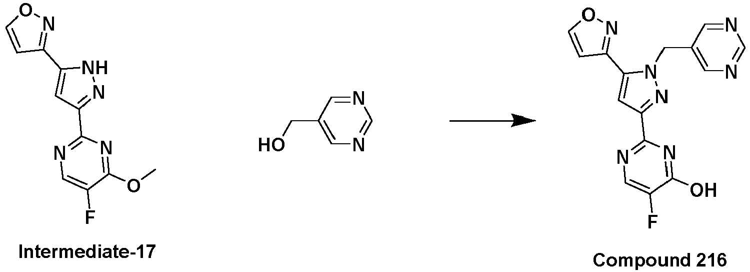

A solution of tert-butyl 3-(3-(5-fluoro-4-methoxypyrimidin-2-yl)-1H-pyrazol-5-yl)isoxazole (1 equiv.), triphenylphosphine (1.5 equiv.), and pyrimidin-5-ylmethanol (1.5 equiv.) in dichloromethane / THF 1:1 (2 ml) was cooled to 0°C. To it was added diethyl azodicarboxylate (1.5 equiv.) and reaction warmed to room temperature overnight. The reaction was concentrated under a stream of nitrogen and the methoxy intermediate purified via reverse phase HPLC utilizing a 30-80% acetonitrile water 0.1% formic acid gradient. The desired fraction was concentrated to dryness and the resulting solid was dissolved in methanol (0.5ml) and conc. aqueous HCl (140ul) and stirred overnight at 60°C. After cooling to ambient temperature, the solvent was removed in vacuo. The crude material was purified via reverse phase HPLC utilizing a 30-80% acetonitrile water 0.1% formic acid gradient to deliver the product (1.9 mg, 10% yield) as a white solid. ¹H NMR (500 MHz, Methanol-*d*4) δ ppm 9.07 (s, 1 H), 8.83 (m, 3 H), 8.02 (d, 1 H), 7.46 (s, 1 H), 6.96 (d, 1 H), 5.98 (s, 2 H).

### Compound 1-215

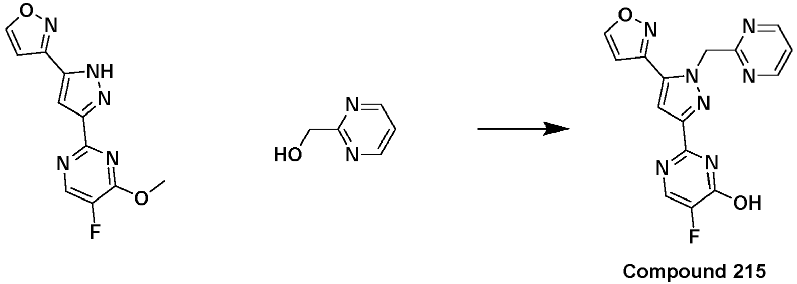

This was synthesized using the same procedure as for **Compound 1-216**, except pyrimidin-2-ylmethanol was used as the alkylating agent to give the product (1.4 mg, 4.3% yield) as a white solid. ¹H NMR (500 MHz, DMSO-*d*6) δ ppm 13.13 (m, 1 H), 9.04 (d, 1 H), 8.73 (d, 2 H), 8.07 (m, 1H), 7.65 (s, 1 H), 7.41 (t, 1 H), 7.23 (s, 1 H), 6.07 (s,2 H).

### Reference Compounds 191 and 192

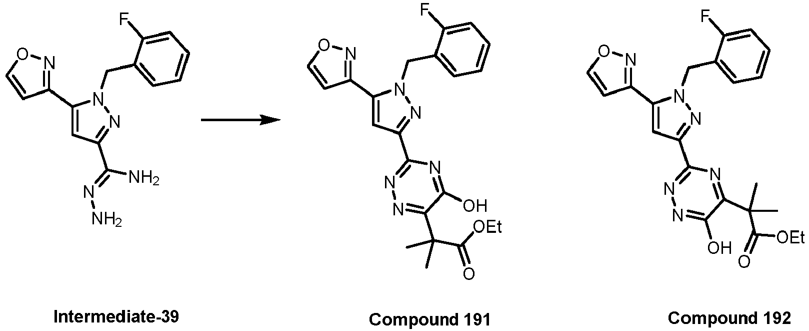

A mixture of **Intermediate-39** (2.0 g, 6.7 mmol, obtained during the synthesis of **Intermediate-28** after addition of hydrazine), diethyl 2,2-dimethyl-3-oxosuccinate (4.3 g, 20.0 mmol) and NaHCO₃ (1.7 g, 20.0 mmol) in toluene (50 mL) was heated overnight at 100°C, then cooled to room temperature and filtered through Celite. The filter cake was rinsed with ethyl acetate and the solvents removed by rotary evaporation. Purification over SiO₂ with a gradient elution of hexane/ethyl acetate gave the two regioisomeric products as solids. **Compound 191** (0.82 g, 27% yield). ¹H-NMR (400 MHz, CDCl₃) δ 10.95 (br s, 1H), 8.56 (d, 1H), 7.48 (s, 1H), 7.3-7.4 (m, 1H), 7.0-7.1 (m, 3H), 6.65 (d, 1H), 5.97 (s, 2H), 4.18 (q, 2H), 1.57 (s, 6H), 1.22 (t, 3H) ppm. **Compound 192** (0.27 g, 9% yield). ¹H-NMR (400 MHz, CDCl₃) δ 11.05 (br s, 1H), 8.52 (d, 1H), 7.28 (s, 1H), 7.2-7.3 (m, 1H), 7.0-7.1 (m, 2H), 6.94 (t, 1H), 6.63 (d, 1H), 6.00 (s, 2H), 4.21 (q, 2H), 1.66 (s, 6H), 1.22 (t, 3H) ppm.

### Reference Compound 209

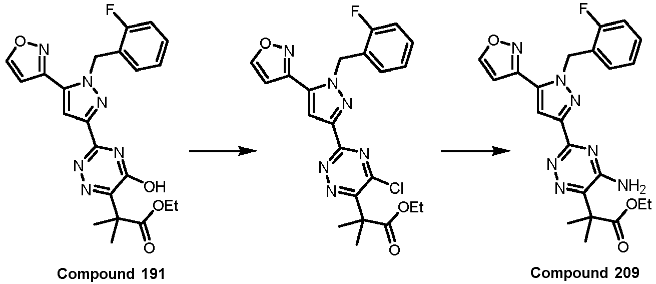

Step 1: **Compound 191** (0.13 g, 0.28 mmol) was dissolved in phosphorus oxychloride (2.0 mL, 21.5 mmol) and the solution was heated for 1 hr at 105°C. The solvent was removed under vacuum and the residue was taken up in ethyl acetate, washed with 3 x 5 mL water, brine, then dried over Na₂SO₄. After the drying agent was filtered off, the crude product was purified over SiO₂ using a gradient elution of hexane/ethyl acetate to give the chlorinated intermediate as a white solid (100 mg, 77% yield). ¹H-NMR (400 MHz, CDC13) δ 8.52 (d, 1H), 7.62 (s, 1H), 7.2-7.3 (m, 2H), 7.07 (t, 1H), 6.64 (d, 1H), 6.07 (s, 2H), 4.22 (q, 2H), 1.82 (s, 6H), 1.23 (t, 3H) ppm.
Step 2: The chlorinated intermediate prepared in step 1 (100 mg, 0.22 mmol) was heated in a sealed vial with ammonia/dioxane (0.5 M, 10 mL, 10 mmol) at 105°C for 2.5 days. The mixture was cooled to room temperature, diluted with ethyl acetate (50 mL), then washed with water (3 x 5 mL), brine and dried over Na₂SO₄. The drying agent was filtered off, the solution was concentrated under vacuum and the residue was purified over SiO₂ using a gradient of dichloromethane/ethyl acetate as eluant. The products was obtained as a white solid (19 mg, 20% yield). ¹H-NMR (400 MHz, CDCl₃) δ 8.47 (s, 1H), 7.68 (br s, 1H), 7.20 (q, 1H), 7.04 (t, 1H), 6.96 (t, 1H), 6.76 (t, 1H), 6.62 (br s, 1H), 6.07 (s, 2H), 6.02 (br s, 2H), 4.17 (q, 2H), 1.72 (s, 6H), 1.20 (t, 3H) ppm.

### Reference Compound 195

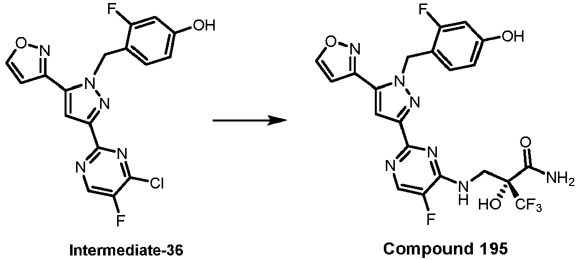

**Intermediate-36** (35 mg, 0.09 mmol), (R)-2-(aminomethyl)-3,3,3-trifluoro-2-hydroxypropanamide (60 mg, 0.35 mmol) and N-ethyl-N-isopropylpropan-2-amine (0.10 mL, 0.56 mmol) were mixed in dimethylsulfoxide (1.5 mL) and heated at 95°C for 8 hr. The solution was cooled to room temperature, diluted with water (2 mL) and the pH taken to 2-3 with 1 N (aq) HCl. The solution was mixed with ethyl acetate (50 mL) and the organic phase was washed with water (2 x 5 mL), brine, then dried over Na2SO4, filtered and concentrated by rotary evaporation. The residue was subjected to preparative reverse phase HPLC using a gradient of water/acetonitrile (0.1% trifluoroacetic acid) as eluant to give the product as a white solid (11 mg, 23% yield). ¹H-NMR (400 MHz, CD₃OD) δ 8.83 (br s, 1H), 8.27 (br s, 1H), 7.49 (br s, 1H), 6.9-7.0 (m, 2H), 6.5-6.6 (m, 2H), 5.86 (s, 2H), 4.35 (d, 1H), 4.16 (d, 1H) ppm. Note: exchangable protons all appeared under the residual HOD peak at 4.91 ppm.

### Reference Compound 194

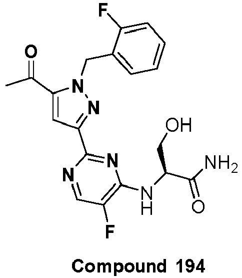

The title compound was prepared following general procedure B, from **Intermediate 1B** and using (S)-2-amino-3-hydroxypropanamide was the amine reactant, and contents were heated to 90 °C for 5 h as a solution in dioxane. The crude material was purified by reverse phase HPLC to deliver the desired compound (S)-2-((2-(5-acetyl-1-(2-fluorobenzyl)-1H-pyrazol-3-yl)-5-fluoropyrimidin-4-yl)amino)-3-hydroxypropanamide (8 mg, 0.019 mmol, 34 % yield). ¹H-NMR (500 MHz, DMSO-*d*6) δ ppm 8.29 (d, 1 H) 7.71 (s, 1 H) 7.62 (s, 1 H) 7.30 - 7.36 (m, 1H) 7.19 - 7.25 (m, 2 H) 7.11 (t, 1 H) 6.81 (t, 1 H) 5.83 (s, 2 H) 4.72 (d, 1 H) 3.77 - 3.86 (m, 2 H), 2.57 (s, 3 H).

### Example 2A: Biological activity measurement by the sGC-HEK-cGMP assay, with LC/MS detection

Human embryonic kidney cells (HEK293), endogenously expressing soluble guanylate cyclase (sGC), were used to evaluate the activity of test compounds. Compounds stimulating the sGC enzyme should cause an increase in the intracellular concentration of cGMP. HEK 293 cells were seeded in Dulbecco's Modification of Eagle's Medium supplemented with fetal bovine serum (10 % final) and penicillin (100U/mL) / streptomycin (100µg/mL) in a 50µL volume at a density of 1.5x10⁴ cells/well in a poly-D-lysine coated 384 well flat bottom plate. Cells were incubated overnight at 37°C in a humidified chamber with 5% CO₂. Medium was aspirated and cells were washed with 1x Hank's Buffered Saline Salt Solution (50µL). Cells were then incubated for 15 minutes at 37°C with 50µL of a 0.5mM 3-isobutyl-1-methylxanthine (IBMX) solution. Test article and Diethylenetriamine NONOate (DETA-NONOate) solutions (x µM concentration for test article solution and 10 µM concentration for DETA-NONOate solution; wherein x is one of the following concentrations);

| |
|---|
| 30000 nM |
| 7500 nM |
| 1875 nM |
| 468.75 nM |
| 117.19 nM |
| 29.29 nM |
| 7.32 nM |
| 1.83 nM |
| 0.46 nM |
| 0.114 nM |
| 0.029 nM |

were then added to the assay mixture and the resulting mixture incubated at 37°C for 20 minutes. After the 20 minute incubation, the assay mixture was aspirated and 10% acetic acid containing 150ng/mL + 3-cGMP (internal standard for LCMS) (50µL) was added to the cells. The plate was incubated at 4°C for 30 minutes in the acetic acid solution to stop the reaction and lyse the cells. The plates were then centrifuged at 1,000g for 3 minutes at 4°C and the supernatant transferred to a clean reaction plate for LCMS analysis.

cGMP concentrations were determined from each sample using the LCMS conditions below (**Table 2**) and calculated standard curve. The standard curve was prepared in 10% acetic acid with 150ng/mL +3cGMP (isotopically labelled cGMP with a weight 3 units higher than wild type) with the following final concentrations of cGMP in ng/mL: 1, 5, 10, 50, 100, 250, 500, 1000, 2000.

**Table 2: LC/MS conditions, Example 2**

| | | | | | | | | |
|---|---|---|---|---|---|---|---|---|
| **MS:** | Thermo Vantage | | | | | | | |
| **Ion Mode:** | ESI⁺ | | | | | | | |
| **Scan Type:** | MRM | | | | | | | |

| **Compound:** | **Transition** | **Dwell Time (msec)** | **Collision Energy (V)** | | **S Lens** | | **Retention Time (min)** | |
|---|---|---|---|---|---|---|---|---|
| cGMP | 346 > 152 | 100 | 32 | | 75 | | 0.6 | |
| (+3) cGMP IS | 349 > 155 | 100 | 32 | | 75 | | 0.6 | |
| **HPLC:** | Waters Acquity UPLC | | | | | | | |
| **Column:** | Thermo Hypersil Gold 2.1 x 50 mm 1.9 micron particle size | | | | | | | |
| **Flow Rate:** | 750 uL/min | | | | | | | |
| **Column Temperature:** | RT | | | | | | | |
| **Autosampler Temperature:** | 6 °C | | | | | | | |
| **Injection Volume:** | 20 uL | | | | | | | |
| **Mobile Phases:** | A = 100% Water + 0.1% Formic Acid | | | | | | | |
| | B = 100% Acetonitrile + 0.1% Formic Acid | | | | | | | |

| **Gradient:** | **Time (min)** | | **% A** | **% B** | | | | |
|---|---|---|---|---|---|---|---|---|
| | 0 | | 100 | 0 | | | | |
| | 0.2 | | 100 | 0 | | | | |
| | 0.3 | | 50 | 50 | | | | |
| | 0.7 | | 50 | 50 | | | | |
| | 0.8 | | 100 | 0 | | | | |

Data were normalized to a high control using the following equation: 100^{∗}Sample - Low Control)/(High Control - Low Control), where the low control is the average of 16 samples treated with 1% DMSO, and the high control is the average of 16 samples treated with 30µM of **Compound Y depicted below.** Data were fit using a 4-parameter fit (log(agonist) vs. response - variable slope) using GraphPad Prism Software v.5. n=2 for all compounds. The Absolute EC₅₀ was interpolated from the curve fit and is defined as the concentration at which a given compound elicits 50% of the high control response. Compounds failing to elicit a minimum response of 50% are reported as >30µM. For compounds run in duplicate or n higher than 2, the result herein given is the geometric mean of the several results obtained. Table 3 summarizes results obtained for selected compounds of the invention in this assay.

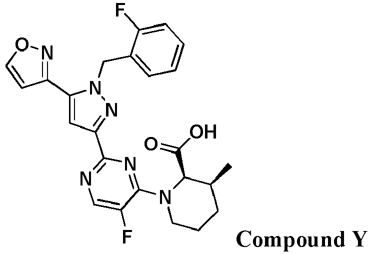

**Table 3A. Whole cell activity in the HEK assay with LC/MS detection (updated assay conditions, Example 2A).**

| **Compound** | **Absolute EC50 (nM) - binned (^{∼})** | **Compound** | **Absolute EC50 (nM)** - **binned (^{∼})** |
|---|---|---|---|
| 1 | C | 51 | B |
| | | 52 | A |
| 3 | ND | | |
| 4 | C | 54 | A |
| 5 | C | 55 | B |
| 6 | C | 56 | C |
| 7 | ND | 57 | ND |
| 8 | B | | |
| 9 | C | 59 | B |
| 10 | C | 60 | A |
| 11 | C | 61 | C |
| 12 | ND | 62 | C |
| 13 | C | 64 | B |
| 14 | B | 65 | A |
| 15 | A | 66 | C |
| 16 | B | 67 | B |
| 17 | ND | 68 | B |
| 19 | C | 69 | A |
| 20 | ND | 70 | B |
| 21 | C | 71 | B |
| 22 | C | 72 | B |
| | | 73 | C |
| 24 | B | 74 | B |
| 25 | NA | 75 | B |
| 26 | B | 76 | B |
| 27 | C | 77 | C |
| 28 | A | 78 | C |
| 29 | A | 79 | B |
| 30 | B | 80 | B |
| | | 81 | C |
| 32 | B | 82 | |
| 33 | C | 83 | C |
| 34 | B | 84 | C |
| 35 | C | 85 | B |
| 36 | B | 86 | C |
| 37 | ND | 87 | B |
| 38 | B | 88 | B |
| 39 | C | 89 | B |
| 40 | C | 90 | A |
| 41 | C | 91 | B |
| 42 | B | 92 | C |
| 43 | C | 93 | B |
| 44 | A | 94 | C |
| 45 | C | 95 | B |
| 46 | C | 96 | B |
| 47 | B | 97 | C |
| 48 | C | 98 | C |
| 49 | C | 99 | B |
| 50 | C | | |
| | | 106 | A |
| 102 | B | 107 | A |
| 103 | B | 108 | B |
| | | 109 | B |
| 105 | ND | | |
| 179 | B | 150 | A |
| 112 | B | 151 | A |
| 113 | B | 152 | A |
| 114 | C | 131 | B |
| 115 | C | 194 | C |
| 116 | B | 127 | A |
| 117 | B | 125 | C |
| 180 | B | 153 | B |
| 181 | B | 126 | ND |
| 118 | C | 154 | B |
| 119 | C | 193 | C |
| 120 | ND | 155 | B |
| 121 | C | 195 | C |
| 122 | C | 156 | B |
| 135 | B | 196 | ND |
| 136 | B | 199 | B |
| 139 | B | 200 | C |
| 140 | C | 202 | B |
| 137 | B | 157 | C |
| 138 | B | 158 | C |
| 123 | B | 206 | A |
| 124 | C | 162 | B |
| 129 | C | 163 | A |
| 182 | C | 164 | A |
| 183 | C | 165 | B |
| 184 | C | 159 | ND |
| 147 | C | 160 | B |
| 148 | A | 161 | B |
| 149 | A | 166 | C |
| 130 | B | | |
| 195 | C | | |
| 194 | C | | |

| | | | |
|---|---|---|---|
| (∼) Code definitions for the sGC enzyme activity values, expressed as Absolute EC₅₀ which is defined as the concentration at which a given compound elicits 50% of the high control response (Compound Y). Compounds failing to elicit a minimum response of 50% are reported as >30µM or ND. EC50Abs ≤ 100 nM = A; 100 nM <EC50Abs ≤ 1000 nM = B; 1000 nM < EC50Abs = C. | | | |

### Example 2B: Biological activity measurement by the cGMP GloSensor cell-based assay, 384-well format

Human embryonic kidney cells (HEK293) cells expressing GloSensor™ 40F cGMP (Part No: CS182801, Promega) were used to evaluate the activity of test compounds. The luminescent biosensors (engineered luciferase) that were incorporated into these cells detect cGMP formed by the compounds stimulating the sGC enzyme and emit luminescence.

cGMP GloSensor cells were maintained in Dulbecco's Modification of Eagle's Medium (DMEM) supplemented with fetal bovine serum (FBS) (10 % final) and hygromycine (200ug/ml). The day before assay, cells were plated in DMEM with 10% FBS in a 50µL volume at a density of 1.5x10⁴ cells/well in a poly-D-lysine coated 384-well flat white-bottom plate (Corning Cat No 35661). Cells were incubated overnight at 37°C in a humidified chamber with 5% CO₂. The next day, medium was removed and cells were replaced with 40ul/well of GloSensor™, 2mM (Promega Cat No E1291). Cells were treated for 90 minutes at 25°C to allow the substrate to equilibrate in the cells. Test compounds and Diethylenetriamine NONOate (DETA-NONOate) was diulted to 3mM (20x) in serum-free CO₂ independent medium and serally diluted at 4x dilutions to create 5X dose curve from which 10 ul was added to the wells (x µM concentration for test compound solution and 10 µM concentration for DETA-NONOate solution; wherein x is one of the following final concentrations).
30000 nM
7500 nM
1785 nM
468.75 nM
117.19 nM
29.29 nM
7.32 nM
1.83 nM
0.46 nM
0.114 nM
0.029 nM

For the kinetics studies, luminescense was measured right away for 0.2 sec per well with Envision (Perkin Elmer model No). For endpoint SAR screening, data were collected after 55 min incubation at room temperature.

Data analysis was carried out as indicated above in Example 2A.

**Table 3B. Whole cell activity in the GloSensor cell-based assay, 384-well format (Example 2B).**

| **Compound** | **sGC_HEK_GloSensor EC50/IC50 Abs (Norm) (nM) Binned** (∼) |
|---|---|
| 128 | C |
| 111 | A |
| 141 | B |
| 142 | C |
| 198 | C |
| 201 | C |
| 143 | C |
| 144 | C |
| 145 | C |
| 204 | B |
| 205 | ND |
| 146 | B |
| 167 | A |
| 168 | A |
| 169 | A |
| 170 | A |
| 132 | A |
| 133 | A |
| 134 | B |
| 171 | A |
| 172 | A |
| 173 | A |
| 174 | A |
| 175 | A |
| 176 | A |
| 177 | A |
| 178 | A |
| 191 | B |
| 192 | C |
| 185 | A |
| 190 | B |
| 187 | ND |
| 189 | C |
| 212 | C |
| 211 | C |
| 215 | C |
| 186 | C |
| 214 | C |
| 216 | C |
| 213 | B |
| 197 | B |
| 208 | B |
| 209 | C |
| 188 | A |
| 192 | C |
| 191 | B |

| | |
|---|---|
| (∼) Code definitions for the sGC enzyme activity values, expressed as Absolute EC₅₀ which is defined as the concentration at which a given compound elicits 50% of the high control response (**Compound Y**). Compounds failing to elicit a minimum response of 50% are reported as >30µM or ND. EC50Abs ≤ 100 nM = A; 100 nM <EC50Abs ≤ 1000 nM = B; 1000 nM < EC50Abs = C. | |

### Example 3A: Biological activity measurement by the thoracic aortic rings assay

Thoracic aortic rings are dissected from anesthetized (isoflurane) male Sprague-Dawley rats weighing 275-299g. Tissues are immediately transferred to ice-cold Krebs-Henseleit solution, which has been aerated with 95% O₂ and 5% CO₂ for 30 minutes. Following removal of connective tissue, aortic sections are cut into 4 rings (∼2 mm each) and suspended on 2 L-shaped hooks, with one hook fixed at the bottom of the tissue bath (Schuler Organ Bath, Harvard Apparatus) and the other connected to a force transducer (F30 Force Transducer, Harvard Apparatus). Baths containing Krebs Henseleit solution (10 mL) are heated to 37 °C and aerated with 95% O₂ and 5% CO₂. Rings are brought to an initial tension of 0.3-0.5 g and gradually raised to a resting tension of 1.0 g over 60 minutes. Rings are rinsed with Krebs Henseleit solution (heated to 37°C and aerated with 95% O2 and 5% CO2) at 15 minute intervals until a stable baseline is obtained. Rings are considered to be stable after a resting tension of 1.0 g is maintained (for approximately 10 minutes) without need for adjustment. Rings are then contracted with 100 ng/mL phenylephrine by adding 100 uL of a 10µg/mL phenylephrine stock solution. Tissues achieving a stable contraction are then treated in a cumulative, dose dependent manner with test compounds prepared in dimethylsulfoxide (DMSO). In some cases, tissues are rinsed three times over a 5 minute period with Krebs-Heinseleit's solution (heated to 37°C and aerated with 95% O2 and 5% CO2), allowed to stabilize at baseline, and then used for characterization of other test articles or DMSO effects. All data are collected using the HSE-ACAD software provided by Harvard Apparatus. Percent relaxation effects are calculated in Microsoft Excel using the recorded tension value of 100ng/mL phenylephrine treatment as 0% inhibition and treatment with 100 µM 3-isobutyl-1-methylxanthine as 100% inhibition. EC50 values are calculated from concentration-response curves generated with GraphPad Prism Software.

### Example 3B: Biological activity measurement by the thoracic aortic rings assay, alternative method

As an alternative thoracic aortic rings assay, the procedure of Example 3 is used except that percent relaxation effects are calculated in Microsoft Excel using the recorded tension value of 100ng/mL phenylephrine treatment as 0% inhibition and, after washing the tissue with buffer, the original resting tesnsion of the tissue is used as 100% inhibition.

### Example 4: Blood pressure change in Sprague-Dawley rats

Male rats (250-350g body weight, supplied by Harlan Laboratories) were anesthetized with ketamine/xylazine and a heparinized saline fluid filled catheter implanted into the right femoral artery. The catheter was exteriorized between the scapula, capped, and the animal allowed to recover for at least 7 days post surgery prior to any compound testing. Prior to testing animals were maintained on normal diet, with free access to drinking water, under a 12 hour light-dark cycle.

On the day of experimentation, under inhaled isoflurane anesthesia, the catheter was uncapped and connected to a tether (Instech Labs) and pressure transducer (Harvard Apparatus). Blood pressure and heart rate were subsequently captured and analyzed with a dedicated data capture system (PowerLab, ADInstruments). Data sampling rates were set at 1 cycle per second. Once connected, each rat was allowed to recover from anesthesia and baseline blood pressure and heart rate levels were established in these conscious, freely-moving animals. Once baseline was established either vehicle (0.5% methylcellulose or 100% PEG400) or test article was administered orally (PO, 10 mg/kg) and the effects on blood pressure and heart rate monitored for up to 24 hours.

Data are reported as hourly averages and changes in blood pressure are calculated from subtracting individual baseline on an hourly basis **(Table 4)**

**Table 4**

| **Compound Number** | **Dose in mpk** | **Rat Mean Arterial Pressure peak change from baseline (at 3 or 10 mpk@)** |
|---|---|---|
| **109** | 10 | B |

| | | |
|---|---|---|
| @Code definitions for Rat Mean Arterial Pressure peak change from baseline at 10 mpk: A = -10 < peak change from baseline at 3 or 10 mpk < 0 B = -20 ≤ peak change from baseline at 3 or 10 mpk ≤ -10 C = peak change from baseline at 3 or 10 mpk < -20 | | |

### Example 5: Purified human recombinant sGC α1β1 enzyme assay performed in the presence of Diethylenetriamine NONOate (DETA-NONOate), a nitric oxide donor.

Purified human recombinant soluble guanylate cyclase enzyme α1β1(h sGC) obtained from Enzo Life Sciences (P/N: ALX-201-177) was used to evaluate the activity of test compounds. The assay reactions contained 0.1 M Tris (pH 8.0), 0.5 mg/mL BSA, 2 mM DTT, 4 mM MgCl₂, 30uM DETA NONOate (Enzo Life Science P/N: ALX-430-014), and 12.5 ng/ml human soluble guanylate cyclase enzyme. Test compounds in DMSO were then added (in a 3-fold titration of compound over a 10-point curve starting at 30uM final concentration, all samples had a 3% DMSO final concentration). Guanosine 5'-triphosphate (Sigma-Aldrich P/N: G8877) was added to a final concentration of 300 µM and enzyme reactions were incubated (100 µL, 384-well plate format) at 37°C for 20 minutes. The controls contained 3% DMSO (low control), or 30uM of **Compound Y** (high control). After the 20 minute incubation, the reaction was stopped with the addition of 100 µL of ice cold 20% acetic acid.

cGMP concentrations in all samples were determined using the cGMP HTRF (Cisbio P/N: 62GM2PEC) assay per manufacturer's instructions. A cGMP standard curve was fit using a 4-parameter fit (log(inhibitor) vs. response - variable slope) using GraphPad Prism Software v.6. Samples were diluted appropriately to ensure that values fell within the linear range of the standard curve.

Data were fit using a 4-parameter fit (log(agonist) vs. response - variable slope) using GraphPad Prism Software v.6. The EC₅₀ was interpolated from the curve fit and is defined as the concentration at which the compound elicits 50% of the maximal response of the 30uM of **Compound Y,** the high control compound.

**Table 5. Enzyme data**

| **compound number** | **sGC_Enz_HTRF_a1b1 EC50/IC50 Abs (Norm) (nM) Binned** | **compound number** | **sGC_Enz_HTRF_a1b1 EC50/IC50 Abs (Norm) (nM) Binned** |
|---|---|---|---|
| 3 | C | 39 | C |
| 4 | C | 44 | B |
| 11 | C | 45 | C |
| 17 | C | 50 | C |
| 27 | C | 56 | C |
| 32 | B | 62 | C |
| 37 | C | 78 | C |
| 38 | C | 83 | C |
| 86 | C | 108 | B |

| | | | |
|---|---|---|---|
| EC50Abs < 100 nM = A ≤ 100 nM < EC50Abs <1000 nM = B ≤ 1000nM = C | | | |

### Example 6: Animal Models descriptions:

### Lamb model of pulmonary hemodynamics using inhaled sGC stimulator

("Inhaled Agonists of Soluble Guanylate Cyclase Induce Selective Pulmonary Vasodilation", Oleg V. et al, American J of Resp and Critical Care Medicine, Vol 176, 2007, p 1138)

It is possible to test whether inhalation of novel dry-powder microparticle formulations containing sGC stimulators would produce selective pulmonary vasodilation in lambs with acute pulmonary hypertension by following a published procedure. It is also possible to evaluate the combined administration of the microparticles of sGC stimulator and inhaled nitric oxide (iNO) in this system. Finally, it is possible to examine whether inhaling microparticles of an sGC stimulator would produce pulmonary vasodilation when the response to iNO (inducible nitric oxide synthase) is impaired.

Protocol: In awake, spontaneously breathing lambs instrumented with vascular catheters and a tracheostomy tube, U-46619 is infused intravenously to increase mean pulmonary arterial pressure to 35 mm Hg. Inhalation of microparticles composed of either BAY 41-2272, BAY 41-8543, or BAY 58-2667 and excipients (dipalmitoylphosphatidylcholine, albumin, lactose) produced dose dependent pulmonary vasodilation and increased transpulmonary cGMP release without significant effect on mean arterial pressure. Inhalation of microparticles containing BAY 41-8543 or BAY 58-2667 increased systemic arterial oxygenation. The magnitude and duration of pulmonary vasodilation induced by iNO were augmented after inhaling BAY 41-8543 microparticles. Intravenous administration of 1H-[1,2,4]oxadiazolo[4,3-a]quinoxalin-1-one (ODQ), which oxidizes the prosthetic heme group of sGC, markedly reduced the pulmonary vasodilator effect of iNO. In contrast, pulmonary vasodilation and transpulmonary cGMP release induced by inhaling BAY 58-2667 microparticles were greatly enhanced after treatment with ODQ. Thus, inhalation of microparticles containing agonists of sGC may provide an effective novel treatment for patients with pulmonary hypertension, particularly when responsiveness to iNO is impaired by oxidation of sGC. Note: BAY 41-2272, BAY 41-8543 are sGC stimulators whereas BAY 58-2667 is an sGC activator.

### Electrical Field Stimulated Guinea Pig Tracheal Smooth Muscle In Vitro (ex vivo) model for the assessment of bronchodilation.

It is possible to assess the bronchodilating effects of sGC stimulators by using the system described below. This system allows us to determine potency, efficacy and duration of action of several sGC stimulators, as well as to assess potential side effects such as blood pressure, or heart rate changes.

Animals: Guinea pig, Dunkin Hartley, male, Full barrier-bred and certified free of specific micro-organisms on receipt 525-609g on the experimental day, Harlan UK Ltd. Guinea pigs are housed in a group of 4 in solid-bottomed cages with Gold Flake bedding in a controlled environment (airflow, temperature and humidity). Food (FD1, Special Diet Services) and water are provided ad libitum.

### Guinea Pig Tracheal Smooth Muscle Contraction in Response to EFS. Assessment of Compound Potency and Efficacy:

On each experimental day, a guinea pig is killed by exposure to a rising concentration of CO2 and the trachea removed. The trachea is cleaned of extraneous tissue and cut open longitudinally in a line opposite the muscle, opened out and cut into strips 2 -3 cartilage rings wide. A cotton loop is attached to one end of each tracheal strip and a length of cotton to the other end. Tracheal strips are then suspended between two platinum electrodes, using tissue holders, in a Myobath system (World Precision Instruments Stevenage, UK). The loop is attached over the hook at the bottom of the tissue holder and the other end attached to the arm of a FORT10 force transducer (World Precision Instruments Stevenage, UK) ensuring that the tissue is positioned between the two platinum electrodes. The whole assembly is then lowered into a 10ml tissue bath containing modified Kreb's-Henseleit buffer, at 37 °C, bubbled with Carbogen. A 1 g tension is applied to each piece of tissue and the tissue washed, followed by a 1 hour stabilization period. Once the tissues has been allowed to stabilize, the apparatus for electrical field stimulation is set to deliver a stimulation of frequency 80Hz pulse width 0.1 ms, with a gated, uni-polar pulse, every 2 minutes using a DS8000 8 channel digital stimulator (World Precision Instruments Stevenage, UK). A voltage response curve is carried out on each tracheal strip at 2, 4, 6, 7, 8, 10, 12 V and a sub-maximal voltage then selected to apply to each tissue during the remainder of the experiment. Guinea pig tracheal smooth muscle (GPTSM) contraction is induced using sub-maximal Electrical Field Stimulation (EFS) (It is also possible to induce contraction by using a spasmogen substance, such as methacholine or histamine as described in Coleman et al.*). Compounds are dissolved in 100% DMSO at 3x10-2M and aliquots stored at -200 C. A separate aliquot is used for each experiment. Tissues are washed with Kreb's buffer and stimulated using the previously determined sub-maximal voltage for 1 hour to establish a stable baseline contraction prior to assessment of compound activity.

A cumulative dose response curve (DRC) to each test substance is then performed and changes in smooth muscle contraction measured. The effect of each test substance in each experiment is expressed as a percentage inhibition of the baseline contraction, normalized to the relevant vehicle controls. The experiment is performed three times, using tissue from three different animals. The data from all three experiments are pooled, the DRC plotted, and the test substance potency and efficacy determined. The potency of Ipratropium bromide is assessed alongside the test compounds and the IC50 determined to be 0.86nM (95% Cl, 0.78-0.94), in agreement with data previously produced in the system.

Novel and Versatile Superfusion System. Its use in the Evaluation of Some Spasmogenic and Spasmolytic Agents Using Guinea pig isolated Tracheal Smooth Muscle.", R. A. Coleman et al., J. Pharmacol. Methods, 21, 71-86, 1989.

### Mouse model for diseases in which altered CFTR-function is causally involved

These diseases comprise cystic fibrosis, pancreatic disorders, gastrointestinal disorders, liver disorders, cystic fibrosis-related diabetes (CFRO), dry eye, dry mouth and Sjoegren's syndrome.

By using transgenic mice expressing or not expressing the delta F508CFTR channel it is possible to measure differences on nasal potential difference and salivation in the presence of a test sGC stimulator by using the literature protocol described below (see WO2011095534).

### Salivary Secretion Assay in delta(.6.)50S-CFTR mice

15 Male and female homozygous, heterozygous .6.50S-CFTR (backcrossed on the FVB genetic background for more than 12 generations, originally obtained from Erasmus University, Rotterdam; 10-14 weeks old and weighing 1S-36g of both sexes were used in this assay. Solutions of Vardenafil in concentrations of 0.07,0.14 and 0.42 mg/kg BW were 20 prepared in sterile saline, whereas the sGC stimulator BAY 41-2272 was dissolved to 0.01, 0.03, 0.1 and 0.3 mg/kg BW in a solvent containing 50% ddH20, 40% PEG 400 (polyethylene glycol 400) and 10% ethanol. The substances or the appropriate vehicles were administered to mice via intraperitoneal injection (5 ml/kg BW) 60 min prior to the salivary secretion assay. After 60 min, mice were anaesthetized with a combination of 25 ketamine and diazepam. The solution was prepared to contain 1 ml of 5 mg/ml diazepam. and 1 ml of 100 mg/ml ketamine in 8 ml sterile saline. Anaesthesia was induced by intraperitoneal injection of the solution (10 ml/kg BW). After anaesthesia, mice were pretreated with a subcutaneous injection of 1 mM atropine (50 1-11) into the left cheek in order to avoid a cross-stimulation of cholinergic receptors. Small strips of Whatman filter 5 paper were placed inside the previously injected cheek for 4 min to absorb any saliva secreted after the injection of atropine. This first piece of filter paper was removed and replaced with a second pre-weighed filter paper. Thereafter, 50 1-11 of a solution containing 100 I-IM isoprenaline and 1 mM atropine was injected into the left cheek at the same site to induce the salivary secretion by adrenergic mechanisms. The time of the 10 isoprenaline injection was taken as time zero, and filter paper stripes were replaced every 10 minutes for a total collection period of 30 minutes. Each piece of filter paper was immediately placed and sealed in a pre-weighed vial. After all samples had been collected, each vial was re-measured and the weights of all samples were recorded. The difference in total weight of vial plus paper measured before and after collecting saliva 15 was taken as the net weight of saliva secreted during the collection period. The total amounts of salivary secretion were calculated as the weight of saliva divided by the number of minutes required for each collection and then normalized to the mass of the mouse in grams. Results are expressed as the mean percentage increase of n mice compared to placebo treatment. Statistics was analyzed by one way ANOVA test 20 followed by post-hoc Bonferoni analysis; */**/*** means statistical significant with p values <0.05/<0.01/0.001 and n.s. means non significant.

These animal studies were carried out with a number of sGC stimulators, sGC activators and PDE5 inhibitors. The results suggests that compounds of the invention are useful for the treatment of cystic fibrosis, pancreatic disorders, gastrointestinal disorders, liver disorders, Cystic Fibrosis-related diabetes (CFRO), dry eye, dry mouth and Sjoegren's syndrome.

### Neuromuscular disorders

It has previously been shown that neuronal Nitric Oxide Synthase (nNOS) mislocalization from the sarcolemmal membrane to the sarcoplasm is observed in a broad range of nondystrophic neuromuscular conditions associated with impaired motility status and catabolic stress. One tool for the evaluation of muscle biopsies of patients with a variety of inherited and acquired forms of neuromuscular disorders is the assessment of sarcolemal localization of nNOS. It was found that the level of nNOS at the sarcolemma correlates with mobility and functional status.

An analogous assessment can be used to determine nNOS localization in animal models of nondystrophic myopathy following the literature protocols described below ("Loss of sarcolemmal nNOS is common in acquired and inherited neuromuscular disorders"; E.L. Finanger Hedderick et al., Neurology, 2011, 76(11), 960-967).

### nNOS mislocalization in mouse models of acquired muscle atrophy

Two mouse models have been described that demonstrate muscle atrophy without compromised mobility: high-dose corticosteroids therapy and short-term starvation. Mice treated with steroids or starved for 48 hours showed significant decreases in overall body mass and in normalized wet skeletal muscle mass. Morphometric analysis of skeletal muscle specimens of both models demonstrated muscle atrophy, as defined by a significant decrease in mean minimal Feret fiber diameter as compared to age-matched controls (n = 5 for each group). Immunofluorescence staining for dystrophin, α-sarcoglycan, and α-1-syntrophin showed normal dystrophin localization suggestive of an intact DGC complex However, both steroid-treated and starved mice showed absent or severely reduced sarcolemmal nNOS staining. Real-time PCR for NOS family proteins (nNOS, eNOS, iNOS) revealed no significant differences in expression levels of any of the 3 transcripts in steroid-treated mice (n = 8 for each group). Moreover, Western blot analysis for nNOS, iNOS, and eNOS showed no differences in protein levels.

These murine animal models could be used to assess the effects of sGC stimulators (for example an sGC stimulator of the invention) in the symptoms of muscle atrophy and related disease states.

Starved mice exhibited a 1-fold decrease of nNOS and iNOS transcript expression as compared to wildtype mice (n = 9 for controls, n = 7 for starved). However, the protein level of nNOS, iNOS, and eNOS revealed no differences between control and starved mice (n = 4 for each group). These data demonstrate that abnormal localization of nNOS occurs in mice with severe muscle atrophy even if overall mobility is preserved, supporting the notion that, in addition to impaired mobility, other triggers such as catabolic stress may be associated with sarcolemmal loss of nNOS.

### Skeletal muscle nNOS localization is maintained during hibernation (studies with squirrels)

Skeletal muscle specimens from hibernating 13-lined ground squirrels have been used to evaluate the impact of immobility and catabolic stress on nNOS localization in the context of maintained muscle homeostasis and integrity. These animals are obligate hibernating mammals that are protected against skeletal muscle atrophy during hibernation. Despite hibernating for 5 months with almost complete immobility and no caloric intake, sarcolemmal expression of nNOS is preserved. These data together with patient and mouse data indicate that biochemical control of nNOS localization is complex and, importantly, that preserved sarcolemmal nNOS may be significant in maintaining muscle homeostasis.

These results also suggest that targeting aberrant NO signaling (for instance with sGC stimulators such as the ones here described) may prove beneficial for a broad group of patients with neuromuscular disorders.

### Mouse models of Muscular Dystrophy (BMD and DMD)

Becker muscular dystrophy (BMD), characterized by progressive skeletal muscle wasting, is caused by mutations of the muscle protein dystrophin. In a human study, Martin et al. (see "Tadalafil Alleviates Muscle Ischemia in Patients with Becker Muscular Dystrophy"; Elizabeth A. Martin et al., Sci. Transl. Med. 4, 162ra155 (2012); "Vascular-targetted therapies for Duchenne muscular dystrophy"; Ennen et al., Skeletal Muscle, 2013, 3:9) assessed exercise-induced attenuation of reflex sympathetic vasoconstriction in the muscles of 10 patients with BMD and 7-age matched healthy male controls. This is a protective mechanism that optimizes perfusion of skeletal muscle to meet the metabolic demands of exercise. Reflex vasoconstriction was induced by simulated orthostatic stress and was measured as the forearm muscles were rested or lightly exercised in the form of rhythmic handgrip. First, the investigators showed that exercise-induced attenuation of reflex vasoconstriction was defective in 9 out of 10 patients with BMD in whom the common dystrophin mutations disrupt targeting of neuronal NO synthase (nNOS) to the muscle sarcolemma. Then, in a double-blind randomized placebo-controlled crossover trial, the authors showed that normal blood flow regulation was restored in eight of nine patients by a single oral dose of 20 mg of tadalafil, a specific PDE5 inhibitor.

It is possible to assess the effects of drugs acting on the NO pathway by using a dystrophin-deficient *mdx* mouse model of related disease Duchene muscular dystrophy (DMD). This model has also shown that inhibitors of phosphodiesterase 5 (PDE5) alleviate some features of the dystrophic phenotype including vasospasm of skeletal muscle microvessels that can lead to muscle injury and fatigue.

With exercise of healthy skeletal muscle, sarcolemmal nNOS derived NO attenuates local α-adrenergic vasoconstriction, thereby optimizing perfusion to meet the metabolic demands of the active muscle. This protective mechanism (termed functional sympatholysis) is lost in *mdx* mice (a model of BMD and DMD), nNOS null mice, and boys with DMD causing functional muscle ischemia. Repeated bouts of functional ischemia could accelerate use-dependent injury of muscle fibers already weakened by dystrophin deficiency.

In the *mdx* mouse, many features of the dystrophic phenotype can be improved by multiple strategies that boost NO signaling, including transgenic expression of nNOS, transgenic expression of dystrophin minigenes that restore sarcolemmal nNOS (and thereby restore functional sympatholysis), administration of the NOS substrate L-arginine (24, 25), treatment with NO-donating drugs, and phosphodiesterase 5A (PDE5A) inhibition with the drug tadalafil or sildenafil. These PDE5A inhibitors, which prolong the halflife of guanosine 3',5'-monophosphate (cGMP)-the downstream target of NO in vascular smooth muscle-were shown in the *mdx* mouse to alleviate muscle ischemia, as well as injury and fatigue, after a brief bout of exercise. Also, these drugs were shown to improve cardiac dynamics in *mdx* mice and to rescue dystrophic skeletal muscle and prolong survival in dystrophin-deficient zebrafish.

These findings support an essential role for sarcolemmal nNOS in modulating sympathetic vasoconstriction in exercising human skeletal muscles and suggests that targeting the aberrant NO pathway (for instance by using an sGC stimulator of the invention) may be a useful therapeutic approach for treating BMD and DMD in humans.

### Sickle Cell Disease

Sickle-cell disease (SCD), or sickle-cell anaemia (SCA) or drepanocytosis, is a hereditary blood disorder, characterized by red blood cells that assume an abnormal, rigid, sickle shape. Sickling decreases the cells' flexibility and results in a risk of various complications. The sickling occurs because of a mutation in the haemoglobin gene. Individuals with one copy of the defunct gene display both normal and abnormal haemoglobin. This is an example of codominance. In 1994, in the US, the average life expectancy of persons with this condition was estimated to be 42 years in males and 48 years in females, but today, thanks to better management of the disease, patients can live into their 70s or beyond.

Sickle-cell anaemia is a form of sickle-cell disease in which there is homozygosity for the mutation that causes HbS. Sickle-cell anaemia is also referred to as "HbSS", "SS disease", "haemoglobin S" or permutations of those names. In heterozygous people, that is, those who have only one sickle gene and one normal adult haemoglobin gene, the condition is referred to as "HbAS" or "sickle cell trait". Other, rarer forms of sickle-cell disease are compound heterozygous states in which the person has only one copy of the mutation that causes HbS and one copy of another abnormal haemoglobin allele. They include sickle-haemoglobin C disease (HbSC), sickle beta-plus-thalassaemia (HbS/β⁺) and sickle beta-zero-thalassaemia (HbS/β⁰).

Although red blood cell (RBC) sickling and rheologic abnormalities are central to the pathophysiology of sickle cell disease, vascular dysfunction resulting from complex interactions between sickled red blood cells (sRBC), endothelial cells, platelets and leukocytes play an equally important role. In sickle cell disease, endothelial activation is associated with sickle cell-mediated hypoxia-reperfusion events (see for example "Advances in understanding of the pathogenesis of cerebrovascular vasculopathy in sicke cell anemia", P. Connes et al., Br. J. Haematol. 2013, 161, 484-98). Red blood cell sickling and adhesion to endothelium initiate vaso-occlusion by impairing blood flow. The subsequent surge of inflammatory mediators and endothelial activation trigger a cascade of events leading to vascular damage. Pathophysiological responses to intermittent hypoxia-reperfusion from these vaso-occlusive events are demonstrated by an increased production of cytokines, leukocyte up-regulation and activation of pro-coagulant and adhesion molecules, with simultaneous inhibition of cytoprotective mediators.

Leukocytosis is correlated with nearly every manifestation of sickle cell disease, emphasizing the influential role of inflammation in the pathophysiology of sickle vasculopathy. Even at baseline, sickle cell disease patients exhibit elevations in pro-inflammatory cytokines, including C-reactive protein (CRP), tumor necrosis factor (TNF), interleukin-1 (IL-1) and interleukin-8 (IL-8). *In vitro* studies have shown that sRBC promote endothelial up-regulation of TNF-α and IL-1-β (8-10). Microarray studies of activated monocytes have shown differential expression of genes involved in inflammation, heme metabolism, cell cycle regulation, anti-oxidant responses, and angiogenesis. More recently, it was shown that differential expression of nuclear factor κ-light-chain-enhancer of activated B cells (NFκB/p65), Kruppel-like factor 2 (KLF2), and other transcription factors that regulate pathways of inflammation in sickle cell disease children at increased risk for stroke.

In transgenic mouse models (see "Novel Therapies Targetting the Endothelium in sickle cell disaease", C.C Hoppe, Hemoglobin, 35(5-6):530-546 (2011) and references cited therein), sickling inducing oxidative stress has been shown to affect microvascular regulatory mechanisms leading to endothelial activation and exaggerated inflammatory and pro-adhesive responses. Oxidative stress occurs through formation of reactive oxygen species (ROS). Depletion of NO occurs through hemoglobin (Hb) mediated scavenging, consumption by ROS and arginase-mediated substrate depletion. In sickle cell disease, the scavenger systems that normally remove circulating free Hb are saturated. Free Hb depletes NO, leading to endothelial dysfunction. Consequently, the normal balance of vasoconstriction and vasodilation is skewed towards vasoconstriction, endothelial activation, oxidative stress and proliferative vasculopathy.

Therapies directed at restoring NO homeostasis have shown promise in preliminary studies in patients with sickle cell disease. Previous *in vitro* studies and studies in other patient populations showed NO-mediated down-regulation of endothelial adhesion molecule expression. Following these observations, the use of inhaled NO was studied in sickle cell disease children presenting with VOE and found associated trends toward lower pain scores, decreased analgesic requirements and a shorter hospital stay.

These findings were reproduced in a recent randomized placebo controlled trial evaluating inhaled NO for the treatment of acute VOE in adult patients with sickle cell disease, showing that inhaled NO significantly reduced pain scores and was associated with a trend towards decreased use of parenteral morphine compared with placebos. Results from a completed phase II trial of adult sickle cell disease patients treated with inhaled NO for acute VOE have not yet been made available (clinicaltrials. gov NCT00023296). Another phase II trial of inhaled NO for VOE treatment in children with sickle cell disease is expected to be completed (clinicaltrials.gov NCT00094887). The possible therapeutic role of inhaled NO for ACS in sickle cell disease is currently being assessed in both children and adults in two separate French phase II/III trials comparing the use of inhaled NO to placebo or standard care in children with ACS (clinicaltrials.gov NCT01089439 and NCT00748423).

Dietary supplementation of the NO synthase substrate, L-arginine, has been studied extensively in sickle cell disease as a means of increase NO bioavailability. In sickle mice, oral L-arginine at high doses has been shown to decrease Gardos channel activity, dense cell formation and hemolysis, as well as functional improvements in vascular reactivity.

Sildenafil, an agent aimed at amplifying the effect of endogenous NO by inhibiting PDE5, a downstream mediator of NO, is used widely in the general population to treat primary PHT. Preliminary studies in sickle cell disease patients with severe PHT reported improvements in PAP and exercise capacity after treatment with sildenafil. A multicenter trial (Treatment of Pulmonary Hypertension and Sickle Cell Disease with Sildenafil Therapy, Walk-PHaSST) testing the safety and efficacy of sildenafil in sickle cell disease patients with Doppler-defined PHT was stopped prematurely due to a higher frequency of serious side effects, including increased rates of VOE, headache, and visual disturbance in the treatment group.

Nitrite and niacin have also been investigated for their direct NO donor properties. In a pilot phase I/II clinical trial, sodium nitrite infusions in adult sickle cell disease patients enhanced forearm blood flow, consistent with a NO donor mechanism of action. A larger phase I/II trial is now investigating whether nitrite infusions administered as adjunctive therapy during acute VOE will improve microvascular blood flow and tissue oxygenation (clinicaltrials.gov NCT01033227). The effect of niacin on improvement in endothelial-dependent vasodilation is also being assessed in a phase II randomized, controlled trial (clinicaltrials.gov NCT 00508989).

The above results suggest that targeting the aberrant NO pathway in sicke cell disease (for instance by using an sGC stimulator of the invention) may be a useful therapy for the treatment of the disease. Murine models of sickle cell anemia that could be used to assess the effect of sGC stimulators (e.g., an sGC stimulator of the invention) in this disease state, are described in Blood, 2001, 98(5), 1577-84; J. Clin. Invest. 2004, 114(8), 1136-45; and Br. J. Haematol., 2004, 124(3), 391-402.

### Bladder dysfunction

It has been shown that the sGC activator BAY 60-2770 ameliorates overactive bladder in obese mice (see "The Soluble Guanylyl Cyclase Activator BAY 60-2770 ameliorates overactive bladder in obese mice", Luiz O Leiria et al., The Journal of Urology, 2013, doi:10.1016/j.juro.2013.09.020.). The animal model described in this publication can analogously be used to assess the effect of an sGC stimulator (for example, an sGC stimulator of the invention) on overactive bladder.

The same group of researchers have also described a rat model of bladder dysfunction (NO-defficient rats, F Z Monica et al., Neurology and Urodynamics, 30, 456-60, 2011) and have shown the protective effects of BAY-2272 (an sGC activator) in this model. The animal model described in this publication can analogously be used to assess the effect of an sGC stimulator (for example, an sGC stimulator of the invention) on bladder dysfunction related to detrusor smooth muscle overactivity.

A number of embodiments have been described. Nevertheless, it will be understood that various modifications may be made without departing from the spirit and scope of the invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has" and "having"), "include" (and any form of include, such as "includes" and "including"), "contain" (and any form contain, such as "contains" and "containing"), and any other grammatical variant thereof, are open-ended linking verbs. As a result, a method or device that "comprises", "has", "includes" or "contains" one or more steps or elements possesses those one or more steps or elements, but is not limited to possessing only those one or more steps or elements. Likewise, a step of a method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

As used herein, the terms "comprising," "has," "including," "containing," and other grammatical variants thereof encompass the terms "consisting of' and "consisting essentially of."

The phrase "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Subject matter incorporated by reference is not considered to be an alternative to any claim limitations, unless otherwise explicitly indicated.

Where one or more ranges are referred to throughout this specification, each range is intended to be a shorthand format for presenting information, where the range is understood to encompass each discrete point within the range as if the same were fully set forth herein.

While several aspects and embodiments of the present invention have been described and depicted herein, alternative aspects and embodiments may be affected by those skilled in the art to accomplish the same objectives. Accordingly, this disclosure and the appended claims are intended to cover all such further and alternative aspects and embodiments as fall within the scope of the invention defined by the appended claims.

## Claims

1. A compound selected from those depicted in Tables A, B, C, and D or a pharmaceutically acceptable salt thereof:

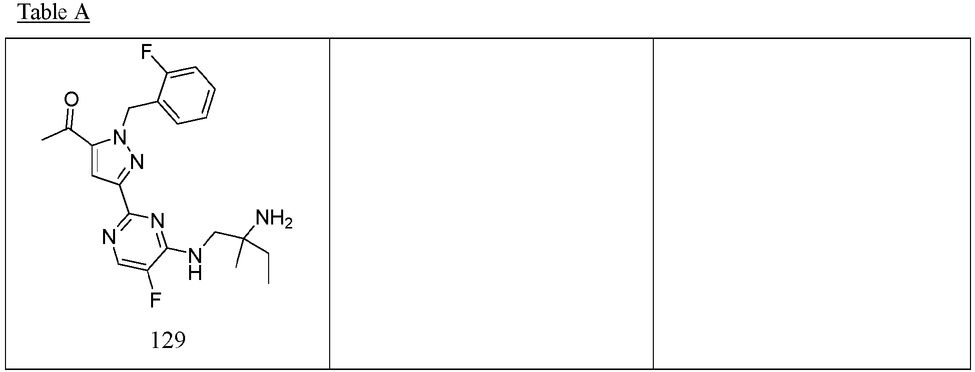

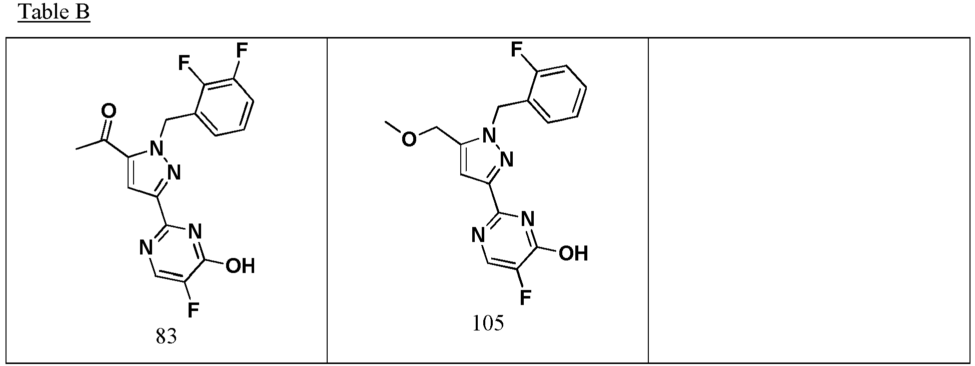

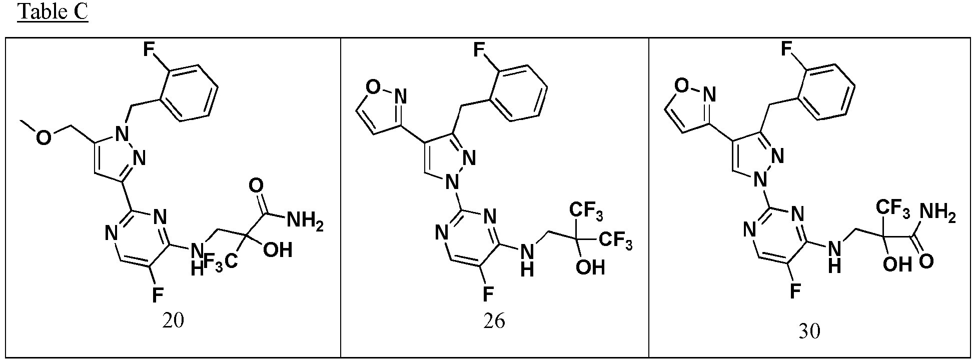

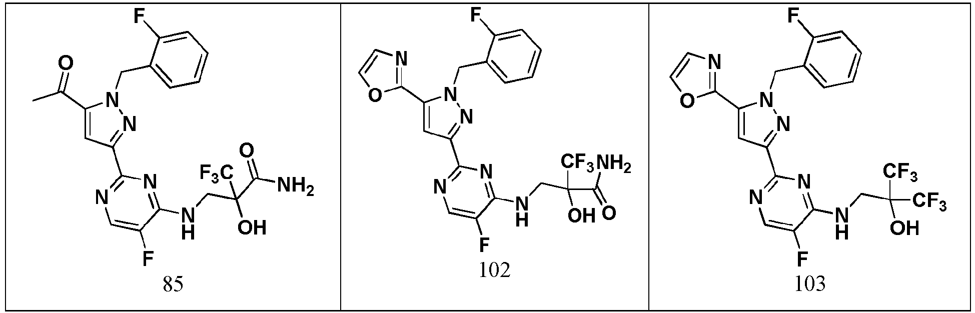

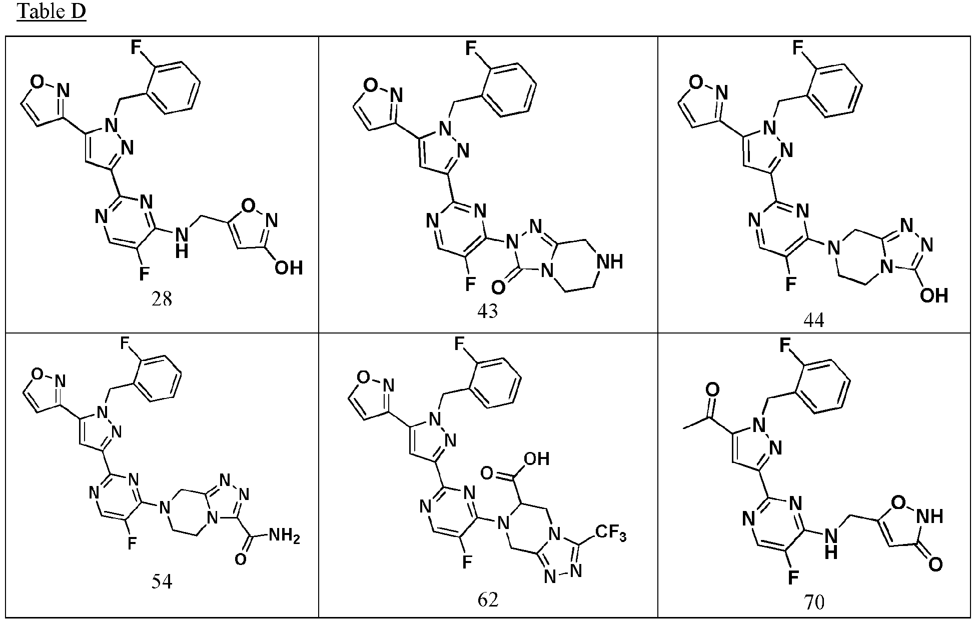

2. A pharmaceutical composition comprising a compound of claim 1, or a pharmaceutically acceptable salt thereof, and one or more excipients.

3. The compound of claim 1, or a pharmaceutically acceptable salt thereof, or a pharmaceutical composition of claim 2, for use in treating a disease, health condition or disorder, wherein the disease, health condition or disorder is selected from:
• disorders related to high blood pressure and decreased coronary blood flow; increased acute and chronic coronary blood pressure; arterial hypertension; vascular disorder resulting from cardiac and renal complications; vascular disorders resulting from heart disease, stroke, cerebral ischemia or renal failure; resistant hypertension; diabetic hypertension; essential hypertension; secondary hypertension; gestational hypertension; pre-eclampsia; portal hypertension; myocardial infarction;
• heart failure, HFPEF, HFREF; acute and chronic HF; more specific forms of HF: acute decompensated HF, right ventricular failure, left ventricular failure, total HF, ischemic cardiomyopathy, dilatated cardiomyopathy, congenital heart defects, HF with valvular defects, mitral valve stenosis, mitral valve insufficiency, aortic valve stenosis, aortic valve insufficiency, tricuspid stenosis, tricuspic insufficiency, pulmonary valve stenosis, pulmonary valve insufficiency, combined valvular defects; diabetic heart failure; alcoholic cardiomyopathy or storage cardiomyopathies; diastolic HF, systolic HF; acute phases of an existing chronic HF (worsening HF); diastolic or systolic dysfunction; coronary insufficiency; arrhythmias; reduction of ventricular preload; cardiac hypertrophy; heart failure/cardiorenal syndrome; portal hypertension; endothelial dysfunction or injury; disturbances of atrial and ventricular rhythm and conduction disturbances: atrioventricular blocks of degree I-III (AVB I-III), supraventricular tachyarrhythmia, atrial fibrillation, atrial flutter, ventricular fibrillation, ventricular flutter, ventricular tachyarrhythmia, torsade-de-pointes tachycardia, atrial and ventricular extrasystoles, AV-junction extrasystoles, sick-sinus syndrome, syncopes, AV-node reentry tachycardia; Wolff-Parkinson-White syndrome or acute coronary syndrome; Boxer cardiomyopathy; premature ventricular contraction; cardiomyopathy; cancer-induced cardiomyopathy;
• thromboembolic disorders and ischemias; myocardial ischemia; infarction; myocardial infarction; heart attack; myocardial insufficiency; endothelial dysfunction; stroke; transient ischemic attacks (TIAs); obstructive thromboanginitis; stable or unstable angina pectoris; coronary spasms or spasms of the peripheral arteries; variant angina; Prinzmetal's angina; cardiac hypertrophy; preeclampsia; thrombogenic disorders; ischemia-reperfusion damage; ischemia-reperfusion associated with organ transplant; ischemia-reperfusion associated with lung transplant, pulmonary transplant, cardiac transplant, venous graft failure; conserving blood substituents in trauma patients;
• peripheral vascular disease; peripheral arterial disease; peripheral occlusive arterial disease; hypertonia; Raynaud's syndrome or phenomenon (primary and secondary); Raynaud's disease; critical limb ischemia; peripheral embolism; intermittent claudication; vaso-occlusive crisis; muscular dystrophy, Duchenne muscular dystrophy, Becker muscular dystrophy; microcirculation abnormalities; control of vascular leakage or permeability; lumbar spinal canal stenosis; occlusive thrombotic vasculitis; thrombotic vasculitis; peripheral perfusion disturbances; arterial and venous thrombosis; microalbuminuria; peripheral and autonomic neuropathies; diabetic microangiopathies;
• edema; renal edema due to heart failure;
• Alzheimer's disease; Parkinson's disease; vascular dementias; vascular cognitive impairment; cerebral vasospasm; congenital myasthenic syndrome; subarachnoid hemorrhage; traumatic brain injury; improving perception, capacity for concentration, capacity for learning or memory performance after cognitive disturbances such as those ocurring in mild cognitive impairment, age-related learning and memory disturbances, age-related memory loss, vascular dementia, head injury, stroke, post-stroke dementia, post-traumatic head injury, general disturbances of concentration and disturbances of concentration in children with learning and memory problems; Lewy body dementia; dementia with frontal lobe degeneration including Pick's syndrome; progressive nuclear palsy; dementia with corticobasal degeneration; Amyotrophic Lateral Sclerosis (ALS); Huntington's disease; demyelination; Multiple Sclerosis; thalamic degeneration; Creutzfeldt-Jakob dementia; HIV-dementia; schizophrenia with dementia or Korsakoff psychosis; Multiple System Atrophy and other forms of Parkinsonism Plus; movement disorders; neuroprotection; anxiety, tension and depression or post-traumatic stress disorder (PTSD); bipolar disorder; schizophrenia; CNS-related sexual dysfunction and sleep disturbances; pathological eating disorders and use of luxury foods and addictive drugs; controlling cerebral perfusion; migraines; prophylaxis and control of consequences of cerebral infarction (apoplexia cerebri); prophylaxis and control of consequences of stroke, cerebral ischemias and head injury;
• shock; cardiogenic shock; sepsis; septic shock; anaphylactic shock; aneurysm; control of leukocyte activation; inhibition or modulation of platelet aggregation; multiple organ dysfunction syndrome (MODS); multiple organ failure (MOF);
• pulmonary/respiratory conditions: pulmonary hypertension (PH); pulmonary arterial hypertension (PAH), and associated pulmonary vascular remodeling; vascular remodeling in the form of localized thrombosis and right heart hypertrophy; pulmonary hypertonia; primary pulmonary hypertension; secondary pulmonary hypertension; familial pulmonary hypertension; sporadic pulmonary hypertension; pre-capillary pulmonary hypertension; idiopathic pulmonary hypertension; other forms of PH; PH associated with left ventricular disease, HIV, SCD, thromboembolism (CTEPH), sarcoidosis, COPD, pulmonary fibrosis, acute respiratory distress syndrome (ARDS), acute lung injury, alpha-1-antitrypsin deficiency (AATD), pulmonary emphysema, smoking-induced emphysema and cystic fibrosis (CF); thrombotic pulmonary arteriopathy; plexogenic pulmonary arteriopathy; cystic fibrosis; bronchoconstriction or pulmonary bronchoconstriction; acute respiratory distress syndrome; lung fibrosis, lung transplant; asthmatic diseases;
• pulmonary hypertension associated with or related to: left ventricular dysfunction, hypoxemia, WHO groups I, II, III, IV and V hypertensions, mitral valve disease, constrictive pericarditis, aortic stenosis, cardiomyopathy, mediastinal fibrosis, pulmonary fibrosis, anomalous pulmonary venous drainage, pulmonary veno-occlusive disease, pulmonary vasculitis, collagen vascular disease, congenital heart disease, pulmonary venous hypertension, interstitial lung disease, sleep-disordered breathing, sleep apnea, alveolar hypoventilation disorders, chronic exposure to high altitude, neonatal lung disease, alveolar-capillary dysplasia, sickle cell disease, other coagulation disorders, chronic thromboembolism, pulmonary embolism; pulmonary embolism due to tumor, parasites or foreign material; connective tissue disease, lupus, lupus nephritis, schistosomiasis, sarcoidosis, chronic obstructive pulmonary disease, asthma, emphysema, chronic bronchitis, pulmonary capillary hemangiomatosis, histiocytosis X, lymphangiomatosis, compressed pulmonary vessels; compressed pulmonary vessels due to adenopathy, tumor or fibrosing mediastinitis;
• arterosclerotic diseases or conditions: atherosclerosis; atherosclerosis associated with endothelial injury, platelet and monocyte adhesion and aggregation, smooth muscle proliferation or migration; restenosis; restenosis developed after thrombolysis therapies, percutaneous transluminal angioplasties (PTAs), transluminal coronary angioplasties (PTCAs), heart transplant, bypass operations or inflammatory processes;
• micro and macrovascular damage (vasculitis); increased levels of fibrinogen and low density DLD; increased concentration of plasminogen activator inhibitor 1 (PA-1);
• metabolic syndrome; metabolic diseases or diseases associated with metabolic syndrome: obesity; excessive subcutaneous fat; excessive adiposity; diabetes; high blood pressure; lipid related disorders, hyperlipidemias, dyslipidemia, hypercholesterolemias, decreased high-density lipoprotein cholesterol (HDL-cholesterol), moderately elevated low-density lipoprotein cholesterol (LDL-cholesterol) levels, hypertriglyceridemias, hyperglyceridemia, hypolipoproteinanemias, sitosterolemia, fatty liver disease, hepatitis; preeclampsia; polycystic kidney disease progression; liver steatosis or abnormal lipid accumulation in the liver; steatosis of the heart, kidneys or muscle; alphabetalipoproteinemia; sitosterolemia; xanthomatosis; Tangier disease; hyperammonemia and related dieases; hepatic encephalopaties; other toxic encephalopaties; Reye syndrome;
• sexual, gynecological and urological disorders of conditions: erectile dysfunction; impotence; premature ejaculation; female sexual dysfunction; female sexual arousal dysfunction; hypoactive sexual arousal disorder; vaginal atrophy; dyspaneuria; atrophic vaginitis; benign prostatic hyperplasia (BPH), prostatic hypertrophy, prostatic enlargement; bladder outlet obstruction; bladder pain syndrome (BPS); interstitial cystitis (IC); overactive bladder; neurogenic bladder and incontinence; diabetic nephropathy; primary and secondary dysmenhorrea; lower urinary tract syndromes (LUTS); endometriosis; pelvic pains; benign and malignant diseases of the organs of the male and female urogenital system;
• chronic kidney disease; acute and chronic renal insufficiency; acute and chronic renal failure; lupus nephritis; underlying or related kidney diseases: hypoperfusion, intradialytic hypotension, obstructive uropathy, glomerulopathies, glomerulonephritis, acute glomerulonephritis, glomerulosclerosis, tubulointerstitial diseases, nephropathic diseases, primary and congenital kidney diseases, nephritis; diseases **characterized by** abnormally reduced creatinine and or water excretion; diseases **characterized by** abnormally increased blood concentrations of urea, nitrogen, potassium and/or creatinine; diseases **characterized by** altered activity of renal enzymes, diseases **characterized by** alterened activity of glutamyl synthetase; diseases **characterized by** altered urine osmolarity or urine volume; diseases **characterized by** increased microalbuminuria, diseases **characterized by** macroalbuminuria; diseases **characterized by** lesions of glomeruli and arterioles, tubular dilatation, hyperphosphatemia and/or need for dialysis; sequelae of renal insufficiency; renal-insufficiency related pulmonary enema; renal-insufficiency related to HF; renal insufficiency related to uremia or anemia; elecrolyte disturbances (herkalemia, hyponatremia); disturbances of bone and carbohydrate metabolism;
• ocular diseases or disorders such as glaucoma, retinopathy and diabetic retinopathy.

4. The compound or composition for use of claim 3, wherein the disease, health condition or disorder is selected from disorders related to high blood pressure and decreased coronary blood flow; increased acute coronary blood pressure; increased chronic coronary blood pressure; arterial hypertension; vascular disorder resulting from cardiac and renal complications; vascular disorder resulting from heart disease, stroke, cerebral ischemia, or renal failure; resistant hypertension; diabetic hypertension; essential hypertension; secondary hypertension; gestational hypertension; pre-eclampsia; portal hypertension; and myocardial infarction.

5. The compound or composition for use of claim 3, wherein the disease, health condition or disorder is selected from heart failure, HFPEF, HFREF; acute and chronic HF; more specific forms of HF: acute decompensated HF, right ventricular failure, left ventricular failure, total HF, ischemic cardiomyopathy, dilatated cardiomyopathy, congenital heart defects, HF with valvular defects, mitral valve stenosis, mitral valve insufficiency, aortic valve stenosis, aortic valve insufficiency, tricuspid stenosis, tricuspic insufficiency, pulmonary valve stenosis, pulmonary valve insufficiency, combined valvular defects; diabetic heart failure; alcoholic cardiomyopathy or storage cardiomyopathies; diastolic HF, systolic HF; acute phases of an existing chronic HF (worsening HF); diastolic or systolic dysfunction; coronary insufficiency; arrhythmias; reduction of ventricular preload; cardiac hypertrophy; heart failure/cardiorenal syndrome; portal hypertension; endothelial dysfunction or injury; disturbances of atrial and ventricular rhythm and conduction disturbances: atrioventricular blocks of degree I-III (AVB I-III), supraventricular tachyarrhythmia, atrial fibrillation, atrial flutter, ventricular fibrillation, ventricular flutter, ventricular tachyarrhythmia, torsade-de-pointes tachycardia, atrial and ventricular extrasystoles, AV-junction extrasystoles, sick-sinus syndrome, syncopes, AV-node reentry tachycardia; Wolff-Parkinson-White syndrome or acute coronary syndrome; Boxer cardiomyopathy; premature ventricular contraction cardiomyopathy; cancer-induced cardiomyopathy.

6. The compound or composition for use of claim 3, wherein the disease, health condition or disorder is selected from thromboembolic disorders and ischemias; myocardial ischemia; infarction; myocardial infarction; heart attack; myocardial insufficiency; endothelial dysfunction; stroke; transient ischemic attacks (TIAs); obstructive thromboanginitis; stable or unstable angina pectoris; coronary spasms or spasms of the peripheral arteries; variant angina; Prinzmetal's angina; cardiac hypertrophy; preeclampsia; thrombogenic disorders; ischemia-reperfusion damage; ischemia-reperfusion associated with organ transplant; ischemia-reperfusion associated with lung transplant, pulmonary transplant, cardiac transplant, venus graft failure; conserving blood substituents in trauma patients.

7. The compound or composition for use of claim 3, wherein the disease, health condition or disorder is selected from peripheral vascular disease; peripheral arterial disease; peripheral occlusive arterial disease; hypertonia; Raynaud's syndrome or phenomenon (primary and secondary); Raynaud's disease; critical limb ischemia; peripheral embolism; intermittent claudication; vaso-occlusive crisis; muscular dystrophy, Duchenne muscular dystrophy, Becker muscular dystrophy; microcirculation abnormalities; control of vascular leakage or permeability; lumbar spinal canal stenosis; occlusive thrombotic vasculitis; thrombotic vasculitis; peripheral perfusion disturbances; arterial and venous thrombosis; microalbuminuria; peripheral and autonomic neuropathies; diabetic microangiopathies.

8. The compound or composition for use of claim 3, wherein the disease, health condition or disorder is selected from edema and renal edema due to heart failure.

9. The compound or composition for use of claim 3, wherein the disease, health condition or disorder is selected from Alzheimer's disease; Parkinson's disease; vascular dementias; vascular cognitive impairment; cerebral vasospasm; congenital myasthenic syndrome; subarachnoid hemorrhage; traumatic brain injury; improving perception, capacity for concentration, capacity for learning or memory performance after cognitive disturbances such as those ocurring in mild cognitive impairment, age-related learning and memory disturbances, age-related memory loss, vascular dementia, head injury, stroke, post-stroke dementia, post-traumatic head injury, general disturbances of concentration and disturbances of concentration in children with learning and memory problems; Lewy body dementia; dementia with frontal lobe degeneration including Pick's syndrome; progressive nuclear palsy; dementia with corticobasal degeneration; Amyotrophic Lateral Sclerosis (ALS); Huntington's disease; demyelination; Multiple Sclerosis; thalamic degeneration; Creutzfeldt-Jakob dementia; HIV-dementia; schizophrenia with dementia or Korsakoff psychosis; Multiple System Atrophy and other forms of Parkinsonism Plus; movement disorders; neuroprotection; anxiety, tension and depression or post-traumatic stress disorder (PTSD); bipolar disorder; schizophrenia; CNS-related sexual dysfunction and sleep disturbances; pathological eating disorders and use of luxury foods and addictive drugs; controlling cerebral perfusion; migraines; prophylaxis and control of consequences of cerebral infarction (apoplexia cerebri); prophylaxis and control of consequences of stroke, cerebral ischemias and head injury.

10. The compound or composition for use of claim 3, wherein the disease, health condition or disorder is selected from shock; cardiogenic shock; sepsis; septic shock; anaphylactic shock; aneurysm; control of leukocyte activation; inhibition or modulation of platelet aggregation; multiple organ dysfunction syndrome (MODS); multiple organ failure (MOF).

11. The compound or composition for use of claim 3, wherein the disease, health condition or disorder is selected from pulmonary/respiratory conditions: pulmonary hypertension (PH); pulmonary arterial hypertension (PAH), and associated pulmonary vascular remodeling; vascular remodeling in the form of localized thrombosis and right heart hypertrophy; pulmonary hypertonia; primary pulmonary hypertension; secondary pulmonary hypertension; familial pulmonary hypertension; sporadic pulmonary hypertension; pre-capillary pulmonary hypertension; idiopathic pulmonary hypertension; other forms of PH; PH associated with left ventricular disease, HIV, SCD, thromboembolism (CTEPH), sarcoidosis, COPD, pulmonary fibrosis, acute respiratory distress syndrome (ARDS), acute lung injury, alpha-1-antitrypsin deficiency (AATD), pulmonary emphysema, smoking-induced emphysema and cystic fibrosis (CF); thrombotic pulmonary arteriopathy; plexogenic pulmonary arteriopathy; cystic fibrosis; bronchoconstriction or pulmonary bronchoconstriction; acute respiratory distress syndrome; lung fibrosis, lung transplant; asthmatic diseases.

12. The compound of claim 1, or a pharmaceutically acceptable salt thereof, or the pharmaceutical composition of claim 2, for use in treating a disease, health condition or disorder, wherein the disease, health condition or disorder is selected from:
• heart muscle inflammation (myocarditis); chronic myocarditis; acute myocarditis; viral myocarditis;
• vasculitis; pancreatitis; peritonitis; rheumatoid diseases;
• inflammatory disease of the kidney; immunological kidney diseases: kidney transplant rejection, immune complex-induced kidney disease, nephropathy induced by toxins, constrast medium-induced nephropathy; diabetic and non-diabetic nephropathy, pyelonephritis, renal cysts, nephrosclerosis, hypertensive nephrosclerosis and nephrotic syndrome;
• chronic interstitial inflammations, inflammatory bowel diseases (IBD), Crohn's, Ulcerative Colitis (UC);
• inflammatory skin diseases;
• inflammatory diseases of the eye, blepharitis, dry eye syndrome, and Sjögren's Syndrome; eye fibrosis.

13. The compound of claim 1, or a pharmaceutically acceptable salt thereof, or the pharmaceutical composition of claim 2, for use in treating a disease, health condition or disorder, wherein the disease, health condition or disorder is selected from wound or ulcer healing in diabetics; microvascular perfusion improvement; microvascular perfusion improvement following injury or to counteract the inflammatory response in perioperative care; anal fissures; diabetic ulcers; diabetic foot ulcers); bone healing; osteoclastic bone resorption and remodeling; and new bone formation.

14. The compound of claim 1, or a pharmaceutically acceptable salt thereof, or the pharmaceutical composition of claim 2, for use in treating a disease, health condition or disorder, wherein the disease, health condition or disorder is selected from:
• urogenital system disorders: diabetic nephropathy; renal fibrosis and renal failure resulting from chronic kidney diseases or insufficiency; renal fibrosis and renal failure due to accumulation/deposition and tissue injury; renal sclerosis; progressive sclerosis; glomerulonephritis; focal segmental glomerulosclerosis; nephrotic syndrome; prostate hypertrophy; kidney fibrosis; interstitial renal fibrosis;
• pulmonary system disorders: pulmonary fibrosis; idiopathic pulmonary fibrosis; cystic fibrosis; progressive massive fibrosis; progressive massive fibrosis thataffects the lungs);
• disorders affecting the heart: endomyocardial fibrosis; old myocardial infarction; atrial fibrosis; cardiac interstitial fibrosis; cardiac remodeling and fibrosis; cardiac hypertrophy;
• disorders of the liver and related organs: liver sclerosis or cirrhosis; liver cirrhosis associated with chronic liver disease; hepatic fibrosis; hepatic stellate cell activation; hepatic fibrous collagen and total collagen accumulation; liver disease of necro-inflammatory and/or of immunological origin; primary biliary cirrhosis; primary sclerosing cholanginitis; other cholestatic liver diseases: those associated with granulomatous liver diseases, liver malignancies, intrahepatic cholestasis of pregnancy, hepatitis, sepsis, drugs or toxins, graft-versus-host disease, post-liver transplantation, choledocholithiasis, bile duct tumors, pancreatic carcinoma, Mirizzi's syndrome, AIDS cholangiopathy or parasites; schistosomiasis;
• digestive diseases or disorders: Crohn's disease; Ulcerative Colitis; sclerosis of the gastro-intestinal tract;
• diseases of the skin or the eyes: nephrogenic fibrosis; keloids; fibrotic topical or skin disorders or conditions; dermal fibrosis; scleroderma, skin fibrosis; morphea; hypertrophic scars; naevi; proliferative vitroretinopathy; sarcoids; granulomas; eye fibrosis;
• diseases affecting the nervous system: Amyotrophic Lateral Sclerosis (ALS); hippocampal sclerosis, multiple sclerosis (MS); focal sclerosis; primary lateral sclerosis;
• diseases of the bones; osteosclerosis;
• otosclerosis; other hearing diseases or disorders; hearing impairment, partial or total hearing loss; partial or total deafness; tinnitus; noise-induced hearing loss;
• other diseases involving autoimmunity, inflammation or fibrosis: scleroderma; localized scleroderma or circumscribed scleroderma; mediastinal fibrosis; fibrosis mediastinitis; myelofibrosis; retroperitoneal fibrosis; arthrofibrosis; Peyronie's disease; Dupuytren's contracture; lichen sclerosus; some forms of adhesive capsulitis; atherosclerosis; tuberous sclerosis; systemic sclerosis; polymyositis; dermatomyositis; polychondritis; oesinophilic fasciitis; Systemic Lupus Erythematosus or lupus; bone marrow fibrosis, myelofibrosis or osteomyelofibrosis; sarcoidosis; uterine fibroids; endometriosis.

15. The compound of claim 1, or a pharmaceutically acceptable salt thereof, or the pharmaceutical composition of claim 2, for use in treating a disease, health condition or disorder, wherein the disease, health condition or disorder is selected from certain types of cancers; Sickle Cell Disease; Sickle Cell Anemia; cancer metastasis; osteoporosis; gastroparesis; functional dyspepsia; diabetic complications; alopecia or hair loss; diseases associated with endothelial dysfunction; neurologic disorders associated with decreased nitric oxide production; arginosuccinic aciduria; neuromuscular diseases; Duchenne muscular dystrophy (DMD); Becker muscular dystrophy (BMD); limb girdle muscular dystrophies; distal myopathies; type I and type II myotonic dystrophies; facio-scapulo-peroneal muscular dystrophy; autosomal and X-linked Emery-Dreifuss muscular dystrophy; oculopharyngeal muscular dystrophy; amyotrophic lateral sclerosis; and spinal muscle atrophy (SMA).

## Patentansprüche

1. Verbindung, ausgewählt aus jenen, die in Tabellen A, B, C und D dargestellt sind, oder pharmazeutisch annehmbares Salz davon:

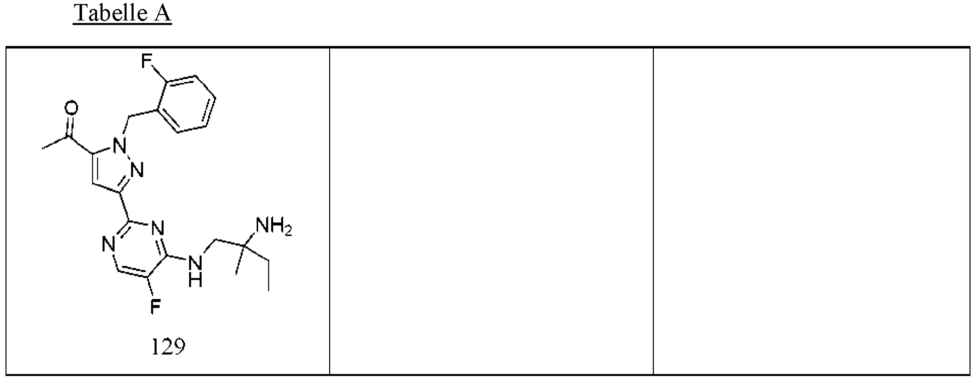

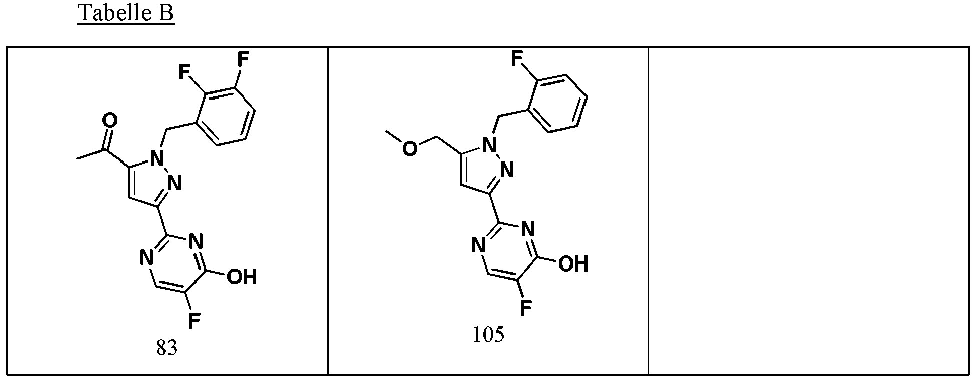

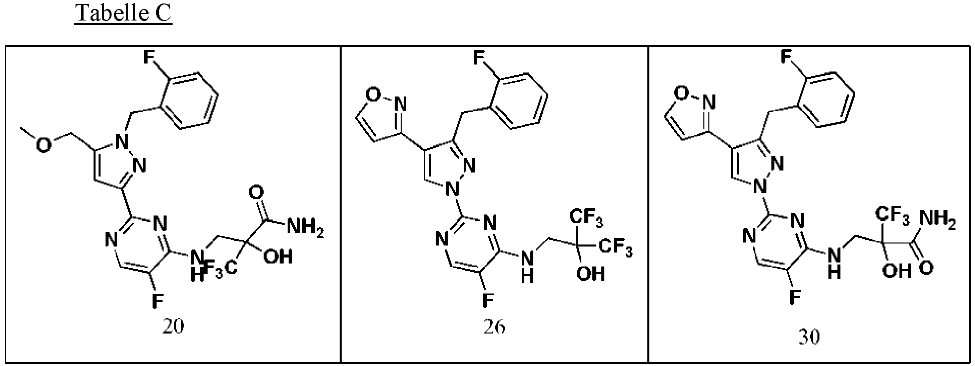

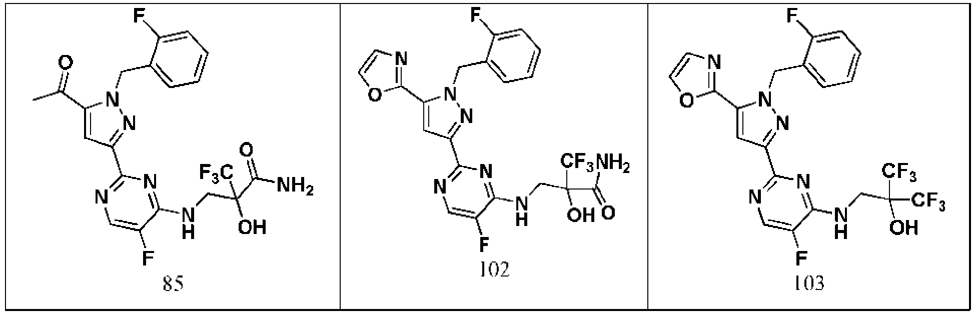

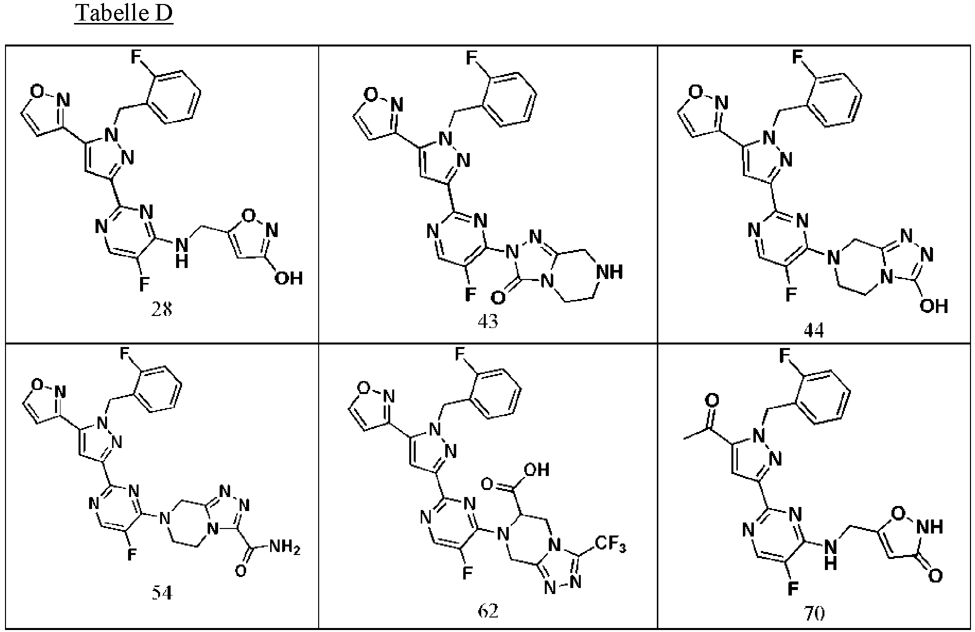

2. Pharmazeutische Zusammensetzung, umfassend eine Verbindung nach Anspruch 1 oder pharmazeutisch annehmbares Salz davon und einen oder mehrere Hilfsstoffe.

3. Verbindung nach Anspruch 1 oder pharmazeutisch annehmbares Salz davon oder pharmazeutische Zusammensetzung nach Anspruch 2 zur Verwendung beim Behandeln einer Krankheit, eines Gesundheitszustands oder einer Störung, wobei die Krankheit, der Gesundheitszustand oder die Störung ausgewählt ist aus:
• Störungen im Zusammenhang mit Bluthochdruck und verminderter Koronardurchblutung; erhöhter akuter und chronischer Koronardruck; arterielle Hypertonie; Gefäßstörungen infolge von Herz- und Nierenkomplikationen; Gefäßstörungen infolge von Herzerkrankungen, Schlaganfall, zerebraler Ischämie oder Nierenversagen; resistenter Hypertonie; diabetische Hypertonie; essentielle Hypertonie; sekundäre Hypertonie; Schwangerschaftshypertonie; Präeklampsie; portale Hypertonie; Myokardinfarkt;
• Herzversagen; HFPEF, HFREF; akutem und chronischem Herzversagen und spezifischeren Formen des Herzversagens; akutem dekompensiertem Herzversagen, Rechtsherzversagen, Linksherzversagen, totalem Herzversagen, ischämischer Kardiomyopathie, dilatierter Kardiomyopathie, kongenitalem Herzfehler, Herzversagen mit Herzklappenfehlern, Mitralklappenstenose, Mitralklappeninsuffizienz, Aortenklappenstenose, Aortenklappeninsuffizienz, Trikuspidstenose, trikuspidaler Insuffizienz, Pulmonalklappenstenose, Pulmonalklappeninsuffizienz, kombiniertem Herzklappenfehler; diabetischem Herzversagen; alkoholischer Kardiomyopathie, Speicherungs-Kardiomyopathien; diastolischem Herzversagen, systolischem Herzversagen, akuten Phasen eines vorliegenden chronischen Herzversagens, degeneratives Herzversagen; diastolischer oder systolischer Dysfunktion; Koronarinsuffizienz; Arrhythmien; Verringerung von ventrikulärer Vorlast; Herzhypertrophie; Herzfehler/kardiorenales Syndrom; portaler Hypertonie; endothelialer Dysfunktion oder Verletzung; Störungen des atrialen und ventrikulären Rhythmus und Leitungsstörungen; Atrioventrikularblöcke vom Grad I-III (AVB I-III); supraventrikulärer Tachyarrhythmie, Vorhofflimmern, Vorhofflattern, Herzkammerflimmern, Herzkammerflattern, ventrikulärer Tachyarrhythmie, Torsade-de-Pointes-Tachykardie, atrialen und ventrikulären Extrasystolen, Extrasystolen der AV-Verbindung, Sick-Sinus-Syndrom, Synkopen, Reentry-Tachykardie des AV-Knotens; Wolff-Parkinson-White-Syndrom oder akutem Koronarsyndrom; Boxer-Kardiomyopathie; vorzeitiger ventrikulärer Kontraktion; Kardiomyopathie; Krebs-induzierte Kardiomyopathie;
• thromboembolischen Störungen und Ischämien, Myokardischämie; Infarzierung; Myokardinfarkt; Herzanfall; Myokardinsuffizienz; endothelialer Dysfunktion, Schlaganfall, transitorischen ischämische Attacken (transient ischemic attacks, TIAs); obstruktiver Thrombangiitis; stabiler oder instabiler Angina pectoris; Koronarspasmen, Spasmen der Becken-Bein-Arterien; Variantangina, Prinzmetal-Angina; Herzhypertrophie; Präeklampsie; thrombogenen Störungen; Ischämie-Reperfusions-Schaden; Ischämie-Reperfusion in Verbindung mit Organtransplantation; Ischämie-Reperfusion in Verbindung mit Lungentransplantation, pulmonaler Transplantation, Herztransplantation; Venentransplantatversagen; Beibehaltung von Blutsubstituenten bei Traumapatienten;
• peripherer Gefäßerkrankung; peripherer arterieller Erkrankung; peripherer arterieller Verschlusskrankheit; Hypertonie; Raynaud-Syndrom oder -Phänomen (primär und sekundär); Raynaud-Krankheit; kritischer Extremitätenischämie; peripherer Embolie; Claudicatio intermittens; vaso-okklusiver Krise; Muskeldystrophie, Duchenne-Muskeldystrophie, Becker-Muskeldystrophie; Mikrozirkulationsanomalien; Kontrolle der vaskulären Leckage oder Permeabilität; lumbaler Spinalkanalstenose; okklusiver thrombotischer Vaskulitis; thrombotischer Vaskulitis; peripheren Perfusionsstörungen; arteriellen und venösen Thrombosen; Mikroalbuminurie; peripheren und autonomen Neuropathien; diabetischen Mikroangiopathien;
• Ödem; Nierenödem wegen Herzversagen;
• Alzheimer-Krankheit; Parkinson-Krankheit; vaskulärer Dementias; vaskulärer kognitiver Beeinträchtigung; zerebraler Vasospasmus; kongenitalem myasthenischem Syndrom; Subarachnoidalblutung; traumatischer Hirnverletzung; Verbessern von Wahrnehmung, Konzentrationsfähigkeit, Lernfähigkeit oder Gedächtnisleistung nach kognitiven Störungen, wie z. B. jene, die bei einer leichten kognitiven Beeinträchtigung, altersbedingten Lern- und Gedächtnisstörungen, altersbedingtem Gedächtnisverlust, vaskulärer Demenz, Kopfverletzung, Schlaganfall, Demenz nach Schlaganfall, posttraumatischer Kopfverletzung, allgemeinen Konzentrationsstörungen und Konzentrationsstörungen bei Kindern mit Lern- und Gedächtnisproblemen vorkommen; Lewy-Körper-Demenz, Demenz mit Frontallappendegeneration einschließlich Pick-Syndrom; progressiver nukleärer Blickparese; Demenz mit kortikobasaler Degeneration; amyotropher Lateralsklerose (ALS); Huntington-Krankheit; Demyelinisierung, multipler Sklerose, thalamische Degeneration; Creutzfeldt-Jakob-Demenz, HIV-Demenz, Schizophrenie mit Demenz oder Korsakoff-Psychose; multipler Systematrophie und anderen Formen von Parkinsonismus Plus; Bewegungsstörungen; Neuroprotektion; Angst, Anspannung und Depression, posttraumatischer Belastungsstörung (PTSD); bipolarer Störung; Schizophrenie; ZNS-bedingter sexueller Dysfunktion und Schlafstörungen; pathologischen Essstörungen und Verwendung von Genussmitteln und Suchtdrogen; Kontrolle von Hirndurchblutung, Kontrolle von Migränen; Prophylaxe und Kontrolle der Folgen von Hirninfarkt (Apoplexia cerebri); Prophylaxe und Kontrolle der Folgen von Schlaganfall, zerebralen Ischämien und Kopfverletzungen;
• Schock; kardiogener Schock; Sepsis; septischer Schock; anaphylaktischer Schock; Aneurysma; Kontrolle der Leukozytenaktivierung; Hemmung oder Modulation der Thrombozytenaggregation; Syndrom multiplem Organdysfunktionssyndrom (MODS); multiples Organversagen (MOF);
• pulmonalen/respiratorischen Bedingungen; pulmonaler Hypertonie (PH), pulmonaler arterieller Hypertonie (PAH) und assoziierter pulmonaler vaskulärer Remodellierung; lokalisierter Thrombose und rechter Ventrikelhypertrophie; pulmonaler Hypertonie; primärer pulmonaler Hypertonie, sekundärer pulmonaler Hypertonie, familiärer pulmonaler Hypertonie, sporadischer pulmonaler Hypertonie, präkapillärer pulmonaler Hypertonie, idiopathischer pulmonaler Hypertonie; anderen Formen von PH; PH assoziiert mit linksventrikulärer Krankheit, HIV, SCD, Thrombembolie (CTEPH), Sarkoidose, COPD, pulmonaler Fibrose; akutem respiratorischem Distresssyndrom (ARDS), akuter Lungenverletzung, Alpha-1-Antitrypsin-Mangel (AATD), pulmonalem Emphysem; durch Rauchen induziertes Emphysem und zystische Fibrose (CF); thrombotischer pulmonaler Arteriopathie; plexogener pulmonaler Arteriopathie; zystischer Fibrose; Bronchokonstriktion oder pulmonaler Bronchokonstriktion; akutem Atemnotsyndrom; Lungenfibrose, Lungentransplantation; asthmatischen Erkrankungen;
• pulmonaler Hypertonie assoziiert mit oder in Bezug auf: linksventrikuläre Dysfunktion, Hypoxämie, WHO-Gruppen I, II, III, IV und V Hypertonien, Mitralklappenkrankheit, konstriktive Perikarditis, Aortastenose, Kardiomyopathie, Mediastinalfibrose, pulmonale Fibrose, anomalen pulmonalen venösen Abfluss, pulmonale Venen-Verschlusskrankheit, pulmonale Vaskulitis, kollagenvaskuläre Krankheit, kongenitale Herzkrankheit, pulmonale Venenhypertonie, interstitielle Lungenkrankheit, schlafbezogene Atmungsstörung, Schlafapnoe, alveoläre Hypoventilationsstörungen, chronische Aussetzung an große Höhe, neonatale Lungenkrankheit, alveoläre kapillare Dysplasie, Sichelzellenkrankheit, andere Koagulationsstörungen, chronische Thrombembolie, pulmonale Embolie, pulmonale Embolie wegen Tumor, Parasiten oder Fremdmaterial; Bindegewebekrankheit, Lupus, Lupusnephritis, Schistosomiasis, Sarkoidose, chronischer obstruktiver pulmonaler Krankheit, Asthma, Emphysem, chronischer Bronchitis, pulmonaler kapillarer Hämangiomatose; Histiozytose X, Lymphangiomatose; komprimierten pulmonalen Gefäßen; komprimierten pulmonalen Gefäßen durch Drüsenerkrankung, Tumor oder fibrosierende Mediastinitis;
• arteriosklerotischen Krankheiten oder Zuständen; Arteriosklerose, Arteriosklerose assoziiert mit endothelialer Verletzung, Thrombozyt- und Monozytadhäsion und -aggregation, Proliferation oder Migration von glattem Muskel; Restenose; Restenose, die sich nach Thrombolyse-Therapien entwickelt hat, perkutanen transluminalen Angioplastien (PTAs), transluminalen Coronar-Angioplastien (PTCAs), Herztransplantation, Bypass-Operationen oder Entzündungsprozesse;
• mikro- und makrovaskulärer Schaden (Vaskulitis); erhöhten Werten von Fibrinogen und DLD niedriger Dichte, erhöhter Konzentration von Plasminogenaktivator-Hemmer 1 (PA-1);
• metabolischem Syndrom; metabolischen Krankheiten oder Krankheiten, die mit dem metabolischen Syndrom verbunden sind: Fettleibigkeit; übermäßigem subkutanem Fett; übermäßigem Adipositas; Diabetes; Bluthochdruck; Fettstoffwechselstörungen, Hyperlipidämien, Dyslipidämien, Hypercholesterinämien, vermindertem High-Density-Lipoprotein-Cholesterin (HDL-Cholesterin), mäßig erhöhten Low-Density-Lipoprotein-Cholesterin-Werten (LDL-Cholesterin), Hypertriglyceridämien, Hyperglyceridämien, Hypolipoproteinanämien, Sitosterolämie, Fettlebererkrankungen, Hepatitis; Präeklampsie; Fortschreiten von polyzystischer Nierenerkrankung; Lebersteatose oder abnorme Lipidansammlung in der Leber; Steatose des Herzens, der Nieren oder des Muskels; Alphabetalipoproteinämie; Sitosterolemie; Xanthomatose; Tanger-Krankheit; Hyperammonämie und verwandte Krankheiten; hepatischen Enzephalopatien; anderen toxischen Enzephalopatien; Reye-Syndrom;
• sexuellen, gynäkologischen und urologischen Störungen von Zuständen; erektiler Dysfunktion; Impotenz; vorzeitiger Ejakulation; weiblicher sexueller Dysfunktion; weiblicher sexueller Erregungsdysfunktion, hypoaktiver sexueller Erregungsstörung, vaginaler Atrophie, Dyspareunie, atrophischer Vaginitis, gutartiger Prostatahyperplasie (BPH), prostatischer Hypertrophie, prostatischer Vergrößerung; Blasenauslassobstruktion; Blasenschmerzsyndrom (BPS); interstitielle Zystitis (IC); überaktiver Blase; neurogener Blase und Inkontinenz; diabetischer Nephropathie; primärer und sekundärer Dysmenorrhö; Syndrome des unteren Harntrakts (LUTS); Endometriose; Beckenschmerzen; gutartigen und bösartigen Krankheiten der Organe des männlichen und weiblichen urogenitalen Systems;
• chronischer Nierenerkrankung; akuter und chronischer Niereninsuffizienz; akutem und chronischem Nierenversagen; zugrundeliegenden oder verwandten Nierenkrankheiten, wie z. B. Hypoperfusion, intradialytische Hypotension, obstruktive Uropathie, Glomerulopathien, Glomerulonephritis, akute Glomerulonephritis, Glomerulosklerose, tubulointerstitielle Krankheiten, nephropathische Krankheiten; primäre und kongenitale Nierenkrankheiten, Nephritis; Krankheiten, die durch eine abnorm reduzierte Kreatinin- und oder Wasserausscheidung gekennzeichnet sind; Krankheiten, die durch abnorm erhöhte Blutkonzentrationen von Harnstoff, Stickstoff, Kalium und/oder Kreatinin gekennzeichnet sind; Krankheiten, die durch eine veränderte Aktivität von Nierenenzymen gekennzeichnet sind, Krankheiten, die durch eine veränderte Aktivität der Glutamylsynthetase gekennzeichnet sind; Krankheiten, die durch eine veränderte Urinosmolarität oder Urinmenge gekennzeichnet sind; Krankheiten, die durch erhöhte Mikroalbuminurie gekennzeichnet sind, Krankheiten, die durch Makroalbuminurie gekennzeichnet sind; Krankheiten, die durch Läsionen der Glomeruli und Arteriolen, tubuläre Dilatation, Hyperphosphatämie und/oder Dialysebedürftigkeit gekennzeichnet sind; Folgeerscheinungen der Niereninsuffizienz; pulmonales Ödem in Zusammenhang mit Niereninsuffizienz; Niereninsuffizienz im Zusammenhang mit Herzversagen; Niereninsuffizienz in Zusammenhang mit Urämie oder Anämie; Elekrolytstörungen (Herkaliämie, Hyponatriämie); Störungen des Knochen- und Kohlenhydratstoffwechsels;
• Augenkrankheiten oder -störungen, wie z. B. Glaukom, Retinopathie und diabetische Retinopathie.

4. Verbindung oder Zusammensetzung zur Verwendung nach Anspruch 3, wobei die Krankheit, der Gesundheitszustand oder die Störung ausgewählt ist aus Störungen in Verbindung mit hohem Blutdruck und vermindertem koronaren Blutfluss; erhöhtem akutem koronarem Blutdruck; erhöhtem chronischem koronarem Blutdruck; arterieller Hypertonie; vaskulärer Störung, die aus kardialen und renalen Komplikationen resultiert; vaskulärer Störung, die aus Herzkrankheit, Schlaganfall, zerebraler Ischämie oder Nierenversagen resultiert; resistenter Hypertonie; diabetischer Hypertonie; essentieller Hypertonie; sekundärer Hypertonie; Schwangerschaftshypertonie; Präeklampsie; portaler Hypertonie; und Myokardinfarkt.

5. Verbindung oder Zusammensetzung zur Verwendung nach Anspruch 3, wobei die Krankheit, der Gesundheitszustand oder die Störung ausgewählt ist aus Herzversagen; HFPEF, HFREF; akutem und chronischem Herzversagen und spezifischeren Formen des Herzversagens; akutem dekompensiertem Herzversagen, Rechtsherzversagen, Linksherzversagen, totalem Herzversagen, ischämischer Kardiomyopathie, dilatierter Kardiomyopathie, kongenitalem Herzfehler, Herzversagen mit Herzklappenfehlern, Mitralklappenstenose, Mitralklappeninsuffizienz, Aortenklappenstenose, Aortenklappeninsuffizienz, Trikuspidstenose, trikuspidaler Insuffizienz, Pulmonalklappenstenose, Pulmonalklappeninsuffizienz, kombiniertem Herzklappenfehler; diabetischem Herzfehler; alkoholischer Kardiomyopathie, Speicherungs-Kardiomyopathien; diastolischem Herzversagen, systolischem Herzversagen, akuten Phasen eines vorliegenden chronischen Herzversagens (degeneratives Herzversagen); diastolischer oder systolischer Dysfunktion; Koronarinsuffizienz; Arrhythmien; Verringerung von ventrikulärer Vorlast; Herzhypertrophie; Herzfehler/kardiorenales Syndrom; portaler Hypertonie; endothelialer Dysfunktion oder Verletzung; Störungen des atrialen und ventrikulären Rhythmus und Leitungsstörungen; Atrioventrikularblöcke vom Grad I-III (AVB I-III); supraventrikulärer Tachyarrhythmie, Vorhofflimmern, Vorhofflattern, Herzkammerflimmern, Herzkammerflattern, ventrikulärer Tachyarrhythmie, Torsade-de-Pointes-Tachykardie, atrialen und ventrikulären Extrasystolen, Extrasystolen der AV-Verbindung, Sick-Sinus-Syndrom, Synkopen, Reentry-Tachykardie des AV-Knotens; Wolff-Parkinson-White-Syndrom oder akutem Koronarsyndrom; Boxer-Kardiomyopathie; Kardiomyopathie mit vorzeitiger ventrikulärer Kontraktion; Krebs-induzierte Kardiomyopathie.

6. Verbindung oder Zusammensetzung zur Verwendung nach Anspruch 3, wobei die Krankheit, der Gesundheitszustand oder die Störung ausgewählt ist aus thromboembolischen Störungen und Ischämien, Myokardischämie; Infarzierung; Myokardinfarkt; Herzanfall; Myokardinsuffizienz; endothelialer Dysfunktion, Schlaganfall, transitorischen ischämische Attacken (transient ischemic attacks, TIAs); obstruktiver Thrombangiitis; stabiler oder instabiler Angina pectoris; Koronarspasmen, Spasmen der Becken-Bein-Arterien; Variantangina, Prinzmetal-Angina; Herzhypertrophie; Präeklampsie; thrombogenen Störungen; Ischämie-Reperfusions-Schaden; Ischämie-Reperfusion in Verbindung mit Organtransplantation; Ischämie-Reperfusion in Verbindung mit Lungentransplantation, pulmonaler Transplantation, Herztransplantation; Venentransplantatversagen; Beibehaltung von Blutsubstituenten bei Traumapatienten.

7. Verbindung oder Zusammensetzung zur Verwendung nach Anspruch 3, wobei die Krankheit, der Gesundheitszustand oder die Störung ausgewählt ist aus peripherer Gefäßerkrankung; peripherer arterieller Erkrankung; peripherer arterieller Verschlusskrankheit; Hypertonie; Raynaud-Syndrom oder -Phänomen (primär und sekundär); Raynaud-Krankheit; kritischer Extremitätenischämie; peripherer Embolie; Claudicatio intermittens; vaso-okklusive Krise; Muskeldystrophie, Duchenne-Muskeldystrophie, Becker-Muskeldystrophie; Mikrozirkulationsanomalien; Kontrolle der vaskulären Leckage oder Permeabilität; lumbaler Spinalkanalstenose; okklusiver thrombotischer Vaskulitis; thrombotischer Vaskulitis; peripheren Perfusionsstörungen; arteriellen und venösen Thrombosen; Mikroalbuminurie; peripheren und autonomen Neuropathien; diabetischen Mikroangiopathien.

8. Verbindung oder Zusammensetzung zur Verwendung nach Anspruch 3, wobei die Krankheit, der Gesundheitszustand oder die Störung ausgewählt ist aus Ödemen und Nierenödemen aufgrund von Herzversagen.

9. Verbindung oder Zusammensetzung zur Verwendung nach Anspruch 3, wobei die Krankheit, der Gesundheitszustand oder die Störung ausgewählt ist aus Alzheimer-Krankheit; Parkinson-Krankheit; vaskulärer Dementias; vaskulärer kognitiver Beeinträchtigung; zerebraler Vasospasmus; kongenitalem myasthenischem Syndrom; Subarachnoidalblutung; traumatischer Hirnverletzung; Verbessern von Wahrnehmung, Konzentrationsfähigkeit, Lernfähigkeit oder Gedächtnisleistung nach kognitiven Störungen, wie z. B. jene, die bei einer leichten kognitiven Beeinträchtigung, altersbedingten Lern- und Gedächtnisstörungen, altersbedingtem Gedächtnisverlust, vaskulärer Demenz, Kopfverletzung, Schlaganfall, Demenz nach Schlaganfall, posttraumatischer Kopfverletzung, allgemeinen Konzentrationsstörungen und Konzentrationsstörungen bei Kindern mit Lern- und Gedächtnisproblemen vorkommen; Lewy-Körper-Demenz, Demenz mit Frontallappendegeneration einschließlich Pick-Syndrom; progressiver nukleärer Blickparese; Demenz mit kortikobasaler Degeneration; amyotropher Lateralsklerose (ALS); Huntington-Krankheit; Demyelinisierung, multipler Sklerose, thalamische Degeneration; Creutzfeldt-Jakob-Demenz, HIV-Demenz, Schizophrenie mit Demenz oder Korsakoff-Psychose; multipler Systematrophie und anderen Formen von Parkinsonismus Plus; Bewegungsstörungen; Neuroprotektion; Angst, Anspannung und Depression, posttraumatischer Belastungsstörung (PTSD); bipolarer Störung; Schizophrenie; ZNS-bedingter sexueller Dysfunktion und Schlafstörungen; pathologischen Essstörungen und Verwendung von Genussmitteln und Suchtdrogen; Kontrolle von Hirndurchblutung, Kontrolle von Migränen; Prophylaxe und Kontrolle der Folgen von Hirninfarkt (Apoplexia cerebri); Prophylaxe und Kontrolle der Folgen von Schlaganfall, zerebralen Ischämien und Kopfverletzung.

10. Verbindung oder Zusammensetzung zur Verwendung nach Anspruch 3, wobei die Krankheit, der Gesundheitszustand oder die Störung ausgewählt ist aus Schock; kardiogenem Schock; Sepsis; septischem Schock; anaphylaktischem Schock; Aneurysma; Kontrolle der Leukozytenaktivierung; Hemmung oder Modulation der Thrombozytenaggregation; Syndrom multiplem Organdysfunktionssyndrom (MODS); multiplem Organversagen (MOF);

11. Die Verbindung oder Zusammensetzung zur Verwendung nach Anspruch 3, wobei die Krankheit, der Gesundheitszustand oder die Störung ausgewählt ist aus pulmonalen/respiratorischen Bedingungen; pulmonaler Hypertonie (PH), pulmonaler arterieller Hypertonie (PAH) und assoziierter pulmonaler vaskulärer Remodellierung; lokalisierter Thrombose und rechter Ventrikelhypertrophie; pulmonaler Hypertonie; primärer pulmonaler Hypertonie, sekundärer pulmonaler Hypertonie, familiärer pulmonaler Hypertonie, sporadischer pulmonaler Hypertonie, präkapillärer pulmonaler Hypertonie, idiopathischer pulmonaler Hypertonie; anderen Formen von PH; PH assoziiert mit linksventrikulärer Krankheit, HIV, SCD, Thrombembolie (CTEPH), Sarkoidose, COPD, pulmonaler Fibrose; akutem respiratorischem Distresssyndrom (ARDS), akuter Lungenverletzung, Alpha-1-Antitrypsin-Mangel (AATD), pulmonalem Emphysem; durch Rauchen induziertes Emphysem und zystische Fibrose (CF); thrombotischer pulmonaler Arteriopathie; plexogener pulmonaler Arteriopathie; zystischer Fibrose; Bronchokonstriktion oder pulmonaler Bronchokonstriktion; akutem Atemnotsyndrom; Lungenfibrose, Lungentransplantation; asthmatischen Erkrankungen.

12. Verbindung nach Anspruch 1 oder pharmazeutisch annehmbares Salz davon oder pharmazeutische Zusammensetzung nach Anspruch 2 zur Verwendung beim Behandeln einer Krankheit, eines Gesundheitszustands oder einer Störung, wobei die Krankheit, der Gesundheitszustand oder die Störung ausgewählt ist aus:
• Herzmuskelentzündung (Myokarditis); chronischer Myokarditis; akuter Myokarditis; viraler Myokarditis;
• Vaskulitis; Pankreatitis; Peritonitis; rheumatischen Erkrankungen;
• entzündlicher Erkrankung der Niere; immunologischen Nierenerkrankungen: Nierentransplantatabstoßung, Immunkomplex-induzierter Nierenerkrankung, toxininduzierter Nephropathie, konstrastmedium-induzierter Nephropathie; diabetischer und nicht-diabetischer Nephropathie, Pyelonephritis, Nierenzysten, Nephrosklerose, hypertensiver Nephrosklerose und nephrotischem Syndrom;
• chronischen interstitiellen Entzündungen, entzündlichen Darmerkrankungen (IBD), Morbus Crohn, Colitis ulcerosa (UC);
• entzündlichen Hauterkrankungen;
• entzündlichen Erkrankungen des Auges, Blepharitis, Syndrom des trockenen Auges und Sjogren-Syndrom; Augenfibrose.

13. Verbindung nach Anspruch 1 oder pharmazeutisch verträgliches Salz davon oder pharmazeutische Zusammensetzung nach Anspruch 2 zur Verwendung beim Behandeln einer Krankheit, eines Gesundheitszustands oder einer Störung, wobei die Krankheit, der Gesundheitszustand oder die Störung ausgewählt ist aus Wund- oder Geschwürheilung bei Diabetikern; Verbesserung der mikrovaskulären Perfusion; Verbesserung der mikrovaskulären Perfusion nach einer Verletzung oder um der Entzündungsreaktion bei der perioperativen Versorgung entgegenzuwirken; Analfissuren; diabetischen Geschwüren; diabetischen Fußgeschwüren); Knochenheilung; osteoklastischer Knochenresorption und -umbau; und neuer Knochenbildung.

14. Verbindung nach Anspruch 1 oder pharmazeutisch annehmbares Salz davon oder pharmazeutische Zusammensetzung nach Anspruch 2 zur Verwendung beim Behandeln einer Krankheit, eines Gesundheitszustands oder einer Störung, wobei die Krankheit, der Gesundheitszustand oder die Störung ausgewählt ist aus:
• Erkrankungen des Urogenitalsystems: diabetische Nephropathie; Nierenfibrose und Nierenversagen infolge chronischer Nierenerkrankungen oder -insuffizienz; Nierenfibrose und Nierenversagen aufgrund von Akkumulation/Deposition und Gewebeschädigung; Nierensklerose; progressive Sklerose; Glomerulonephritis; fokal segmentale Glomerulosklerose; nephrotisches Syndrom; Prostatahypertrophie; Nierenfibrose; interstitielle Nierenfibrose;
• Erkrankungen des pulmonalen Systems: pulmonale Fibrose; idiopathische pulmonale Fibrose; zystische Fibrose; progressive massive Fibrose; progressive massive Fibrose, die die Lungen betrifft);
• Störungen, die das Herz betreffen: Endomyokardfibrose; alter Herzinfarkt; Vorhoffibrose; kardiale interstitielle Fibrose; kardiales Remodeling und Fibrose; kardiale Hypertrophie;
• Erkrankungen der Leber und verwandter Organe: Lebersklerose oder -zirrhose; Leberzirrhose in Verbindung mit einer chronischen Lebererkrankung; Leberfibrose; Aktivierung der hepatischen Sternzellen; hepatisches fibröses Kollagen und Gesamtkollagenakkumulation; Lebererkrankungen nekro-inflammatorischen und/oder immunologischen Ursprungs; primär biliäre Zirrhose; primär sklerosierende Cholanginitis; andere cholestatische Lebererkrankungen: jene, die mit granulomatösen Lebererkrankungen, Lebermalignomen, intrahepatischer Cholestase in der Schwangerschaft, Hepatitis, Sepsis, Medikamenten oder Toxinen, Transplantat-gegen-Wirt-Krankheit, nach Lebertransplantation, Choledocholithiasis, Gallengangstumoren, Pankreaskarzinom, Mirizzi-Syndrom, AIDS-Cholangiopathie oder Parasiten verbunden sind; Schistosomiasis;
• Krankheiten oder Störungen des Verdauungssystems: Morbus Crohn; Colitis ulcerosa; Sklerose des Magen-Darm-Trakts;
• Erkrankungen der Haut oder der Augen: nephrogene Fibrose; Keloide; fibrotische Erkrankungen oder Zustände der Haut; dermale Fibrose; Sklerodermie, Hautfibrose; Morphea; hypertrophe Narben; Naevi; proliferative Vitroretinopathie; Sarkoide; Granulome; Augenfibrosen;
• Krankheiten, die das Nervensystem betreffen: Amyotrophe Lateralsklerose (ALS); Hippocampussklerose, Multiple Sklerose (MS); fokale Sklerose; primäre Lateralsklerose;
• Erkrankungen der Knochen; Osteosklerose;
• Otosklerose; andere Erkrankungen oder Störungen des Gehörs; Schwerhörigkeit, teilweiser oder vollständiger Hörverlust; teilweise oder vollständige Taubheit; Tinnitus; lärmbedingter Hörverlust;
• anderen Krankheiten mit Autoimmunität, Entzündung oder Fibrose: Sklerodermie; lokalisierte Sklerodermie oder umschriebene Sklerodermie; Mediastinalfibrose; Fibrosemediastinitis; Myelofibrose; retroperitoneale Fibrose; Arthrofibrose; Peyronie-Krankheit; Dupuytren'sche Kontraktur; Lichen sclerosus; einige Formen der adhäsiven Kapsulitis; Atherosklerose; tuberöse Sklerose; systemische Sklerose; Polymyositis; Dermatomyositis; Polychondritis; eosinophile Fasziitis; systemischer Lupus erythematodes oder Lupus; Knochenmarkfibrose, Myelofibrose oder Osteomyelofibrose; Sarkoidose; Uterusmyome; Endometriose.

15. Verbindung nach Anspruch 1 oder ein pharmazeutisch annehmbares Salz davon oder pharmazeutische Zusammensetzung nach Anspruch 2 zur Verwendung beim Behandeln einer Krankheit, eines Gesundheitszustands oder einer Störung, wobei die Krankheit, der Gesundheitszustand oder die Störung ausgewählt ist aus bestimmten Arten von Krebs; Sichelzellenkrankheit; Sichelzellenanämie; Krebsmetastasen; Osteoporose; Gastroparese; funktioneller Dyspepsie; diabetischen Komplikationen; Alopezie oder Haarausfall; Krankheiten, die mit endothelialer Dysfunktion verbunden sind; neurologischen Erkrankungen, die mit einer verminderten Stickoxid-Produktion einhergehen; Arginosuccinsäureurie; neuromuskulären Erkrankungen; Duchenne-Muskeldystrophie (DMD); Becker-Muskeldystrophie (BMD); Gliedergürtel-Muskeldystrophien; distalen Myopathien; Myotone Dystrophien Typ I und Typ II; fazio-skapulo-peroneale Muskeldystrophie; autosomaler und X-chromosomaler Emery-Dreifuss-Muskeldystrophie; okulopharyngealer Muskeldystrophie; amyotropher Lateralsklerose; und spinaler Muskelatrophie (SMA).

## Revendications

1. Composé choisi parmi ceux décrits dans les tableaux A, B, C et D ou sel pharmaceutiquement acceptable de celui-ci :

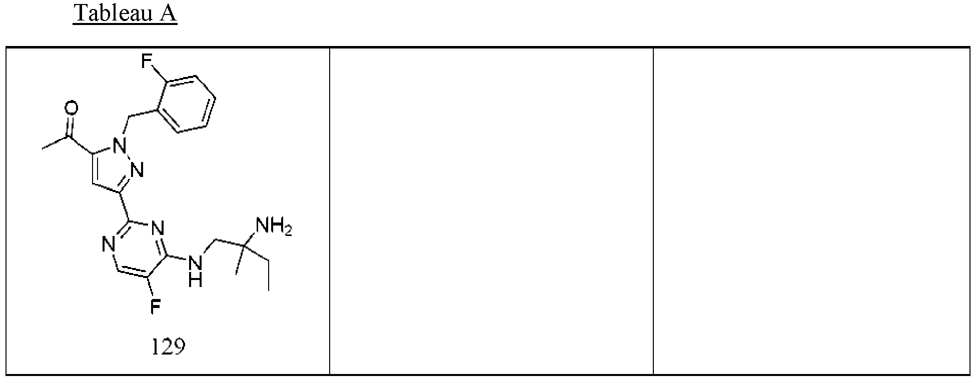

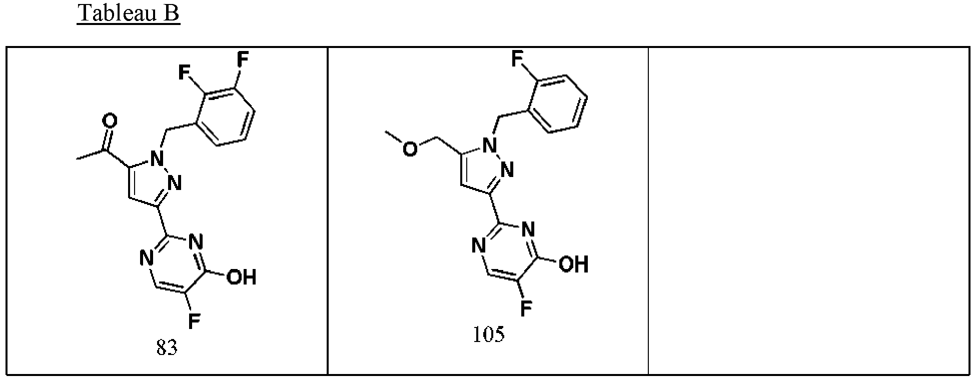

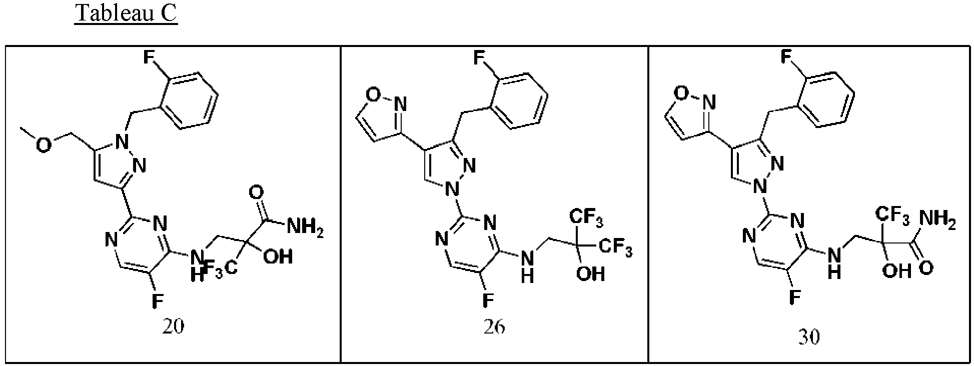

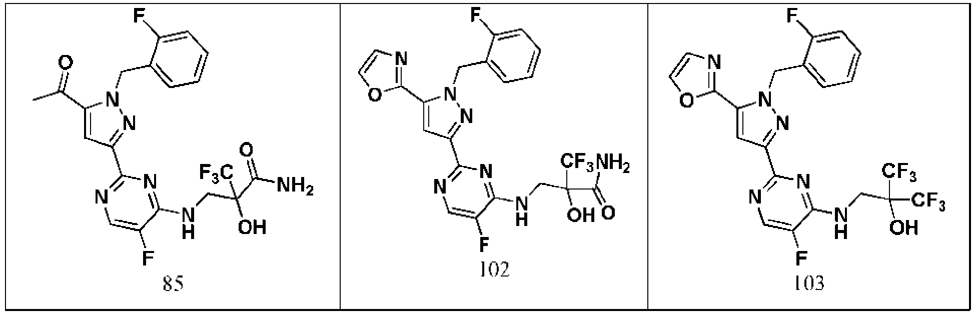

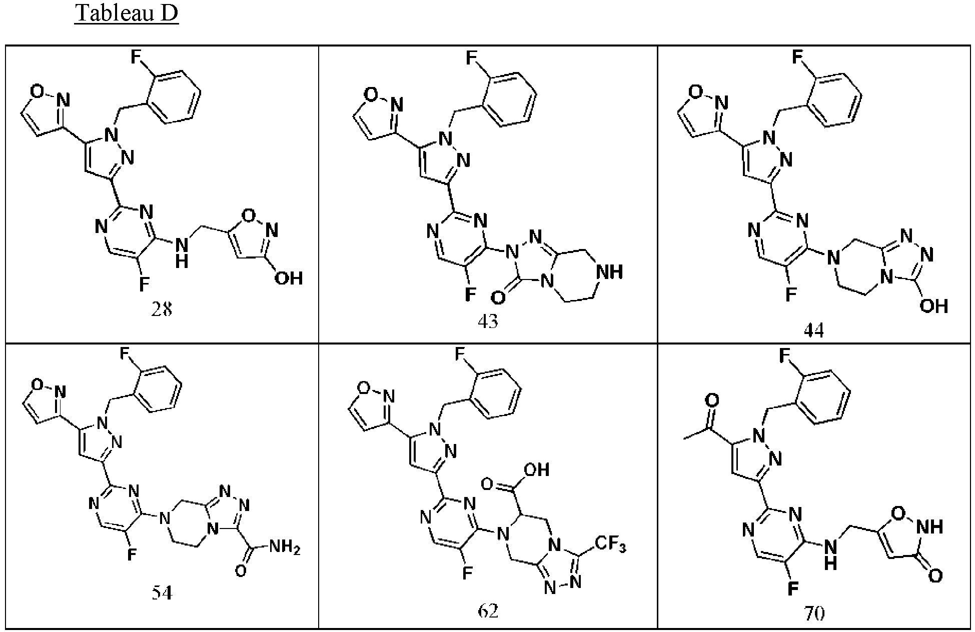

2. Composition pharmaceutique comprenant un composé selon la revendication 1 ou un sel pharmaceutiquement acceptable de celui-ci, et un ou plusieurs excipients.

3. Composé selon la revendication 1, ou sel pharmaceutiquement acceptable de celui-ci, ou composition pharmaceutique selon la revendication 2, pour une utilisation dans le traitement d'une maladie, d'un état de santé ou d'un trouble, ladite maladie, ledit état de santé ou ledit trouble étant choisi parmi :
• les troubles associés à une pression artérielle élevée et une diminution du flux sanguin coronaire ; une augmentation de la pression artérielle coronaire chronique et aiguë ; l'hypertension artérielle ; un trouble vasculaire suite à des complications rénales ou cardiaques ; les troubles vasculaires suite à une cardiopathie, un accident vasculaire cérébral, une ischémie cérébrale ou une insuffisance rénale ; une hypertension résistante ; une hypertension diabétique ; une hypertension essentielle ; une hypertension secondaire ; une hypertension gestationnelle ; une prééclampsie ; une hypertension portale ; un infarctus du myocarde ;
• une insuffisance cardiaque, une ICFEP, une ICFER ; une IC aiguë ou chronique ; des formes plus spécifiques d'IC : une IC décompensée aiguë, une insuffisance ventriculaire droite, une insuffisance ventriculaire gauche, une IC totale, une cardiomyopathie ischémique, une cardiomyopathie dilatée, des malformations cardiaques congénitales, une IC avec malformations valvulaires, une sténose de la valvule mitrale, une insuffisance de la valvule mitrale, une sténose de la valvule aortique, une insuffisance de la valvule aortique, une sténose de la valvule tricuspide, une insuffisance de la valvule tricuspide, une sténose de la valvule pulmonaire, une insuffisance de la valvule pulmonaire, des malformations valvulaires combinées ; une insuffisance cardiaque diabétique ; une cardiomyopathie alcoolique ou des cardiomyopathies de surcharge ; une IC diastolique ; une IC systolique ; des phases aiguës d'une IC chronique existante (IC en cours d'aggravation) ; un dysfonctionnement diastolique ou systolique ; une insuffisance coronarienne ; des arythmies ; une réduction de précharge ventriculaire ; une hypertrophie cardiaque ; une insuffisance cardiaque/un syndrome cardio-rénal ; une hypertension portale ; une lésion ou un dysfonctionnement endothélial ; des perturbations du rythme auriculaire et ventriculaire et des perturbations de conductance : des blocs auriculo-ventriculaires de degré I-III (AVB I-III), une tachyarythmie supraventriculaire, une fibrillation auriculaire, un flutter auriculaire, une fibrillation ventriculaire, un flutter ventriculaire, une tachyarythmie ventriculaire, une tachycardie à torsades de pointe, des extrasystoles auriculaire et ventriculaire, des extrasystoles de jonction auriculo-ventriculaire, la maladie du sinus, les syncopes, une tachycardie par réentrée nodale auriculo-ventriculaire ; le syndrome de Wolff-Parkinson-White ou un syndrome coronarien aigu ; la cardiomyopathie de Boxer ; une contraction ventriculaire prématurée ; une cardiomyopathie ; une cardiomyopathie induite par un cancer ;
• les troubles et les ischémies thromboemboliques ; une ischémie myocardique ; un infarctus ; un infarctus du myocarde ; une crise cardiaque ; une insuffisance du myocarde ; un dysfonctionnement endothélial ; un accident vasculaire cérébral ; des accidents ischémiques transitoires (AIT) ; une thromboangéite obstructive ; une angine de poitrine stable ou instable ; des spasmes coronariens ou des spasmes des artères périphériques ; un angor variant ; un angor de Prinzmetal ; une hypertrophie cardiaque ; une prééclampsie ; des troubles thrombogènes ; une lésion ischémique de reperfusion ; une reperfusion ischémique associée à une greffe d'organe ; une reperfusion ischémique associée à une rejet de greffe de poumon, de greffe pulmonaire, de greffe cardiaque, de greffe veineuse ; la conservation de substituts sanguins chez des patients souffrant de traumatismes ;
• une maladie vasculaire périphérique ; une maladie artérielle périphérique ; une maladie artérielle occlusive périphérique ; une hypertonie ; le syndrome ou phénomène de Raynaud (primaire et secondaire) ; la maladie de Raynaud ; une ischémie critique des membres ; une embolie périphérique ; une claudication intermittente ; une crise vaso-occlusive ; une dystrophie musculaire, la dystrophie musculaire de Duchenne, la dystrophie musculaire de Becker ; les anomalies de microcirculation ; le contrôle de la perméabilité ou des fuites vasculaires ; une sténose du canal rachidien lombaire ; une vascularité thrombotique occlusive ; une vascularité thrombotique ; des perturbations de perfusion périphérique ; une thrombose artérielle et veineuse ; une microalbuminurie ; des neuropathies périphériques et autonomes ; des microangiopathies diabétiques ;
• un œdème ; un œdème rénal dû à une insuffisance cardiaque ;
• la maladie d'Alzheimer ; la maladie de Parkinson ; les démences vasculaires ; un déficit cognitif vasculaire ; un vasospasme cérébral ; le syndrome myasthénique congénital ; une hémorrhagie subarachnoïde ; une lésion cérébrale traumatique ; une amélioration de la perception, de la capacité de concentration, de la capacité d'apprentissage ou des performances de la mémoire après des perturbations cognitives comme celles se produisant dans un trouble cognitif léger, des perturbations d'apprentissage et de la mémoire liées à l'âge, une perte de mémoire liée à l'âge, une démence vasculaire, un traumatisme crânien, un accident vasculaire cérébral, une démence après un accident vasculaire cérébral, un traumatisme crânien post-traumatique, des troubles généraux de concentration et des troubles de concentration chez l'enfant avec des problèmes d'apprentissage et de mémoire ; la démence à corps de Lewy ; une démence avec dégénérescence du lobe frontal y compris le syndrome de Pick ; une paralysie nucléaire progressive ; une démence avec dégénérescence corticobasale ; la sclérose latérale amyotrophique (SLA) ; la maladie de Huntington ; une démyélinisation ; la sclérose en plaques ; une dégénérescence thalamique ; la démence de Creutzfeldt-Jakob ; une démence associée au VIH ; une schizophrénie avec démence ou psychose de Korsakoff; une atrophie multisystématisée et d'autres formes de parkinsonisme plus ; des troubles du mouvement ; une neuroprotection ; l'anxiété, la tension et la dépression ou un trouble de stress post-traumatique (TSPT) ; un trouble bipolaire ; une schizophrénie ; un dysfonctionnement sexuel associé au SNC et des troubles du sommeil ; des troubles alimentaires pathologiques et l'utilisation de denrées de luxe et de drogues addictives ; le contrôle de l'irrigation sanguine cérébrale ; les migraines ; la prophylaxie et le contrôle des conséquences d'un infarctus cérébral (apoplexie cérébrale) ; la prophylaxie et le contrôle des conséquences d'un accident vasculaire cérébral, d'ischémies cérébrales et d'un traumatisme crânien ;
• une commotion ; un choc cardiogène ; une septicémie ; un choc septique ; un choc anaphylactique ; un anévrisme ; le contrôle de l'activation des leucocytes ; l'inhibition ou la modulation de l'agrégation plaquettaire ; le syndrome de défaillance multiviscérale (SDMV) ; une défaillance multiviscérale (DMV) ;
• les états pulmonaires/respiratoires: l'hypertension pulmonaire (HP) ; l'hypertension artérielle pulmonaire (HAP) et le remodelage vasculaire pulmonaire associé ; le remodelage vasculaire sous la forme d'une thrombose localisée et d'une hypertrophie cardiaque droite ; l'hypertonie pulmonaire ; l'hypertension pulmonaire primaire ; l'hypertension pulmonaire secondaire ; l'hypertension pulmonaire familiale ; l'hypertension pulmonaire sporadique ; l'hypertension pulmonaire précapillaire ; l'hypertension pulmonaire idiopathique ; les autres formes de HP ; une HP associée à une maladie du ventricule gauche, au VIH, à la SCD, à une thromboembolie (CTEPH), une sarcoïdose, la BPCO, une fibrose pulmonaire, le syndrome de détresse respiratoire aiguë (SDRA), une lésion pulmonaire aiguë, un déficit en alpha-1-antitrypsine (DAAT), un emphysème pulmonaire, un emphysème induit par la fumée de cigarette et la mucoviscidose (MV) ; une artériopathie pulmonaire thrombotique ; une artériopathie pulmonaire plexogène ; la mucoviscidose ; une bronchoconstriction ou une bronchoconstriction pulmonaire ; le syndrome de détresse respiratoire aiguë ; une fibrose pulmonaire, une greffe de poumon ; les maladies asthmatiques ;
• l'hypertension pulmonaire associée ou liée à : un dysfonctionnement du ventricule gauche, l'hypoxémie, les hypertensions des groupes I, II, III, IV et V selon l'OMS, une valvulopathie mitrale, une péricardite constrictive, une sténose aortique, une cardiomyopathie, une fibrose médiastinale, une fibrose pulmonaire, un drainage veineux pulmonaire anormal, une maladie pulmonaire veino-occlusive, une vascularité pulmonaire, une maladie vasculaire du collagène, une maladie cardiaque congénitale, l'hypertension veineuse pulmonaire, une pneumopathie interstitielle, un trouble respiratoire du sommeil, l'apnée du sommeil, les troubles d'hypoventilation alvéolaire, une exposition chronique à de hautes altitudes, une pneumopathie néonatale, une dysplasie alvéolo-capillaire, une drépanocytose, les autres troubles de la coagulation, une thomboembolie chronique, une embolie pulmonaire ; une embolie pulmonaire due à une tumeur, des parasites ou un corps étranger ; une maladie du tissu conjonctif, un lupus, une néphropathie lupique, une schistosomiase, une sarcoïdose, la bronchopneumopathie chronique obstructive, l'asthme, un emphysème, une bronchite chronique, une hémangiomatose capillaire pulmonaire, une histiocytose X, une lymphangiomatose, des vaisseaux pulmonaires comprimés ; des vaisseaux pulmonaires comprimés en raison d'une adénopathie, d'une tumeur ou d'une médiastinite fibrosante ;
• les maladies ou états artérioscléreux : l'athérosclérose ; l'athérosclérose associée à une lésion endothéliale, à l'adhérence et l'agrégation des monocytes et des plaquettes, à la prolifération ou la migration des muscles lisses ; une resténose ; une resténose développée après des thérapies par thrombolyse, des angioplasties transluminales percutanées (ATP), des angioplasties coronariennes transluminales (ACTP), une greffe de cœur, des opérations de pontage ou des processus inflammatoires ;
• les dommages microvasculaires et macrovasculaires (vasculite) ; une augmentation des taux de fibrinogène et de DLD de faible densité ; une augmentation de la concentration de l'inhibiteur 1 de l'activateur du plasminogène (PA-1) ;
• un syndrome métabolique ; les maladies métaboliques ou les maladies associées à un syndrome métabolique : l'obésité ; un excès de graisse sous-cutanée ; une adiposité excessive ; le diabète ; une tension artérielle élevée ; des troubles associés aux lipides, l'hyperlipidémie, la dyslipidémie, l'hypercholestérolémie, une diminution du taux de cholestérol à lipoprotéines haute densité (HDL-cholestérol), des taux de cholestérol à lipoprotéines basse densité (LDL-cholestérol) modérément élevés, l'hypertriglycéridémie, l'hyperglycéridémie, l'hypolipoprotéinémie, la sitostérolémie, une stéatose hépatique, une hépatite ; une prééclampsie ; l'évolution d'une polykystose rénale ; une stéatose hépatique ou une accumulation anormale de lipides dans le foie ; une stéatose du cœur, des reins ou d'un muscle ; l'alphabêtalipoprotéinémie ; la sitostérolémie ; la xanthomatose ; la maladie de Tangier ; l'hyperammoniémie et les maladies associées ; les encéphalopathies hépatiques ; les autres encéphalopathies toxiques ; le syndrome de Reye ;
• les troubles sexuels, gynécologiques et urologiques d'états : un dysfonctionnement érectile ; l'impuissance ; l'éjaculation précoce ; un dysfonctionnement sexuel féminin ; un dysfonctionnement de l'excitation sexuelle chez la femme ; un trouble de baisse de l'excitation sexuelle ; une atrophie vaginale ; une dyspareunie ; une vaginite atrophique ; une hyperplasie prostatique bénigne (HPB), une hypertrophie prostatique, un agrandissement de la prostate ; une obstruction de la sortie de la vessie ; le syndrome de la vessie douloureuse (SVD) ; la cystite interstitielle (CI) ; une vessie hyperactive ; une vessie neurogène et l'incontinence ; une néphropathie diabétique ; une dysménorrhée primaire et secondaire ; les syndromes de la voie urinaire inférieure (LUTS) ; l'endométriose ; les douleurs pelviennes ; les maladies bénignes et malignes des organes du système urogénital mâle et femelle ;
• une néphropathie chronique ; une insuffisance rénale aiguë et chronique ; une défaillance rénale chronique et aiguë ; une néphropathie lupique ; les maladies rénales associées ou sous-jacentes : l'hypoperfusion, l'hypotension intradialytique, une uropathie obstructive, les glomérulopathies, une glomérulonéphrite, une glomérulonéphrite aiguë, une glomérulosclérose, les maladies tubulointerstitielles, les maladies néphropathiques, les maladies rénales primaires et congénitales, une néphrite ; les maladies **caractérisées par** une excrétion de la créatine et ou de l'eau anormalement réduite ; les maladies **caractérisées par** des concentrations sanguines anormalement élevées d'urée, d'azote, de potassium et/ou de créatine ; les maladies **caractérisées par** une activité modifiée des enzymes rénales, les maladies **caractérisées par** une activité modifiée de la glutamyl synthétase ; les maladies **caractérisées par** une osmolarité urinaire ou un volume urinaire modifié ; les maladies **caractérisées par** une augmentation de la microalbuminurie, les maladies **caractérisées par** une macroalbuminurie ; les maladies **caractérisées par** des lésions des glomérules et des artérioles, une dilatation tubulaire, une hyperphosphatémie et/ou un besoin de dialyse ; les séquelles d'une insuffisance rénale ; une énéma pulmonaire associée à une insuffisance rénale ; une insuffisance rénale associée à une IC ; une insuffisance rénale associée à une urémie ou une anémie ; des perturbations élecrolytiques (herkalémie, hyponatrémie) ; les perturbations du métabolisme osseux et glucidique ;
• les maladies ou les troubles oculaires tels qu'un glaucome, une rétinopathie et une rétinopathie diabétique.

4. Composé ou composition pour une utilisation selon la revendication 3, ladite maladie, ledit état de santé ou ledit trouble étant choisi parmi les troubles associés à une pression artérielle élevée et une diminution du flux sanguin coronaire ; une augmentation de la pression artérielle coronaire aiguë ; une augmentation de la pression artérielle coronaire chronique ; l'hypertension artérielle ; un trouble vasculaire suite à des complications rénales ou cardiaques ; un trouble vasculaire suite à une cardiopathie, un accident vasculaire cérébral, une ischémie cérébrale ou une insuffisance rénale ; une hypertension résistante ; une hypertension diabétique ; une hypertension essentielle ; une hypertension secondaire ; une hypertension gestationnelle ; une prééclampsie ; une hypertension portale ; et un infarctus du myocarde.

5. Composé ou composition pour une utilisation selon la revendication 3, ladite maladie, ledit état de santé ou ledit trouble étant choisi parmi une insuffisance cardiaque, une ICFEP, une ICFER ; une IC aiguë et chronique ; des formes plus spécifiques d'IC ; une IC décompensée aiguë, une insuffisance ventriculaire droite, une insuffisance ventriculaire gauche, une IC totale, une cardiomyopathie ischémique, une cardiomyopathie dilatée, des malformations cardiaques congénitales, une IC avec malformations valvulaires, une sténose de la valvule mitrale, une insuffisance de la valvule mitrale, une sténose de la valvule aortique, une insuffisance de la valvule aortique, une sténose de la valvule tricuspide, une insuffisance de la valvule tricuspide, une sténose de la valvule pulmonaire, une insuffisance de la valvule pulmonaire, des malformations valvulaires combinées ; une insuffisance cardiaque diabétique ; une cardiomyopathie alcoolique ou des cardiomyopathies de surcharge ; une IC diastolique ; une IC systolique ; des phases aiguës d'une IC chronique existante (IC en cours d'aggravation) ; un dysfonctionnement diastolique ou systolique ; une insuffisance coronarienne ; des arythmies ; une réduction de précharge ventriculaire ; une hypertrophie cardiaque ; une insuffisance cardiaque/un syndrome cardio-rénal ; une hypertension portale ; une lésion ou un dysfonctionnement endothélial ; des perturbations du rythme auriculaire et ventriculaire et des perturbations de conductance : des blocs auriculo-ventriculaires de degré I-III (AVB I-III), une tachyarythmie supraventriculaire, une fibrillation auriculaire, un flutter auriculaire, une fibrillation ventriculaire, un flutter ventriculaire, une tachyarythmie ventriculaire, une tachycardie à torsades de pointe, des extrasystoles auriculaire et ventriculaire, des extrasystoles de jonction auriculo-ventriculaire, la maladie du sinus, les syncopes, une tachycardie par réentrée nodale auriculo-ventriculaire ; le syndrome de Wolff-Parkinson-White ou un syndrome coronarien aigu ; la cardiomyopathie de Boxer ; une contraction ventriculaire prématurée une cardiomyopathie ; une cardiomyopathie induite par un cancer.

6. Composé ou composition pour une utilisation selon la revendication 3, ladite maladie, ledit état de santé ou ledit trouble étant choisi parmi les troubles et les ischémies thromboemboliques ; une ischémie myocardique ; un infarctus ; un infarctus du myocarde ; une crise cardiaque ; une insuffisance du myocarde ; un dysfonctionnement endothélial ; un accident vasculaire cérébral ; des accidents ischémiques transitoires (AIT) ; une thromboangéite obstructive ; une angine de poitrine stable ou instable ; des spasmes coronariens ou des spasmes des artères périphériques ; un angor variant ; un angor de Prinzmetal ; une hypertrophie cardiaque ; une prééclampsie ; des troubles thrombogènes ; une lésion ischémique de reperfusion ; une reperfusion ischémique associée à une greffe d'organe ; une reperfusion ischémique associée à un rejet de greffe de poumon, de greffe pulmonaire, de greffe cardiaque, de greffe veineuse ; la conservation de substituts sanguins chez des patients souffrant de traumatismes.

7. Composé ou composition pour une utilisation selon la revendication 3, ladite maladie, ledit état de santé ou ledit trouble étant choisi parmi une maladie vasculaire périphérique ; une maladie artérielle périphérique ; une maladie artérielle occlusive périphérique ; une hypertonie ; le syndrome ou phénomène de Raynaud (primaire et secondaire) ; la maladie de Raynaud ; une ischémie critique des membres ; une embolie périphérique ; une claudication intermittente ; une crise vaso-occlusive ; une dystrophie musculaire, la dystrophie musculaire de Duchenne, la dystrophie musculaire de Becker ; les anomalies de microcirculation ; le contrôle de la perméabilité ou des fuites vasculaires ; une sténose du canal rachidien lombaire ; une vascularité thrombotique occlusive ; une vascularité thrombotique ; des perturbations de perfusion périphérique ; une thrombose artérielle et veineuse ; une microalbuminurie ; des neuropathies périphériques et autonomes ; des microangiopathies diabétiques.

8. Composé ou composition pour une utilisation selon la revendication 3, ladite maladie, ledit état de santé ou ledit trouble étant choisi parmi un œdème et un œdème rénal dû à une insuffisance cardiaque.

9. Composé ou composition pour une utilisation selon la revendication 3, ladite maladie, ledit état de santé ou ledit trouble étant choisi parmi la maladie d'Alzheimer ; la maladie de Parkinson ; les démences vasculaires ; un déficit cognitif vasculaire ; un vasospasme cérébral ; le syndrome myasthénique congénital ; une hémorrhagie subarachnoïde ; une lésion cérébrale traumatique ; une amélioration de la perception, de la capacité de concentration, de la capacité d'apprentissage ou des performances de la mémoire après des perturbations cognitives comme celles se produisant dans un trouble cognitif léger, des perturbations d'apprentissage et de la mémoire liées à l'âge, une perte de mémoire liée à l'âge, une démence vasculaire, un traumatisme crânien, un accident vasculaire cérébral, une démence après un accident vasculaire cérébral, un traumatisme crânien post-traumatique, des troubles généraux de concentration et des troubles de concentration chez l'enfant avec des problèmes d'apprentissage et de mémoire ; la démence à corps de Lewy ; une démence avec dégénérescence du lobe frontal y compris le syndrome de Pick ; une paralysie nucléaire progressive ; une démence avec dégénérescence corticobasale ; la sclérose latérale amyotrophique (SLA) ; la maladie de Huntington ; une démyélinisation ; la sclérose en plaques ; une dégénérescence thalamique ; la démence de Creutzfeldt-Jakob ; une démence associée au VIH ; une schizophrénie avec démence ou psychose de Korsakoff ; une atrophie multisystématisée et d'autres formes de parkinsonisme plus ; des troubles du mouvement ; une neuroprotection ; l'anxiété, la tension et la dépression ou un trouble de stress post-traumatique (TSPT) ; un trouble bipolaire ; la schizophrénie ; un dysfonctionnement sexuel associé au SNC et des troubles du sommeil ; des troubles alimentaires pathologiques et l'utilisation de denrées de luxe et de drogues addictives ; le contrôle de l'irrigation sanguine cérébrale ; les migraines ; la prophylaxie et le contrôle des conséquences d'un infarctus cérébral (apoplexie cérébrale) ; la prophylaxie et le contrôle des conséquences d'un accident vasculaire cérébral, d'ischémies cérébrales et d'un traumatisme crânien.

10. Composé ou composition pour une utilisation selon la revendication 3, ladite maladie, ledit état de santé ou ledit trouble étant choisi parmi une commotion ; un choc cardiogène ; une septicémie ; un choc septique ; un choc anaphylactique ; un anévrisme ; le contrôle de l'activation des leucocytes ; l'inhibition ou la modulation de l'agrégation plaquettaire ; le syndrome de défaillance multiviscérale (SDMV) ; une défaillance multiviscérale (DMV).

11. Composé ou composition pour une utilisation selon la revendication 3, ladite maladie, ledit état de santé ou ledit trouble étant choisi parmi les états pulmonaires/respiratoires: l'hypertension pulmonaire (HP) ; l'hypertension artérielle pulmonaire (HAP) et le remodelage vasculaire pulmonaire associé ; le remodelage vasculaire sous la forme d'une thrombose localisée et d'une hypertrophie cardiaque droite ; l'hypertonie pulmonaire ; l'hypertension pulmonaire primaire ; l'hypertension pulmonaire secondaire ; l'hypertension pulmonaire familiale ; l'hypertension pulmonaire sporadique ; l'hypertension pulmonaire précapillaire ; l'hypertension pulmonaire idiopathique ; les autres formes de HP ; une HP associée à une maladie du ventricule gauche, au VIH, à la SCD, à une thromboembolie (CTEPH), une sarcoïdose, la BPCO, une fibrose pulmonaire, le syndrome de détresse respiratoire aiguë (SDRA), une lésion pulmonaire aiguë, un déficit en alpha-1-antitrypsine (DAAT), un emphysème pulmonaire, un emphysème induit par la fumée de cigarette et la mucoviscidose (MV) ; une artériopathie pulmonaire thrombotique ; une artériopathie pulmonaire plexogène ; la mucoviscidose ; une bronchoconstriction ou une bronchoconstriction pulmonaire ; le syndrome de détresse respiratoire aiguë ; une fibrose pulmonaire, une greffe de poumon ; les maladies asthmatiques.

12. Composé selon la revendication 1, ou sel pharmaceutiquement acceptable de celui-ci, ou composition selon la revendication 2, pour une utilisation dans le traitement d'une maladie, d'un état de santé ou d'un trouble, ladite maladie, ledit état de santé ou ledit trouble étant choisi parmi :
• une inflammation du muscle cardiaque (myocardite) ; une myocardite chronique ; une myocardite aiguë ; une myocardite virale ;
• une vascularité ; une pancréatite ; une péritonite ; les maladies rhumatoïdes ;
• une maladie inflammatoire du rein ; les maladies rénales immunologiques : un rejet de greffe de rein, une maladie rénale induite par des complexes immuns, une néphropathie induite par des toxines, une néphropathie induite par un milieu de contraste ; une néphropathie diabétique et non diabétique, une pyélonéphrite, des kystes rénaux, une néphrosclérose, une néphrosclérose hypertensive et un syndrome néphrotique ;
• les inflammations interstitielles chroniques, les maladies intestinales inflammatoires (MII), la maladie de Crohn, la colite ulcéreuse (CU) ;
• les maladies cutanées inflammatoires ;
• les maladies inflammatoires des yeux, une blépharite, le syndrome de l'œil sec et le syndrome de Gougerot-Sjogren ; et une fibrose de l'œil.

13. Composé selon la revendication 1, ou sel pharmaceutiquement acceptable de celui-ci, ou composition pharmaceutique selon la revendication 2, pour une utilisation dans le traitement d'une maladie, d'un état de santé ou d'un trouble, ladite maladie, ledit état de santé ou ledit trouble étant choisi parmi la cicatrisation des plaies et des ulcères chez les diabétiques ; l'amélioration de la perfusion microvasculaire ; l'amélioration de la perfusion microvasculaire suite à une lésion ou pour contrer la réponse inflammatoire dans des soins périopératoires ; les fissures anales ; les ulcères diabétiques ; les ulcères du pied diabétique) ; la cicatrisation osseuse ; la résorption et le remodelage osseux ostéoclastiques ; et la formation de nouveaux os.

14. Composé selon la revendication 1, ou sel pharmaceutiquement acceptable de celui-ci, ou composition pharmaceutique selon la revendication 2, pour une utilisation dans le traitement d'une maladie, d'un état de santé ou d'un trouble, ladite maladie, ledit état de santé ou ledit trouble étant choisi parmi :
• les troubles du système urogénital : une néphropathie diabétique ; une fibrose rénale et une insuffisance rénale suite à une insuffisance ou des maladies rénales chroniques ; une fibrose rénale et une insuffisance rénale en raison d'une accumulation/d'un dépôt et d'une lésion tissulaire ; une sclérose rénale ; une sclérose progressive ; une glomérulonéphrite ; une glomérulosclérose segmentaire focale ; un syndrome néphrotique ; une hypertrophie de la prostate ; une fibrose rénale ; une fibrose rénale interstitielle ;
• les troubles du système pulmonaire : une fibrose pulmonaire ; une fibrose pulmonaire idiopathique ; la mucoviscidose ; la fibrose massive progressive ; la fibrose massive progressive qui touchent les poumons) ;
• les troubles touchant le cœur : une fibrose endomyocardique ; un ancien infarctus du myocarde ; une fibrose auriculaire ; une fibrose interstitielle cardiaque ; un remodelage et une fibrose cardiaques ; une hypertrophie cardiaque ;
• les troubles du foie et des organes associés : une sclérose ou une cirrhose du foie ; une cirrhose du foie associée à une maladie hépatique chronique ; une fibrose hépatique ; l'activation des cellules étoilées hépatiques ; l'accumulation hépatique du collagène fibreux et du collagène total ; une maladie hépatique d'origine nécro-inflammatoire et/ou immunologique ; une cirrhose biliaire primaire ; une angiocholite sclérosante primitive ; les autres maladies hépatiques cholestatiques : celles associées à des maladies hépatiques granulomateuses, des tumeurs malignes du foie, une cholestase intrahépatique de la grossesse, une hépatite, une septicémie, des médicaments ou des toxines, une réaction du greffon contre l'hôte, après greffe du foie, une cholédocholithiase, des tumeurs du canal biliaire, un carcinome pancréatique, le syndrome de Mirizzi, une cholangiopathie liée au SIDA ou des parasites ; une schistosomiase ;
• les maladies ou troubles digestifs : la maladie de Crohn ; une colite ulcéreuse ; une sclérose du tractus gastro-intestinal ;
• les maladies de la peau ou des yeux : une fibrose néphrogène ; des chéloïdes ; des troubles ou des états cutanés ou topiques fibrotiques ; une fibrose dermique ; un scléroderme, une fibrose cutanée ; une morphée ; des cicatrices hypertrophiques ; les naevi ; une vitrorétinopathie proliférante ; des sarcoïdes ; des granulomes ; une fibrose de l'œil ;
• les maladies touchant le système nerveux : la sclérose latérale amyotrophique (SLA) ; une sclérose hippocampique, la sclérose en plaques (SEP) ; une sclérose focale ; une sclérose latérale primitive ;
• les maladies des os ; l'ostéosclérose ;
• l'otospongiose ; les autres maladies ou troubles auditifs ; une déficience auditive une perte d'audition partielle ou totale ; une surdité partielle ou totale ; un acouphène ; une perte d'audition induite par un bruit ;
• les autres maladies impliquant l'autoimmunité, une inflammation ou une fibrose : un scléroderme ; un scléroderme localisé ou un scléroderme circonscrit ; une fibrose médiastinale ; une fibrose médiastinite ; une myélofibrose ; une fibrose rétropéritonéale ; un arthrofibrose ; une maladie de Peyronie ; une contracture de Dupuyturen ; un lichen scléreux ; des formes d'une capsulite rétractile ; une athérosclérose ; une sclérose tubéreuse ; une sclérose systémique ; une polymyosite ; une dermatomyosite ; une polychondrite ; une fasciite à éosinophiles ; un lupus érythémateux disséminé ou un lupus ; une fibrose médullaire, une myélofibrose ou une ostéomyélofibrose ; une sarcoïdose ; des fibroïdes utérins ; un endométriose.

15. Composé selon la revendication 1, ou sel pharmaceutiquement acceptable de celui-ci, ou composition pharmaceutique selon la revendication 2, pour une utilisation dans le traitement d'une maladie, d'un état de santé ou d'un trouble, ladite maladie, ledit état de santé ou ledit trouble étant choisi parmi certains types de cancers ; une drépanocytose ; une anémie falciforme ; des métastases cancéreuses ; l'ostéoporose ; la gastroparésie ; une dyspepsie fonctionnelle ; les complications diabétiques ; l'alopécie ou la perte de cheveux ; les maladies associées à un dysfonctionnement endothélial ; les troubles neurologiques associés à une diminution de la production d'oxyde nitrique ; l'acidurie arginosuccinique ; les maladies neuromusculaires ; la dystrophie musculaire de Duchenne (DMD) ; la dystrophie musculaire de Becker (DMB) ; les dystrophies musculaires capulo-humérales ; les myopathies distales ; les dystrophies myotoniques de type I et de type II ; une dystrophie musculaire facio-scapulo-péronière ; une dystrophie musculaire d'Emery-Dreifuss autosomique et liée au chromosome X ; une dystrophie musculaire oculopharyngienne ; la sclérose latérale amyotrophique ; et une atrophie musculaire spinale (AMS).
